# EUROPÄISCHE PATENTANMELDUNG

(11) **EP 2 258 867 A2**
(43) Veröffentlichungstag der Anmeldung: **08.12.2010**
(21) Anmeldenummer: 10010161.7
(22) Anmeldetag: 11.05.2005
(51) Int. Cl.: C12Q 1/68

(54) **Identifizierung von Oberflächen-assoziierten Antigenen für die Tumordiagnose und -therapie**

(30) Priorität: 11.05.2004 DE 102004023187
(62) Teilanmeldung aus: 10005074.9
(71) Anmelder: Ganymed Pharmaceuticals AG, 55131 Mainz (DE)
(72) Erfinder: Türeci, Özlem, 55116 Mainz (DE); Sahin, Ugur, 55116 Mainz (DE); Schneider, Sandra, 76297 Stutensee (DE); Helftenbein, Gerd, 35329 Gemünden (DE); Schlüter, Volker, 82061 Neuried (DE); Usener, Dirk, 65193 Wiesbaden (DE); Thiel, Philippe, 82152 Planegg (DE); Koslowski, Michael, 65933 Frankfurt (DE)
(74) Vertreter: Schnappauf, Georg

(57) **Zusammenfassung**

Erfindungsgemäß wurden Tumor-assoziiert exprimierte Genprodukte und die dafür kodierenden Nukleinsäuren identifiziert. Die vorliegende Erfindung betrifft die Therapie und Diagnose von Erkrankungen, bei denen diese Tumor-assoziiert exprimierten Genprodukte aberrant exprimiert werden. Des weiteren betrifft die Erfindung Proteine, Polypeptide und Peptide, die Tumor-assoziiert exprimiert werden und die dafür kodierenden Nukleinsäuren.

## Beschreibung

Trotz interdisziplinärer Ansätze und Ausreizung klassischer Therapiemodalitäten gehören Krebserkrankungen weiterhin zu den führenden Todesursachen. Neuere therapeutische Konzepte zielen darauf ab, das patienteneigene Immunsystem durch Einsatz von rekombinanten Tumorvakzinen und anderen spezifischen Maßnahmen wie Antikörpertherapie in das therapeutische Gesamtkonzept mit einzubeziehen. Voraussetzung für den Erfolg einer solchen Strategie ist die Erkennung von Tumor-spezifischen oder Tumor-assoziierten Antigenen bzw. Epitopen durch das Immunsystem des Patienten, dessen Effektorfunktionen verstärkt werden sollen. Tumorzellen unterscheiden sich biologisch wesentlich von ihren nichtmalignen Ursprungszellen. Diese Differenzen sind durch während der Tumorentwicklung erworbene genetische Veränderungen bedingt und führen u.a. auch zur der Bildung qualitativ oder quantitativ veränderter molekularer Strukturen in den Krebszellen. Werden solche Tumor-assoziierten Strukturen vom spezifischen Immunsystem des tumortragenden Wirtes erkannt, spricht man von Tumor-assoziierten Antigenen.

An der spezifischen Erkennung von Tumor-assoziierten Antigenen sind zelluläre und humorale Mechanismen beteiligt, die zwei miteinander funktionell vernetzte Einheiten darstellen: CD4⁺ und CD8⁺ T-Lymphozyten erkennen prozessierte Antigene, die auf den Molekülen der MHC- (Major Histocompatibility complex = Histokompatibilitäts-Antigene) Klassen II bzw. I präsentiert werden, während B-Lymphozyten zirkulierende Antikörpermoleküle produzieren, die direkt an unprozessierte Antigene binden.

Die potentielle klinisch-therapeutische Bedeutung von Tumor-assoziierten Antigenen ergibt sich aus der Tatsache, dass die Erkennung von Antigenen auf neoplastischen Zellen durch das Immunsystem zur Initiierung von cytotoxischen Effektormechanismen führt und bei Vorhandensein von T-Helferzellen die Elimination der Krebszellen bewirken kann (Pardoll, Nat. Med. 4:525-31, 1998). Entsprechend ist es eine zentrale Zielsetzung der Tumorimmunologie, diese Strukturen molekular zu definieren. Die molekulare Natur dieser Antigene blieb lange enigmatisch. Erst als entsprechende Klonierungstechniken entwickelt wurden, gelang es, durch Analyse der Zielstrukturen von cytotoxischen T-Lymphozyten (CTL) (van der Bruggen et al., Science 254:1643-7, 1991) bzw. mit zirkulierenden Autoantikörpern (Sahin et al., Curr. Opin. Immunol. 9:709-16, 1997) als Sonden cDNA-Expressionsbanken von Tumoren systematisch auf Tumör-assozüerte Antigene zu analysieren. Hierzu wurden cDNA-Expressionsbanken aus frischem Tumorgewebe hergestellt und in geeigneten Systemen als Proteine rekombinant exprimiert. Aus Patienten isolierte Immuneffektoren, nämlich CTL-Klone mit Tumor-spezifischem Lysemuster, oder zirkulierende Autoantikörper wurden genutzt, um die respektiven Antigene zu klonieren.

Durch diese Ansätze sind in den letzten Jahren eine Vielzahl von Antigenen in verschiedenen Neoplasien definiert worden. Von großem Interesse ist dabei die Klasse der Cancer/Testis-Antigene (CTA). CTA und sie kodierende Gene (Cancer/Testis-Gene oder CTG) sind durch ihr charakteristisches Expressionsmuster definiert (Türeci et al, Mol. Med. Today 3:342-9, 1997). Sie finden sich nicht in Normalgeweben bis auf Testis bzw. Keimzellen, werden jedoch in einer Reihe von humanen Malignomen exprimiert und zwar nicht tumortypspezifisch, sondern mit unterschiedlicher Häufigkeit in Tumorentitäten ganz unterschiedlicher Herkunft (Chen & Old, Cancer J. Sci. Am. 5:16-7, 1999). Antikörper gegen CTA sind in gesunden Personen nicht nachweisbar, wohl aber in Tumorpatienten. Insbesondere aufgrund ihrer Gewebeverteilung ist diese Antigenklasse von besonderem Wert für immuntherapeutische Vorhaben und wird in derzeit laufenden klinischen Patientenstudien getestet (Marchand et al., Int. J. Cancer 80:219-30, 1999; Knuth et al., Cancer Chemother. Pharmacol. 46: S46-51, 2000).

Allerdings nutzen die oben dargestellten klassischen Verfahren zur Antigenidentifizierung Immuneffektoren (zirkulierende Autoantikörper oder CTL-Klone) aus Patienten mit in der Regel bereits fortgeschrittenem Krebs als Sonden. Aus einer Reihe von Daten geht hervor, dass Tumore z.B. zur Tolerisierung und Anergisierung von T-Zellen führen können und gerade im Verlauf der Erkrankung diejenigen Spezifitäten aus dem Immuneffektorenrepertoire verloren gehen, die eine effektive Immunerkennung bewirken könnten. Aus laufenden Patientenstudien hat sich noch kein gesicherter Beweis für eine tatsächliche Wirkung der bisher entdeckten und genutzten Tumor-assoziierten Antigene ergeben. Entsprechend kann nicht ausgeschlossen werden, dass spontane Immunantworten evozierende Proteine die falschen Zielstrukturen sind.

Es war die Aufgabe der vorliegenden Erfindung Zielstrukturen für eine Diagnose und Therapie von Krebserkrankungen bereitzustellen.

Diese Aufgabe wird erfindungsgemäß durch den Gegenstand der Patentansprüche gelöst.

Erfindungsgemäß wurde eine Strategie für eine Identifizierung und Bereitstellung Tumor-assoziierter Antigene und der dafür kodierenden Nukleinsäuren verfolgt. Diese Strategie beruht auf der Auswertung humaner Protein- und Nukleinsäuredatenbanken im Hinblick auf potenzielle, krebsspezifische Antigene, die auf der Zelloberfläche zugänglich sind. Die Definition der dafür notwendigen Filterkriterien zusammen mit einer Hochdurchsatz-Methodik zur Analyse möglichst aller Gene bildet den zentralen Bestandteil der Erfindung. Durch Datamining wird zunächst eine möglichst komplette Liste aller bekannter Gene aufgestellt, die dem Grundprinzip Gen zu mRNA zu Protein folgend auf das Vorhandensein einer oder mehrerer Transmembrandomänen hin untersucht werden. Hieran schließen sich eine Homologiesuche, eine Einteilung der Treffer in gewebsspezifische Gruppen (u.a. Tumorgewebe) und eine Überprüfung der realen Existenz der mRNA an. Schließlich werden die so identifizierten Proteine z.B. durch Expressionsanalysen und proteinchemische Verfahren auf ihre aberrante Aktivierung in Tumoren evaluiert.

Datamining ist ein bekanntes Verfahren zur Identifizierung von Tumor-assoziierten Genen. Bei den herkömmlichen Strategien werden allerdings in der Regel Transkriptome von Normalgewebebanken elektronisch von Tumorgewebsbanken unter der Annahme subtrahiert, dass die verbleibenden Gene Tumor-spezifisch sind (Schmitt et al., Nucleic Acids Res. 27:4251-60, 1999; Vasmatzis et al., Proc. Natl. Acad. Sci. USA. 95:300-4, 1998; Scheurle et al., Cancer Res. 60:4037-43, 2000).

Das erfindungsgemäße Konzept beruht jedoch darauf, Datamining zur elektronischen Extraktion aller Gene, die für auf der Zelloberfläche zugängliche, krebsspezifische Antigene kodieren, zu nutzen und diese sodann auf ektope Expression in Tumoren zu evaluieren.

Somit betrifft die Erfindung in einem Aspekt eine Strategie zur Identifizierung von differentiell in Tumoren exprimierten Genen. Diese kombiniert Datamining von öffentlichen Sequenzbanken ("*in silico*") mit darauffolgenden laborexperimentellen ("wet bench") Untersuchungen.

Eine kombinierte Strategie basierend auf unterschiedlichen bioinformatischen Skripten ermöglichte erfindungsgemäß die Identifizierung von Genen, die für auf der Zelloberfläche zugängliche, krebsspezifische Antigene kodieren. Die Identifizierung und Bereitstellung dieser Tumor-assozüerten Gene und der dadurch kodierten Genprödukte erfolgte erfindungsgemäß unabhängig von einer immunogenen Wirkung.

Die erfindungsgemäß identifizierten Tumor-assoziierten Antigene weisen eine Aminosäuresequenz auf, die von einer Nukleinsäure kodiert wird, die aus der Gruppe ausgewählt ist bestehend aus (a) einer Nukleinsäure, die eine Nukleinsäuresequenz umfasst, die aus der Gruppe bestehend aus SEQ ID NOs: 1, 3, 5, 7, 9, 11, 13, 15, 17, 19, 21, 23, 25, 27, 29, 69, 71, 73, 75, 79, 80, 85, 87, 102, 104, 106, 108, 110, 112, einem Teil oder Derivat davon ausgewählt ist, (b) einer Nukleinsäure, die unter stringenten Bedingungen mit der Nukleinsäure unter (a) hybridisiert, (c) einer Nukleinsäure, die in Bezug auf die Nukleinsäure unter (a) oder (b) degeneriert ist, und (d) einer Nukleinsäure, die zu der Nukleinsäure unter (a), (b) oder (c) komplementär ist. In einer bevorzugten Ausführungsform weist ein erfindungsgemäß identifiziertes Tumor-assoziiertes Antigen eine Aminosäuresequenz auf, die von einer Nukleinsäure kodiert wird, die aus der Gruppe bestehend aus SEQ ID NOs: 1, 3, 5, 7, 9, 11, 13, 15, 17, 19, 21, 23, 25, 27, 29, 69, 71, 73, 75, 79, 80, 85, 87, 102, 104, 106, 108, 110, 112, ausgewählt ist. In einer weiteren bevorzugten Ausführungsform umfasst ein erfindungsgemäß identifiziertes Tumor-assoziiertes Antigen eine Aminosäuresequenz, die aus der Gruppe bestehend aus SEQ ID NOs: 2, 4, 6, 8, 10, 12, 14, 16, 18, 20, 22, 24, 26, 28, 30, 61 bis 68, 70, 72, 74, 76, 81, 82, 86, 88, 96 bis 101, 103, 105, 107, 109, 111, 113, einem Teil oder Derivat davon ausgewählt ist.

Die vorliegende Erfindung betrifft allgemein die Verwendung von erfindungsgemäß identifizierten Tumor-assoziierten Antigenen oder von Teilen davon, von dafür kodierenden Nukleinsäuren oder von Nukleinsäuren, die gegen die kodierenden Nukleinsäuren gerichtet sind, oder von Antikörpern, die gegen die erfindungsgemäß identifizierten Tumor-assoziierten Antigene oder Teile davon gerichtet sind, für die Therapie und Diagnose. Diese Nutzung kann einzelne, aber auch Kombinationen von mehreren dieser Antigene, funktionalen Fragmente, Nukleinsäuren, Antikörper etc. betreffen, in einer Ausführungsform auch in Kombination mit anderen Tumor-assoziierten Genen und Antigenen für eine Diagnose, Therapie und Verlaufskontrolle.

Die Eigenschaft der erfindungsgemäß identifizierten Tumor-assoziierten Antigene, dass sie auf oder an der Zelloberfläche lokalisiert sind, qualifiziert sie als geeignete Ziele oder Mittel für die Therapie und Diagnose. Besonders geeignet hierfür ist ein Teil der erfindungsgemäß identifizierten Tumor-assoziierten Antigene, der dem nicht-Transmembrananteil, insbesondere dem extrazellulären Anteil der Antigene entspricht oder davon umfasst wird. Somit ist erfindungsgemäß ein Teil der erfindungsgemäß identifizierten Tumor-assozüerten Antigene, der dem nicht-Transmembrananteil der Antigene entspricht oder davon umfasst ist, oder ein entsprechender Teil der für die erfindungsgemäß identifizierten Antigene kodierenden Nukleinsäuren für eine Therapie oder Diagnose bevorzugt. Ähnlich ist die Verwendung von Antikörpern bevorzugt, die gegen einen Teil der erfindungsgemäß identifizierten Tumor-assoziierten Antigene gerichtet sind, der dem nicht-Transmembrananteil der Antigene entspricht oder davon umfasst ist.

Bevorzugte Erkrankungen für eine Therapie und/oder Diagnose sind solche, bei denen eine selektive Expression oder abnormale Expression von einem oder mehreren der erfindungsgemäß identifizierten Tumor-assoziierten Antigene vorliegt.

Die Erfindung betrifft auch solche Nukleinsäuren und Genprodukte, die tumorzellassozüert exprimiert werden und durch verändertes Spleißen (Spleißvarianten) der Nukleinsäuren der erfmdungsgemäß identifizierten Tumor-assoziierten Antigene bzw. durch veränderte Translation unter Nutzung alternativer offener Leserahmen entstehen. Die erfindungsgemäßen Spleißvarianten sind erfindungsgemäß als Targets für die Diagnostik und Therapie von Tumorerkrankungen verwendbar.

Für die Entstehung von Spleißvarianten können verschiedenste Mechanismen ursächlich sein, beispielsweise
- die Nutzung variabler Transkriptionsinitiationsstellen
- die Nutzung zusätzlicher Exons
- vollständiges oder unvollständiges Ausspleißen von einzelnen oder mehreren Exons,
- über Mutation veränderte Spleißregulatorsequenzen (Deletion bzw. Schaffung neuer Donoi/Acceptorsequenzen),
- die unvollständige Elimination von Intionsequenzen.

Das veränderte Spleißen eines Gens führt zu einer veränderten Transkriptsequenz (Spleißvariante). Wird eine Spleißvariante im Bereich ihrer veränderten Sequenz translatiert, resultiert ein verändertes Protein, welches sich von dem ursprünglichen in Struktur und Funktion deutlich unterscheiden kann. Bei tumorassoziierten Spleißvarianten können tumorassoziierte Transkripte und tumorassoziierte Proteine/Antigene entstehen. Diese können als molekulare Marker sowohl zum Nachweis von Tumorzellen als auch zum therapeutischen Targeting von Tumoren genutzt werden. Die Detektion von Tumorzellen z.B. im Blut, Serum, Knochenmark, Sputum, Bronchial-Lavage, Körpersekreten und Gewebsbiopsien kann erfindungsgemäß z.B. nach Extraktion, von Nukleinsäuren durch PCR-Amplifikation mit Spleißvarianten-spezifischen Oligonukleotiden erfolgen.

Zum Nachweis eignen sich erfindungsgemäß alle Sequenz-abhängigen Detektionssysteme. Neben der PCR sind diese z.B. Genchip-/Microarraysysteme, Northern-Blot, RNAse protection assays (RDA) und andere. Allen Detektionssystemen ist gemeinsam, dass die Detektion auf einer spezifischen Hybridisierung mit mindestens einer Spleißvarianten-spezifischen Nukleinsäuresequenz basiert. Die Detektion von Tumorzellen kann jedoch auch erfindungsgemäß durch Antikörper erfolgen, die ein durch die Spleißvariante kodiertes spezifisches Epitop erkennen. Für die Herstellung der Antikörper können Peptide zur Immunisierung verwendet werden, die für diese Spleißvariante spezifisch sind. Für die Immunisierung eignen sich besonders die Aminosäuren, die deutliche Epitopunterschiede zu der/den Variante(n) des Genprodukts aufweisen, welche bevorzugt in gesunden Zellen gebildet wird/werden. Der Nachweis der Tumorzellen mit Antikörper kann dabei an einer vom Patienten isolierten Probe oder als Imaging mit intravenös applizierten Antikörpern erfolgen.

Neben der diagnostischen Nutzbarkeit stellen Spleißvarianten, die neue oder veränderte Epitope aufweisen, attraktive Targets für die Immuntherapie dar. Die erfindungsgemäßen Epitope können zum Targeting von therapeutisch wirksamen monoklonalen Antikörpern oder T-Lymphozyten genutzt werden. Bei der passiven Immuntherapie werden hierbei Antikörper oder T-Lymphozyten adoptiv transferiert, die Spleißvarianten-spezifische Epitope erkennen. Die Generierung von Antikörpern kann wie bei anderen Antigenen auch unter Nutzung von Standardtechnologien (Immunisierung von Tieren, Panningstrategien zur Isolation von rekombinanten Antikörpern) unter Nutzung von Polypeptiden, die diese Epitope beinhalten, erfolgen. Alternativ können zur Immunisierung Nukleinsäuren genutzt werden, die für Oligo- oder Polypeptide kodieren, die diese Epitope beinhalten. Verschiedene Techniken zur in vitro oder in vivo Generierung von epitopspezifischen T-Lymphozyten sind bekannt und ausführlich beschrieben (z.B. Kessler JH, et al. 2001, Sahin et al., 1997) und basieren ebenfalls auf der Nutzung von Oligo- oder Polypeptide, die die Spleißvarianten-spezifischen Epitope beinhalten, oder Nukleinsäuren, die für diese kodieren. Oligo- oder Polypeptide, die die Spleißvarianten-spezifischen Epitope beinhalten, oder Nukleinsäuren, die für diese Polypeptide kodieren, sind auch für die Nutzung als pharmazeutisch wirksame Substanzen bei der aktiven Immuntherapie (Vakzinierung, Vakzintherapie) verwendbar.

In einem weiteren Aspekt betrifft die Erfindung auch posttranslational modifizierte Proteindomänen wie zum Beispiel Glykosylierungen oder Myristilierungen. Diese Art von Modifikationen kann zu einem differentiellen Erkennungsmuster eines Antigens durch z.B. einen Antikörper führen und verschiedene, unter Umständen krankheitsassoziierte Zustände erkennen. Vor allem durch den Einsatz von Antikörpern kann diese Differenzierung eines Antigens sowohl diagnostisch als auch therapeutisch genutzt werden. Für Tumorzellen ist publiziert, dass die tumorassoziierte zelluläre Entartung zu veränderten posttranslalionalen Modifikationen führen kann (Durand & Seta. 2000. Clin Chem 46: 795-805; Granovsky et al. 2000. Nat Med 6: 306-312). Insbesondere Glykosylierungsmuster sind auf Tumorzellen stark verändert. Erfindungsgemäß können diese speziellen Epitope diagnostisch Tumorzellen von nicht karzinogenen Zellen unterscheiden. Sollte ein posttranslational modifizierbares Epitop in normalen, nicht entarteten Zellen glykosyliert und in Tumorzellen deglykosyliert sein, erlaubt diese Situation die Entwicklung eines tumorspezifischen therapeutischen Antikörpers im Sinne der Erfindung.

In einem Aspekt betrifft die Erfindung eine pharmazeutische Zusammensetzung, umfassend ein Mittel, das das erfindungsgemäß identifizierte Tumor-assoziierte Antigen erkennt und vorzugsweise selektive für Zellen ist, die eine Expression oder abnormale Expression eines erfindungsgemäß identifizierten Tumor-assoziierten Antigens aufweisen. Das Mittel kann in bestimmten Ausführungsformen die Induktion des Zelltods, die Reduktion des Zellwachstums, die Schädigung der Zellmembran oder die Sekretion von Zytokinen bewirken und weist vorzugsweise eine tumorhemmende Aktivität auf In einer Ausführungsform ist das Mittel eine Antisense-Nukleinsäure, die selektiv mit der Nukleinsäure hybridisiert, die für das Tumor-assoziierte Antigen kodiert. In einer weiteren Ausführungsform ist das Mittel ein Antikörper, der selektiv an das Tumor-assoziierte Antigen bindet, insbesondere ein komplementaktivierter Antikörper, der selektiv an das Tumor-assoziierte Antigen bindet. In einer weiteren Ausführungsform umfasst das Mittel mehrere Mittel, die jeweils selektiv verschiedene Tumor-assoziierte Antigene erkennen, wobei mindestens eines der Tumor-assoziierten Antigene ein erfindungsgemäß identifiziertes Tumor-assozüertes Antigen ist. Die Erkennung muss nicht direkt mit einer Hemmung von Aktivität oder Expression des Antigens einhergehen. In diesem Aspekt der Erfindung dient das selektiv auf Tumoren beschränkte Antigen vorzugsweise als Markierung zur Rekrutierung von Effektormechanismen an diesen spezifischen Ort. In einer bevorzugten Ausführungsform ist das Mittel ein cytotoxischer T-Lymphozyt, der das Antigen auf einem HLA-Molekül erkennt und die derartig markierte Zelle lysiert. In einer weiteren Ausführungsform ist das Mittel ein Antikörper, der selektiv an das Tumor-assozüerte Antigen bindet und somit natürliche oder artifizielle Effektormechanismen zu dieser Zelle rekrutiert. In einer weiteren Ausführungsform ist das Mittel ein Helfer-T-Lymphozyt, der Effektorfunktionen von anderen Zellen, die spezifisch dieses Antigen erkennen, stärkt.

In einem Aspekt betrifft die Erfindung eine pharmazeutische Zusammensetzung, umfassend ein Mittel, das die Expression oder Aktivität eines erfindungsgemäß identifizierten Tumor-assoziierten Antigens hemmt. In einer bevorzugten Ausführungsform ist das Mittel eine Antisense-Nukleinsäure, die selektiv mit der Nukleinsäure hybridisiert, die für das Tumor-assoziierte Antigen kodiert. In einer weiteren Ausführungsform ist das Mittel ein Antikörper, der selektiv an das Tumor-assoziierte Antigen bindet. In einer weiteren Ausftlhrungsform umfasst das Mittel mehrere Mittel, die jeweils selektiv die Expression oder Aktivität verschiedener Tumor-assozüerter Antigene hemmen, wobei mindestens eines der Tumor-assoziierten Antigene ein erfindungsgemäß identifiziertes Tumor-assoziiertes Antigen ist.

Die Aktivität eines erfindungsgemäß identifizierten Tumor-assoziierten Antigens kann eine jegliche Aktivität eines Proteins oder Peptids sein. Somit können die erfindungsgemäßen Therapie- und Diagnosverfahren auch auf Hemmung oder Reduktion dieser Aktivität oder auf ein Testen dieser Aktivität abzielen.

Des weiteren betrifft die Erfindung eine pharmazeutische Zusammensetzung, die ein Mittel umfasst, das bei einer Verabreichung selektiv die Menge an Komplexen zwischen einem HLA-Molekül und einem Peptidepitop aus dem erfindungsgemäß identifizierten Tumor-assozüerten Antigen erhöht. Das Mittel umfasst in einer Ausführungsform einen oder mehrere Bestandteile, die aus der Gruppe ausgewählt sind bestehend aus (i) dem Tumor-assoziierten Antigen oder einem Teil davon, (ii) einer Nukleinsäure, die für das Tumor-assozüerte Antigen oder einen Teil davon kodiert, (iii) einer Wirtszelle, die das Tumor-assoziierte Antigen oder einen Teil davon exprimiert, und (iv) isolierten Komplexen zwischen Peptidepitopen aus dem Tumor-assoziierten Antigen und einem MHC-Molekül. In einer Ausführmgsform umfasst das Mittel mehrere Mittel, die jeweils selektiv die Menge an Komplexen zwischen MHC-Molekülen und Peptidepitopen verschiedener Tumor-assoziierter Antigene erhöhen, wobei mindestens eines der Tumor-assoziierten Antigene ein erfindungsgemäß identifiziertes Tumor-assoziiertes Antigen ist.

Des weiteren betrifft die Erfindung eine pharmazeutische Zusammensetzung, die einen oder mehrere Bestandteile umfasst, die aus der Gruppe ausgewählt sind bestehend aus (i) einem erfindungsgemäß identifizierten Tumor-assoziierten Antigen oder einem Teil davon, (ii) einer Nukleinsäure, die für ein erfindungsgemäß identifiziertes Tumor-assoziiertes Antigen oder einen Teil davon kodiert, (iii) einem Antikörper, der an ein erfindungsgemäß identifiziertes Tumor-assoziiertes Antigen oder einen Teil davon bindet, (iv) einer Antisense-Nukleinsäure, die spezifisch mit einer Nukleinsäure, die für ein erfindungsgemäß identifiziertes Tumor-assoziiertes Antigen kodiert, hybridisiert, (v) einer Wirtszelle, die ein erfindungsgemäß identifiziertes Tumor-assozüertes Antigen oder einen Teil davon exprimiert, und (vi) isolierten Komplexen zwischen einem erfindungsgemäß identifizierten Tumor-assoziierten Antigen oder einem Teil davon und einem HLA-Molekül.

Eine Nukleinsäure, die für ein erfindungsgemäß identifiziertes Tumor-assozüertes Antigen oder einen Teil davon kodiert, kann in der pharmazeutische Zusammensetzung in einem Expressionsvektor vorliegen und funktionell mit einem Promotor verbunden sein.

Eine in einer erfindungsgemäßen pharmazeutischen Zusammensetzung enthaltene Wirtszelle kann das Tumor-assoziierte Antigen oder den Teil davon sekretieren, auf der Oberfläche exprimieren oder kann zusätzlich ein HLA-Molekül exprimieren, das an das Tumor-assoziierte Antigen oder den Teil davon bindet. In einer Ausführungsform exprimiert die Wirtszelle das HLA-Molekül endogen. In einer weiteren Ausführungsform exprimiert die Wirtszelle das HLA-Molekül und/oder das Tumor-assozüerte Antigen oder den Teil davon rekombinant. Vorzugsweise ist die Wirtszelle nicht-proliferativ. In einer bevorzugten Ausführungsform ist die Wirtszelle eine Antigen-präsentierende Zelle, insbesondere eine dendritische Zelle, ein Monozyt oder ein Makrophage.

Ein in einer erfindungsgemäßen pharmazeutischen Zusammensetzung enthaltener Antikörper kann ein monoklonaler Antikörper sein. In weiteren Ausführungsformen ist der Antikörper ein chimärer oder humanisierter Antikörper, ein Fragment eines natürlichen Antikörpers oder ein synthetischer Antikörper, die durch kambinatorische Techniken hergestellt werden können. Der Antikörper kann mit einem therapeutisch oder diagnostisch nützlichen Mittel gekoppelt sein.

Eine in einer erfindungsgemäßen pharmazeutischen Zusammensetzung enthaltene Antisense-Nukleinsäure kann eine Sequenz von 6-50, insbesondere 10-30, 15-30 oder 20-30 zusammenhängenden Nukleotiden aus der Nukleinsäure, die für das erfindungsgemäß identifizierte Tumor-assoziierte Antigen kodiert, umfassen.

In weiteren Ausführungsformen bindet ein durch eine erfindungsgemäße pharmazeutische Zusammensetzung entweder direkt oder durch die Expression einer Nukleinsäure bereitgestelltes Tumor-assozüertes Antigen oder ein Teil davon an MHC-Moleküle auf der Oberfläche von Zellen, wobei die Bindung vorzugsweise eine cytolytische Reaktion hervorruft und/oder eine Zytokinausschüttung induziert.

Eine erfindungsgemäße pharmazeutische Zusammensetzung kann einen pharmazeutisch verträglichen Träger und/oder ein Adjuvans umfassen. Das Adjuvans kann aus Saponin, GM-CSF, CpG-Oligonukleoüden, RNA, einem Zytokin oder einem Chemokin ausgewählt sein. Eine erfindungsgemäße pharmazeutische Zusammensetzung wird vorzugsweise zur Behandlung einer Erkrankung eingesetzt, die sich durch die selektive Expression oder abnormale Expression eines Tumor-assoziierten Antigens auszeichnet. In einer bevorzugten Ausführungsform ist die Erkrankung Krebs.

Des Weiteren betrifft die Erfindung Verfahren zur Behandlung oder Diagnose einer Erkrankung, die sich durch die Expression oder abnormale Expression eines oder mehrerer Tumor-assoziierter Antigene auszeichnet. In einer Ausführungsform umfasst die Behandlung die Verabreichung einer erfindungsgemäßen pharmazeutischen Zusammensetzung.

In einem Aspekt betrifft die Erfindung ein Verfahren zur Diagnose einer Erkrankung, die sich durch die Expression oder abnormale Expression eines erfindungsgemäß identifizierten Tumor-assoziierten Antigens auszeichnet. Das Verfahren umfasst den Nachweis (i) einer Nukleinsäure, die für das Tumor-assoziierte Antigen kodiert, oder eines Teils davon und/oder (ii) den Nachweis des Tumor-assoziierten Antigens oder eines Teils davon, und/oder (iii) den Nachweis eines Antikörpers gegen das Tumor-assoziierte Antigen oder einen Teil davon und/oder (iv) den Nachweis von cytotoxischen oder Helfer-T-Lymphozyten, die für das Tumor-assoziierte Antigen oder einen Teil davon spezifisch sind in einer aus einem Patienten isolierten biologischen Probe. In bestimmten Ausführungsformen umfasst der Nachweis (i) die Kontaktierung der biologischen Probe mit einem Mittel, das spezifisch an die Nukleinsäure, die für das Tumor-assoziierte Antigen kodiert, oder den Teil davon, an das Tumor-assoziierte Antigen oder den Teil davon, an den Antikörper oder an cytotoxische oder Helfer-T-Lymphozyten, die für das Tumor-assoziierte Antigen oder Teile davon spezifisch sind, bindet und (ii) den Nachweis der Komplexbildung zwischen dem Mittel und der Nukleinsäure oder dem Teil davon, dem Tumor-assoziierten Antigen oder dem Teil davon, dem Antikörper oder den zytotoxischen oder Helfer-T-Lymphozyten. In einer Ausführungsform zeichnet sich die Erkrankung durch die Expression oder abnormale Expression mehrerer verschiedener Tumor-assoziierter Antigene aus und der Nachweis umfasst einen Nachweis mehrerer Nukleinsäuren, die für die mehreren verschiedenen Tumor-assoziierten Antigene kodieren, oder von Teilen davon, den Nachweis der mehreren verschiedenen Tumor-assoziierten Antigene oder von Teilen davon, den Nachweis mehrerer Antikörper, die an die mehreren verschiedenen Tumor-assoziierten Antigene oder an Teile davon binden, oder den Nachweis mehrerer cytotoxischer oder Helfer-T-Lymphozyten, die für die mehreren verschiedenen Tumor-assoziierten Antigene spezifisch sind. In einer weiteren Ausführungsform wird die isolierte biologische Probe aus dem Patienten mit einer vergleichbaren normalen biologischen Probe verglichen.

Die erfmdungsgemäßen Diagnoseverfahren können auch eine Nutzung der erfindungsgemäß identifizierten Tumor-assoziierten Antigene als prognostische Marker betreffen, um eine Metastatisierung z.B. durch Testen des Migrationsverhalten von Zellen und daher einen verschlechterten Krankheitsverlauf zu prädizieren, wodurch unter anderem die Planung einer aggressiveren Therapie ermöglicht wird.

In einem weiteren Aspekt betrifft die Erfindung ein Verfahren zur Bestimmung der Regression, des Verlaufs oder des Ausbruchs einer Erkrankung, die sich durch die Expression oder abnormale Expression eines erfindungsgemäß identifizierten Tumor-assoziierten Antigens auszeichnet, umfassend die Überwachung einer Probe aus einem Patienten, der die Erkrankung aufweist oder in Verdacht steht, an der Erkrankung zu erkranken in Bezug auf einen oder mehrere Parameter, die aus der Gruppe ausgewählt sind bestehend aus (i) der Menge der Nukleinsäure, die für das Tumor-assoziierte Antigen kodiert, oder eines Teil davon, (ii) der Menge des Tumor-assoziierten Antigens oder eines Teils davon, (üi) der Menge an Antikörpern, die an das Tumor-assoziierte Antigen oder einen Teil davon binden, und (iv) der Menge an cytolytischen T-Zellen oder Helfer-T-Zellen, die für einen Komplex zwischen dem Tumor-assoziierten Antigen oder einem Teil davon und einem MHC-Molekül spezifisch sind. Vorzugsweise umfasst das Verfahren die Bestimmung des oder der Parameter zu einem ersten Zeitpunkt in einer ersten Probe und zu einem zweiten Zeitpunkt in einer weiteren Probe, wobei durch einen Vergleich der beiden Proben oder Verlauf der Erkrankung ermittelt wird. In bestimmten Ausführungsformen zeichnet sich die Erkrankung durch die Expression oder abnormale Expression mehrerer verschiedener Tumor-assoziierter Antigene aus und die Überwachung umfasst eine Überwachung (i) der Menge mehrerer Nukleinsäuren, die für die mehreren verschiedenen Tumor-assoziierten Antigene kodieren, oder von Teilen davon und/oder (ii) der Menge der mehreren verschiedenen Tumor-assoziierten Antigene oder von Teilen davon und/oder (iii) der Menge mehrerer Antikörper, die an die mehreren verschiedenen Tumor-assoziierten Antigene oder an Teile davon binden, und/oder (iv) der Menge mehrerer cytolytischer T-Zellen oder Helfer-T-Zellen, die für Komplexe zwischen den mehreren verschiedenen Tumor-assoziierten Antigenen oder von Teilen davon und MHC-Molekülen spezifisch sind.

Ein Nachweis einer Nukleinsäure oder eines Teils davon oder eine Überwachung der Menge einer Nukleinsäure oder eines Teils davon kann erfindungsgemäß mit einer Polynukleotid-Sonde erfolgen, die spezifisch mit der Nukleinsäure oder dem Teil davon hybridisiert, oder kann durch selektive Amplifikation der Nukleinsäure oder des Teils davon erfolgen. In einer Ausführungsform umfasst die Polynukleotid-Sonde eine Sequenz von 6-50, insbesondere 10-30, 15-30 oder 20-30 zusammenhängenden Nukleotiden aus der Nukleinsäure.

Ein Nachweis eines Tumor-assoziierten Antigens oder eines Teils davon oder eine Überwachung der Menge eines Tumor-assoziierten Antigens oder eines Teils davon kann erfindungsgemäß mit einem Antikörper erfolgen, der spezifisch an das Tumor-assoziierte Antigen oder den Teil davon bindet.

In bestimmten Ausführungsformen liegt das nachzuweisende Tumor-assoziierte Antigen oder der Teil davon in einem Komplex mit einem MHC-Molekül, insbesondere einem HLA-Molekül vor.

Ein Nachweis eines Antikörpers oder die Überwachung der Menge an Antikörpern kann erfindungsgemäß mit einem Protein oder Peptid erfolgen, das spezifisch an den Antikörper bindet.

Ein Nachweis von cytolytischen T-Zellen oder Helfer-T-Zellen oder die Überwachung der Menge an cytolytischen T-Zellen oder Helfer-T-Zellen, die für Komplexe zwischen einem Antigen oder einem Teil davon und MHC-Molekülen spezifisch sind, kann erfindungsgemäß mit einer Zelle erfolgen, die den Komplex zwischen dem Antigen oder dem Teil davon und einem MHC-Molekül präsentiert.

Die für einen Nachweis oder für eine Überwachung verwendete Polynukleotid-Sonde, der Antikörper, das Protein oder Peptid oder die Zelle sind vorzugsweise nachweisbar markiert. In bestimmten Ausführungsformen ist der nachweisbare Marker ein radioaktiver Marker oder ein Enzymmarker. Der Nachweis von T-Lymphozyten kann zusätzlich durch Nachweis ihrer Proliferation, ihrer Zytokinproduktion, sowie ihrer cytotoxischen Aktivität erfolgen, die durch die spezifische Stimulation mit dem Komplex aus MHC und Tumor-assoziiertem Antigen oder Teilen davon ausgelöst wird. Der Nachweis von T-Lymphozyten kann ferner durch ein rekombinantes MHC-Molekül oder auch einen Komplex aus mehreren MHC-Molekülen, die mit dem jeweiligen immunogenen Fragment aus einem oder mehreren der Tumor-assoziierten Antigene beladen sind, und durch Kontaktierung des spezifischen T-Zell-Rezeptors erfolgen, der spezifische T-Lymphozyten identifizieren kann.

In einem weiteren Aspekt betrifft die Erfindung ein Verfahren zur Behandlung, Diagnose oder Überwachung einer Erkrankung, die sich durch die Expression oder abnormale Expression eines erfindungsgemäß identifizierten Tumor-assoziierten Antigens auszeichnet, umfassend die Verabreichung eines Antikörpers, der an das Tumor-assoziierte Antigen oder einen Teil davon bindet und mit einem therapeutischen oder diagnostischen Mittel gekoppelt ist. Der Antikörper kann ein monoklonaler Antikörper sein. In weiteren Ausführungsformen ist der Antikörper ein chimärer oder humanisierter Antikörper oder ein Fragment eines natürlichen Antikörpers.

Die Erfindung betrifft auch ein Verfahren zur Behandlung eines Patienten mit einer Erkrankung, die sich durch die Expression oder abnormale Expression eines erfindungsgemäß identifizierten Tumor-assoziierten Antigens auszeichnet, umfassend (i) die Entfernung einer Probe mit immunreaktiven Zellen aus dem Patienten, (ii) die Kontaktierung der Probe mit einer Wirtszelle, die das Tumor-assoziierte Antigen oder einen Teil davon exprimiert, unter Bedingungen, die eine Produktion cytolytischer T-Zellen gegen das Tumor-assoziierte Antigen oder einen Teil davon begünstigen, und (üi) das Einbringen der cytolytischen T-Zellen in den Patienten in einer Menge, die geeignet ist, Zellen zu lysieren, die das Tumor-assoziierte Antigen oder einen Teil davon exprimieren. Die Erfindung betrifft ebenfalls die Klonierung des T-Zell-Rezeptors von cytolytischen T-Zellen gegen das Tumor-assoziierte Antigen. Dieser kann in andere T-Zellen transferiert werden, die damit die erwünschte Spezifität erhalten und wie unter (iii) in den Patienten eingebracht werden können.

In einer Ausführungsform exprimiert die Wirtszelle ein HLA-Molekül endogen. In einer weiteren Ausführungsform exprimiert die Wirtszelle ein HLA-Molekül und/oder das Tumor-assoziierte Antigen oder den Teil davon rekombinant. Vorzugsweise ist die Wirtszelle nicht-proliferativ. In einer bevorzugten Ausführungsform ist die Wirtszelle eine Antigen-präsentierende Zelle, insbesondere eine dendritische Zelle, ein Monozyt oder ein Makrophage.

In einem weiteren Aspekt betrifft die Erfindung ein Verfahren zur Behandlung eines Patienten mit einer Erkrankung, die sich durch die Expression oder abnormale Expression eines Tumor-assoziierten Antigens auszeichnet, umfassend (i) die Identifizierung einer für ein erfindungsgemäß identifiziertes Tumor-assoziiertes Antigen kodierenden Nukleinsäure, die von Zellen exprimiert wird, die mit der Erkrankung assoziiert sind, (ii) die Transfektion einer Wirtszelle mit der Nukleinsäure oder einem Teil davon, (iii) die Kultivierung der transfizierten Wirtszelle für eine Expression der Nukleinsäure (dies ist bei Erreichen einer hohen Transfektionsrate nicht obligat) und (iv) das Einbringen der Wirtszellen oder eines Extrakts davon in den Patienten in einer Menge, die geeignet ist, die Immunreaktion gegen die Zellen des Patienten, die mit der Erkrankung assoziiert sind, zu erhöhen. Das Verfahren kann ferner die Identifizierung eines MHC-Moleküls, das das Tumor-assoziierte Antigen oder einen Teil davon präsentiert, umfassen, wobei die Wirtszelle das identifizierte MHC-Molekül exprimiert und das Tumor-assoziierte Antigen oder einen Teil davon präsentiert. Die Immunreaktion kann eine B-Zellen-Reaktion oder eine T-Zellen-Reaktion umfassen. Des weiteren kann eine T-Zellen-Reaktion die Produktion von cytolytischen T-Zellen und/oder Helfer-T-Zellen umfassen, die spezifisch für die Wirtszellen sind, die das Tumor-assoziierte Antigen oder einen Teil davon präsentieren oder spezifisch für Zellen des Patienten sind, die das Tumor-assoziierte Antigen oder einen Teil davon exprimieren.

Die Erfindung betrifft auch ein Verfahren zur Behandlung einer Erkrankung, die sich durch die Expression oder abnormale Expression eines erfindungsgemäß identifizierten Tumor-assoziierten Antigens auszeichnet, umfassend (i) die Identifikation von Zellen aus dem Patienten, die abnormale Mengen des Tumor-assoziierten Antigens exprimieren, (ii) die Isolierung einer Probe der Zellen, (iii) die Kultivierung der Zellen und (iv) das Einbringen der Zellen in den Patienten in einer Menge, die geeignet ist, eine Immunreaktion gegen die Zellen auszulösen.

Vorzugsweise sind die erfindungsgemäß verwendeten Wirtszellen nicht-proliferativ oder werden nicht-proliferativ gemacht. Eine Erkrankung, die sich durch die Expression oder abnormale Expression eines Tumor-assoziierten Antigens auszeichnet, ist insbesondere Krebs.

Des weiteren betrifft die vorliegende Erfindung eine Nukleinsäure, die aus der Gruppe ausgewählt ist bestehend aus (a) einer Nukleinsäure, die eine Nukleinsäuresequenz umfasst, die aus der Gruppe bestehend aus SEQ ID NOs: 69, 71, 73, 79, 80, 85, 87, 102, 104, 106, 108, 110, 112, einem Teil oder Derivat davon ausgewählt ist, (b) einer Nukleinsäure, die unter stringenten Bedingungen mit der Nukleinsäure unter (a) hybridisiert, (c) einer Nukleinsäure, die in Bezug auf die Nukleinsäure unter (a) oder (b) degeneriert ist, und (d) einer Nukleinsäure, die zu der Nukleinsäure unter (a), (b) oder (c) komplementär ist. Des weiteren betrifft die Erfindung eine Nukleinsäure, die für ein Protein oder Polypeptid kodiert, das eine Aminosäuresequenz umfasst, ausgewählt aus der Gruppe bestehend aus SEQ ID NOs: 61 bis 68, 70, 72, 74, 81, 82, 86, 88, 96 bis 101, 103, 105, 107, 109, 111, 113, einem Teil oder Derivat davon.

In einem weiteren Aspekt betrifft die Erfindung ein rekombinantes Nukleinsäuremolekül, insbesondere DNA- oder RNA-Molekül, das eine erfindungsgemäße Nukleinsäure umfasst. Die Erfindung betrifft auch Wirtszellen, die eine erfindungsgemäße Nukleinsäure oder ein rekombinantes Nukleinsäuremolekül, das eine erfindungsgemäße Nukleinsäure umfasst, enthalten.

Die Wirtszelle kann ferner eine Nukleinsäure umfassen, die für ein HLA-Molekül kodiert. In einer Ausführungsform exprimiert die Wirtszelle das HLA-Molekül endogen. In einer weiteren Ausführungsform exprimiert die Wirtszelle das HLA-Molekül und/oder die erfindungsgemäße Nukleinsäure oder einen Teil davon rekombinant. Vorzugsweise ist die Wirtszelle nicht-proliferativ. In einer bevorzugten Ausführungsform ist die Wirtszelle eine Antigen-präsentierende Zelle, insbesondere eine dendritische Zelle, ein Monozyt oder ein Makrophage.

In einer weiteren Ausführungsform betrifft die Erfindung Oligonukleotide, die mit einer erfindungsgemäß identifizierten Nukleinsäure hybridisieren und als genetische Sonden oder als "Antisense-Moleküle" verwendet werden können. Nukleinsäuremoleküle in der Form von Oligonukleotid-Primem oder kompetenten Proben, die mit einer erfindungsgemäß identifizierten Nukleinsäure oder Teilen davon hybridisieren, können zum Auffinden von Nukleinsäuren verwendet werden, die zu der erfindungsgemäß identifizierten Nukleinsäure homolog sind. PCR-Amplifikation, Southern- und Northern-Hybridisierung können zum Auffinden homologer Nukleinsäuren eingesetzt werden. Die Hybridisierung kann unter niedrig-, besser unter mittel- und am besten unter hoch-stringenten Bedingungen erfolgen. Der Begriff "stringente Bedingungen" betrifft erfindungsgemäß Bedingungen, die eine spezifische Hybridisierung zwischen Polynukleotiden erlauben.

In einem weiteren Aspekt betrifft die Erfindung ein Protein oder Polypeptid, das von einer Nukleinsäure kodiert wird, die aus der Gruppe ausgewählt ist bestehend aus (a) einer Nukleinsäure, die eine Nukleinsäuresequenz umfasst, die aus der Gruppe bestehend aus SEQ ID NOs: 69, 71, 73, 79, 80, 85, 87, 102, 104, 106, 108, 110, 112, einem Teil oder Derivat davon ausgewählt ist, (b) einer Nukleinsäure, die unter stringenten Bedingungen mit der Nukleinsäure unter (a) hybridisiert, (c) einer Nukleinsäure, die in Bezug auf die Nukleinsäure unter (a) oder (b) degeneriert ist, und (d) einer Nukleinsäure, die zu der Nukleinsäure unter (a), (b) oder (c) komplementär ist. In einer bevorzugten Ausführungsform betrifft die Erfindung ein Protein oder Polypeptid, das eine Aminosäuresequenz umfasst, ausgewählt aus der Gruppe bestehend aus SEQ ID NOs: 61 bis 68, 70, 72, 74, 81, 82, 86, 88, 96 bis 101, 103, 105, 107, 109, 111, 113, einem Teil oder Derivat davon.

In einem weiteren Aspekt betrifft die Erfindung ein immunogenes Fragment eines erfindungsgemäß identifizierten Tumor-assoziierten Antigens. Das Fragment bindet vorzugsweise an einen menschlichen HLA-Rezeptor oder menschlichen Antikörper. Vorzugsweise umfasst ein erfindungsgemäßes Fragment eine Sequenz von mindestens 6, insbesondere mindestens 8, mindestens 10, mindestens 12, mindestens 15, mindestens 20, mindestens 30 oder mindestens 50 Aminosäuren. Insbesondere umfasst ein erfindungsgemäßes immunogenes Fragment eine Aminosäuresequenz, die aus der Gruppe bestehend aus SEQ ID NOs: 61 bis 68, 81, 82 und 96 bis 101, einem Teil oder Derivat davon ausgewählt ist.

In einem weiteren Aspekt betrifft die Erfindung ein Mittel, das an ein erfindungsgemäß identifiziertes Tumor-assoziiertes Antigen oder an einen Teil davon bindet. In einer bevorzugten Ausihrungsform ist das Mittel ein Antikörper. In weiteren Ausführungsformen ist der Antikörper ein chimärer, ein humanisierter oder mit kombinatorischen Techniken hergestellte Antikörper oder ein Fragment eines Antikörpers. Des weiteren betrifft die Erfindung einen Antikörper, der selektiv an einen Komplex aus (i) einem erfindungsgemäß identifizierten Tumor-assoziierten Antigen oder einem Teil davon und (ii) einem MHC-Molekül bindet, an das das erfindungsgemäß identifizierte Tumor-assoziierte Antigen oder der Teil davon bindet, wobei der Antiköper nicht alleine an (i) oder (ii) bindet. Ein erfindungsgemäßer Antikörper kann ein monoklonaler Antikörper sein. In weiteren Ausführungsformen ist der Antikörper ein chimärer oder humanisierter Antikörper oder ein Fragment eines natürlichen Antikörpers.

Des weiteren betrifft die Erfindung ein Konjugat zwischen einem erfindungsgemäßen Mittel, das an ein erfindungsgemäß identifiziertes Tumor-assoziiertes Antigen oder an einen Teil davon bindet, oder einem erfindungsgemäßen Antikörper und einem therapeutischen oder diagnostischen Mittel. In einer Ausführungsform ist das therapeutische oder diagnostische Mittel ein Toxin.

In einem weiteren Aspekt betrifft die Erfindung einen Kit zum Nachweis der Expression oder abnormalen Expression eines erfindungsgemäß identifizierten Tumor-assoziierten Antigens, umfassend Mittel zum Nachweis (i) der Nukleinsäure, die für das Tumor-assoziierte Antigen kodiert, oder eines Teils davon, (ii) des Tumor-assoziierten Antigens oder eines Teils davon, (iii) von Antikörpern, die an das Tumor-assoziierte Antigen oder einen Teil davon binden, und/oder (iv) von T-Zellen, die für einen Komplex zwischen dem Tumor-assoziierten Antigen oder einem Teil davon und einem MHC-Molekül spezifisch sind. In einer Ausführungsform sind die Mittel zum Nachweis der Nukleinsäure oder des Teils davon Nukleinsäuremoleküle für die selektive Amplifikation der Nukleinsäure, die insbesondere eine Sequenz von 6-50, insbesondere 10-30, 15-30 oder 20-30 zusammenhängenden Nukleotiden aus der Nukleinsäure umfassen.

Die Erfindung betrifft insbesondere folgendes:
1. Pharmazeutische Zusammensetzung, umfassend ein Mittel, das die Expression oder Aktivität eines Tumor-assoziierten Antigens hemmt, wobei das Tumor-assoziierte Antigen eine Sequenz aufweist, die von einer Nukleinsäure kodiert wird, die aus der Gruppe ausgewählt ist, bestehend aus:
   (a) einer Nukleinsäure, die eine Nukleinsäuresequenz umfasst, die aus der Gruppe bestehend aus SEQ ID NOs: 1, 3, 5, 7, 9, 11, 13, 15, 17, 19, 21, 23, 25, 27, 29, 69, 71, 73, 75, 79, 80, 85, 87, 102, 104, 106, 108, 110, 112, einem Teil oder Derivat davon ausgewählt ist,
   (b) einer Nukleinsäure, die unter stringenten Bedingungen mit der Nukleinsäure unter (a) hybridisiert,
   (c) einer Nukleinsäure, die in Bezug auf die Nukleinsäure unter (a) oder (b) degeneriert ist und
   (d) einer Nukleinsäure, die zu der Nukleinsäure unter (a), (b) oder (c) komplementär ist.
2. Pharmazeutische Zusammensetzung, umfassend ein Mittel mit tumorhemmender Aktivität, das selektiv ist für Zellen, die eine Expression oder abnormale Expression eines tumorassoziierten Antigens aufweisen, wobei das Tumor-assoziierte Antigen eine Sequenz aufweist, die von einer Nukleinsäure kodiert wird, die aus der Gruppe ausgewählt ist, bestehend aus
   (a) einer Nukleinsäure, die eine Nukleinsäuresequenz umfasst, die aus der Gruppe bestehend aus SEQ ID NOs: 1, 3, 5, 7, 9, 11, 13, 15, 17, 19, 21, 23, 25, 27, 29, 69, 71, 73, 75, 79, 80, 85, 87, 102, 104, 106, 108, 110, 112, einem Teil oder Derivat davon ausgewählt ist,
   (b) einer Nukleinsäure, die unter stringenten Bedingungen mit der Nukleinsäure unter (a) hybridisiert,
   (c) einer Nukleinsäure, die in Bezug auf die Nukleinsäure unter (a) oder (b) degeneriert ist und
   (d) einer Nukleinsäure, die zu der Nukleinsäure unter (a), (b) oder (c) komplementär ist.
3. Pharmazeutische Zusammensetzung nach Ziffer 2, wobei das Mittel die Induktion des Zelltods, die Reduktion des Zellwachstums, eine Schädigung der Zellmembran oder eine Sekretion von Zytokinen bewirkt.
4. Pharmazeutische Zusammensetzung nach Ziffer 1 oder 2, wobei das Mittel eine Antisense-Nukleinsäure ist, die selektiv mit der Nukleinsäure hybridisiert, die für das Tumor-assozüerte Antigen kodiert.
5. Pharmazeutische Zusammensetzung nach Ziffer 1 oder 2, wobei das Mittel ein Antikörper ist, der selektiv an das Tumor-assoziierte Antigen bindet.
6. Pharmazeutische Zusammensetzung nach Ziffer 2, wobei das Mittel ein komplementaktivierender Antikörper ist, der selektiv an das Tumor-assoziierter Antigen bindet.
7. Pharmazeutische Zusammensetzung, umfassend ein Mittel, das bei einer Verabreichung selektiv die Menge an Komplexen zwischen einem HLA-Molekül und einem Tumor-assoziierten Antigen oder einem Teil davon erhöht, wobei das Tumor-assoziierte Antigen eine Sequenz aufweist, die von einer Nukleinsäure kodiert wird, die aus der Gruppe ausgewählt ist, bestehend aus:
   (a) einer Nukleinsäure, die eine Nukleinsäuresequenz umfasst, die aus der Gruppe bestehend aus SEQ CD NOs: 1, 3, 5, 7, 9, 11, 13, 15, 17, 19, 21, 23, 25, 27, 29, 69, 71, 73, 75, 79, 80, 85, 87, 102, 104, 106, 108, 110, 112, einem Teil oder Derivat davon ausgewählt ist,
   (b) einer Nukleinsäure, die unter stringenten Bedingungen mit der Nukleinsäure unter (a) hybridisiert,
   (c) einer Nukleinsäure, die in Bezug auf die Nukleinsäure unter (a) oder (b) degeneriert ist und
   (d) einer Nukleinsäure, die zu der Nukleinsäure unter (a), (b) oder (c) komplementär ist.
8. Pharmazeutische Zusammensetzung nach Ziffer 7, wobei das Mittel einen oder mehrere Bestandteile umfasst, die aus der Gruppe ausgewählt sind, bestehend aus:
   (i) dem Tumor-assoziierten Antigen oder einem Teil davon,
   (ii) einer Nukleinsäure, die für das Tumor-assoziierte Antigen oder einen Teil davon kodiert,
   (iii) einer Wirtszelle, die das Tumor-assoziierte Antigen oder einen Teil davon exprimiert, und
   (iv) isolierten Komplexen zwischen dem Tumor-assoziierten Antigen oder einem Teil davon und einem HLA-Molekül.
9. Pharmazeutische Zusammensetzung nach Ziffer 1, 2 oder 7, wobei das Mittel mehrere Mittel umfasst, die jeweils selektiv die Expression oder Aktivität verschiedener Tumor-assozüerter Antigene hemmen, jeweils selektiv für Zellen sind, die verschiedene Tumor-assoziierte Antigene exprimieren oder die Menge an Komplexen zwischen HLA-Molekülen und verschiedenen Tumor-assoziierten Antigenen oder Teilen davon erhöhen, wobei mindestens eines der Tumör-assozüerten Antigene eine Sequenz aufweist, die von einer Nukleinsäure kodiert wird, die aus der Gruppe ausgewählt ist, bestehend aus:
   (a) einer Nukleinsäure, die eine Nukleinsäuresequenz umfasst, die aus der Gruppe bestehend aus SEQ ID NOs: 1, 3, 5, 7, 9, 11, 13, 15, 17, 19, 21, 23, 25, 27, 29, 69, 71, 73, 75, 79, 80, 85, 87, 102, 104, 106, 108, 110, 112, einem Teil oder Derivat davon ausgewählt ist,
   (b) einer Nukleinsäure, die unter stringenten Bedingungen mit der Nukleinsäure unter (a) hybridisiert,
   (c) einer Nukleinsäure, die in Bezug auf die Nukleinsäure unter (a) oder (b) degeneriert ist und
   (d) einer Nukleinsäure, die zu der Nukleinsäure unter (a), (b) oder (c) komplementär ist.
10. Pharmazeutische Zusammensetzung umfassend einen oder mehrer Bestandteile, die aus der Gruppe ausgewählt sind, bestehend aus:
   (i) einem Tumor-assoziierten Antigen oder einem Teil davon,
   (ii) einer Nukleinsäure, die für ein Tumor-assoziiertes Antigen oder einen Teil davon kodiert,
   (iii) einem Antikörper, der an ein Tumor-assoziiertes Antigen oder einen Teil davon bindet,
   (iv) einer Antisense-Nukleinsäure, die spezifisch mit einer Nukleinsäure, die für ein Tumor-assoziiertes Antigen kodiert, hybridisiert,
   (v) einer Wirtszelle, die ein Tumor-assozüertes Antigen oder einen Teil davon exprimiert, und
   (vi) isolierten Komplexen zwischen einem Tumor-assozüerten Antigen oder einem Teil davon und einem HLA-Molekül,
   wobei das Tumor-assozüerte Antigen eine Sequenz aufweist, die von einer Nukleinsäure kodiert wird, die aus der Gruppe ausgewählt ist, bestehend aus:
   (a) einer Nukleinsäure, die eine Nukleinsäuresequenz umfasst, die aus der Gruppe bestehend aus SEQ ID NOs: 1, 3, 5, 7, 9, 11, 13, 15, 17, 19, 21, 23, 25, 27, 29, 69, 71, 73, 75, 79, 80, 85, 87, 102, 104, 106, 108, 110, 112, einem Teil oder Derivat davon ausgewählt ist,
   (b) einer Nukleinsäure, die unter stringenten Bedingungen mit der Nukleinsäure unter (a) hybridisiert,
   (c) einer Nukleinsäure, die in Bezug auf die Nukleinsäure unter (a) oder (b) degeneriert ist und
   (d) einer Nukleinsäure, die zu der Nukleinsäure unter (a), (b) oder (c) komplementär ist.
11. Pharmazeutische Zusammensetzung nach Ziffer 8 oder 10, wobei die Nukleinsäure unter (ii) in einem Expressionsvektor vorliegt.
12. Pharmazeutische Zusammensetzung nach Ziffer 8 oder 10, wobei die Nukleinsäure unter (ii) funktionell mit einem Promotor verbunden ist.
13. Pharmazeutische Zusammensetzung nach Ziffer 8 oder 10, wobei die Wirtszelle das Tumor-assoziierte Antigen oder den Teil davon sekretiert.
14. Pharmazeutische Zusammensetzung nach Ziffer 8 oder 10, wobei die Wirtszelle zusätzlich ein HLA-Molekül exprimiert, das an das Tumor-assoziierte Antigen oder den Teil davon bindet.
15. Pharmazeutische Zusammensetzung nach Ziffer 14, wobei die Wirtszelle das HLA-Molekül und/oder das Tumor-assozüerte Antigen oder den Teil davon rekombinant exprimiert.
16. Pharmazeutische Zusammensetzung nach Ziffer 14, wobei die Wirtszelle das HLA-Molekül endogen exprimiert.
17. Pharmazeutische Zusammensetzung nach Ziffer 8, 10, 14 oder 16, wobei die Wirtszelle eine Antigen-präsentierende Zelle ist.
18. Pharmazeutische Zusammensetzung nach Ziffer 17, wobei die Antigen-präsentierende Zelle eine dendritische Zelle oder ein Makrophage ist
19. Pharmazeutische Zusammensetzung nach einer der Ziffern 8, 10 und 13-18, wobei die Wirtszelle nicht-proliferativ ist.
20. Pharmazeutische Zusammensetzung nach Ziffer 5 oder 10, wobei der Antikörper ein monoklonaler Antikörper ist.
21. Pharmazeutische Zusammensetzung nach Ziffer 5 oder 10, wobei der Antikörper ein chimärer oder humanisierter Antikörper ist.
22. Pharmazeutische Zusammensetzung nach Ziffer 5 oder 10, wobei der Antikörper ein Fragment eines natürlichen Antikörpers ist.
23. Pharmazeutische Zusammensetzung nach Ziffer 5 oder 10, wobei der Antikörper mit einem therapeutischen oder diagnostischen Mittel gekoppelt ist.
24. Pharmazeutische Zusammensetzung nach Ziffer 4 oder 10, wobei die Antisense-Nukleinsäure eine Sequenz von 6-50 zusammenhängenden Nukleotiden aus der Nukleinsäure, die für das Tumor-assoziierte Antigen kodiert, umfasst.
25. Pharmazeutische Zusammensetzung nach einer der Ziffern 8 und 10-13, wobei das durch die pharmazeutische Zusammensetzung bereitgestellte Tumor-assoziierte Antigen oder der Teil davon an MHC-Moleküle auf der Oberfläche von Zellen bindet, die eine abnormale Menge des Tumor-assoziierten Antigens oder eines Teils davon exprimieren.
26. Pharmazeutische Zusammensetzung nach Ziffer 25, wobei die Bindung eine cytolytische Reaktion hervorruft und/ oder eine Cytokinausschüttung induziert
27. Pharmazeutische Zusammensetzung nach einer der Ziffern 1-26, ferner umfassend einen pharmazeutisch verträglichen Träger und/oder ein Adjuvans.
28. Pharmazeutische Zusammensetzung nach Ziffer 27, wobei das Adjuvans Saponin, GM-CSF, CpG, Zytokin oder ein Chemokin ist.
29. Pharmazeutische Zusammensetzung nach einer der Ziffern 1-28, die zur Behandlung einer Erkrankung eingesetzt werden kann, die sich durch die Expression oder abnormale Expression eines Tumor-assoziierten Antigens auszeichnet.
30. Pharmazeutische Zusammensetzung nach Ziffer 29, wobei die Erkrankung Krebs ist.
31. Pharmazeutische Zusammensetzung nach Ziffer 29, wobei die Erkrankung ein Lungentumor, ein Brusttumor, ein Prostatatumor, ein Melanom, ein Colontumor, eine Metastase eines Colontumors, ein Nierenzellkarzinom oder ein Zervixkarzinom, ein Coloncarcinom oder ein Mammacarcinom ist.
32. Pharmazeutische Zusammensetzung nach einer der Ziffern 1-31, wobei das Tumor-assoziierte Antigen eine Aminosäuresequenz umfasst, die aus der Gruppe bestehend aus SEQ ID NOs: 2, 4, 6, 8, 10, 12, 14, 16, 18, 20, 22, 24, 26, 28, 30, 61 bis 68, 70, 72, 74, 76, 81, 82, 86, 88, 96 bis 101, 103, 105, 107, 109, 111, 113, einem Teil oder Derivat davon ausgewählt ist.
33. Verfahren zur Diagnose einer Erkrankung, die sich durch die Expression oder abnormale Expression eines Tumor-assoziierten Antigens auszeichnet, umfassend
   (i) den Nachweis einer Nukleinsäure, die für das Tumor-assoziierte Antigen kodiert, oder eines Teils davon; und/oder
   (ii) den Nachweis des Tumor-assoziierten Antigens oder eines Teils davon, und/oder
   (iii) den Nachweis eines Antikörpers gegen das Tumor-assoziierte Antigen oder eines Teils davon und/oder
   (iv) den Nachweis von cytotoxischen oder Helfer-T-Lymphozyten, die für das Tumor-assoziierte Antigen oder einen Teil davon spezifisch sind in einer aus einem Patienten isolierten biologischen Probe, wobei
   das Tumor-assoziierte Antigen eine Sequenz aufweist, die von einer Nukleinsäure kodiert wird, die aus der Gruppe ausgewählt ist, bestehend aus:
   (a) einer Nukleinsäure, die eine Nukleinsäuresequenz umfasst, die aus der Gruppe bestehend aus SEQ ID NOs: 1, 3, 5, 7, 9, 11, 13, 15, 17,19, 21, 23, 25, 27, 29, 69, 71, 73, 75, 79, 80, 85, 87, 102, 104, 106, 108, 110, 112, einem Teil oder Derivat davon ausgewählt ist,
   (b) einer Nukleinsäure, die unter stringenten Bedingungen mit der Nukleinsäure unter (a) hybridisiert,
   (c) einer Nukleinsäure, die in Bezug auf die Nukleinsäure unter (a) oder (b) degeneriert ist
      und
   (d) einer Nukleinsäure, die zu der Nukleinsäure unter (a), (b) oder (c) komplementär ist.
34. Verfahren nach Ziffer 33, wobei der Nachweis
   (i) die Kontaktierung der biologischen Probe mit einem Mittel, das spezifisch an die Nukleinsäure, die für das Tumor-assoziierte Antigen kodiert, oder den Teil davon, an das Tumor-assoziierte Antigen oder den Teil davon, an den Antikörper oder an die cytotoxischen oder Helfer-T-Lymphozyten bindet, und
   (ii) den Nachweis der Komplexbildung zwischen dem Mittel und der Nukleinsäure oder dem Teil davon, dem Tumor-assoziierten Antigen oder, dem Teil davon, dem Antikörper oder den cytotoxischen oder Helfer-T-Lymphozyten umfasst.
35. Verfahren nach Ziffer 33 oder 34, wobei der Nachweis mit dem Nachweis in einer vergleichbaren normalen biologischen Probe verglichen wird.
36. Verfahren nach einer der Ziffern 33-35, wobei sich die Erkrankung durch die Expression oder abnormale Expression mehrerer verschiedener Tumor-assoziierter Antigene auszeichnet und der Nachweis einen Nachweis mehrerer Nukleinsäuren, die für die mehreren verschiedenen Tumor-assoziierten Antigene kodieren, oder von Teilen davon, den Nachweis der mehreren verschiedenen Tumor-assoziierten Antigene oder von Teilen davon, den Nachweis mehrerer Antikörper, die an die mehreren verschiedenen Tumor-assoziierten Antigene oder an Teile davon binden, oder den Nachweis mehrerer cytotoxischer oder Helfer-T-Lymphozyten, die für die mehreren verschiedenen Tumor-assoziierten Antigene spezifisch sind, umfasst.
37. Verfahren nach einer der Ziffern 33-36, wobei der Nachweis der Nukleinsäure oder des Teils davon mit einer Polynukleotid-Sonde erfolgt, die spezifisch mit der Nukleinsäure oder dem Teil davon hybridisiert.
38. Verfahren nach Ziffer 37, wobei die Polynukleotid-Sonde eine Sequenz von 6-50 zusammenhängenden Nukleotiden aus der Nukleinsäure, die für das Tumor-assoziierte Antigen kodiert, umfasst.
39. Verfahren nach einer der Ziffern 33-36, wobei der Nachweis der Nukleinsäure oder des Teils davon durch selektive Amplifikation der Nukleinsäure oder des Teils davon erfolgt.
40. Verfahren nach einer der Ziffern 33-36, wobei das nachzuweisende Tumor-assoziierte Antigen oder der Teil davon in einem Komplex mit einem MHC-Molekül vorliegt.
41. Verfahren nach Ziffer 40, wobei das MHC-Molekül ein HLA-Molekül ist.
42. Verfahren nach einer der Ziffern 33-36 und 40-41, wobei der Nachweis des Tumor-assoziierten Antigens oder des Teils davon mit einem Antikörper erfolgt, der spezifisch an das Tumor-assoziierte Antigen oder den Teil davon bindet.
43. Verfahren nach einer der Ziffern 33-36, wobei der Nachweis des Antikörpers mit einem Protein oder Peptid erfolgt, das spezifisch an den Antikörper bindet.
44. Verfahren zur Bestimmung der Regression, des Verlaufs oder des Ausbruchs einer Erkrankung, die sich durch die Expression oder abnormale Expression eines Tumor-assoziierten Antigens auszeichnet, umfassend die Überwachung einer Probe aus einem Patienten, der die Erkrankung aufweist oder in Verdacht steht, an der Erkrankung zu erkranken in Bezug auf einen oder mehrere Parameter, ausgewählt aus der Gruppe, bestehend aus:
   (i) der Menge der Nukleinsäure, die für das Tumor-assoziierte Antigen kodiert, oder eines Teil davon,
   (ii) der Menge des Tumor-assoziierten Antigens oder eines Teils davon,
   (iii) der Menge an Antikörpern, die an das Tumor-assozüerte Antigen oder einen Teil davon binden, und
   (iv) der Menge an cytolytischen oder Cytokin-ausschüttenden T-Zellen, die für einen Komplex zwischen dem Tumor-assoziierten Antigen oder einem Teil davon und einem MHC-Molekül spezifisch sind, wobei das Tumor-assoziierte Antigen eine Sequenz aufweist, die von einer Nukleinsäure kodiert wird, die aus der Gruppe ausgewählt ist, bestehend aus:
      (a) einer Nukleinsäure, die eine Nukleinsäuresequenz umfasst, die aus der Gruppe bestehend aus SEQ ID NOs: 1, 3, 5, 7, 9, 11, 13, 15, 17,19, 21, 23, 25, 27, 29, 69, 71, 73, 75, 79, 80, 85, 87, 102, 104, 106, 108, 110, 112, einem Teil oder Derivat davon ausgewählt ist,
      (b) einer Nukleinsäure, die unter stringenten Bedingungen mit der Nukleinsäure unter (a) hybridisiert,
      (c) einer Nukleinsäure, die in Bezug auf die Nukleinsäure unter (a) oder (b) degeneriert ist und
      (d) einer Nukleinsäure, die zu der Nukleinsäure unter (a), (b) oder (c) komplementär ist.
45. Verfahren nach Ziffer 44, wobei das Verfahren die Bestimmung des oder der Parameter zu einem ersten Zeitpunkt in einer ersten Probe und zu einem zweiten Zeitpunkt in einer weiteren Probe umfasst und durch einen Vergleich der beiden Proben der Verlauf der Erkrankung ermittelt wird.
46. Verfahren nach Ziffer 44 oder 45, wobei die Erkrankung sich durch die Expression oder abnormale Expression mehrerer verschiedener Tumor-assozüerter Antigene auszeichnet und die Überwachung eine Überwachung
   (i) der Menge mehrerer Nukleinsäuren, die für die mehreren verschiedenen Tumor-assozüerten Antigene kodieren, oder von Teilen davon,
   (ii) der Menge der mehreren verschiedenen Tumor-assoziierten Antigene oder von Teilen davon,
   (iii) der Menge mehrerer Antikörper, die an die mehreren verschiedenen Tumor-assoziierten Antigene oder an Teile davon binden, und/oder
   (iv) der Menge mehrerer cytolytischer oder Cytokine-ausschüttender T-Zellen, die für Komplexe zwischen den mehreren verschiedenen Tumor-assoziierten Antigenen oder von Teilen davon und MHC-Molekülen spezifisch sind, umfasst.
47. Verfahren nach einer der Ziffern 44-46, wobei die Überwachung der Menge der Nukleinsäure oder des Teils davon mit einer Polynukleotid-Sonde erfolgt, die spezifisch mit der Nukleinsäure oder dem Teil davon hybridisiert.
48. Verfahren nach Ziffer 47, wobei die Polynukleotid-Sonde eine Sequenz von 6-50 zusammenhängenden Nukleotiden aus der Nukleinsäure, die für das Tumor-assozüerte Antigen kodiert, umfasst.
49. Verfahren nach einer der Ziffern 44-46, wobei die Überwachung der Menge der Nukleinsäure oder des Teils davon durch selektive Amplifikation der Nukleinsäure oder des Teils davon erfolgt.
50. Verfahren nach einer der Ziffern 44-46, wobei die Überwachung der Menge des Tumor-assoziierten Antigens oder des Teils davon mit einem Antikörper erfolgt, der spezifisch an das Tumor-assoziierte Antigen oder den Teil davon bindet.
51. Verfahren nach einer der Ziffern 44-46, wobei die Überwachung der Menge an Antikörpern mit einem Protein oder Peptid erfolgt, das spezifisch an den Antikörper bindet.
52. Verfahren nach einer der Ziffern 44-46, wobei die Überwachung der Menge an cytolytischen oder Cytokin-aussschüttenden T-Zellen mit einer Zelle erfolgt, die den Komplex zwischen dem Tumor-assoziierten Antigen oder dem Teil davon und einem MHC-Molekül präsentiert.
53. Verfahren nach einer der Ziffern 37-38, 42-43, 47-48 und 50-52, wobei die Polynukleotid-Sonde, der Antikörper, das Protein oder Peptid oder die Zelle nachweisbar markiert sind.
54. Verfahren nach Ziffer 53, wobei der nachweisbare Marker ein radioaktiver Marker oder ein Enzymmarker ist.
55. Verfahren nach einer der Ziffern 33-54, wobei die Probe Körperflüssigkeit und/oder Körpergewebe umfasst.
56. Verfahren zur Behandlung einer Erkrankung, die sich durch die Expression oder abnormale Expression eines Tumor-assoziierten Antigens auszeichnet, umfassend die Verabreichung einer pharmazeutischen Zusammensetzung nach einer der Ziffern 1-32, wobei das Tumor-assoziierte Antigen eine Sequenz aufweist, die von einer Nukleinsäure kodiert wird, die aus der Gruppe ausgewählt ist, bestehend aus:
   (a) einer Nukleinsäure, die eine Nukleinsäuresequenz umfasst, die aus der Gruppe bestehend aus SEQ ID NOs: 1, 3, 5, 7, 9, 11, 13, 15, 17,19, 21, 23, 25, 27, 29, 69, 71, 73, 75, 79, 80, 85, 87, 102, 104, 106, 108, 110, 112, einem Teil oder Derivat davon ausgewählt ist,
   (b) einer Nukleinsäure, die unter stringenten Bedingungen mit der Nukleinsäure unter (a) hybridisiert,
   (c) einer Nukleinsäure, die in Bezug auf die Nukleinsäure unter (a) oder (b) degeneriert ist und
   (d) einer Nukleinsäure, die zu der Nukleinsäure unter (a), (b) oder (c) komplementär ist.
57. Verfahren zur Behandlung, Diagnose oder Überwachung einer Erkrankung, die sich durch die Expression oder abnormale Expression eines Tumor-assoziierten Antigens auszeichnet, umfassend die Verabreichung eines Antikörpers, der an das Tumor-assoziierte Antigen oder einen Teil davon bindet und mit einem therapeutischen oder diagnostischen Mittel gekoppelt ist, wobei das Tumor-assoziierte Antigen eine Sequenz aufweist, die von einer Nukleinsäure kodiert wird, die aus der Gruppe ausgewählt ist, bestehend aus:
   (a) einer Nukleinsäure, die eine Nukleinsäuresequenz umfasst, die aus der Gruppe bestehend aus SEQ ID NOs: 1, 3, 5, 7, 9,11, 13, 15, 17,19, 21, 23, 25, 27, 29, 69, 71, 73, 75, 79, 80, 85, 87, 102, 104, 106, 108, 110, 112, einem Teil oder Derivat davon ausgewählt ist,
   (b) einer Nukleinsäure, die unter stringenten Bedingungen mit der Nukleinsäure unter (a) hybridisiert,
   (c) einer Nukleinsäure, die in Bezug auf die Nukleinsäure unter (a) oder (b) degeneriert ist und
   (d) einer Nukleinsäure, die zu der Nukleinsäure unter (a), (b) oder (c) komplementär ist
58. Verfahren nach Ziffer 42, 50 oder 57, wobei der Antikörper ein monoklonaler Antikörper ist.
59. Verfahren nach Ziffer 42, 50 oder 57, wobei der Antikörper ein chimärer oder humanisierter Antikörper ist.
60. Verfahren nach Ziffer 42, 50 oder 57, wobei der Antikörper ein Fragment eines natürlichen Antikörpers ist.
61. Verfahren zur Behandlung eines Patienten mit einer Erkrankung, die sich durch die Expression oder abnormale Expression eines Tumor-assoziierten Antigens auszeichnet, umfassend:
   (i) die Entfernung einer Probe mit immunreaktiver Zellen aus dem Patienten,
   (ii) die Kontaktierung der Probe mit einer Wirtszelle, die das Tumor-assoziierte Antigen oder einen Teil davon exprimiert, unter Bedingungen, die eine Produktion cytolytischer oder Cytokine-ausschüttender T-Zellen gegen das Tumor-assoziierte Antigen oder einen Teil davon begünstigen, und
   (iii) das Einbringen der cytolytischen oder Cytokine-ausschüttenden T-Zellen in den Patienten in einer Menge, die geeignet ist, Zellen zu lysieren, die das Tumor-assoziierte Antigen oder einen Teil davon exprimieren, wobei das Tumor-assoziierte Antigen eine Sequenz aufweist, die von einer Nukleinsäure kodiert wird, die aus der Gruppe ausgewählt ist, bestehend aus:
      (a) einer Nukleinsäure, die eine Nukleinsäuresequenz umfasst, die aus der Gruppe bestehend aus SEQ ID NOs: 1, 3, 5, 7, 9, 11, 13, 15, 17, 19, 21, 23, 25, 27, 29, 69, 71, 73, 75, 79, 80, 85, 87, 102, 104, 106, 108, 110, 112, einem Teil oder Derivat davon ausgewählt ist,
      (b) einer Nukleinsäure, die unter stringenten Bedingungen mit der Nukleinsäure unter (a) hybridisiert,
      (c) einer Nukleinsäure, die in Bezug auf die Nukleinsäure unter (a) oder (b) degeneriert ist und
      (d) einer Nukleinsäure, die zu der Nukleinsäure unter (a), (b) oder (c) komplementär ist.
62. Verfahren nach Ziffer 61, wobei die Wirtszelle ein HLA-Molekül rekombinant exprimiert, das an das Tumor-assoziierte Antigen oder einen Teil davon bindet.
63. Verfahren nach Ziffer 62, wobei die Wirtszelle ein HLA-Molekül endogen exprimiert, das an das Tumor-assoziierte Antigen oder einen Teil davon bindet.
64. Verfahren zur Behandlung eines Patienten mit einer Erkrankung, die sich durch die Expression oder abnormale Expression eines Tumor-assoziierten Antigens auszeichnet, umfassend:
   (i) die Identifizierung einer Nukleinsäure, die von Zellen exprimiert wird, die mit der Erkrankung assoziiert sind, wobei die Nukleinsäure aus der Gruppe ausgewählt ist, bestehend aus:
      (a) einer Nukleinsäure, die eine Nukleinsäuresequenz umfasst, die aus der Gruppe bestehend aus SEQ ID NOs: 1, 3, 5, 7, 9, 11, 13, 15, 17, 19, 21, 23, 25, 27, 29, 69, 71, 73, 75, 79, 80, 85, 87, 102, 104, 106, 108, 110, 112, einem Teil oder Derivat davon ausgewählt ist,
      (b) einer Nukleinsäure, die unter stringenten Bedingungen mit der Nukleinsäure unter (a) hybridisiert,
      (c) einer Nukleinsäure, die in Bezug auf die Nukleinsäure unter (a) oder (b) degeneriert ist und
      (d) einer Nukleinsäure, die zu der Nukleinsäure unter (a), (b) oder (c) komplementär ist,
   (ii) die Transfektion einer Wirtszelle mit der Nukleinsäure oder einem Teil davon,
   (iii) die Kultivierung der transfizierten Wirtszelle für eine Expression der Nukleinsäure, und
   (iv) das Einbringen der Wirtszellen oder eines Extrakts davon in den Patienten in einer Menge, die geeignet ist, die Immunreaktion gegen die Zellen des Patienten, die mit der Erkrankung assoziiert sind, zu erhöhen.
65. Verfahren nach Ziffer 64, ferner umfassend die Identifizierung eines MHC-Moleküls, das das Tumor-assoziierte Antigen oder einen Teil davon präsentiert, wobei die Wirtszelle das identifizierte MHC-Molekül exprimiert und das Tumor-assoziierte Antigen oder einen Teil davon präsentiert.
66. Verfahren nach Ziffer 64 oder 65, wobei die Immunreaktion eine B-Zellen-Reaktion oder eine T-Zellen-Reaktion umfasst.
67. Verfahren nach Ziffer 66, wobei die Immunreaktion eine T-Zellen-Reaküon ist, umfassend die Produktion cytolytischer oder Cytokine-ausschüttenden T-Zellen, die spezifisch für die Wirtszellen sind, die das Tumor-assoziierte Antigen oder einen Teil davon präsentieren oder spezifisch für Zellen des Patienten sind, die das Tumor-assoziierte Antigen oder einen Teil davon exprimieren.
68. Verfahren nach einer der Ziffern 61-67, wobei die Wirtszellen nicht-proliferativ sind.
69. Verfahren zur Behandlung einer Erkrankung, die sich durch die Expression oder abnormale Expression eines Tumor-assoziierten Antigens auszeichnet, umfassend:
   (i) die Identifikation von Zellen aus dem Patienten, die abnormale Mengen des Tumor-assoziierten Antigens exprimieren,
   (ii) die Isolierung einer Probe der Zellen,
   (iii) die Kultivierung der Zellen, und
   (iv) das Einbringen der Zellen in den Patienten in einer Menge, die geeignet ist, eine Immunreaktion gegen die Zellen auszulösen, wobei das Tumor-assoziierte Antigen eine Sequenz aufweist, die von einer Nukleinsäure kodiert wird, die aus der Gruppe ausgewählt ist, bestehend aus:
      (a) einer Nukleinsäure, die eine Nukleinsäuresequenz umfasst, die aus der Gruppe bestehend aus SEQ ID NOs: 1, 3, 5, 7, 9, 11,13,15,17,19,21,23, 25,27,29, 69, 71, 73, 75, 79, 80, 85, 87, 102, 104, 106, 108, 110, 112, einem Teil oder Derivat davon ausgewählt ist,
      (b) einer Nukleinsäure, die unter stringenten Bedingungen mit der Nukleinsäure unter (a) hybridisiert,
      (c) einer Nukleinsäure, die in Bezug auf die Nukleinsäure unter (a) oder (b) degeneriert ist und
      (d) einer Nukleinsäure, die zu der Nukleinsäure unter (a), (b) oder (c) komplementär ist.
70. Verfahren nach einer der Ziffern 33-69, wobei die Erkrankung Krebs ist.
71. Verfahren zur Hemmung der Entwicklung von Krebs bei einem Patienten, umfassend die Verabreichung einer wirksamen Menge einer pharmazeutischen Zusammensetzung nach einer der Ziffern 1-32.
72. Verfahren nach einer der Ziffern 33-71, wobei das Tumor-assozüerte Antigen eine Aminosäuresequenz umfasst, die aus der Gruppe bestehend aus SEQ ID NOs: 2, 4, 6, 8, 10, 12, 14, 16, 18, 20, 22, 24, 26, 28, 30, 61 bis 68, 70, 72, 74, 76, 81, 82, 86, 88, 96 bis 101, 103, 105, 107, 109, 111, 113, einem Teil oder Derivat davon ausgewählt ist.
73. Nukleinsäure, die aus der Gruppe ausgewählt ist, bestehend aus:
   (a) einer Nukleinsäure, die eine Nukleinsäuresequenz umfasst, die aus der Gruppe bestehend aus SEQ ID NOs: 69, 71, 73, 79, 80, 85, 87, 102, 104, 106, 108, 110, 112, einem Teil oder Derivat davon ausgewählt ist,
   (b) einer Nukleinsäure, die unter stringenten Bedingungen mit der Nukleinsäure unter (a) hybridisiert,
   (c) einer Nukleinsäure, die in Bezug auf die Nukleinsäure unter (a) oder (b) degeneriert ist und
   (d) einer Nukleinsäure, die zu der Nukleinsäure unter (a), (b) oder (c) komplementär ist.
74. Nukleinsäure, die für ein Protein oder Polypeptid kodiert, das eine Aminosäuresequenz umfasst, ausgewählt aus der Gruppe bestehend aus SEQ ID NOs: 61 bis 68, 70, 72, 74, 81, 82, 86, 88, 96 bis 101, 103, 105, 107, 109, 111, 113, einem Teil oder Derivat davon.
75. Rekombinantes DNA- oder RNA-Molekül, das eine Nukleinsäure nach Ziffer 73, 74, 85, 87, umfasst.
76. Rekombinantes DNA-Molekül nach Ziffer 75, wobei das rekombinante DNA-Molekül ein Vektor ist.
77. Rekombinantes DNA-Molekül nach Ziffer 76, wobei der Vektor ein viraler Vektor oder ein Bakteriophage ist.
78. Rekombinantes DNA-Molekül nach einer der Ziffern 75-77, das ferner Expressionskontrollsequenzen umfasst, die die Expression der Nukleinsäure steuern.
79. Rekombinantes DNA-Molekül nach Ziffer 78, wobei die Expressionskontrollsequenzen homo- oder heterolog zu der Nukleinsäure sind.
80. Wirtszelle, die eine Nukleinsäure nach Ziffer 73 oder 74 oder ein rekombinantes DNA-Molekül nach einer der Ziffern 75-79 umfasst.
81. Wirtszelle nach Ziffer 80, die ferner eine Nukleinsäure umfasst, die für ein HLA-Molekül kodiert.
82. Protein oder Polypeptid, das von einer Nukleinsäure nach Ziffer 73 kodiert wird.
83. Protein oder Polypeptid, das eine Aminosäuresequenz umfasst, ausgewählt aus der Gruppe bestehend aus SEQ ID NOs: 61 bis 68, 70, 72, 74, 81, 82, 86, 88, 96 bis 101, 103, 105, 107, 109, 111, 113, einem Teil oder Derivat davon.
84. Immunogenes Fragment des Proteins oder Polypeptids nach Ziffer 82 oder 83.
85. Fragment des Proteins oder Polypeptids nach Ziffer 82 oder 83, das an menschlichen HLA-Rezeptor oder menschlichen Antikörper bindet.
86. Mittel, das spezifisch an ein Protein oder Polypeptid oder an einen Teil davon bindet, wobei das Protein oder Polypeptid von einer Nukleinsäure kodiert wird, die aus der Gruppe ausgewählt ist, bestehend aus:
   (a) einer Nukleinsäure, die eine Nukleinsäuresequenz umfasst, die aus der Gruppe bestehend aus SEQ ID NOs: 1, 3, 5, 7, 9, 11,13, 15, 17, 19, 21, 23, 25, 27, 29, 69, 71, 73, 75, 79, 80, 85, 87, 102, 104, 106, 108, 110, 112, einem Teil oder Derivat davon ausgewählt ist,
   (b) einer Nukleinsäure, die unter stringenten Bedingungen mit der Nukleinsäure unter (a) hybridisiert,
   (c) einer Nukleinsäure, die in Bezug auf die Nukleinsäure unter (a) oder (b) degeneriert ist und
   (d) einer Nukleinsäure, die zu der Nukleinsäure unter (a), (b) oder (c) komplementär ist.
87. Mittel nach Ziffer 86, wobei das Protein oder Polypeptid eine Aminosäuresequenz umfasst, die aus der Gruppe ausgewählt ist, bestehend aus SEQ ID NOs: 2, 4, 6, 8, 10, 12, 14, 16, 18, 20, 22, 24, 26, 28, 30, 61 bis 68, 70, 72, 74, 76, 81, 82, 86, 88, 96 bis 101, 103, 105, 107, 109, 111, 113, einem Teil oder Derivat davon.
88. Mittel nach Ziffer 86 oder 87, wobei das Mittel ein Antikörper ist.
89. Mittel nach Ziffer 88, wobei der Antikörper ein monoklonaler, chimärer oder humanisierter Antikörper oder ein Fragment eines Antikörpers ist.
90. Antikörper, der selektiv an einen Komplex aus:
   (i) einem Protein oder Polypeptid oder einem Teil davon und
   (ii) einem MHC-Molekül bindet, an das das Protein oder Polypeptid oder der Teil davon bindet, wobei der Antiköper nicht alleine an (i) oder (ii) bindet und das Protein oder Polypeptid von einer Nukleinsäure kodiert wird, die aus der Gruppe ausgewählt ist, bestehend aus:
      (a) einer Nukleinsäure, die eine Nukleinsäuresequenz umfasst, die aus der Gruppe bestehend aus SEQ ID NOs: 1, 3, 5, 7, 9, 11, 13, 15, 17, 19, 21, 23, 25, 27, 29, 69, 71, 73, 75, 79, 80, 85, 87, 102, 104, 106, 108, 110, 112, einem Teil oder Derivat davon ausgewählt ist,
      (b) einer Nukleinsäure, die unter stringenten Bedingungen mit der Nukleinsäure unter (a) hybridisiert,
      (c) einer Nukleinsäure, die in Bezug auf die Nukleinsäure unter (a) oder (b) degeneriert ist und
      (d) einer Nukleinsäure, die zu der Nukleinsäure unter (a), (b) oder (c) komplementär ist.
91. Antikörper nach Ziffer 90, wobei das Protein oder Polypeptid eine Aminosäuresequenz umfasst, die aus der Gruppe ausgewählt ist, bestehend aus SEQ ID NOs: 2, 4, 6, 8, 10, 12, 14, 16, 18, 20, 22, 24, 26, 28, 30, 61 bis 68, 70, 72, 74, 76, 81, 82, 86, 88, 96 bis 101, 103, 105, 107, 109, 111, 113, einem Teil oder Derivat davon.
92. Antikörper nach Ziffer 90 oder 91, wobei der Antikörper ein monoklonaler, chimärer oder humanisierter Antikörper oder ein Fragment eines Antikörpers ist.
93. Konjugat zwischen einem Mittel nach einer der Ziffern 86-89 oder einem Antikörper nach einer der Ziffern 90-92 und einem therapeutischen oder diagnostischen Mittel.
94. Konjugat nach Ziffer 93, wobei das therapeutische oder diagnostische Mittel ein Toxin ist
95. Kit zum Nachweis der Expression oder abnormalen Expression eines Tumor-assozüerten Antigens, umfassend Mittel zum Nachweis
   (i) der Nukleinsäure, die für das Tumor-assoziierte Antigen kodiert, oder eines Teils davon,
   (ii) des Tumor-assoziierten Antigens oder eines Teils davon,
   (iii) von Antikörpern, die an das Tumor-assozüerte Antigen oder einen Teil davon binden, und/oder
   (iv) von T-Zellen, die für einen Komplex zwischen dem Tumor-assoziierten Antigen oder einem Teil davon und einem MHC-Molekül spezifisch sind, wobei das Tumor-assoziierte Antigen eine Sequenz aufweist, die von einer Nukleinsäure kodiert wird, die aus der Gruppe ausgewählt ist, bestehend aus:
      (a) einer Nukleinsäure, die eine Nukleinsäuresequenz umfasst, die aus der Gruppe bestehend aus SEQ ID NOs: 1, 3, 5, 7, 9, 11, 13, 15, 17, 19, 21, 23, 25, 27, 29, 69, 71, 73, 75, 79, 80, 85, 87, 102, 104, 106, 108, 110,112, einem Teil oder Derivat davon ausgewählt ist,
      (b) einer Nukleinsäure, die unter stringenten Bedingungen mit der Nukleinsäure unter (a) hybridisiert,
      (c) einer Nukleinsäure, die in Bezug auf die Nuldeinsäure unter (a) oder (b) degeneriert ist und
      (d) einer Nukleinsäure, die zu der Nukleinsäure unter (a), (b) oder (c) komplementär ist.
96. Kit nach Ziffer 95, wobei die Mittel zum Nachweis der Nukleinsäure, die für das Tumor-assoziierte Antigen kodiert, oder eines Teils davon Nukleinsäuremoleküle für die selektive Amplifikation der Nukleinsäure sind.
97. Kit nach Ziffer 96, wobei die Nukleinsäuremoleküle für die selektive Amplifikation der Nukleinsäure eine Sequenz von 6-50 zusammenhängenden Nukleotiden aus der Nukleinsäure, die für das Tumor-assoziierte Antigen kodiert, umfassen.

### Detaillierte Beschreibung der Erfindung

Erfindungsgemäß werden Gene beschrieben, die in Tumorzellen selektiv exprimiert oder aberrant exprimiert werden und Tumor-assoziierte Antigene darstellen.

Erfindungsgemäß sind diese Gene oder ihre Derivate bevorzugte Zielstrukturen für therapeutische Ansätze. Konzeptionell können die therapeutischen Ansätze auf eine Hemmung der Aktivität des selektiv exprimierten Tumor-assozüerten Genproduktes zielen. Dies ist dann sinnvoll, wenn die aberrante respektive selektive Expression funktionell von tumorpathogenetischer Bedeutung ist und ihre Unterbindung mit einer selektiven Schädigung der entsprechenden Zellen einhergeht. Andere therapeutische Konzepte betrachten Tumor-assoziierte Antigene als Markierungen, die Effektormechanismen mit zellschädigendem Potential selektiv zu Tumorzellen rekrutieren. Hierbei ist die Funktion des Zielmoleküls selbst und seine Rolle bei der Tumorentstehung vollkommen unerheblich.

Mit "Derivat" einer Nukleinsäure ist erfindungsgemäß gemeint, dass einzelne oder multiple Nukleotidsubstitiitionen, -deletionen und/oder -additionen in der Nukleinsäure vorliegen. Weiterhin umfasst der Begriff "Derivat" auch eine chemische Derivatisierung einer Nukleinsäure an einer Base, einem Zucker oder Phosphat eines Nukleotids. Der Begriff "Derivat" umfasst auch Nukleinsäuren, die nicht in der Natur vorkommende Nukleotide und Nukleotidanaloga enthalten.

Eine Nukleinsäure ist erfindungsgemäß vorzugsweise Desoxyribonukleinsäure (DNA) oder Ribonukleinsäure (RNA). Nukleinsäuren umfassen erfindungsgemäß genomische DNA, cDNA, mRNA, rekombinant hergestellte und chemisch synthetisierte Moleküle. Eine Nukleinsäure kann erfindungsgemäß als einzelsträngiges oder doppelsträngiges und lineares oder kovalent kreisförmig geschlossenes Molekül vorliegen.

Die erfindungsgemäß beschriebenen Nukleinsäuren sind vorzugsweise isoliert. Der Begriff "isolierte Nukleinsäure" bedeutet erfindungsgemäß, dass die Nukleinsäure (i) *in vitro* amplifiziert wurde, zum Beispiel durch Polymerase-Kettenreaktion (PCR), (ii) rekombinant durch Klonierung produziert wurde, (iii) gereinigt wurde, zum Beispiel durch Spaltung und gelelektrophoretische Auftrennung, oder (iv) synthetisiert wurde, zum Beispiel durch chemische Synthese. Eine isolierte Nukleinsäure ist eine Nukleinsäure, die für eine Manipulierung durch rekombinante DNA-Techniken zur Verfügung steht.

Eine Nukleinsäure ist dann zu einer anderen Nukleinsäure "komplementär", wenn die beiden Sequenzen miteinander hybridisieren und ein stabiles Duplexmolekül eingehen können, wobei die Hybridisierung vorzugsweise unter Bedingungen erfolgt, die eine spezifische Hybridisierung zwischen Polynukleotiden erlauben (stringente Bedingungen). Stringente Bedingungen sind beispielsweise in Molecular Cloning: A Laboratory Manual, J. Sambrook et al., Hrsg., 2. Auflage, Cold Spring Harbor Laboratory Press, Cold Spring Harbor, New York, 1989, oder Current Protocols in Molecular Biology, F.M. Ausubel et al., Hrsg., John Wiley & Sons; Inc., New York beschrieben und betreffen beispielsweise die Hybridisierung bei 65°C in Hybridisierungspuffer (3,5 x SSC, 0,02% Ficoll, 0,02% Polyvinylpyrrolidon, 0,02% Rinderserumalbumin, 2,5 mM NaH₂PO₄ (pH 7), 0,5% SDS, 2 mM EDTA). SSC ist eine Lösung mit jeweils 0,15 M Natriumchlorid und Natriumcitrat, pH 7. Nach der Hybridisierung wird die Membran, auf die die DNA übertragen wurde, beispielsweise in 2 x SSC bei Raumtemperatur und sodann in 0,1 - 0,5 x SSC / 0,1 x SDS bei Temperaturen bis 68°C gewaschen.

Komplementäre Nukleinsäuren weisen erfindungsgemäß mindestens 40%, insbesondere mindestens 50%, mindestens 60%, mindestens 70%, mindestens 80%, mindestens 90% und vorzugsweise mindestens 95%, mindestens 98% oder mindestens 99% Identität der Nukleotide auf.

Nukleinsäuren, die für Tumor-assoziierte Antigene kodieren, können erfindungsgemäß alleine oder in Kombination mit anderen Nukleinsäuren, insbesondere heterologen Nukleinsäuren, vorliegen. In bevorzugten Ausführungsformen liegt eine Nukleinsäure funktionell in Verbindung mit Expressionskontrollsequenzen oder regulatorischen Sequenzen vor, die in Bezug zu der Nukleinsäure homolog oder heterolog sein können. Eine kodierende Sequenz und eine regulatorische Sequenz sind dann "funktionell" miteinander verbunden, falls sie derart kovalent miteinander verknüpft sind, dass die Expression oder Transkription der kodierenden Sequenz unter der Kontrolle oder unter dem Einfluss der regulatorischen Sequenz steht. Falls die kodierende Sequenz in ein funktionelles Protein translatiert werden soll, führt bei einer funktionellen Verbindung einer regulatorischen Sequenz mit der kodierenden Sequenz eine Induktion der regulatorischen Sequenz zu einer Transkription der kodierenden Sequenz, ohne dass es zu einer Leserasterverschiebung in der kodierenden Sequenz oder zu einem Unvermögen der kodierenden Sequenz kommt, in das gewünschte Protein oder Peptid translatiert zu werden.

Der Begriff "Expxessionskontrollsequenz" oder "regulatorische Sequenz" umfasst erfindungsgemäß Promotoren, Enhancer und andere Kontrollelemente, die die Expression eines Gens steuern. In bestimmten erfindungsgemäßen Ausführungsformen sind die Expressionskontrollsequenzen regulierbar. Die genaue Struktur von regulatorischen Sequenzen kann speziesabhängig oder zelltypusabhängig variieren, umfasst jedoch im allgemeinen 5'-nicht-transkribierte und 5'-nicht-translatierte Sequenzen, die an der Initiation der Transkription bzw. Translation beteiligt sind wie TATA-Box, Capping-Sequenz, CAAT-Sequenz und ähnliches. Insbesondere umfassen 5'-nicht-transkribierte Regulationssequenzen eine Promotorregion, die eine Promotorsequenz für eine transkriptionelle Kontrolle des funktionell verbundenen Gens einschließt. Regulatorische Sequenzen können auch EnhancerSequenzen oder stromaufwärts gelegene Aktivatorsequenzen umfassen.

Zum einen können also die hier dargestellten Tumorassoziierten Antigene mit beliebigen Expressionskontrollsequenzen und Promotoren kombiniert werden. Zum anderen aber können erfindungsgemäß die Promotoren der hier dargestellten Tumor-assoziierten Genprodukte mit beliebigen anderen Genen kombiniert werden. Dies erlaubt, die selektive Aktivität dieser Promotoren zu nutzen.

Des weiteren kann eine Nukleinsäure erfindungsgemäß in Verbindung mit einer anderen Nukleinsäure vorliegen, die für ein Polypeptid kodiert, das eine Sekretion des durch die Nukleinsäure kodierten Proteins oder Polypeptids aus einer Wirtszelle steuert. Auch kann eine Nukleinsäure erfindungsgemäß in Verbindung mit einer anderen Nukleinsäure vorliegen, die für ein Polypeptid kodiert, das eine Verankerung des kodierten Proteins oder Polypeptids auf der Zellmembran der Wirtszelle oder seine Kompartimentalisierung in bestimmte Organellen dieser Zelle herbeiführt.

In einer bevorzugten Ausführungsform ist ein rekombinantes DNA-Molekül erfindungsgemäß ein Vektor, gegebenenfalls mit einem Promotor, der die Expression einer Nukleinsäure, z.B. einer Nukleinsäure, die für ein erfindungsgemäßes Tumor-assoziiertes Antigen kodiert, steuert. Der Begriff "Vektor" wird dabei in seiner allgemeinsten Bedeutung verwendet und umfasst jegliche intermediären Vehikel für eine Nukleinsäure, die es z.B. ermöglichen, die Nukleinsäure in prokaryontische und/oder in eukaryontische Zellen einzubringen und gegebenenfalls in ein Genom zu integrieren. Solche Vektoren werden vorzugsweise in der Zelle repliziert und/oder exprimiert. Ein intermediäres Vehikel kann z.B. für den Gebrauch bei der Elektroporation, beim Mikroprojektilbeschuss, bei der liposomalen Verabreichung, beim Transfer mit Hilfe von Agrobakterien oder bei der Insertion über DNA- oder RNA-Viren angepasst sein. Vektoren umfassen Plasmide, Phagemide, Bacteriophage oder Virusgenome.

Die Nukleinsäuren, die für ein erfindungsgemäß identifiziertes Tumor-assozüertes Antigen kodieren, können für eine Transfektion von Wirtszellen eingesetzt werden. Mit Nukleinsäuren ist dabei sowohl rekombinante DNA wie auch RNA gemeint. Rekombinante RNA kann durch in vitro-Transkription von einer DNA-Matritze hergestellt werden. Sie kann des weiteren vor Applikation durch stabilisierende Sequenzen, Capping und Poly-Adenylierung modifiziert werden. Der Begriff "Wirtszelle" betrifft erfindungsgemäß jede Zelle, die mit einer exogenen Nukleinsäure transformierbar oder transfizierbar ist. Der Begriff "Wirtszellen" umfasst erfindungsgemäß prokaryontische (z.B. *E. coli*) oder eukaryontische (z.B. dendritische Zellen, B-Zellen, CHO-Zellen, COS-Zellen, K562-Zellen, Hefezellen und Insektenzellen). Besonders bevorzugt sind Säugerzellen wie Zellen aus Mensch, Maus, Hamster, Schwein, Ziege und Primaten. Die Zellen können aus einer Vielzahl von Gewebetypen abgeleitet sein und umfassen primäre Zellen und Zelllinien. Spezifische Beispiele umfassen Keratinozyten, periphere Blutleukozyten, Stammzellen des Knochenmarks und embryonale Stammzellen. In weiteren Ausführungsformen ist die Wirtszelle eine Antigen-präsentierende Zelle, insbesondere eine dendritische Zelle, ein Monozyt oder Makrophage. Eine Nukleinsäure kann in der Wirtszelle in einer einzigen oder in mehreren Kopien vorliegen und wird in einer Ausftihrungsform in der Wirtszelle exprimiert.

Der Begriff "Expression" wird erfindungsgemäß in seiner allgemeinsten Bedeutung verwendet und umfasst die Produktion von RNA oder von RNA und Protein. Er umfasst auch eine teilweise Expression von Nukleinsäuren. Des Weiteren kann die Expression transient oder stabil erfolgen. Bevorzugte Expressionssysteme in Säugerzellen umfassen pcDNA3.1 und pRc/CMV (Invitrogen, Carlsbad, CA), die einen selektierbaren Marker enthalten wie ein Gen, das eine Resistenz gegenüber G418 verleiht (und somit eine Selektion stabil transfizierter Zelllinien ermöglicht), und die Enhancer-Promotor-Sequenzen von Cytomegalovirus (CMV).

In den Fällen der Erfindung, in denen ein HLA-Molekül ein Tumor-assoziiertes Antigen oder einen Teil davon präsentiert, kann ein Expressionsvektor auch eine Nukleinsäuresequenz umfassen, die für das HLA-Molekül kodiert. Die Nukleinsäuresequenz, die für das HLA-Molekül kodiert, kann auf demselben Expressionsvektor wie die Nukleinsäure, die für das Tumor-assozüerte Antigen oder den Teil davon kodiert, vorliegen oder beide Nukleinsäuren können auf verschiedenen Expressionsvektoren vorliegen. Im letzteren Fall können die beiden Expressionsvektoren in eine Zelle cotransfiziert werden. Falls eine Wirtszelle weder das Tumor-assozüerte Antigen oder den Teil davon noch das HLA-Molekül exprimiert, werden beide dafür kodierenden Nukleinsäuren entweder auf demselben Expressionsvektor oder auf verschiedenen Expressionsvektoren in die Zelle transfiziert. Falls die Zelle bereits das HLA-Molekül exprimiert, kann nur die Nukleinsäuresequenz, die für das Tumor-assoziierte Antigen oder den Teil davon kodiert, in die Zelle transfiziert werden.

Erfindungsgemäß umfasst sind Kits zur Amplifikation einer Nukleinsäure, die für ein Tumor-assoziiertes Antigen kodiert. Solche Kits umfassen beispielsweise ein Paar von Amplifikationsprimern, die an die Nukleinsäure hybridisieren, die für das Tumor-assoziierte Antigen kodiert. Die Primer umfassen vorzugsweise eine Sequenz von 6-50, insbesondere 10-30, 15-30 oder 20-30 zusammenhängenden Nukleotiden aus der Nukleinsäure und sind nichtüberlappend, um die Bildung von Primer-Dimeren zu vermeiden. Einer der Primer wird an einen Strang der Nukleinsäure hybridisieren, die für das Tumor-assoziierte Antigen kodiert, und der andere Primer wird an den komplementären Strang in einer Anordnung hybridisieren, die eine Amplifikation der Nukleinsäure, die für das Tumor-assoziierte Antigen kodiert, erlaubt.

"Antisense"-Moleküle oder "Antisense"-Nukleinsäuren können zur Regulierung, insbesondere der Reduktion der Expression einer Nukleinsäure verwendet werden. Der Begriff "Antisense-Molekül" oder "Antisense-Nukleinsäure" betrifft erfindungsgemäß ein Oligonukleotid, das ein Oligoribonukleotid, Oligodesoxyribonukleotid, modifiziertes Oligoribonukleotid oder modifiziertes Oligodesoxyribonukleotid ist und das unter physiologischen Bedingungen an DNA, die ein bestimmtes Gen umfasst, oder mRNA dieses Gens hybridisiert, wodurch die Transkription dieses Gens und/oder die Translation dieser mRNA gehemmt wird. Ein "Antisense-Molekül" umfasst erfindungsgemäß auch ein Konstrukt, das eine Nukleinsäure oder einen Teil davon in reverser Orientierung in Bezug auf ihren natürlichen Promotor enthält. Ein Antisense-Transkript einer Nukleinsäure oder eines Teils davon kann ein Duplexmolekül mit der natürlich vorkommenden mRNA, die das Enzym spezifiziert, eingehen und so eine Akkumulation von oder die Translation der mRNA in das aktive Enzym verhindern. Eine weitere Möglichkeit ist die Verwendung von Ribozymen zur Inaktivierung einer Nukleinsäure. Bevorzugte erfindungsgemäße Antisense-Oligonukleotide weisen eine Sequenz von 6-50, insbesondere 10-30, 15-30 oder 20-30 zusammenhängenden Nukleotiden aus der Ziel-Nukleinsäure auf und sind vorzugsweise vollständig zu der Ziel-Nukleinsäure oder einem Teil davon komplementär.

In bevorzugten Ausführungsformen hybridisiert das Antisense-Oligonukleotid mit einer N-terminalen oder 5'-stromaufwärts gelegenen Stelle wie einer Translationsinitiations-, Transkriptionsinitiations- oder Promotorstelle. In weiteren Ausführungsformen hybridisiert das Antisense-Oligonukleotid mit einer 3'-nicht-translatierten Region oder mRNA-SpleißStelle.

In einer Ausführungsform besteht ein erfindungsgemäßes Oligonukleotid aus Ribonukleotiden, Desoxyribonukleotiden oder einer Kombination davon. Dabei sind das 5'-Ende eines Nukleotids und das 3'-Ende eines anderen Nukleotids durch eine Phosphodiesterbindung miteinander verknüpft. Diese Oligonukleotide können in herkömmlicher Weise synthetisiert oder rekombinant produziert werden.

In bevorzugten Ausführungsformen ist ein erfindungsgemäßes Oligonukleotid ein "modifiziertes" Oligonukleotid. Dabei kann das Oligonukleotid, um beispielsweise seine Stabilität oder therapeutische Wirksamkeit zu erhöhen, auf verschiedenste Art und Weise modifiziert sein ohne dass seine Fähigkeit, an sein Ziel zu binden, beeinträchtigt wird. Der Begriff "modifiziertes Oligonukleotid" bedeutet erfindungsgemäß ein Oligonukleotid, bei dem (i) mindestens zwei seiner Nukleotide durch eine synthetische Internukleosidbindung (d.h. eine Internukleosidbindung, die keine Phosphodiesterbindung ist) miteinander verknüpft sind und/oder (ii) eine chemische Gruppe kovalent mit dem Oligonukleotid verbunden ist, die normalerweise nicht bei Nukleinsäuren auftritt. Bevorzugte synthetische Intemukleosidbindungen sind Phosphorothioate, Alkylphosphonate, Phosphorodithioate, Phosphatester, Alkylphosphonothioate, Phosphoramidate, Carbamate, Carbonate, Phosphattriester, Acetamidate, Carboxymethylester und Peptide.

Der Begriff "modifiziertes Oligonukleotid" umfasst auch Oligonukleotide mit einer kovalent modifizierten Base und/oder Zucker. "Modifizierte Oligonukleotide" umfassen beispielsweise Oligonukleotide mit Zuckerresten, die kovalent an organische Gruppen mit einem geringen Molekulargewicht gebunden sind, die keine Hydroxylgruppe an der 3'-Position und keine Phosphatgruppe an der 5'-Position sind. Modifizierte Oligonukleotide können beispielsweise einen 2'-O-alkylierten Riboserest oder einen anderen Zucker anstelle von Ribose wie Arabinose umfassen.

Die erfindungsgemäß beschriebenen Proteine und Polypeptide sind vorzugsweise isoliert. Die Begriffe "isoliertes Protein" oder "isoliertes Polypeptid" bedeuten, dass das Protein oder Polypeptid von seiner natürlichen Umgebung getrennt ist. Ein isoliertes Protein oder Polypeptid kann in einem im wesentlichen aufgereinigten Zustand vorliegen. Der Begriff "im wesentlichen aufgereinigt" bedeutet, dass das Protein oder Polypeptid im wesentlichen frei von anderen Substanzen vorliegt, mit denen es in der Natur oder *in vivo* vorliegt.

Solche Proteine und Polypeptide dienen beispielsweise der Herstellung von Antikörpern und sind in einem immunologischen oder diagnostischen Assay oder als Therapeutika einsetzbar. Erfindungsgemäß beschriebene Proteine und Polypeptide können aus biologischen Proben wie Gewebe- oder Zellhomogenaten isoliert werden und können auch rekombinant in einer Vielzahl pro- oder eukaryontischer Expressionssysteme exprimiert werden.

"Derivate" eines Proteins oder Polypeptids oder einer Aminosäuresequenz im Sinne dieser Erfindung umfassen Aminosäure-Insertionsvarianten, Aminosäure-Deletionsvarianten und/oder Aminosäure-Substitutionsvarianten.

Aminosäure-Insertionsvarianten umfassen amino- und/oder carboxyterminale Fusionen, sowie Insertionen von einzelnen oder mehreren Aminosäuren in einer bestimmten Aminosäuresequenz. Bei Aminosäure-Sequenzvarianten mit einer Insertion werden ein oder mehrere Aminosäurereste in eine vorbestimmte Stelle in einer Aminosäuresequenz eingebracht, obwohl eine zufällige Insertion mit geeignetem Screening des resultierenden Produkts auch möglich ist. Aminosäure-Deletionsvarianten sind durch das Entfernen von einer oder mehreren Aminosäuren aus der Sequenz charakterisiert. Aminosäure-Substitutionsvarianten zeichnen sich dadurch aus, dass wenigstens ein Rest in der Sequenz entfernt und ein anderer Rest an dessen Stelle eingefügt wird. Vorzugsweise befinden sich die Modifikationen an Positionen in der Aminosäuresequenz, die zwischen homologen Proteinen oder Polypeptiden nicht konserviert sind. Vorzugsweise werden Aminosäuren durch andere mit ähnlichen Eigenschaften, wie Hydrophobizität, Hydrophilizität, Elektronegativität, Volumen der Seitenkette und ähnliches, ersetzt (konservative Substitution). Konservative Substitutionen betreffen beispielsweise den Austausch einer Aminosäure durch eine andere, wobei beide Aminosäuren in derselben nachstehenden Gruppe aufgeführt sind:
1. kleine aliphatische, nicht-polare oder leicht-polare Reste: Ala, Ser, Thr (Pro, Gly)
2. negativ geladene Reste und ihre Amide: Asn, Asp, Glu, Gln
3. positiv geladene Reste: His, Arg, Lys
4. große aliphatische, nicht-polare Reste: Met, Leu, Ile, Val (Cys)
5. große aromatische Reste: Phe, Tyr, Trp.

Drei Reste sind aufgrund ihrer besonderen Rolle für die Proteinarchitektur in Klammern gesetzt. Gly ist der einzige Rest ohne eine Seitenkette und verleiht der Kette somit Flexibilität. Pro besitzt eine ungewöhnliche Geometrie, die die Kette stark einschränkt. Cys kann eine Disulfidbrücke bilden.

Die oben beschriebenen Aminosäure-Varianten können leicht mit Hilfe von bekannten Peptidsynthesetechniken wie z.B. durch "Solid Phase Synthesis" (Merrifield, 1964) und ähnliche Verfahren oder durch rekombinante DNA-Manipulation hergestellt werden. Techniken, um Substitutionsmutationen an vorbestimmten Stellen in DNA einzubringen, die eine bekannte oder teilweise-bekannte Sequenz besitzt, sind gut bekannt und umfassen z.B. M13-Mutagenese. Die Manipulation von DNA-Sequenzen zur Herstellung von Proteinen mit Substitutionen, Insertionen oder Deletionen ist z.B. in Sambrook et. al. (1989) ausführlich beschrieben.

"Derivate" von Proteinen oder Polypeptiden umfassen erfindungsgemäß auch einzelne oder multiple Substitutionen, Deletionen und/oder Additionen jeglicher Moleküle, die mit dem Enzym assoziiert sind, wie Kohlenhydrate, Lipide und/oder Proteine oder Polypeptide. Ferner erstreckt sich der Begriff "Derivat" auch auf alle funktionellen chemischen Äquivalente der Proteine oder Polypeptide.

Ein Teil oder Fragment eines Tumor-assoziierten Antigens weist erfindungsgemäß eine funktionelle Eigenschaft des Polypeptids auf, aus dem es abgeleitet ist. Solche funktionellen Eigenschaften umfassen die Interaktion mit Antikörpern, die Interaktion mit anderen Polypeptiden oder Proteinen, die selektive Bindung von Nukleinsäuren und eine enzymatische Aktivität. Eine bedeutende Eigenschaft ist die Fähigkeit, einen Komplex mit HLA einzugehen und gegebenenfalls eine Immunreaktion zu erzeugen. Diese Immunreaktion kann auf Stimulation von cytotoxischen oder Helfer-T-Zellen beruhen. Vorzugsweise umfasst ein erfindungsgemäßer Teil oder Fragment eines Tumor-assoziierten Antigens eine Sequenz von mindestens 6, insbesondere mindestens 8, mindestens 10, mindestens 12, mindestens 15, mindestens 20, mindestens 30 oder mindestens 50 aufeinanderfolgenden Aminosäuren aus dem Tumor-assoziierten Antigen. Ein Teil oder Fragment eines Tumor-assoziierten Antigens ist vorzugsweise ein Teil des Tumor-assoziierten Antigens, der dem nicht-Transmembrananteil, insbesondere dem extrazellulären Anteil des Antigens entspricht oder davon umfasst wird.

Ein Teil oder ein Fragment einer Nukleinsäure, die für ein Tumor-assoziiertes Antigen kodiert, betrifft erfindungsgemäß den Teil der Nukleinsäure, der zumindest für das Tumor-assoziierte Antigen und/oder für einen Teil oder ein Fragment des Tumor-assoziierten Antigens wie vorstehend definiert kodiert. Vorzugsweise ist ein Teil oder Fragment einer Nukleinsäure, die für ein Tumor-assoziiertes Antigen kodiert, derjenige Teil, der dem offenen Leserahmen, insbesondere wie im Sequenzprotokoll angegeben entspricht.

Die Isolierung und Identifizierung von Genen, die für Tumor-assoziierte Antigene kodieren, ermöglicht auch die Diagnose einer Erkrankung, die sich durch die Expression von einem oder mehreren Tumor-assoziierten Antigenen auszeichnet. Diese Verfahren umfassen die Bestimmung einer oder mehrerer Nukleinsäuren, die für ein Tumor-assoziiertes Antigen kodieren, und/oder die Bestimmung der kodierten Tumor-assoziierten Antigene und/oder von davon abgeleiteten Peptiden. Eine Bestimmung der Nukleinsäure kann in herkömmlicher Weise erfolgen, einschließlich durch Polymerase-Kettenreaktion oder Hybridisierung mit einer markierten Sonde. Eine Bestimmung von Tumor-assoziierten Antigenen oder davon abgeleiteten Peptiden kann durch ein Screening von Patienten-Antiseren in Bezug auf eine Erkennung des Antigens und/oder der Peptide erfolgen. Sie kann auch durch ein Screening von T-Zellen des Patienten auf Spezifität für das entsprechende Tumor-assoziierte Antigen erfolgen.

Die vorliegende Erfindung ermöglicht auch die Isolierung von Proteinen, die an hier beschriebene Tumor-assoziierte Antigene binden, einschließlich Antikörper und zelluläre Bindepartner der Tumor-assoziierten Antigene.

Erfindungsgemäß werden auch in bestimmten Ausführungsformen "dominant negative" Polypeptide bereitgestellt, die von Tumor-assoziierten Antigenen abgeleitet sind. Ein dominant negatives Polypeptid ist eine inaktive Variante eines Proteins, die durch Interaktion mit der zellulären Maschinerie ein aktives Protein von seiner Interaktion mit der zellulären Maschinerie verdrängt oder mit dem aktiven Protein kompetitiert, wodurch die Wirkung des aktiven Proteins verringert wird. Zum Beispiel kann ein dominant negativer Rezeptor, der einen Liganden bindet, jedoch kein Signal in Reaktion auf die Bindung des Liganden erzeugt, die biologische Wirkung des Liganden verringern. In ähnlicher Weise kann eine dominant negative katalytisch-inaktive Kinase, die normalerweise mit Zielproteinen interagiert, jedoch die Zielproteine nicht phosphoryliert, die Phosphorylierung der Zielproteine in Reaktion auf ein zelluläres Signal verringern. In ähnlicher Weise kann ein dominant negativer Transkriptionsfaktor, der an eine Promotorstelle in der Kontrollregion eines Gens bindet, jedoch die Transkription des Gens nicht erhöht, die Wirkung eines normalen Transkriptionsfaktors durch die Besetzung von Promotorbindestellen ohne eine Erhöhung der Transkription verringern.

Das Ergebnis der Expression eines dominant negativen Polypeptids in einer Zelle ist eine Verringerung der Funktion aktiver Proteine. Der Fachmann kann dominant negative Varianten eines Proteins beispielsweise durch herkömmliche Mutageneseverfahren und Bewerten der dominant negativen Wirkung des Varianten-Polypeptids herstellen.

Erfindungsgemäß umfasst sind auch Stoffe wie Polypeptide, die an Tumor-assoziierte Antigene binden. Solche Bindestoffe können z.B. in Screening-Assays für einen Nachweis von Tumor-assoziierten Antigenen und Komplexen von Tumor-assoziierten Antigenen mit ihren Bindepartnem sowie bei einer Aufreinigung der Tumor-assoziierten Antigene und von Komplexen davon mit ihren Bindepartnern Verwendung finden. Solche Stoffe können auch für eine Hemmung der Aktivität Tumor-assoziierter Antigene beispielsweise durch Bindung an solche Antigene Verwendung finden.

Erfindungsgemäß umfasst sind daher Bindestoffe wie z.B. Antikörper oder Antikörperfragmente, die die Fähigkeit aufweisen, selektiv an Tumor-assoziierte Antigene zu binden. Antikörper umfassen polyklonale und monoklonale Antikörper, die in herkömmlicher Weise hergestellt werden.

Es ist bekannt, dass nur ein kleiner Teil eines Antikörpermoleküls, das Paratop, an der Bindung des Antikörpers an sein Epitop beteiligt ist (vgl. Clark, W.R. (1986), The Experimental Foundations of Modern Immunology, Wiley & Sons, Inc., New York; Roitt, I. (1991), Essential Immunology, 7. Auflage, Blackwell Scientific Publications, Oxford). Die pFc'- und Fc-Regionen sind z.B. Effektoren der Komplementkaskade, sind jedoch nicht an der Antigenbindung beteiligt. Ein Antikörper, von dem die pFc'-Region enzymatisch abgespalten wurde oder der ohne die pFc'-Region hergestellt wurde, bezeichnet als F(ab')₂-Fragment, trägt beide Antigenbindestellen eines vollständigen Antikörpers. In ähnlicher Weise trägt ein Antikörper, von dem die Fc-Region enzymatisch abgespalten wurde oder der ohne die Fc-Region hergestellt wurde, bezeichnet als Fab-Fragment, eine Antigenbindestelle eines intakten Antikörpermoleküls. Des weiteren bestehen Fab-Fragmente aus einer kovalent gebundenen leichten Kette eines Antikörpers und einem Teil der schweren Kette des Antikörpers, bezeichnet als Fd. Die Fd-Fragmente sind die Haupt-Determinanten der Antikörper-Spezifität (ein einzelnes Fd-Fragment kann mit bis zu zehn verschiedenen leichten Ketten assoziiert werden, ohne die Spezifität des Antikörpers zu verändern) und Fd-Fragmente behalten bei einer Isolierung die Fähigkeit, an ein Epitop zu binden.

Innerhalb des Antigen-bindenden Teils eines Antikörpers befinden sich komplementaritätsbestimmende Regionen (CDRs), die direkt mit dem Epitop des Antigens wechselwirken, und Gerüstregionen (FRs), die die Tertiärstruktur des Paratops aufrechterhalten. Sowohl in dem Fd-Fragment der schweren Kette als auch in der leichten Kette von IgG-Immunglobulinen befinden sich vier Gerüstregionen (FR1 bis FR4), die jeweils durch drei komplementaritätsbestimmende Regionen (CDR1 bis CDR3) getrennt sind. Die CDRs und insbesondere die CDR3-Regionen und noch mehr die CDR3-Region der schweren Kette sind größtenteils für die Antikörper-Spezifität verantwortlich.

Man weiß, dass die Nicht-CDR-Regionen eines Säuger-Antikörpers durch ähnliche Regionen von Antikörpern mit der gleichen oder einer anderen Spezifität ersetzt werden können, wobei die Spezifität für das Epitop des ursprünglichen Antikörpers erhalten bleibt. Dies ermöglichte die Entwicklung sogenannter "humanisierter" Antikörper, bei denen nicht-menschliche CDRs kovalent mit menschlichen FR- und/oder Fc/pFc'-Regionen für die Herstellung eines funktionellen Antikörpers verbunden sind.

Zum Beispiel beschreibt die WO 92/04381 die Herstellung und Verwendung von humanisierten RSV-Antikörpern aus Maus, bei denen mindestens ein Teil der FR-Regionen aus Maus durch FR-Regionen eines menschlichen Ursprungs ersetzt wurden. Solche Antikörper, einschließlich Fragmente intakter Antikörper mit einer Antigen-Bindefähigkeit werden oft als "chimäre" Antikörper bezeichnet.

Erfindungsgemäß werden auch F(ab')₂-, Fab-, Fv- und Fd-Fragmente von Antikörpern, chimäre Antikörper, bei denen die Fc- und/oder FR- und/oder CDR1- und/oder CDR2-und/oder leichte Kette-CDR3-Regionen durch homologe menschliche oder nicht-menschliche Sequenzen ersetzt wurden, chimäre F(ab')₂-Fragment-Antikörper, bei denen die FR- und/oder CDR1- und/oder CDR2- und/oder leichte Kette-CDR3-Regionen durch homologe menschliche oder nicht-menschliche Sequenzen ersetzt wurden, chimäre Fab-Fragment-Antikörper, bei denen die FR- und/oder CDR1- und/oder CDR2- und/oder leichte Kette-CDR3-Regionen durch homologe menschliche oder nicht-menschliche Sequenzen ersetzt wurden, und chimäre Fd-Fragment-Antikörper, bei denen die FR- und/oder CDR1- und/oder CDR2-Regionen durch homologe menschliche oder nicht-menschliche Sequenzen ersetzt wurden, bereitgestellt. Erfindungsgemäß umfasst sind auch sogenannte einzelkettige Antikörper.

Vorzugsweise ist ein erfindungsgemäß verwendeter Antikörper gegen eine der in SEQ ID NOs: 2, 4, 6, 8, 10, 12, 14, 16, 18, 20, 22, 24, 26, 28, 30, 61 bis 68, 70, 72, 74, 76, 81, 82, 86, 88, 96 bis 101, 103, 105, 107, 109, 111, 113 dargestellten Sequenzen oder einen Teil oder ein Derivat davon gerichtet und/oder kann durch Immunisierung mit diesen Peptiden erhalten werden.

Erfindungsgemäß umfasst sind auch Polypeptide, die spezifisch an Tumor-assoziierte Antigene binden. Beispielsweise können solche Polypeptid-Bindestoffe durch degenerierte Peptid-Bibliotheken bereitgestellt werden, die einfach in Lösung in einer immobilisierten Form oder als Phagen-Display-Bibliotheken hergestellt werden können. Kombinatorische Bibliotheken aus Peptiden mit einer oder mehreren Aminosäuren können ebenfalls hergestellt werden. Ferner können Bibliotheken aus Peptoiden und nicht-peptidischen synthetischen Resten hergestellt werden.

Phagen-Display kann besonders wirksam bei der Identifizierung erfindungsgemäßer Bindepeptide sein. Dabei wird beispielsweise eine Phagen-Bibliothek (durch Verwendung beispielsweise des m13-, fd- oder lambda-Phagen) hergestellt, die Inserts einer Länge von 4 bis etwa 80 Aminosäureresten präsentiert. Es werden sodann Phagen ausgewählt, die Inserts tragen, die an das Tumor-assoziierte Antigen binden. Dieser Prozess kann über mehrere Zyklen einer Rückselektion von Phagen wiederholt werden, die an das Tumor-assoziierte Antigen binden. Wiederholte Runden führen zu einer Anreicherung von Phagen, die bestimmte Sequenzen tragen. Es kann eine Analyse von DNA-Sequenzen erfolgen, um die Sequenzen der exprimierten Polypeptide zu identifizieren. Der kleinste lineare Anteil der Sequenz, der an das Tumor-assoziierte Antigen bindet, kann bestimmt werden. Das "twohybrid-System" aus Hefe kann auch für die Identifizierung von Polypeptiden eingesetzt werden, die an ein Tumor-assoziiertes Antigen binden. Erfindungsgemäß beschriebene Tumor-assoziierte Antigene oder Fragmente davon können für ein Screening von Peptid-Bibliotheken, einschließlich Phagen-Display-Bibliotheken, eingesetzt werden, um Peptid-Bindepartner der Tumor-assoziierten Antigene zu identifizieren und selektieren. Solche Moleküle können beispielsweise für Screening-Assays, Aufreinigungsprotokolle, für eine Interferenz mit der Funktion des Tumor-assoziierten Antigens und für andere Zwecke, die dem Fachmann bekannt sind, verwendet werden.

Die vorstehend beschriebenen Antikörper und andere Bindemoleküle können beispielsweise für die Identifizierung von Gewebe verwendet werden, das ein Tumor-assoziiertes Antigen exprimiert. Antikörper können auch an spezifische diagnostische Stoffe für eine Darstellung von Zellen und Geweben gekoppelt werden, die Tumor-assoziierte Antigene exprimieren. Sie können ferner an therapeutisch nützliche Stoffe gekoppelt werden. Diagnostische Stoffe umfassen in nicht begrenzender Weise Bariumsulfat, Iocetaminsäure, Iopansäure, Calcium-Ipodat, Natrium-Diatrizoat, Meglumin-Diatrizoat, Metrizamid, Natrium-Tyropanoat und Radiodiagnostika, einschließlich Positronen-Emitter wie Fluor-18 und Kohlenstoff-11, gamma-Emitter wie Iod-123, Technetium-99m, Iod-131 und Indium-111, Nuklide für magnetische Kernresonanz wie Fluor und Gadolinium. Der Begriff "therapeutisch nützlicher Stoff" meint erfindungsgemäß jedes therapeutische Molekül, das wunschgemäß selektiv zu einer Zelle geführt wird, die ein oder mehrere Tumor-assoziierte Antigene exprimiert, einschließlich Antikrebsmittel, mit radioaktivem Iod versehene Verbindungen, Toxine, cytostatische oder cytolytische Arzneistoffe, usw. Antikrebsmittel umfassen beispielsweise Aminoglutethimid, Azathioprin, Bleomycinsulfat, Busulfan, Carmustin, Chlorambucil, Cisplatin, Cyclophosphamid, Cyclosporin, Cytarabin, Dacarbazin, Dactinomycin, Daunorubin, Doxorubicin, Taxol, Etoposid, Fluoruracil, Interferon-α, Lomustin, Mercaptopurin, Methotrexat, Mitotan, Procarbazin-HCl, Thioguanin, Vinblastinsulfat und Vincristinsulfat. Weitere Antikrebsmittel sind beispielsweise in Goodman und Gilman, "The Pharmacological Basis of Therapeutics", 8. Auflage, 1990, McGraw-Hill, Inc., insbesondere Kapitel 52 (Antineoplastic Agents (Paul Calabresi und Bruce A. Chabner)) beschrieben. Toxine können Proteine wie Pokeweed-antivirales Protein, Choleratoxin, Pertussistoxin, Ricin, Gelonin, Abrin, Diphtherie-Exotoxin oder *Pseudomonas*-Exotoxin sein. Toxinreste können auch Hochenergie-emittierende Radionuklide wie Kobalt-60 sein.

Der Begriff "Patient" bedeutet erfindungsgemäß Mensch, nicht menschlicher Primat oder ein anderes Tier, insbesondere Säugetier wie Kuh, Pferd, Schwein, Schaf, Ziege, Hund, Katze oder Nagetier wie Maus und Ratte. In einer besonders bevorzugten Ausführungsform ist der Patient ein Mensch.

Der Begriff "Erkrankung" betrifft erfindungsgemäß jeden pathologischen Zustand, bei dem Tumor-assoziierte Antigene exprimiert oder abnormal exprimiert werden. "Abnormale Expression" bedeutet erfindungsgemäß, dass die Expression gegenüber dem Zustand bei einem gesunden Individuum verändert, vorzugsweise erhöht ist. Eine Erhöhung der Expression betrifft eine Erhöhung um mindestens 10%, insbesondere mindestens 20%, mindestens 50% oder mindestens 100%. In einer Ausführungsform wird das Tumor-assoziierte Antigen nur in Gewebe eines erkrankten Individuums exprimiert, während die Expression bei einem gesunden Individuum reprimiert ist. Ein Beispiel einer solchen Erkrankung ist Krebs, insbesondere Seminome, Melanome, Teratome, Gliome, Colon-, Rektal-, Nieren-, Brust-, Prostata-, Gebärmutter-, Ovarial-, Endometrial-, Speiseröhren-, Blut-, Leber-, Pankreas-, Haut-, Gehirn- und Lungenkrebs, Lymphome und Neuroblastome. Beispiele hierfür sind Lungen-, Brust-, Prostata-, Colontumor, Nierenzell-, Zervix-, Colon- und Mammakarzinom oder Metastasen der vorstehenden Krebsarten oder Tumore.

Eine biologische Probe kann erfindungsgemäß eine Gewebe- und/oder zelluläre Probe sein und kann für eine Verwendung in den verschiedenen, hier beschriebenen Verfahren in herkömmlicher Weise gewonnen werden, wie durch Gewebebiopsie, einschließlich Stanzbiopsie, und Entnahme von Blut, Bronchialaspirat, Urin, Fäces oder anderen Körperflüssigkeiten.

Der Begriff "immunreaktive Zelle" bedeutet erfindungsgemäß eine Zelle, die in eine Immunzelle (wie B-Zelle, Helfer-T-Zelle oder cytolytische T-Zelle) bei geeigneter Stimulierung reifen kann. Immunreaktive Zellen umfassen CD34⁺ hämatopoietische Stammzellen, unreife und' reife T-Zellen sowie unreife und reife B-Zellen. Falls die Herstellung cytolytischer oder Helfer-T-Zellen, die ein Tumor-assoziiertes Antigen erkennen, gewünscht ist, wird die immunreaktive Zelle mit einer Zelle, die ein Tumor-assoziiertes Antigen exprimiert, unter Bedingungen in Kontakt gebracht, die eine Produktion, Differenzierung und/oder Selektion von cytolytischen sowie Helfer-T-Zellen begünstigen. Die Differenzierung von T-Zell-Vorläufern in eine cytolytische T-Zelle bei einer Exposition gegenüber einem Antigen ist ähnlich zur klonalen Selektion des Immunsystems.

Manche therapeutische Verfahren beruhen auf einer Reaktion des Immunsystems eines Patienten, die zu einer Lyse Antigen-präsentierender Zellen führt, wie Krebszellen, die ein oder mehrere Tumor-assoziierte Antigene präsentieren. Dabei werden beispielsweise autologe cytotoxische T-Lymphozyten, die für einen Komplex aus einem Tumor-assoziierten Antigen und einem MHC-Molekül spezifisch sind, an einen Patienten mit einer Zellabnormalie verabreicht. Die Produktion solcher cytotoxischer T-Lymphozyten *in vitro* ist bekannt. Ein Beispiel für ein Verfahren zur Differenzierung von T-Zellen findet sich in der WO-A-96/33265. Im Allgemeinen wird eine Probe mit Zellen wie Blutzellen aus dem Patienten entnommen und die Zellen werden mit einer Zelle in Kontakt gebracht, die den Komplex präsentiert und eine Vermehrung von cytotoxischen T-Lymphozyten auslösen kann (z.B. dendritische Zellen). Die Zielzelle kann eine transfizierte Zelle wie eine COS-Zelle sein. Diese transfizierten Zellen präsentieren den gewünschten Komplex auf ihrer Oberfläche und stimulieren bei einer Kontaktierung mit cytotoxischen T-Lymphozyten deren Vermehrung. Die klonal expandierten autologen cytotoxischen T-Lymphozyten werden sodann an den Patienten verabreicht.

Bei einem anderen Verfahren zur Selektion Antigen-spezifischer cytotoxischer T-Lymphozyten werden fluorogene Tetramere von MHC-Klasse 1-Molekül/Peptid-Komplexen für einen Nachweis spezifischer Klone von cytotoxischen T-Lymphozyten verwendet (Altman et al., Science 274:94-96, 1996; Dunbar et al., Curr. Biol. 8:413-416, 1998). Lösliche MHC-Klasse I-Moleküle werden *in vitro* in Gegenwart von β₂-Mikroglobulin und eines Peptid-Antigens, das an das Klasse I-Molekül bindet, gefaltet. Nach Aufreinigung der MHC/Peptid-Komplexe werden diese mit Biotin markiert. Tetramere werden durch Mischen der biotinylierten Peptid-MHC-Komplexe mit markiertem Avidin (z.B. Phycoerythrin) bei einem molaren Verhältnis von 4:1 gebildet. Tetramere werden sodann mit cytotoxischen T-Lymphozyten wie peripherem Blut oder Lymphknoten in Kontakt gebracht. Die Tetramere binden an cytotoxische T-Lymphozyten, die den Peptid-Antigen/MHC-Klasse 1-Komplex erkennen. Zellen, die an die Tetramere gebunden werden, können durch Fluoreszenzgesteuerte Zellsortierung für eine Isolierung reaktiver cytotoxischer T-Lymphozyten sortiert werden. Die isolierten cytotoxischen T-Lymphozyten können sodann *in vitro* vermehrt werden.

Bei einem therapeutischen Verfahren, das als adoptiver Transfer bezeichnet wird (Greenberg, J. Immunol. 136(5):1917, 1986; Riddel et al., Science 257:238, 1992; Lynch et al., Eur. J. Immunol. 21:1403-1410, 1991; Kast et al., Cell 59:603-614, 1989), werden Zellen, die den gewünschten Komplex präsentieren (z.B. dendritische Zellen), mit cytotoxischen T-Lymphozyten des zu behandelnden Patienten kombiniert, was zu einer Vermehrung spezifischer cytotoxischer T-Lymphozyten führt. Die vermehrten cytotoxischen T-Lymphozyten werden sodann an einen Patienten mit einer zellulären Abnormalie verabreicht, die sich durch bestimmte abnormale Zellen auszeichnet, die den spezifischen Komplex präsentieren. Die cytotoxischen T-Lymphozyten lysieren sodann die abnormalen Zellen, wodurch eine gewünschte therapeutische Wirkung erreicht wird.

Oft lassen sich aus dem T-Zell-Repertoire eines Patienten lediglich niedrig-affine T-Zellen gegen einen solchen spezifischen Komplex vermehren, da die hochaffinen durch Toleranzentwicklung ausgelöscht worden sind. Eine Alternative kann hier ein Transfer des T-Zell-Rezeptors selbst sein. Hierfür werden ebenfalls Zellen, die den gewünschten Komplex präsentieren (z.B. dendritische Zellen), mit cytotoxischen T-Lymphozyten von Gesunden kombiniert. Dies führt zu einer Vermehrung hochaffiner spezifischer cytotoxischer T-Lymphozyten, wenn der Spender mit dem spezifischen Komplex bisher keinen Kontakt hatte. Der hochaffine T-Zell-Rezeptor aus diesen vermehrten spezifischen T-Lymphozyten wird kloniert und kann durch Gentransfer z.B. mit retroviralen Vektoren beliebig in T-Zellen von anderen Patienten transduziert werden. Adoptiver Transfer erfolgt dann mit diesen genetisch veränderten T-Lymphozyten (Stanislawski et al., Nat. Immunol. 2:962-70, 2001 ; Kessels et al., Nat. Immunol. 2:957-61, 2001).

Die vorstehenden therapeutischen Aspekte gehen davon aus, dass zumindest manche der abnormalen Zellen des Patienten einen Komplex aus einem Tumor-assoziierten Antigen und einem HLA-Molekül präsentieren. Eine Identifizierung solcher Zellen kann in an sich bekannter Weise erfolgen. Sobald Zellen, die den Komplex präsentieren, identifiziert wurden, können sie mit einer Probe aus dem Patienten, die cytotoxische T-Lymphozyten enthält, kombiniert werden. Falls die Zellen, die den Komplex präsentieren, durch die cytotoxischen T-Lymphozyten lysiert werden, kann angenommen werden, dass ein Tumor-assoziiertes Antigen präsentiert wird.

Der adoptive Transfer ist nicht die einzige Therapieform, die erfindungsgemäß anwendbar ist. Cytotoxische T-Lymphozyten können auch *in vivo* in an sich bekannter Weise erzeugt werden. Bei einem Verfahren werden nicht-proliferative Zellen verwendet, die den Komplex exprimieren. Die Zellen, die dabei verwendet werden, werden diejenigen sein, die normalerweise den Komplex exprimieren, wie bestrahlte Tumorzellen oder Zellen, die mit einem oder beiden Genen transfiziert wurden, die für eine Präsentation des Komplexes notwendig sind (d.h. das Antigen-Peptid und das präsentierende HLA-Molekül). Verschiedene Zelltypen können eingesetzt werden. Des weiteren können Vektoren verwendet werden, die eines oder beide der interessierenden Gene tragen. Virale oder bakterielle Vektoren sind besonders bevorzugt. Zum Beispiel können Nukleinsäuren, die für ein Tumor-assoziiertes Antigen oder einen Teil davon kodieren, funktionell mit Promotor- und Enhancersequenzen verknüpft werden, die eine Expression des Tumor-assoziierten Antigens oder eines Fragments davon in bestimmten Geweben oder Zelltypen steuern. Die Nukleinsäure kann in einen Expressionsvektor eingebaut werden. Expressionsvektoren können, nicht-modifizierte extrachromosomale Nukleinsäuren, Plasmide oder virale Genome sein, in die eine Insertion exogener Nukleinsäuren möglich ist. Nukleinsäuren, die für ein Tumor-assoziiertes Antigen kodieren, können auch in ein retrovirales Genom inseriert werden, wodurch die Integration der Nukleinsäure in das Genom des Zielgewebes oder der Zielzelle ermöglicht wird. Bei diesen Systemen trägt ein Mikroorganismus wie Vacciniavirus, Poxvirus, Herpes simplex-Virus, Retrovirus oder Adenovirus das interessierende Gen und "infiziert" de facto Wirtszellen. Eine weitere bevorzugte Form ist die Einbringung des Tumor-assoziierten Antigens in Form von rekombinanter RNA. Diese kann z.B. durch liposomalen Transfer oder durch Elektroporation in Zellen eingebracht werden. Die resultierenden Zellen präsentieren den interessierenden Komplex und werden von autologen cytotoxischen T-Lymphozyten erkannt, die sich sodann vermehren.

Eine ähnliche Wirkung kann durch Kombination des Tumor-assoziierten Antigens oder eines Fragments davon mit einem Adjuvans erreicht werden, um einen Einbau in Antigen-präsentierende Zellen *in vivo* zu ermöglichen. Das Tumor-assoziierte Antigen oder ein Fragment davon können als Protein, als DNA (z.B. innerhalb eines Vektors) oder als RNA repräsentiert sein. Das Tumor-assoziierte Antigen wird prozessiert, um einen Peptidpartner für das HLA-Molekül zu ergeben, während ein Fragment davon präsentiert werden kann, ohne dass eine weitere Prozessierung erforderlich ist. Letzteres ist insbesondere der Fall, wenn diese an HLA-Moleküle binden können. Verabreichungsformen, bei denen das Gesamt-Antigen *in vivo* von einer dendritischen Zelle prozessiert wird, sind bevorzugt, da hier auch Helfer-T-Zell-Antworten entstehen können. Eine effektive Immunantwort benötigt diese (Ossendorp et al., Immunol. Lett. 74:75-9, 2000; Ossendorp et al., J. Exp. Med. 187:693-702, 1998). Im allgemeinen kann eine wirksame Menge des Tumor-assoziierten Antigens an einen Patienten z.B. durch eine intradermale Injektion verabreicht werden. Die Injektion kann aber auch intranodal in einen Lymphknoten erfolgen (Maloy et al., Proc. Natl. Acad. Sci. USA 98:3299-303, 2001). Sie kann auch in Kombination mit Reagenzien erfolgen, die eine Aufnahme in dendritische Zellen erleichtern. Bevorzugte Tumor-assoziierte Antigene umfassen diejenigen, die mit allogenen Krebs-Antiseren oder mit T-Zellen vieler Krebs-Patienten reagieren. Von besonderem Interesse sind aber auch solche, gegen die keine spontanen Immunantworten vorbestehen. Gegen diese können nachweislich Immunantworten induziert werden, die Tumoren lysieren können (Keogh et al., J. Immunol. 167:787-96, 2001; Appella et al., Biomed. Pept. Proteins Nucleic Acids 1:177-84, 1995; Wentworth et al., Mol. Immunol. 32:603-12, 1995).

Die erfindungsgemäß beschriebenen pharmazeutischen Zusammensetzungen können auch als Vakzinen für die Immunisierung eingesetzt werden. Die Begriffe "Immunisierung" oder "Vakzinierung" bedeuten erfindungsgemäß eine Erhöhung oder Aktivierung einer Immunreaktion gegenüber einem Antigen. Tiermodelle können für ein Testen einer immunisierenden Wirkung gegenüber Krebs durch Verwendung eines Tumor-assoziierten Antigens oder einer dafür kodierenden Nukleinsäure eingesetzt werden. Zum Beispiel können menschliche Krebszellen in eine Maus für die Schaffung eines Tumors eingebracht werden und eine oder mehrere Nukleinsäuren, die für Tumor-assoziierte Antigene kodieren, können verabreicht werden. Die Wirkung auf die Krebszellen (beispielsweise Verringerung der Tumorgröße) kann als Maß für die Wirksamkeit einer Immunisierung durch die Nukleinsäure gemessen werden.

Als Teil der Zusammensetzung für eine Immunisierung werden eines oder mehrere Tumor-assoziierte Antigene oder stimulierende Fragmente davon mit einem oder mehreren Adjuvanzien für eine Induktion einer Immunreaktion oder eine Erhöhung einer Immunreaktion verabreicht. Ein Adjuvans ist eine Substanz, die in das Antigen eingebaut oder gemeinsam mit diesem verabreicht wird und die Immunreaktion verstärkt. Adjuvanzien können die Immunreaktion durch Bereitstellen eines Antigen-Reservoirs (extrazellulär oder in Makrophagen), Aktivierung von Makrophagen und Stimulierung bestimmter Lymphozyten verstärken. Adjuvanzien sind bekannt und umfassen in nicht begrenzender Weise Monophosphoryl-Lipid-A (MPL, SmithKline Beecham), Saponine wie QS21 (SmithKline Beecham), DQS21 (SmithKline Beecham; WO 96/33739), QS7, QS17, QS18 und QS-L1 (So et al., Mol. Cells 7:178-186, 1997), unvollständiges Freundsches Adjuvans, vollständiges Feundsches Adjuvans, Vitamin E, Montanid, Alaun, CpG-Oligonukleotide (vgl. Krieg et al., Nature 374:546-9, 1995) und verschiedene Wasser-in-Öl-Emulsionen, die aus biologisch abbaubaren Ölen wie Squalen und/oder Tocopherol hergestellt werden. Vorzugsweise werden die Peptide in einer Mischung mit DQS21/MPL verabreicht Das Verhältnis von DQS21 zu MPL beträgt typischerweise etwa 1:10 bis 10:1, vorzugsweise etwa 1:5 bis 5:1 und insbesondere etwa 1:1. Für eine Verabreichung an den Menschen sind DQS21 und MPL typischerweise in einer Vakzine-Formulierung in einem Bereich von etwa 1 µg bis etwa 100 µg vorhanden.

Andere Stoffe, die eine Immunreaktion des Patienten stimulieren, können auch verabreicht werden. Zum Beispiel sind Zytokine bei einer Vakzinierung aufgrund ihrer regulatorischen Eigenschaften auf Lymphozyten verwendbar. Solche Zytokine umfassen z.B. Interleukin-12 (IL-12), von dem gezeigt wurde, dass es die schützenden Wirkungen von Vakzinen verstärkt (vgl. Science 268:1432-1434, 1995), GM-CSF und IL-18.

Es gibt eine Reihe von Verbindungen, die eine Immunreaktion verstärken und die daher bei einer Vakzinierung eingesetzt werden können. Diese umfassen co-stimulierende Moleküle, die in Form von Proteinen oder Nukleinsäuren bereitgestellt werden. Solche co-stimulierenden Moleküle sind beispielsweise B7-1 und B7-2 (CD80 bzw. CD86), die auf dendritischen Zellen (DC) exprimiert werden und mit dem auf den T-Zellen exprimierten CD28-Molekül interagieren. Diese Interaktion stellt eine Co-Stimulierung (Signal 2) für eine Antigen/MHC/TCR-stimulierte (Signal 1) T-Zelle bereit, wodurch die Vermehrung der T-Zelle und die Effektorfunktion verstärkt wird. B7 interagiert auch mit CTLA4 (CD152) auf T-Zellen und Untersuchungen, die CTLA4- und B7-Liganden einbeziehen, zeigen, dass die B7-CTLA4-Interaktion eine Antitumor-Immunität und CTL-Vermehrung verstärken kann (Zheng, P. et al., Proc. Natl. Acad. Sci. USA 95(11):6284-6289 (1998)).

B7 wird typischerweise nicht auf Tumorzellen exprimiert, so dass diese keine wirksamen Antigen-präsentierenden Zellen (APCs) für T-Zellen sind. Eine Induktion der B7-Expression würde ermöglichen, dass Tumorzellen wirksamer eine Vermehrung von cytotoxischen T-Lymphozyten und eine Effektorfunktion stimulieren. Eine Co-Stimulierung durch eine Kombination von B7/IL-6/IL-12 zeigte eine Induktion des IFN-gamma- und Th1-Zytokin-Profils in einer T-Zell-Population, was zu einer weiter verstärkten T-Zell-Aktivität führt (Gajewski et al., J. Immunol. 154:5637-5648 (1995)).

Eine vollständige Aktivierung von cytotoxischen T-Lymphozyten und eine vollständige Effektorfunktion erfordert eine Mitwirkung von T-Helferzellen durch die Interaktion zwischen dem CD40-Liganden auf den T-Helferzellen und dem CD40-Molekül, das von dendritischen Zellen exprimiert wird (Ridge et al., Nature 393:474 (1998), Bennett et al., Nature 393:478 (1998), Schönberger et al., Nature 393:480 (1998)). Der Mechanismus dieses co-stimulierenden Signals betrifft wahrscheinlich die Steigerung der B7- und assoziierten IL-6/IL-12-Produktion durch die dendritischen Zellen (Antigen-präsentierenden Zellen). Die CD40-CD40L-Interaktion komplementiert so die Interaktionen des Signals 1 (Antigen/MHC-TCR) und des Signals 2 (B7-CD28).

Die Verwendung von anti-CD40-Antikörpern für eine Stimulierung von dendritischen Zellen würde erwartungsgemäß direkt eine Reaktion gegenüber Tumor-Antigenen verstärken, die normalerweise außerhalb des Bereichs einer entzündlichen Reaktion liegen oder von nichtprofessionellen Antigen-präsentierenden Zellen (Tumorzellen) präsentiert werden. In diesen Situationen werden T-Helfer- und B7-co-stimulierende Signale nicht bereitgestellt. Dieser Mechanismus könnte im Zusammenhang mit Therapien verwendet werden, die auf Antigengepulsten dendritischen Zellen basieren, oder in Situationen, bei denen T-Helfer-Epitope nicht in bekannten TRA-Vorläufern definiert wurden.

Erfindungsgemäß vorgesehen ist auch eine Verabreichung von Nukleinsäuren, Polypeptiden oder Peptiden. Eine Verabreichung von Polypeptiden und Peptiden kann in an sich bekannter Weise erfolgen. In einer Ausführungsform erfolgt die Verabreichung von Nukleinsäuren durch *ex vivo*-Verfahren, d.h. durch Entfernung von Zellen aus einem Patienten, genetische Veränderung der Zellen, um ein Tumor-assoziiertes Antigen einzubauen, und Wiedereinbringung der veränderten Zellen in den Patienten. Dies umfasst im Allgemeinen das Einbringen einer funktionellen Kopie eines Gens in die Zellen eines Patienten *in vitro* und die Rückführung der genetisch veränderten Zellen in den Patienten. Die funktionelle Kopie des Gens steht unter funktioneller Kontrolle von regulatorischen Elementen, die eine Expression des Gens in den genetisch veränderten Zellen erlauben. Transfektions- und Transduktionsverfahren sind dem Fachmann bekannt. Erfindungsgemäß vorgesehen ist auch eine Verabreichung von Nukleinsäuren *in vivo* durch die Verwendung von Vektoren wie Viren und zielgesteuerten Liposomen.

In einer bevorzugten Ausführungsform ist ein viraler Vektor für die Verabreichung einer Nukleinsäure, die für ein Tumor-assoziiertes Antigen kodiert, aus der Gruppe ausgewählt bestehend aus Adenoviren, Adeno-assoziierten Viren, Poxviren, einschließlich Vacciniavirus und attenuierten Poxviren, Semliki-Forest-Virus, Retroviren, Sindbis-Virus und Ty-Virusähnlichen Partikeln. Besonders bevorzugt sind Adenoviren und Retroviren. Die Retroviren sind üblicherweise replikationsdefizient (d.h. sie sind unfähig, infektiöse Partikel zu erzeugen).

Verschiedene Verfahren können eingesetzt werden, um erfindungsgemäß Nukleinsäuren in Zellen *in vitro* oder *in vivo* einzubringen. Solche Verfahren umfassen die Transfektion von Nukleinsäure-Kalziumphosphat-Präzipitaten, die Transfektion von Nukleinsäuren, die mit DEAE assoziiert sind, die Transfektion oder Infektion mit den vorstehenden Viren, die die interessierenden Nukleinsäuren tragen, die Liposomen-vermittelte Transfektion und ähnliches. In bestimmten Ausführungsformen ist eine Steuerung der Nukleinsäure an bestimmte Zellen bevorzugt. In solchen Ausführungsformen kann ein Träger, der für die Verabreichung einer Nukleinsäure an eine Zelle (z.B. ein Retrovirus oder ein Liposom) eingesetzt wird, ein gebundenes Zielsteuerungsmolekül aufweisen. Zum Beispiel kann ein Molekül wie ein Antikörper, der für ein Oberflächenmembran-Protein auf der Zielzelle spezifisch ist, oder ein Ligand für einen Rezeptor auf der Zielzelle in den Nukleinsäureträger eingebaut oder daran gebunden werden. Bevorzugte Antikörper umfassen Antikörper, die selektiv ein Tumor-assoziiertes Antigen binden. Falls eine Verabreichung einer Nukleinsäure durch Liposomen erwünscht ist, können Proteine, die an ein Oberflächenmembran-Protein binden, das mit der Endozytose assoziiert ist, in die Liposomenformulierung eingebaut werden, um eine Zielsteuerung und/oder Aufnahme zu ermöglichen. Solche Proteine umfassen Kapsid-Proteine oder Fragmente davon, die für einen bestimmten Zelltyp spezifisch sind, Antikörper gegen Proteine, die internalisiert werden, Proteine, die eine intrazelluläre Stelle ansteuern, und ähnliches.

Die erfindungsgemäßen therapeutischen Zusammensetzungen können in pharmazeutisch verträglichen Zubereitungen verabreicht werden. Solche Zubereitungen können gewöhnlich pharmazeutisch verträgliche Konzentrationen von Salzen, Pufferstoffen, Konservierungsstoffen, Trägern, ergänzenden immunitätssteigernden Stoffen wie Adjuvanzien (z.B. CpG-Oligonukleotide) und Zytokine und gegebenenfalls andere therapeutische Wirkstoffe enthalten.

Die erfindungsgemäßen therapeutischen Wirkstoffe können auf jedem herkömmlichen Weg verabreicht werden, einschließlich durch Injektion oder durch Infusion. Die Verabreichung kann beispielsweise oral, intravenös, intraperitoneal, intramuskulär, subkutan oder transdermal erfolgen. Eine therapeutische Verabreichung von Antikörpern erfolgt vorzugsweise durch ein Lungenaerosol. Die Verabreichung von Antisense-Nukleinsäuren erfolgt vorzugsweise durch langsame intravenöse Verabreichung.

Die erfindungsgemäßen Zusammensetzungen werden in wirksamen Mengen verabreicht. Eine "wirksame Menge" betrifft die Menge, die alleine oder zusammen mit weiteren Dosen eine gewünschte Reaktion oder eine gewünschte Wirkung erzielt. Im Fall einer Behandlung einer bestimmten Erkrankung oder eines bestimmten Zustands, der sich durch die Expression eines oder mehrerer Tumor-assoziierter Antigene auszeichnet, betrifft die gewünschte Reaktion die Hemmung des Krankheitsverlaufs. Dies umfasst die Verlangsamung des Fortschreitens der Erkrankung und insbesondere eine Unterbrechung des Fortschreitens der Erkrankung. Die gewünschte Reaktion bei einer Behandlung einer Erkrankung oder eines Zustands kann auch die Verzögerung des Ausbruchs oder eine Verhinderung des Ausbruchs der Erkrankung oder des Zustands sein.

Eine wirksame Menge einer erfindungsgemäßen Zusammensetzung wird von dem zu behandelnden Zustand, der Schwere der Krankheit, den individuellen Parametern des Patienten, einschließlich Alter, physiologischer Zustand, Größe und Gewicht, der Dauer der Behandlung, der Art einer begleitenden Therapie (falls vorhanden), dem spezifischen Verabreichungsweg und ähnlichen Faktoren abhängen.

Die erfindungsgemäßen pharmazeutischen Zusammensetzungen sind vorzugsweise steril und enthalten eine wirksame Menge der therapeutisch wirksamen Substanz für die Erzeugung der gewünschten Reaktion oder der gewünschten Wirkung.

Die Dosen der erfindungsgemäßen Zusammensetzungen, die verabreicht werden, können von verschiedenen Parametern wie der Verabreichungsart, dem Zustand des Patienten, dem gewünschten Verabreichungszeitraum, usw. abhängen. Für den Fall, dass eine Reaktion bei einem Patienten bei einer anfänglichen Dosis unzureichend ist, können höhere Dosen (oder effektiv höhere Dosen, die durch einen anderen, stärker lokalisierten Verabreichungsweg erzielt werden) eingesetzt werden.

Im Allgemeinen werden für eine Behandlung oder für eine Erzeugung oder Erhöhung einer Immunreaktion Dosen des Tumor-assoziierten Antigens von 1 ng bis 1 mg, vorzugsweise von 10 ng bis 100 µg formuliert und verabreicht. Falls die Verabreichung von Nukleinsäuren (DNA sowie RNA), die für Tumor-assoziierte Antigene kodieren, erwünscht ist, werden Dosen von 1 ng bis 0,1 mg formuliert und verabreicht.

Die erfindungsgemäßen pharmazeutischen Zusammensetzungen werden im Allgemeinen in pharmazeutisch verträglichen Mengen und in pharmazeutisch verträglichen Zusammensetzungen verabreicht. Der Begriff "pharmazeutisch verträglich" betrifft ein nichttoxisches Material, das nicht mit der Wirkung des aktiven Bestandteils der pharmazeutischen Zusammensetzung wechselwirkt. Solche Zubereitungen können gewöhnlich Salze, Pufferstoffe, Konservierungsstoffe, Träger und gegebenenfalls andere therapeutische Wirkstoffe enthalten. Bei einer Verwendung in der Medizin sollten die Salze pharmazeutisch verträglich sein. Nicht-pharmazeutisch verträgliche Salze können jedoch für die Herstellung pharmazeutisch verträglicher Salze davon verwendet werden und sind erfindungsgemäß umfasst. Solche pharmakologisch und pharmazeutisch verträglichen Salze umfassen in nicht begrenzender Weise diejenigen, die aus den nachstehenden Säuren hergestellt werden: Chlorwasserstoff-, Bromwasserstoff-, Schwefel-, Salpeter-, Phosphor-, Malein-, Essig-, Salicyl-, Citronen-, Ameisen-, Malon-, Bernsteinsäure und ähnliches. Pharmazeutisch verträgliche Salze können auch als Alkalimetall- oder Erdalkalimetallsalze wie Natrium-, Kalium- oder Calciumsalze hergestellt werden.

Eine erfindungsgemäße pharmazeutische Zusammensetzung kann einen pharmazeutisch verträglichen Träger umfassen. Der Begriff "pharmazeutisch verträglicher Träger" betrifft erfindungsgemäß einen oder mehrere kompatible feste oder flüssige Füllstoffe, Verdünnungsmittel oder Kapselsubstanzen, die für eine Verabreichung an einen Menschen geeignet sind. Der Begriff "Träger" betrifft einen organischen oder anorganischen Bestandteil, natürlicher oder synthetischer Natur, in dem der aktive Bestandteil kombiniert wird, um eine Anwendung zu erleichtern. Die Bestandteile der erfindungsgemäßen pharmazeutischen Zusammensetzung sind gewöhnlich derart, dass keine Interaktion auftritt, die die gewünschte pharmazeutische Wirksamkeit wesentlich beeinträchtigt.

Die erfindungsgemäßen pharmazeutischen Zusammensetzungen können geeignete Pufferstoffe wie Essigsäure in einem Salz, Citronensäure in einem Salz, Borsäure in einem Salz und Phosphorsäure in einem Salz enthalten.

Die pharmazeutischen Zusammensetzungen können auch gegebenenfalls geeignete Konservierungsstoffe wie Benzalkoniumchlorid, Chlorbutanol, Parabene und Thimerosal enthalten.

Die pharmazeutischen Zusammensetzungen werden gewöhnlich in einer einheitlichen Dosisform dargeboten und können in an sich bekannter Weise hergestellt werden. Erfindungsgemäße pharmazeutische Zusammensetzungen können beispielsweise in Form von Kapseln, Tabletten, Lutschpastillen, Lösungen, Suspensionen, Sirupen, Elixieren oder als Emulsion vorliegen.

Zusammensetzungen, die für eine parenterale Verabreichung geeignet sind, umfassen gewöhnlich eine sterile wässrige oder nicht-wässrige Zubereitung des Wirkstoffs, die vorzugsweise mit dem Blut des Empfängers isotonisch ist. Verträgliche Träger und Lösungsmittel sind beispielsweise Ringer-Lösung und isotonische Natriumchloridlösung. Zusätzlich werden gewöhnlich sterile, fixierte Öle als Lösungs- oder Suspensionsmedium eingesetzt.

Die vorliegende Erfindung wird durch die nachstehenden Abbildungen und Beispiele ausführlich beschrieben, die ausschließlich der Erläuterung dienen und nicht begrenzend zu verstehen sind. Dem Fachmann sind aufgrund der Beschreibung und der Beispiele weitere Ausführungsf]ormen zugänglich, die ebenfalls erfindungsgemäß umfasst sind.

### Abbildungen

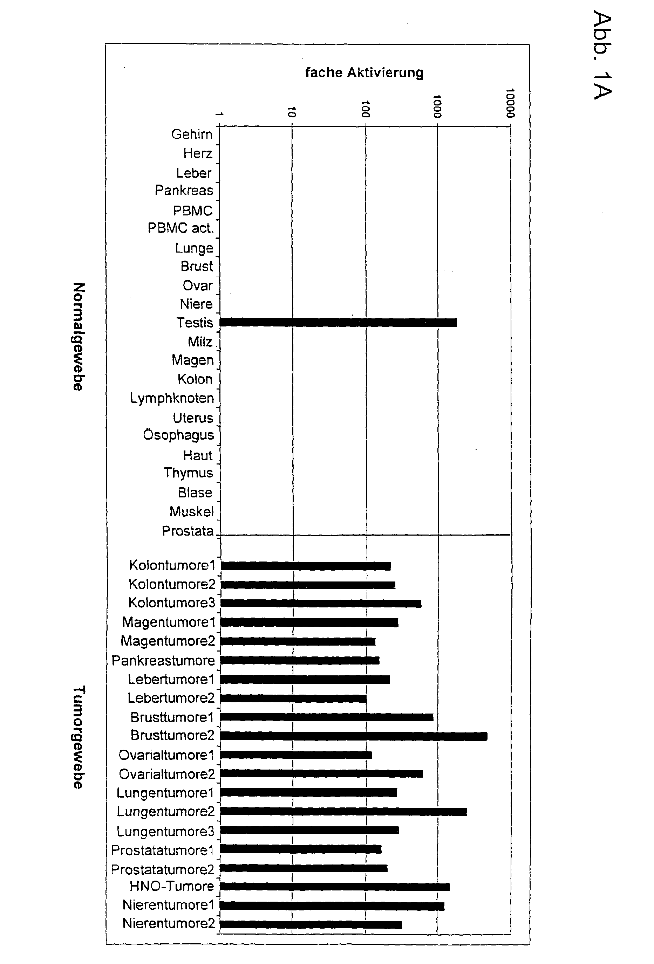

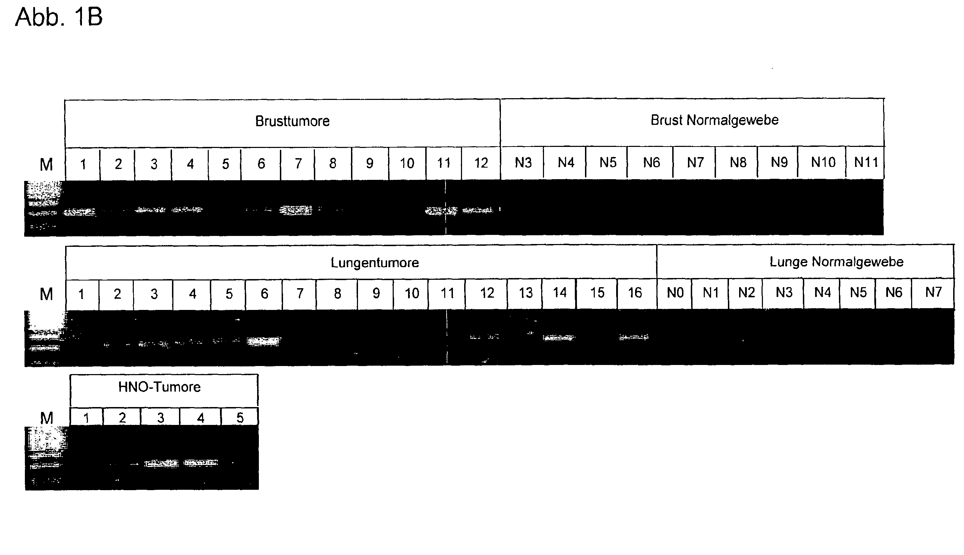

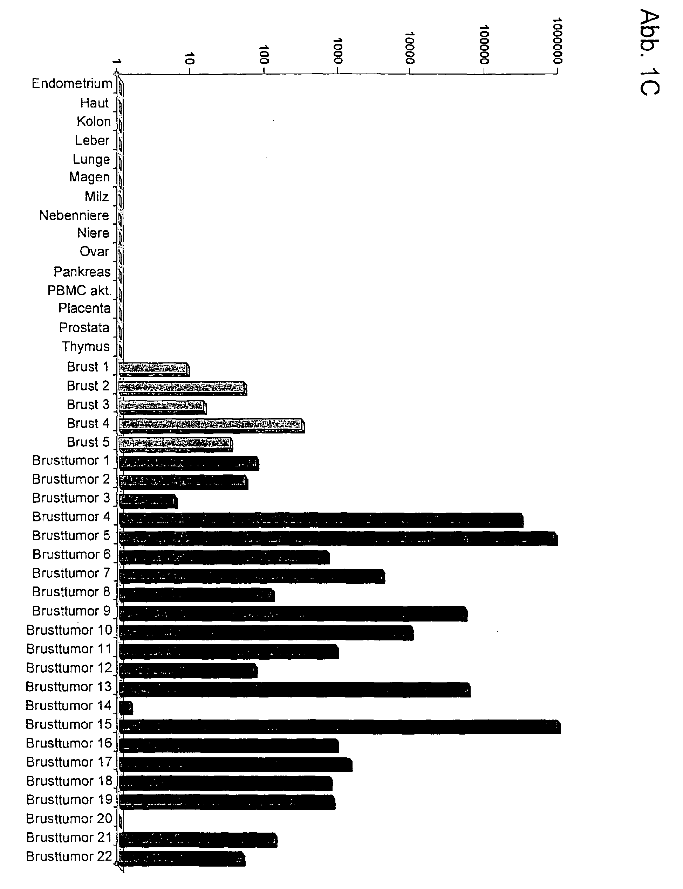

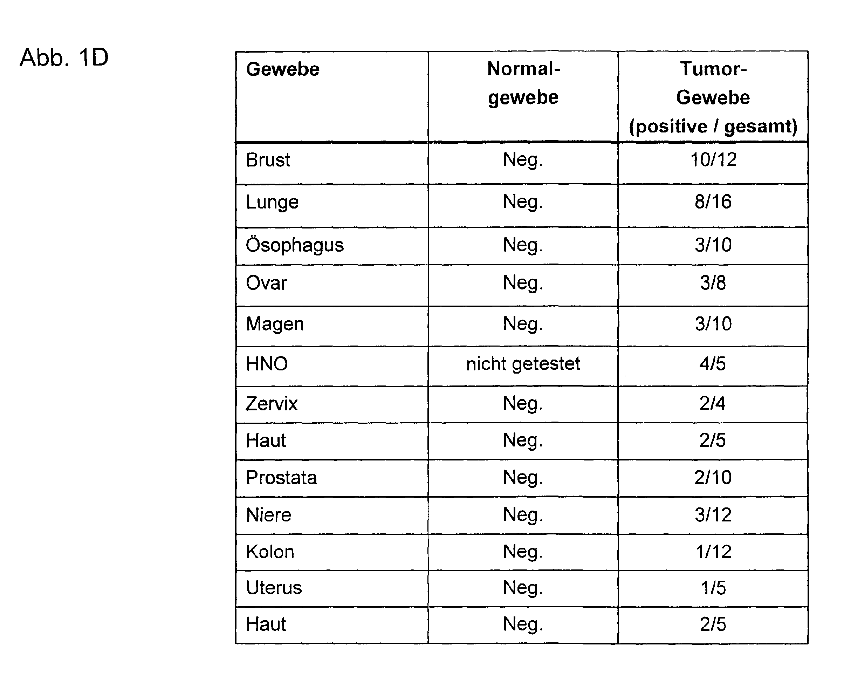

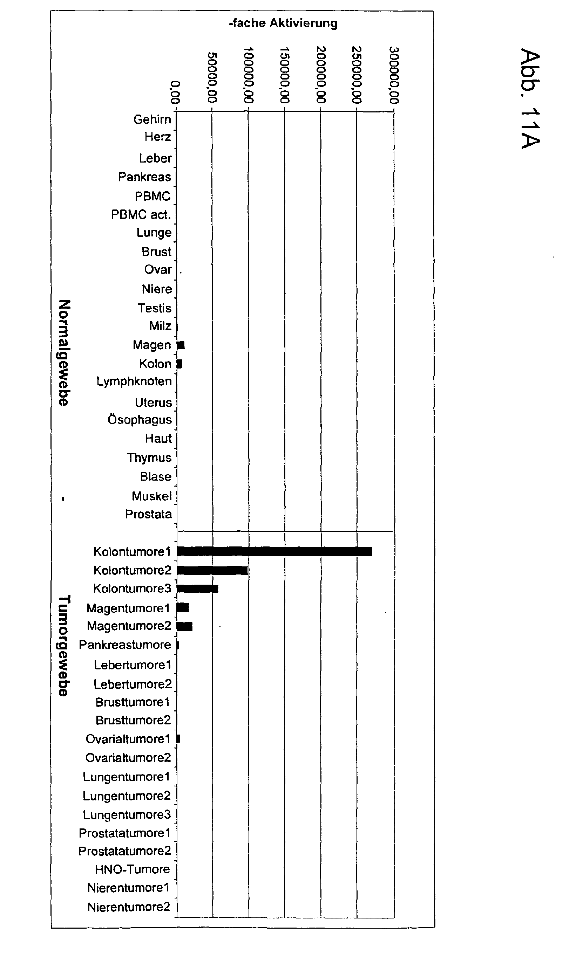

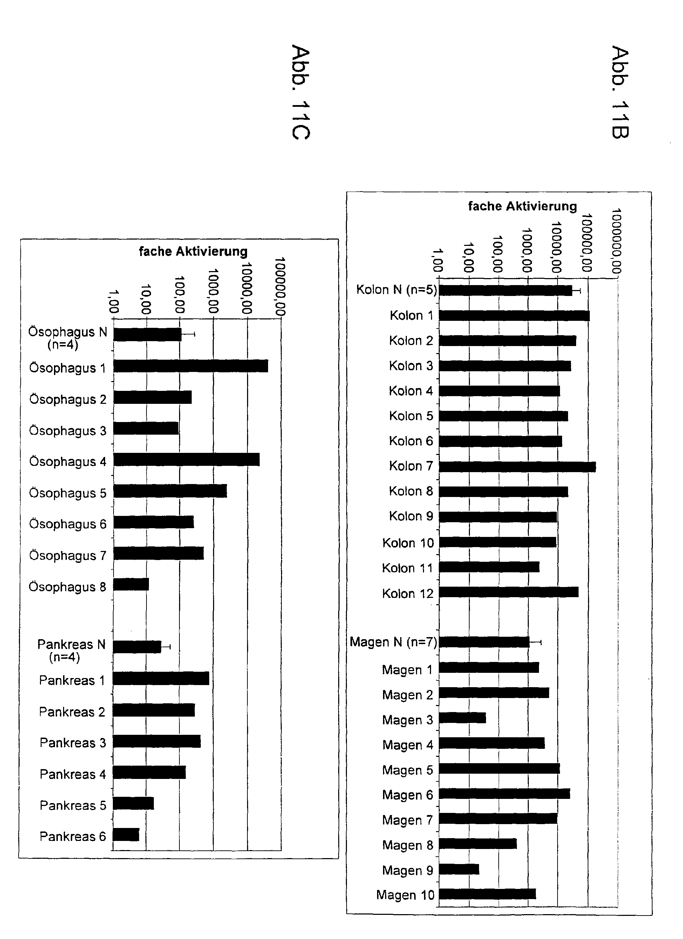

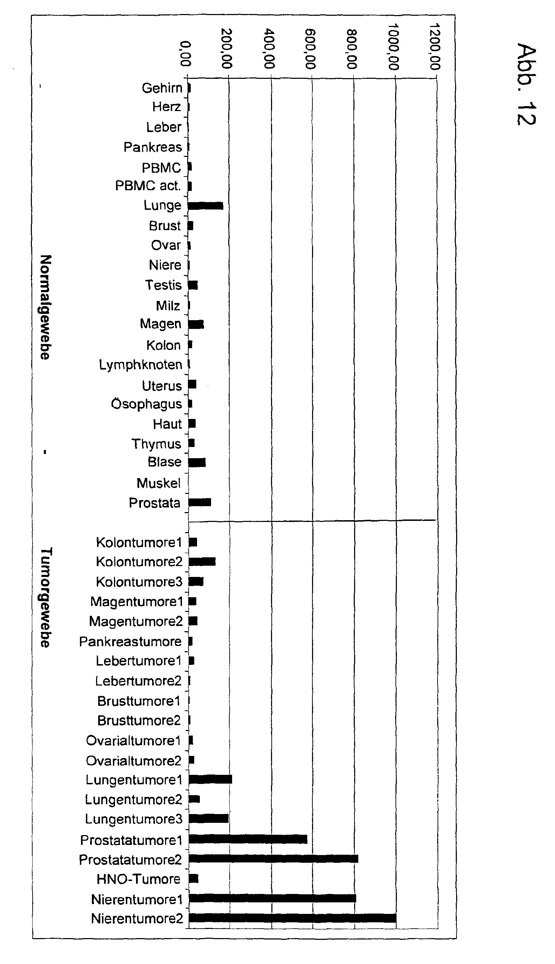

**Abb. 1****: PCR-Analyse des Gens FLJ31461**
   **A:** Quantitative Expressionsanalyse von FLJ31461 in Normalgeweben (linke Seite) und in verschiedenen Tumoren (Pools bestehend aus jeweils 3-4 Einzelproben, rechte Seite) in logarithmischer Darstellung der relativen Expression (-fache Aktivierung). In den meisten Tumoren findet sich eine mindestens 100-fache Überexpression von FLJ31461 im Vergleich zu den Expressionsspiegeln in den gesunden Geweben.
   **B:** Gelbild einer konventionellen RT-PCR-Analyse von FLJ31461 in Brust-, Lungen- und HNO-Tumoren mit den zugehörigen Normalgeweben Nₓ; M: DNA-Längenmarker.
   **C:** Quantitative Expressionsanalyse in verschiedenen Normalgeweben (linke Seite) und in Brusttumoren in logarithmischer Darstellung der relativen Expression (-fache Aktivierung). In fast allen Brusttumoren findet sich eine mindestens 100-fache Überexpression von FLJ31461 im Vergleich zu den Expressionsspiegeln in den gesunden Geweben.
   **D:** Zusammenfassung der FLJ31461-spezifischen Expression in verschiedenen analysierten Tumoren. Angegeben ist die Anzahl positiv getesteter Tumorproben/Gesamtzahl der analysierten Tumorproben. Während alle untersuchten somatischen Normalgewebe (jeweils 3-10 Gewebe, je nach Gewebstyp) keine Expression von FLJ31461 aufweisen, ist das Gen in sehr vielen Tumoren mit unterschiedlicher Häufigkeit exprimiert.
**Abb. 2****: Protein-Lokalisation**
   Darstellung der zellulären Lokalisation des FLJ31461-Proteins. Die Abbildung zeigt die endogene Proteinexpression der Brusttumor-Zelllinie MCF7.
**Abb. 3****: Immunhistochemische Analyse**
   **A:** Normalgewebe Testis (positive Membranlokalisation), Kolon und Niere (negative Membranlokalisation).
   **B:** Nachweis des FLJ31461-Proteins in einem Bronchialkarzinom, einem Zervixkarzinom sowie einer Lymphknoten-Metastase eines Brusttumors in Übersicht (linke Spalte) und im Detail (rechte Spalte).
   **C:** Zusammenfassung der immunhistochemischen Analysen des FLJ31461-Proteins. Angegeben ist die Anzahl positiv getesteter Tumorproben/Gesamtzahl der analysierten Tumorproben. Während alle untersuchten somatischen Normalgewebe keine Expression von FLJ31461 aufweisen, ist das Protein in sehr vielen Tumoren mit unterschiedlicher Häufigkeit an der Zelloberfläche zu finden.

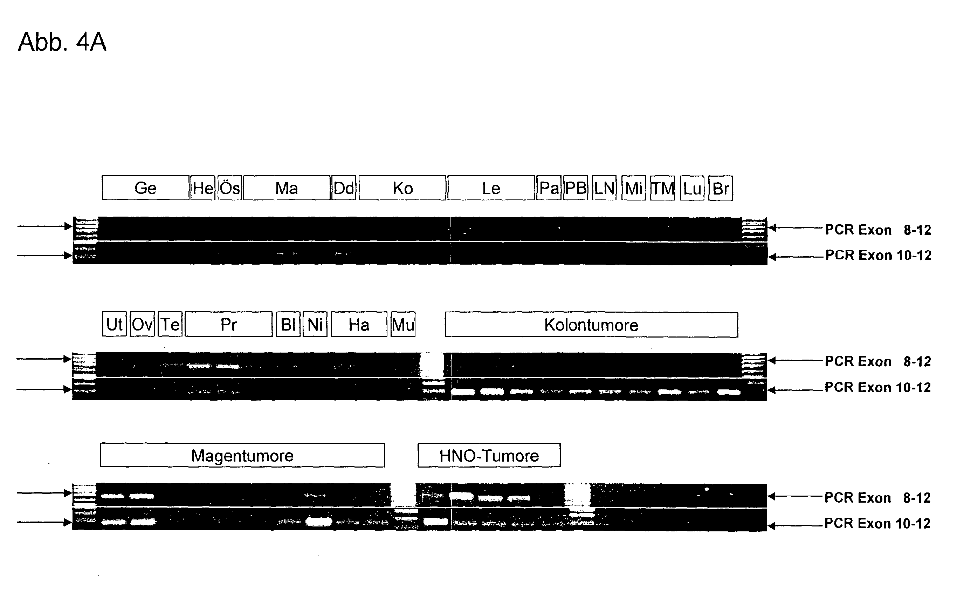

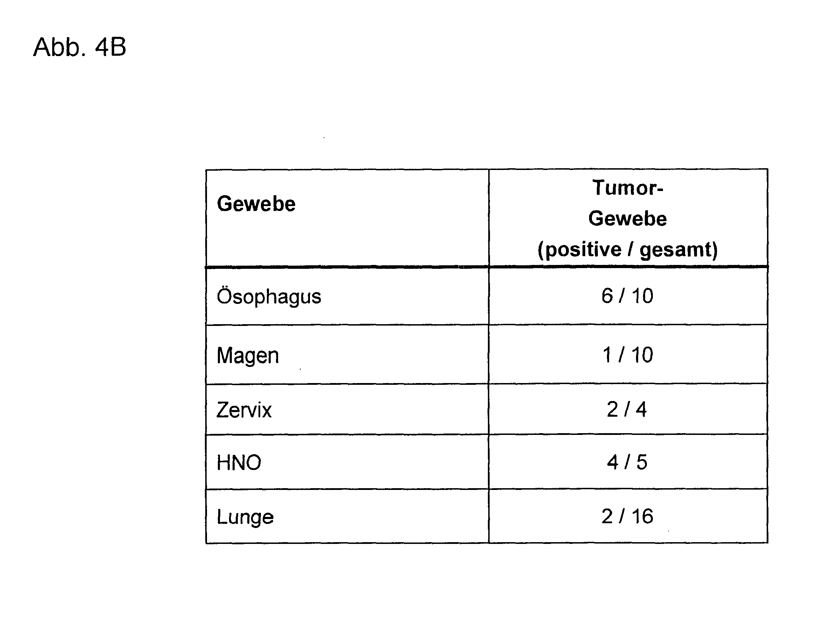

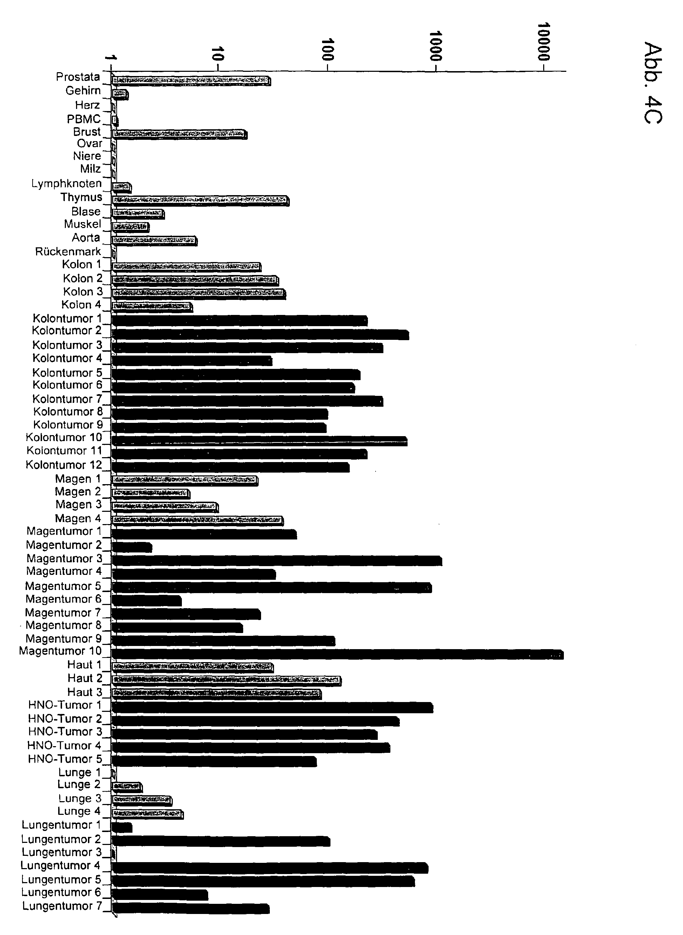

**Abb. 4****: PCR-Analyse von DSG4-Spleissvarianten in Normalgeweben und Tumoren**
   **A:** Die PCR an Normalgeweben und diversen Tumoren erfolgte mit DSG4-spezifischen Oligonukleotiden in Exons 8-12 und Exons 10-12. Erkennbar ist die dominante Epression des Transkripts der Exons 10-12 in Kolontumoren, während das Transkript der Exons 8-12 auch in Normalgeweben deutlich exprimiert ist. Ge: Gehirn, Dd: Duodenum, Pa: Pankreas, Mi: Milz, Te: Testis, He: Herz, Ko: Kolon, LN: Lymphknoten, TM: Thymus, Pr: Prostata, Ös: Ösophagus, Le: Leber, PB: akt. PBMC, Lu: Lunge, B1: Blase, Ma: Magen, Br: Brust, Ut: Uterus, Ov: Ovar, Ni: Niere, Ha: Haut, Mu: Muskel.
   **B:** Zusammenfassung der spezifischen Expression der DSG4-Exons 10-12 in verschiedenen analysierten Tumoren. Angegeben ist die Anzahl positiv getesteter Tumorproben/Gesamtzahl. Während fast alle untersuchten somatischen Normalgewebe keine Expression von DSG4 aufweisen, ist dieser Genabschnitt in sehr vielen Tumoren mit unterschiedlicher Häufigkeit nachzuweisen.
   **C:** Quantitative Expressionsanalyse des Transkriptabschnitts der DSG4-Exons 10-12 in Normalgeweben (linke Seite) und in Kolon-, Magen- und HNO-Tumoren in logarithmischer Darstellung der relativen Expression (-fache Aktivierung). In den meisten Tumoren findet sich eine mindestens 50-fache Überexpression der DSG4-Exons 10-12 im Vergleich zu den Expressionsspiegeln in den gesunden Geweben.

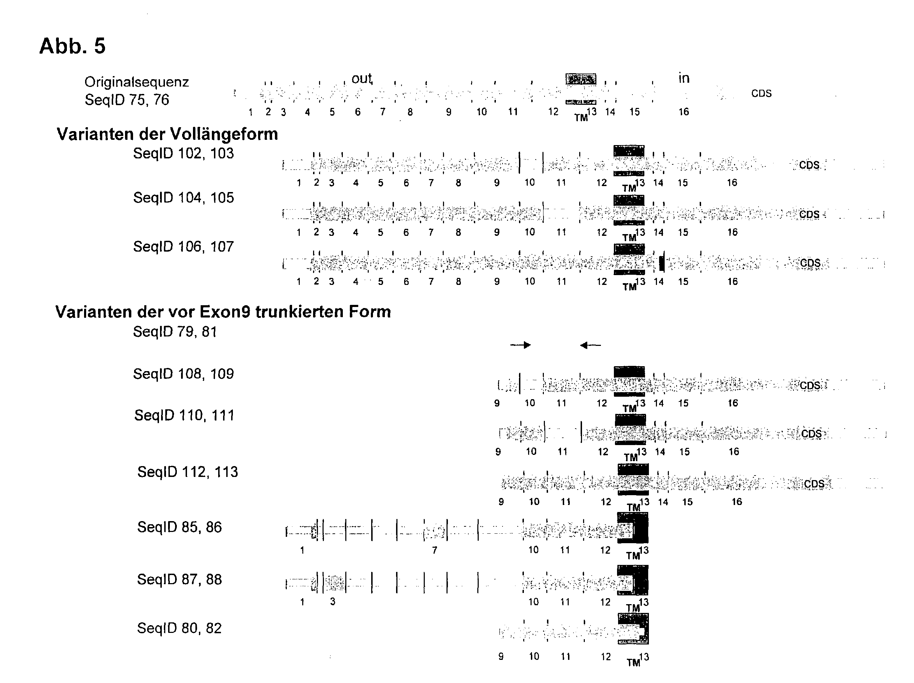

**Abb. 5****: Übersicht über die putativen Transkriptvarianten des DSG4-Gens**

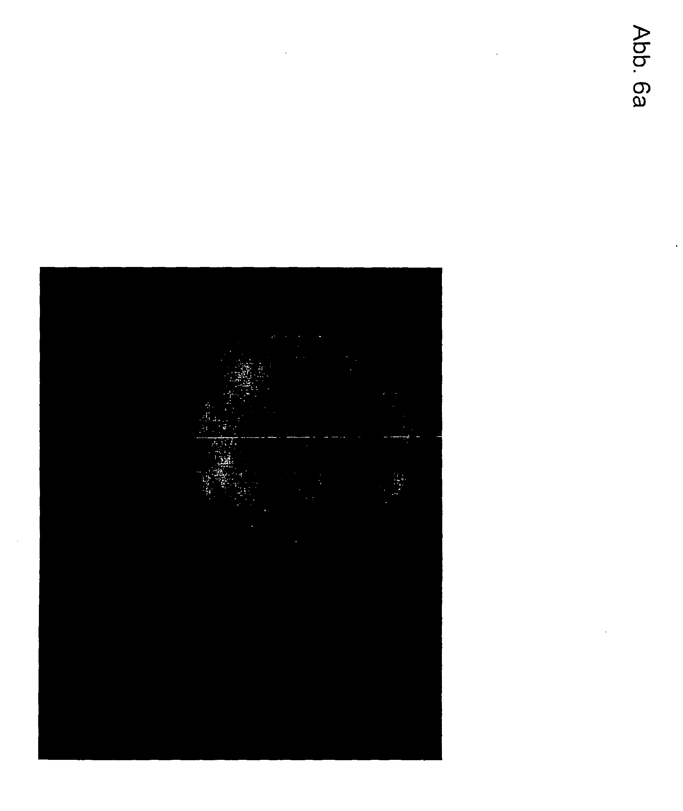

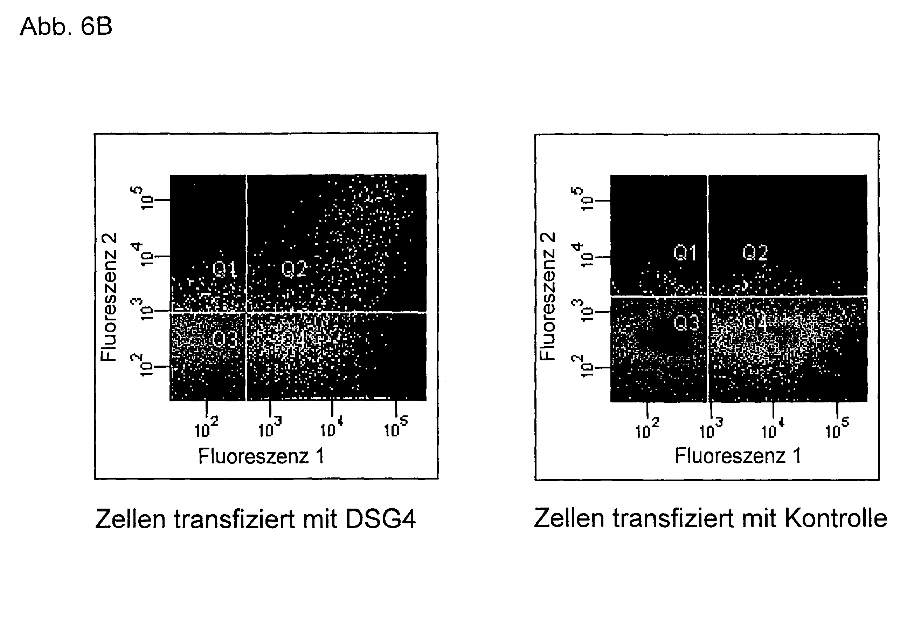

**Abb. 6****: Protein-Lokalisation**
   **A:** Darstellung der zellulären Lokalisation des DSG4-Proteins mittels Immunfluoreszenz an einer DSG4-transfizierten Zelle.
   **B:** FACS-Analyse von DSG4-transfizierten Zellen mit DSG4-spezifischen Antikörpern (linke Abb.) und von Mock-transfizierten Zellen mit DSG4-spezifischen Antikörpern (Negativkontrolle, rechte Abb.). Deutlich ist die spezifische, oberflächenspezifische Anfärbung zu erkennen.

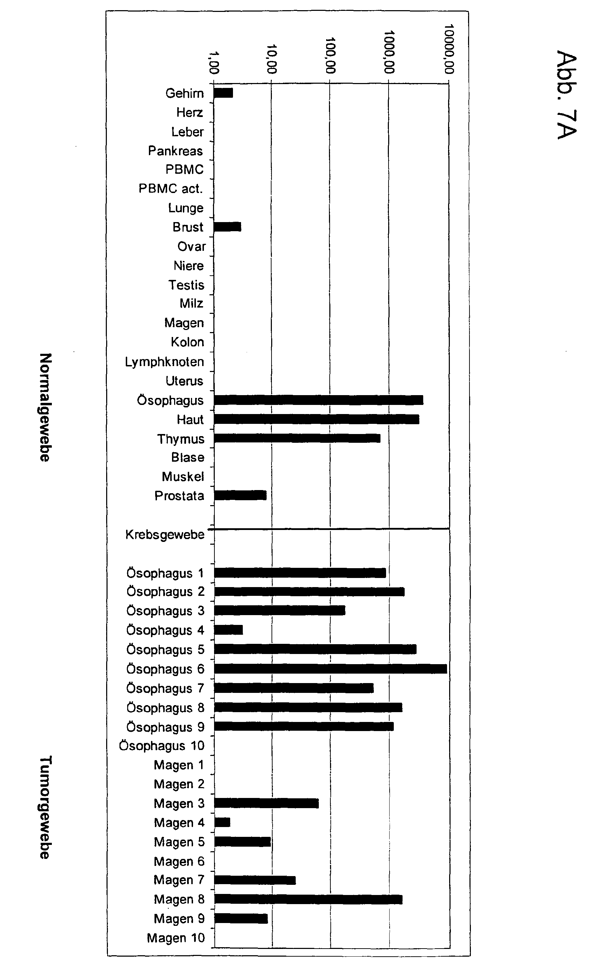

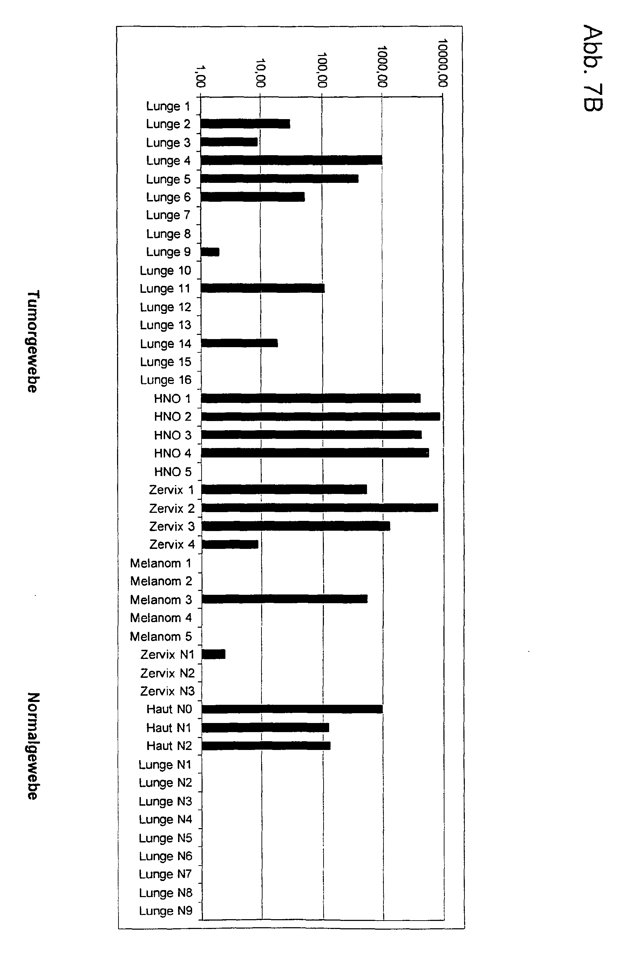

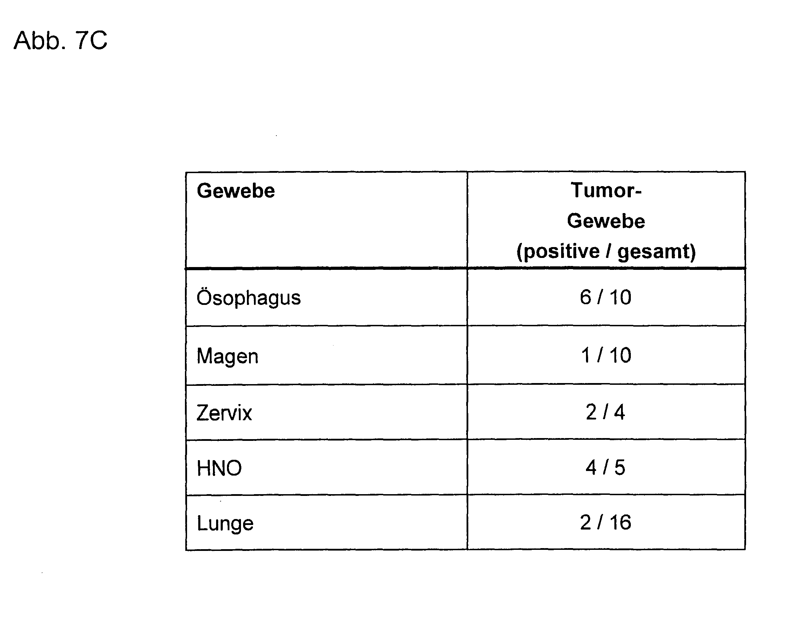

**Abb. 7****: qPCR-Analyse der DSG3-spezifischen Expression in Normalgeweben und in Tumoren**
   **A:** Quantitative Expressionsanalyse von DSG3 in Normalgeweben (linke Seite) und in verschiedenen Tumoren (Pools bestehend aus jeweils 3-4 Einzelproben, rechte Seite) in logarithmischer Darstellung der relativen Expression (-fache Aktivierung). Erkennbar ist die deutliche Überexpression in Ösophagustumoren im Vergleich zu den meisten Normalgeweben.
   **B:** Quantitative Expressionsanalyse von DSG3 in verschiedenen Zervix- und Lungentumoren sowie in HNO-Tumoren im Vergleich zur Expression in den zugehörigen Normalgeweben (n=3 (Zervix); n=9 (Lunge)). Logarithmische Darstellung.
   **C:** Zusammenfassung der DSG3-spezifischen Expression in verschiedenen analysierten Tumoren. Angegeben ist die Anzahl positiv getesteter Tumorproben/Gesamtzahl der analysierten Tumorproben. Während alle untersuchten somatischen Normalgewebe (jeweils 3-10 Gewebe, je nach Gewebstyp) keine Expression von DSG3 aufweisen, ist das Gen in sehr vielen Tumoren mit unterschiedlicher Häufigkeit exprimiert.

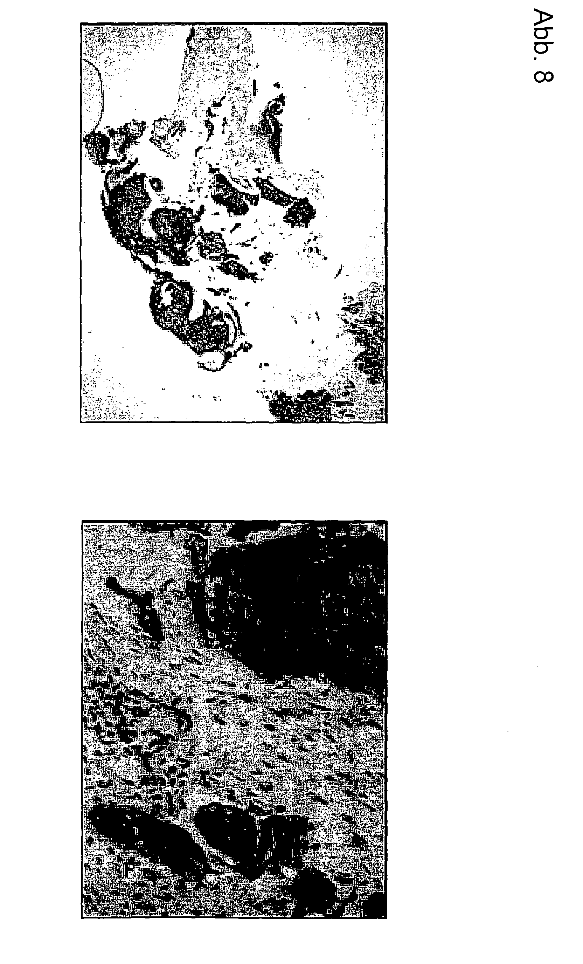

**Abb. 8****: Immunhistochemische Analyse**
   Die Abbildung zeigt in Übersicht (linke Seite) und im Detail die homogene DSG3-Lokalisation in einem HNO-Tumor.

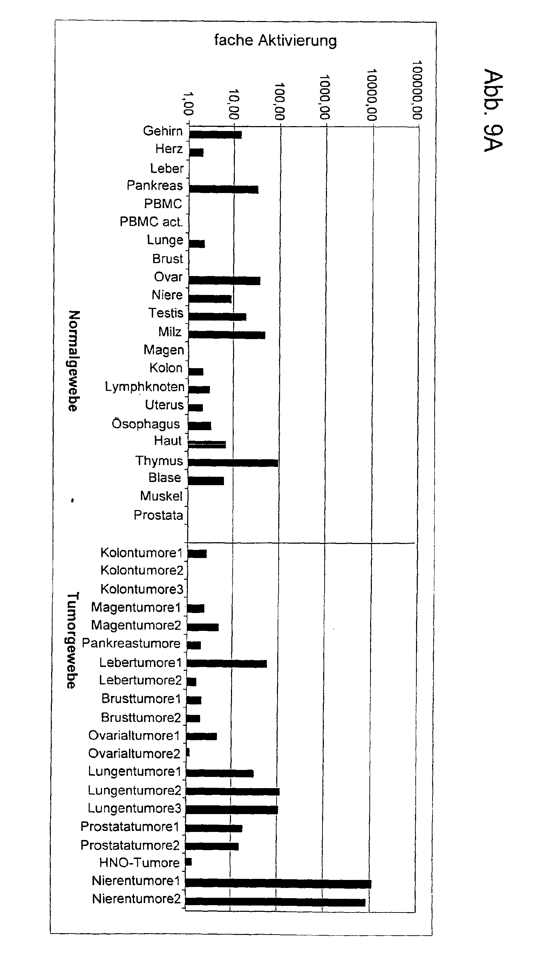

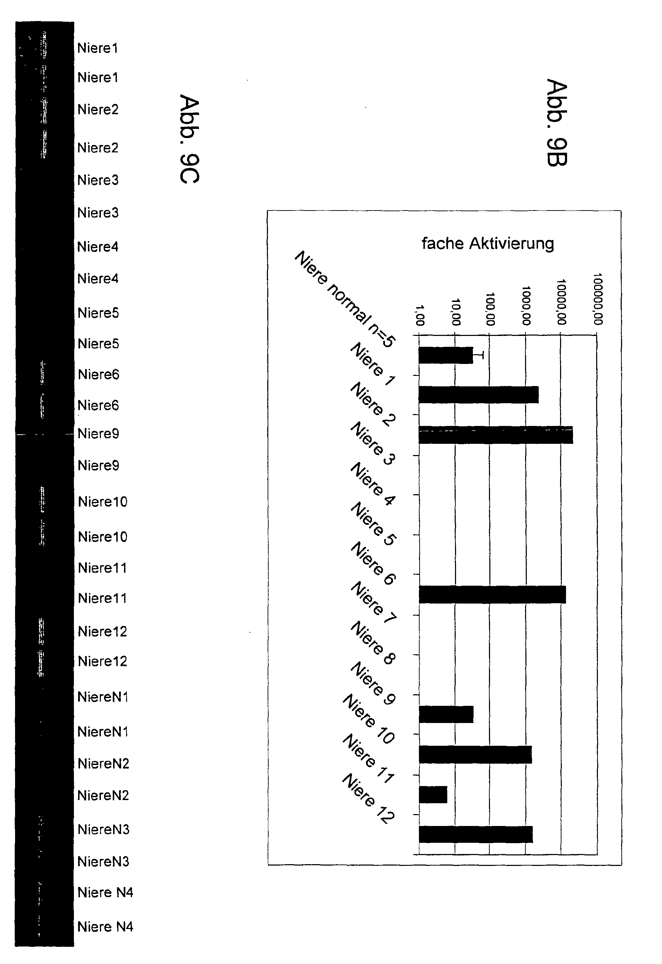

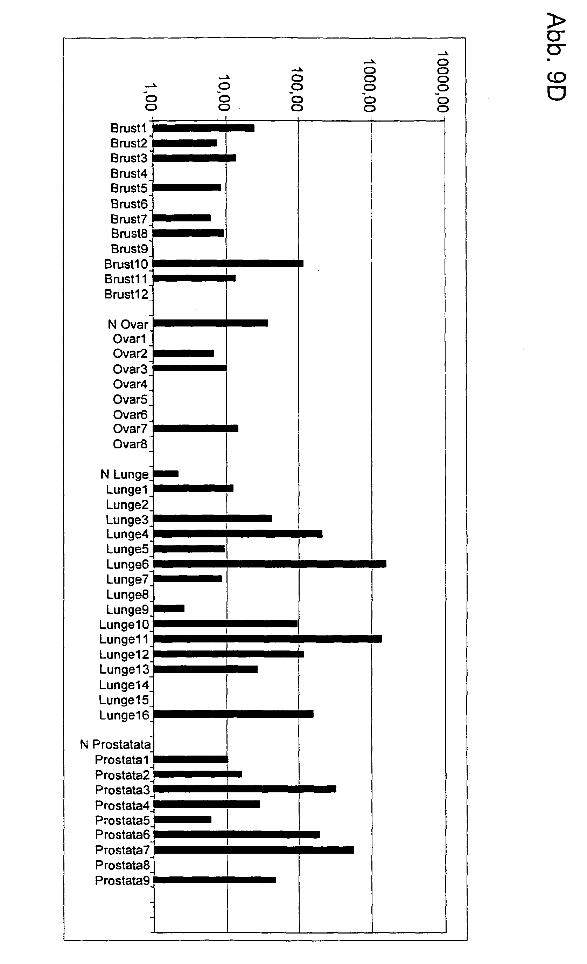

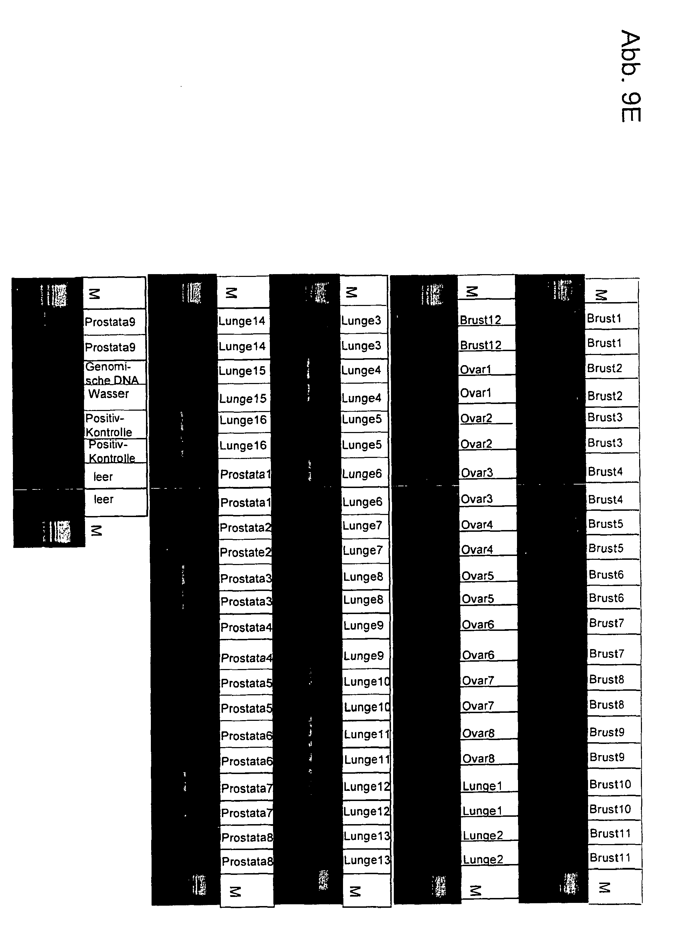

**Abb. 9****: qPCR-Analyse von SLC6A3**
   **A:** Quantitative Expressionsanalyse von SLC6A3 in Normalgeweben (links) und in Tumorproben (Pools bestehend aus jeweils 3-4 Einzelproben, rechts) in logarithmischer Darstellung der relativen Expression (-fache Aktivierung).
   **B:** Quantitative Expressionsanalyse von SLC6A3 in verschiedenen Nierentumoren im Vergleich zur Expression in der normalen Niere (n=5). Logarithmische Darstellung der relativen Expression.
   **C:** Konventionelle Endpunkt-RT-PCR-Analyse von SLC6A3-spezifischen Transkripten (Doppelbestimmung) in Nierentumoren und verschiedenen normalen Nierengeweben. Bild nach gelelektrophoretischer Auftrennung der SLC6A3-spezifischen Fragmente.
   **D:** Quantitative Expressionsanalyse von SLC6A3 in Brust-, Ovarial-, Lungen- und Prostatakarzinomen; Logarithmische Darstellung der relativen Expression (-fache Aktivierung). "Gewebe" N: Normalgewebe; "Gewebe": Tumorgewebe.
   E: Konventionelle RT-PCR-Analyse von SLC6A3 in Brust-, Ovarial-, Lungen- und Prostatatumoren nach gelelektrophoretischer Auftrennung in einer Doppelbestimmung. M: DNA-Längenmarker.

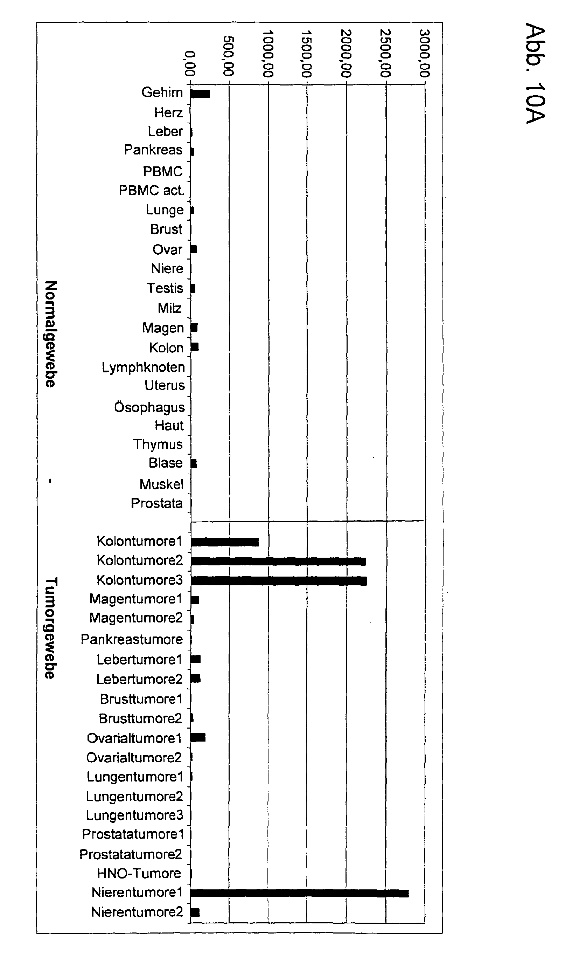

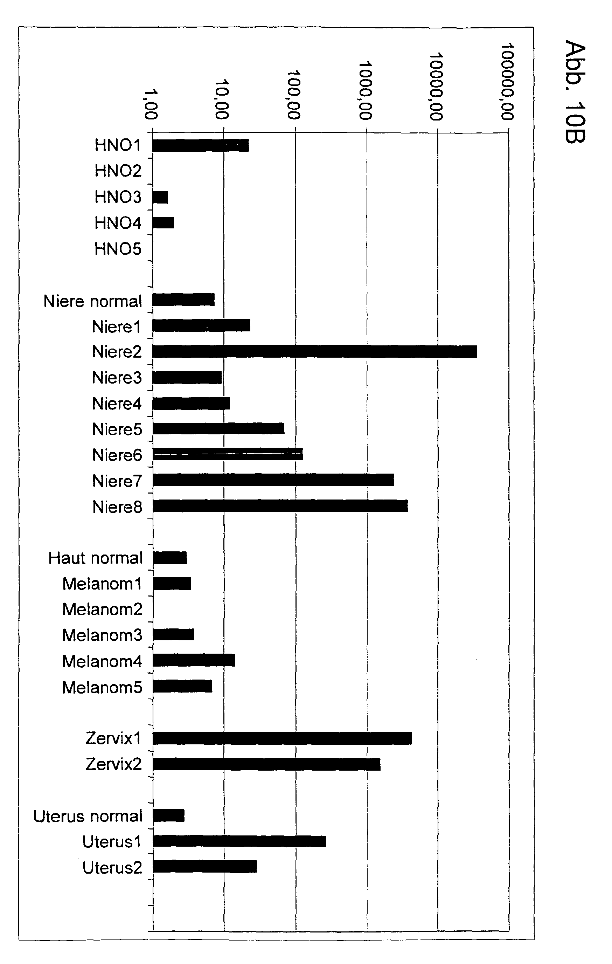

**Abb. 10****: qPCR-Analyse von GRM8**
   **A:** Quantitative Expressionsanalyse von GRM8 in Normalgeweben (links) und Tumorgeweben (Pools bestehend aus jeweils 3-4 Einzelproben, rechts) in linearer Darstellung der relativen Expression (-fache Aktivierung).
   **B:** Quantitative Expressionsanalyse von GRM8 in verschiedenen Nieren- und Uterustumoren im Vergleich zur Expression in normale Niere und Uterus, sowie relative Expression in HNO- und Zervixtumoren und Melanomen. Logarithmische Darstellung der relativen Expression. -
**Abb. 11****: qPCR-Analyse von CDH17**
   **A:** Quantitative Expressionsanalyse von CDH17 in Normalgeweben (links) und in Tumorgeweben (Pools bestehend aus jeweils 3-4 Einzelproben, rechts) in linearer Darstellung der relativen Expression (-fache Aktivierung).
   **B:** Quantitative Expressionsanalyse von CDH17 in verschiedenen Kolon- und Magentumoren im Vergleich zur Expression in den zugehörigen Normalgeweben. Logarithmische Darstellung.
   **C:** Quantitative Expressionsanalyse von CDH17 in verschiedenen Ösophagus- und Pankreastumoren im Vergleich zur Expression in den zugehörigen Normalgeweben. Logarithmische Darstellung
**Abb. 12****: qPCR-Analyse des ABC-Transporters ABCC4**
   Quantitative Expressionsanalyse von ABCC4 in Normalgeweben (links) und in Tumoren (Pools bestehend aus jeweils 3-4 Einzelproben, rechts) in linearer Darstellung der relativen Expression (-fache Aktivierung).

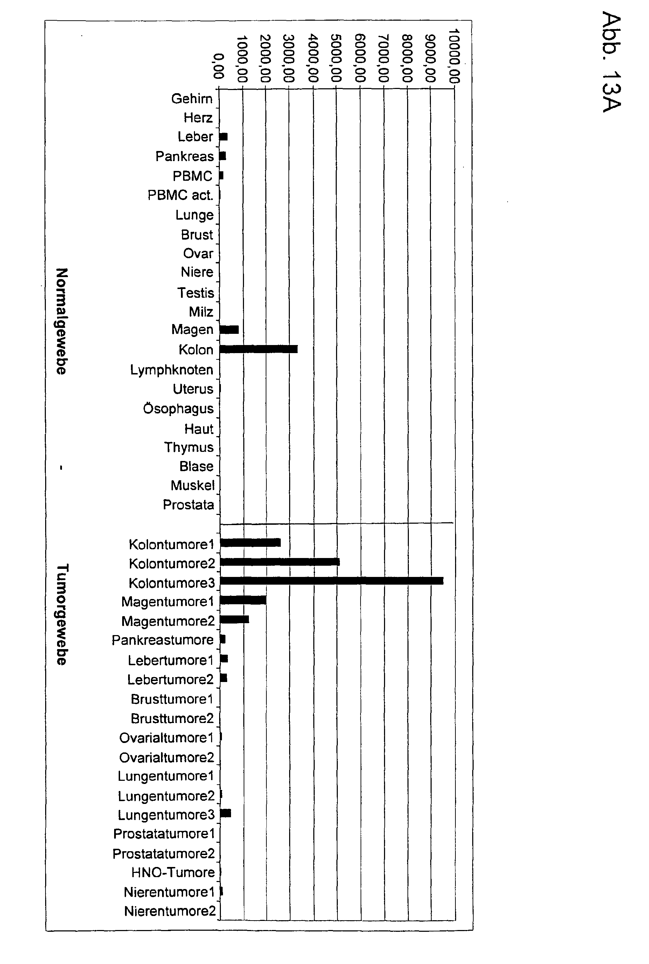

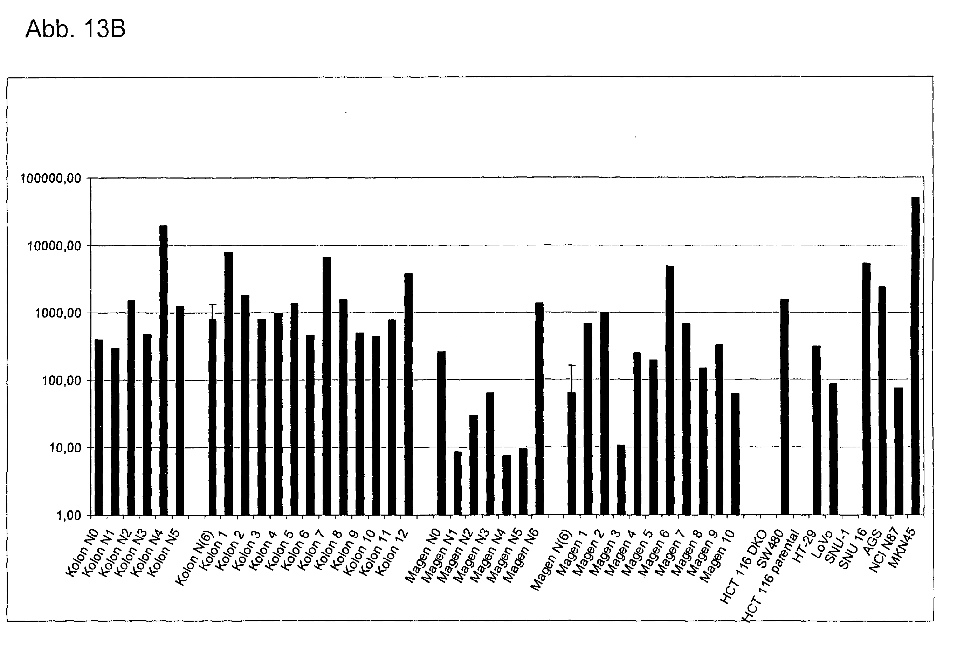

**Abb. 13****: qPCR-Analyse von Villin1 (VIL1).**
   **A:** Quantitative Expressionsanalyse von VIL1 in Normalgeweben (links) und Tumorgeweben (Pools bestehend aus jeweils 3-4 Einzelproben, rechts) in linearer Darstellung der relativen Expression (-fache Aktivierung).
   **B:** Quantitative Expressionsanalyse von VIL1 in verschiedenen Kolon- und Magentumoren im Vergleich zur Expression in den zugehörigen Normalgeweben. Logarithmische Darstellung.

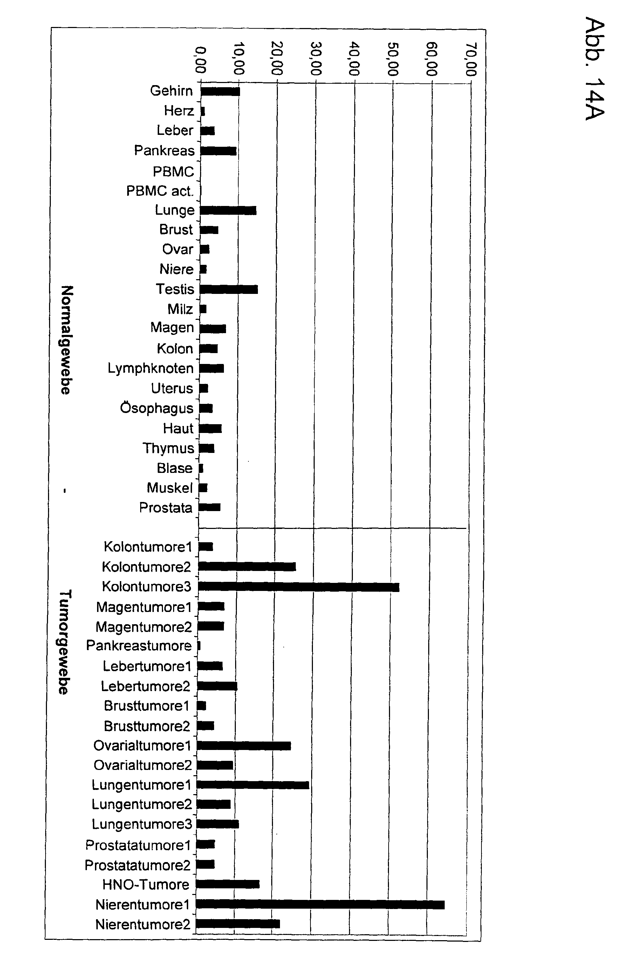

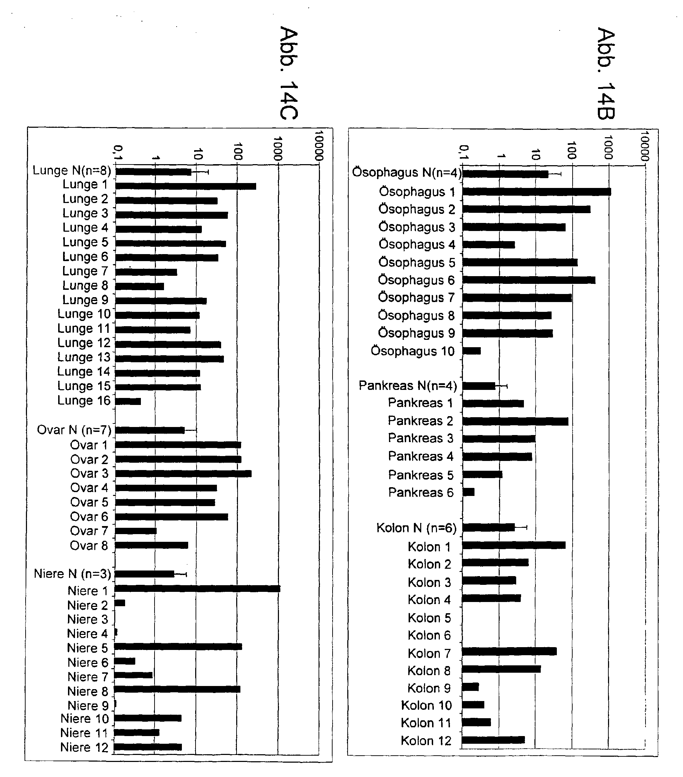

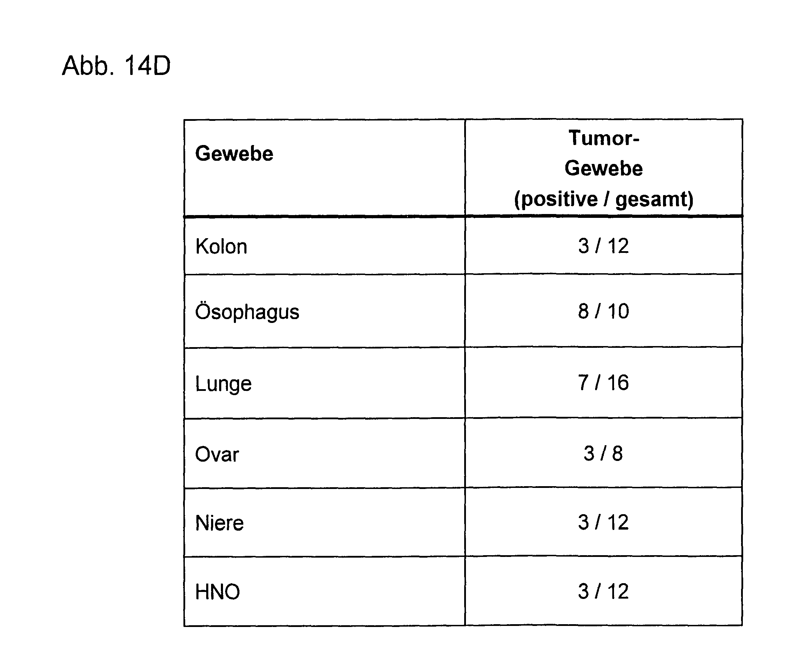

**Abb. 14****: qPCR-Analyse des hypothetischen Proteins MGC34032**
   **A:** Quantitative Expressionsanalyse von MGC34032 in Normalgeweben (links) und verschiedenen Tumoren (Pools bestehend aus jeweils 3-4 Einzelproben, rechts) in linearer Darstellung der relativen Expression (-fache Aktivierung).
   **B:** Quantitative Expressionsanalyse von MGC34032 in verschiedenen Ösophagus-, Pankreas- und Kolontumoren im Vergleich zur Expression in den zugehörigen Normalgeweben. Logarithmische Darstellung.
   **C:** Quantitative Expressionsanalyse von MGC34032 in verschiedenen Lungen-, Ovarial- und Nierentumoren im Vergleich zur Expression in den zugehörigen Normalgeweben. Logarithmische Darstellung.
   **D:** Zusammenfassung der MGC34032-spezifischen Expression in verschiedenen analysierten Tumoren. Angegeben ist die Anzahl positiv getesteter Tumorproben/Gesamtzahl der analysierten Tumorproben. Während alle untersuchten somatischen Normalgewebe (jeweils 3-10 Gewebe, je nach Gewebstyp) eine signifikant niedrigere Expression von MGC34032 aufweisen, ist das Gen in sehr vielen Tumoren mit unterschiedlicher Häufigkeit überexprimiert.

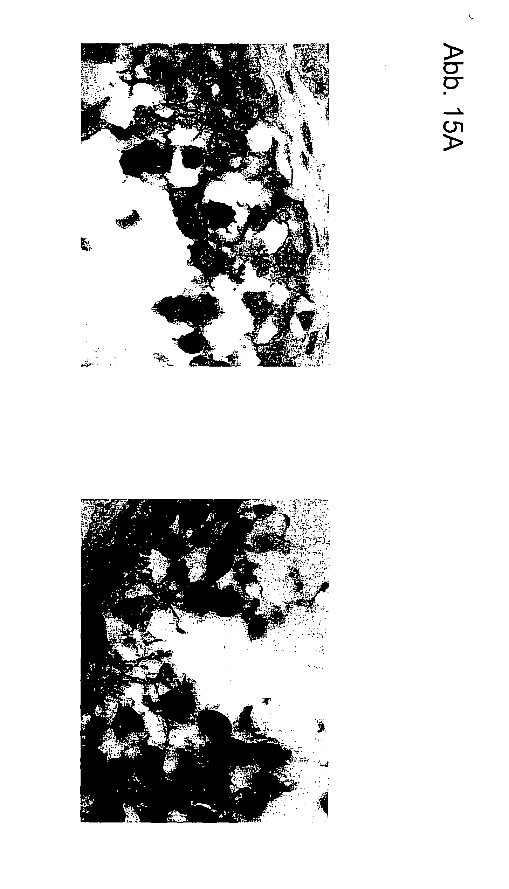

**Abb. 15****: Immunhistochemische Analyse**
   Die Abbildung zeigt 2 Detailansichten der zellulären Lokalisation des MGC34032-Proteins in einem menschlichen Testisgewebe.

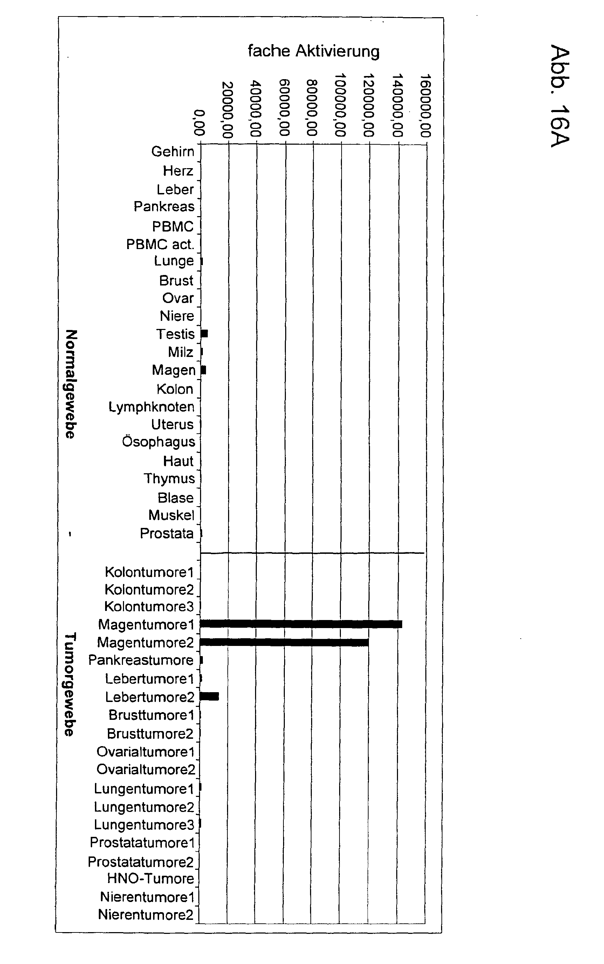

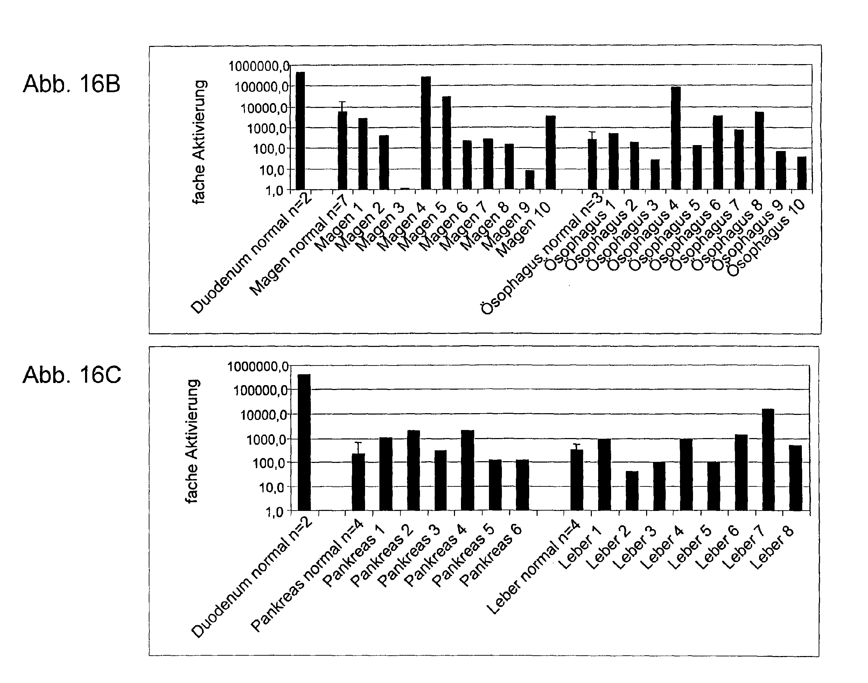

**Abb. 16****: Expressionsanalyse der Enterokinase (PRSS7)**
   **A:** Quantitative Expressionsanalyse von PRSS7 in Normalgeweben (links) und verschiedenen Tumorgeweben (Pools bestehend aus jeweils 3-4 Einzelproben, rechts) in linearer Darstellung der relativen Expression (-fache Aktivierung).
   **B:** Quantitative Expressionsanalyse von PRSS7 in verschiedenen Magen- und Ösophagustumoren im Vergleich zur Expression in den zugehörigen Normalgeweben (Magen: n=7; Ösophagus: n=3). Als Vergleich wurde die Expression im normalen Duodenum gemessen (n=2). Logarithmische Darstellung.
   **C:** Quantitative Expressionsanalyse von PRSS7 in verschiedenen Pankreas- und Lebertumoren im Vergleich zur Expression in den zugehörigen Normalgeweben (jeweils n=4). Als Vergleich wurde die Expression im normalen Duodenum gemessen (n=2). Logarithmische Darstellung.

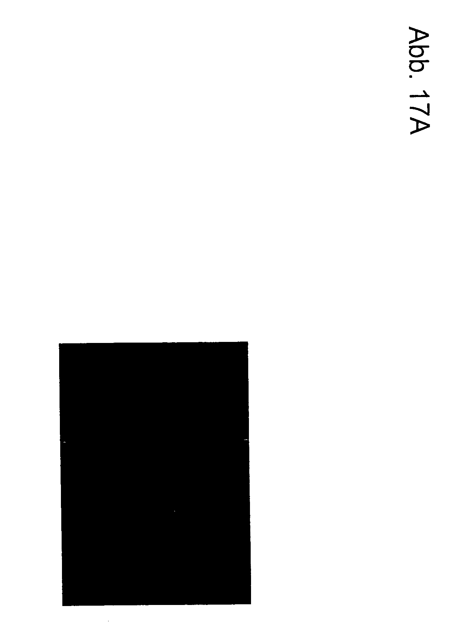

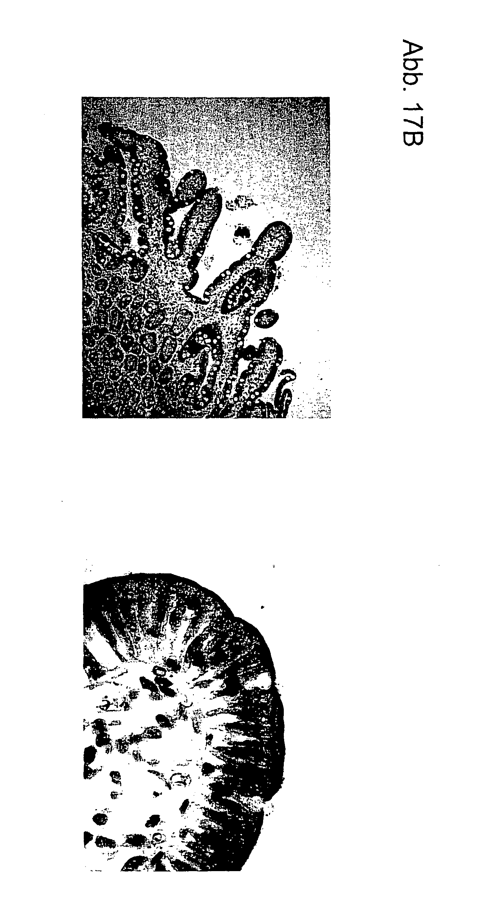

**Abb. 17****: Protein-Lokalisation**
   **A:** Darstellung der zellulären Lokalisation des PRSS7-Proteins an PRSS7-transfizierten Zellen.
   **B:** Nachweis des PRSS7-Proteins in Übersicht (links) und im Detail (rechts).

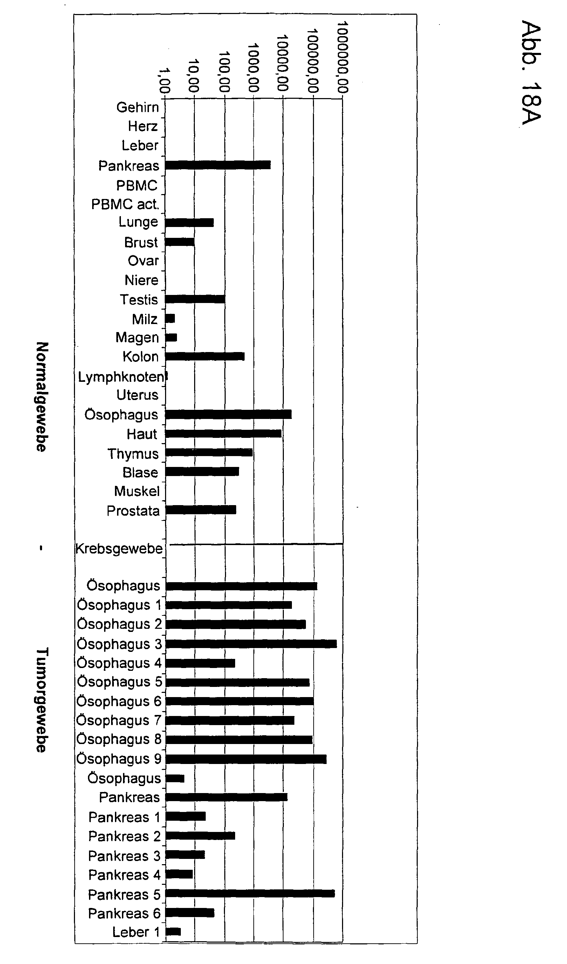

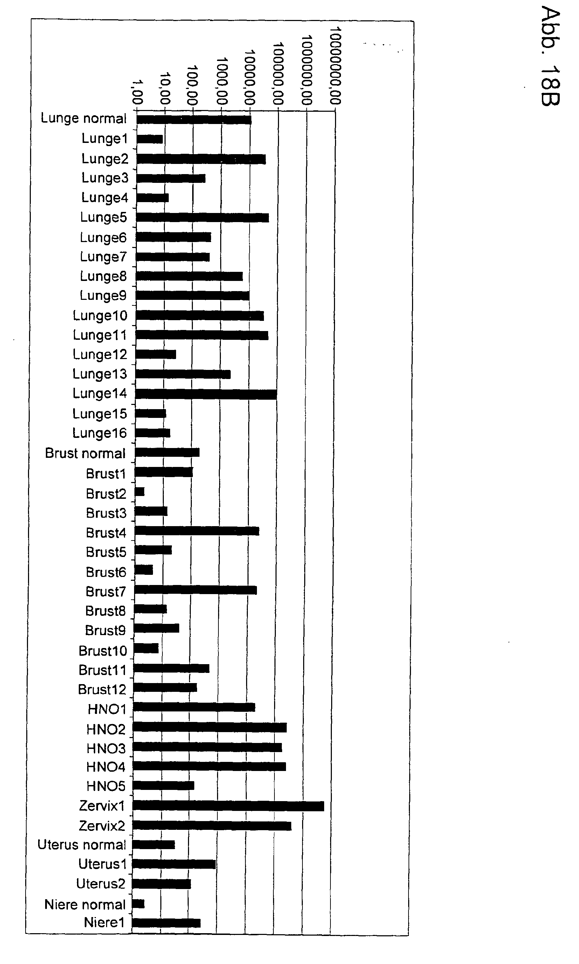

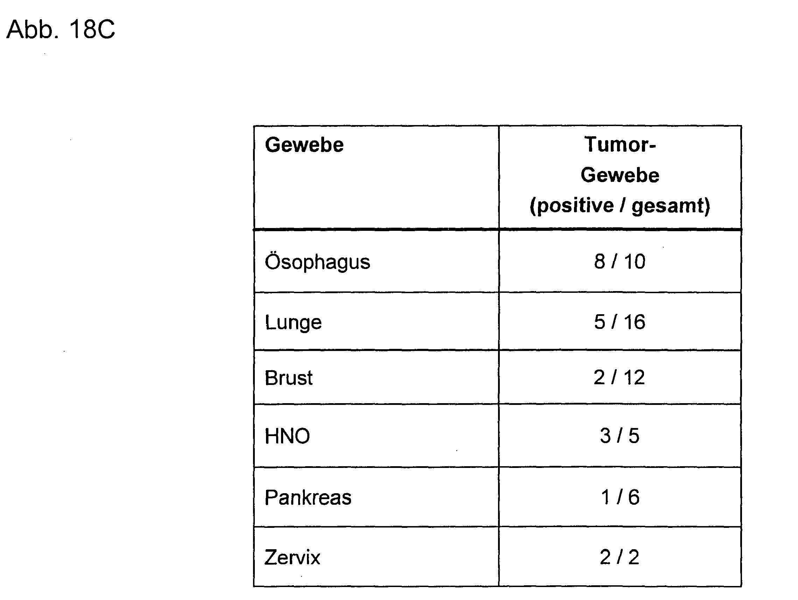

**Abb. 18****: qPCR-Analyse von CLCA2**
   **A:** Quantitative Expressionsanalyse von CLCA2 in Normalgeweben (links) und verschiedenen Tumoren (Pools bestehend aus jeweils 3-4 Einzelproben, rechts) in logarithmischer Darstellung der relativen Expression (-fache Aktivierung).
   **B:** Quantitative Expressionsanalyse von CLCA2 in verschiedenen Lungen-, HNO-, Brust-, Zervix- und Uterustumoren im Vergleich zur Expression in den zugehörigen Normalgeweben. Logarithmische Darstellung.
   **C:** Zusammenfassung der CLCA2-spezifischen Expression in verschiedenen analysierten Tumoren. Angegeben ist die Anzahl positiv getesteter Tumorproben/Gesamtzahl der analysierten Tumorproben. Während alle untersuchten somatischen Normalgewebe eine signifikant niedrigere Expression von CLCA2 aufweisen, ist das Gen in sehr vielen Tumoren mit unterschiedlicher Häufigkeit überexprimiert.

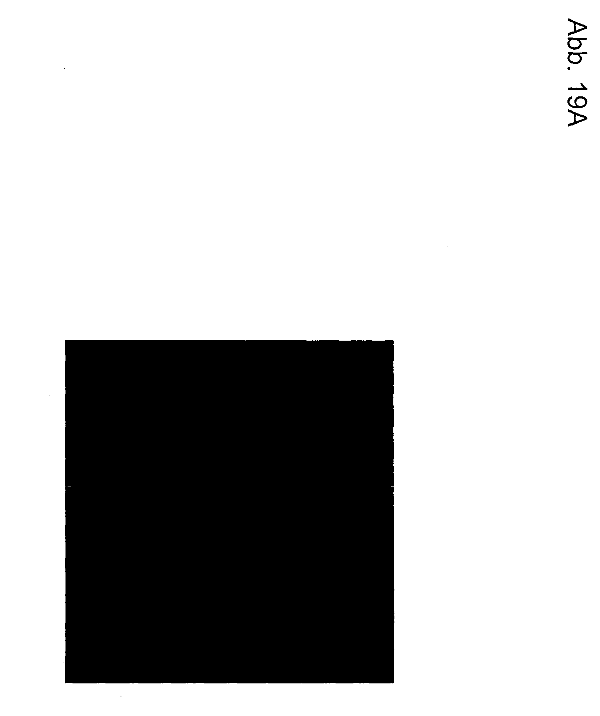

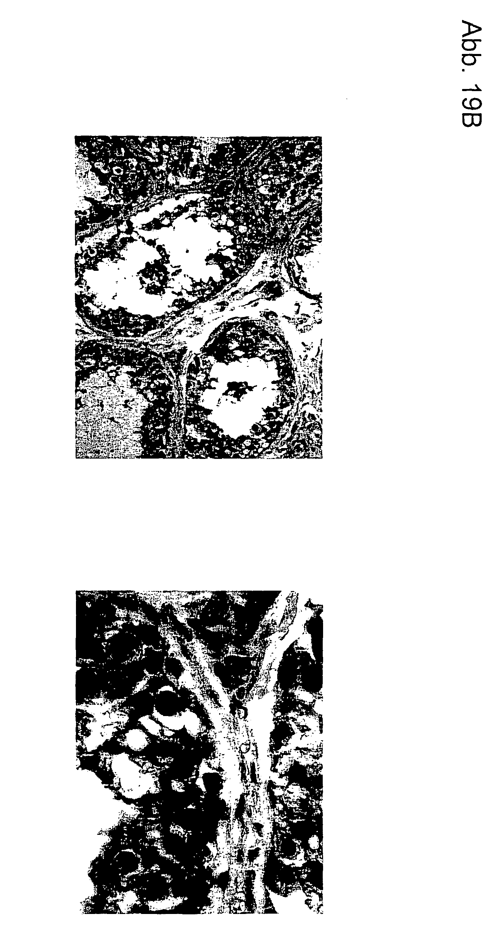

**Abb. 19****: Protein-Lokalisation**
   **A:** Darstellung der membranständigen Lokalisation des CLCA2-Proteins an CLCA2-transfizierten Zellen.
   **B:** Die Abbildung zeigt die immunhistochemische Analyse des CLCA2-Proteins.

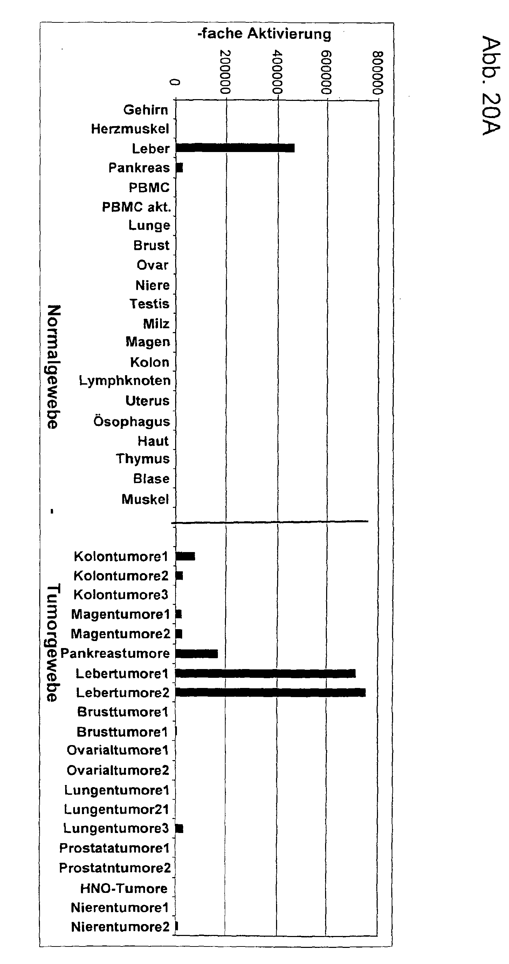

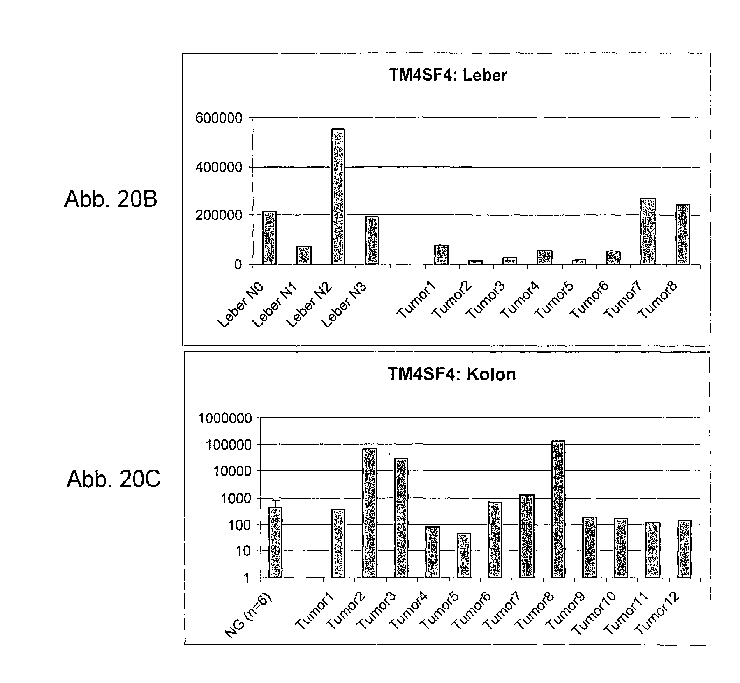

**Abb. 20****: qPCR-Analyse von TM4SF4**
   **A:** Quantitative Expressionsanalyse von TM4SF4 in Normalgeweben (links) und verschiedenen Tumoren (Pools bestehend aus jeweils 3-4 Einzelproben, rechts) in linearer Darstellung der relativen Expression (-fache Aktivierung).
   **B**: Quantitative Expressionsanalyse von TM4SF4 in verschiedenen Lebertumoren im Vergleich zu 4 verschiedenen Normalgeweben der Leber (NO bis N3); lineare Darstellung. **C:** Logarithmische Darstellung der relativen Expression von TM4SF4 in 12 verschiedenen Kolontumoren im Vergleich zu normalen Kolon-Proben (NG: Normalgewebe; 6 verschiedene Normalgewebe untersucht).

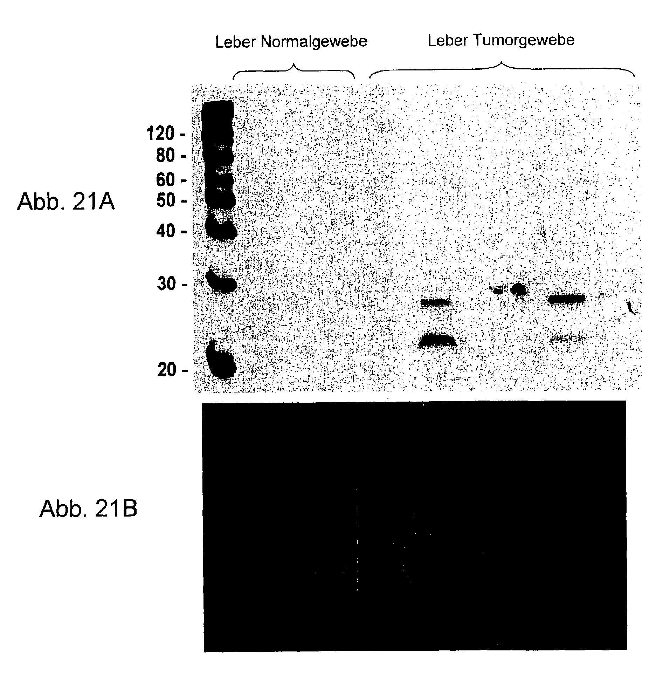

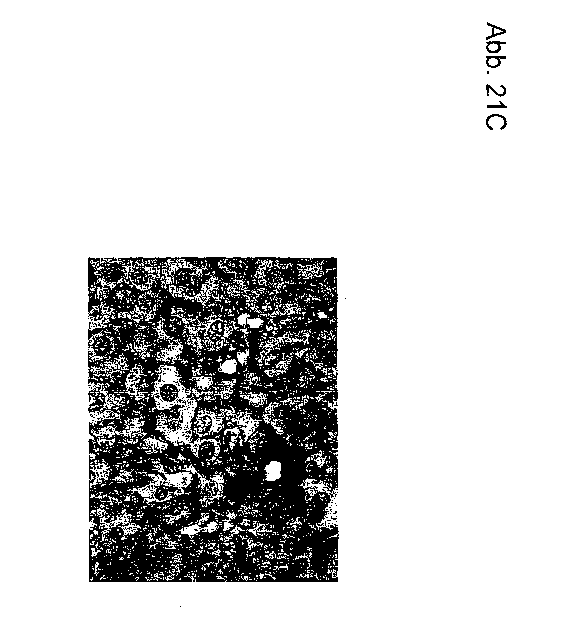

**Abb. 21****: Protein-Analyse**
   **A:** Die Darstellung zeigt einen Immunblot mit TM4SF4-spezifischen Antikörpern an normalem Lebergewebe und Leber-Tumorgewebe. Zu erkennen sind 2 putative Glykosilierungs größen.
   **B:** Die Abbildung zeigt die membranständige Lokalisation des TM4SF4-Proteins an TM4SF4-transfizierten Zellen.
   **C:** Die immunhistochemische Analyse konnte die in der PCR gezeigte Expressionsselektivität bestätigen.

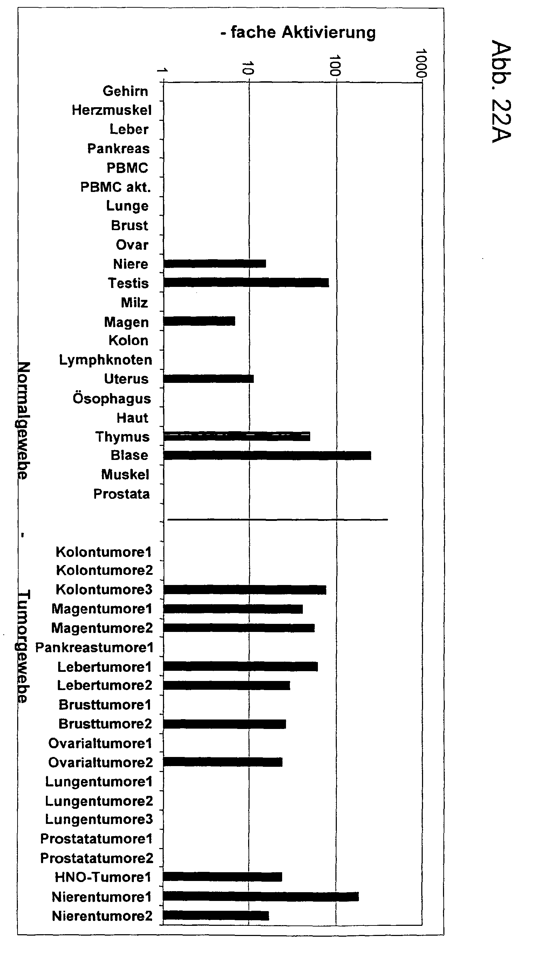

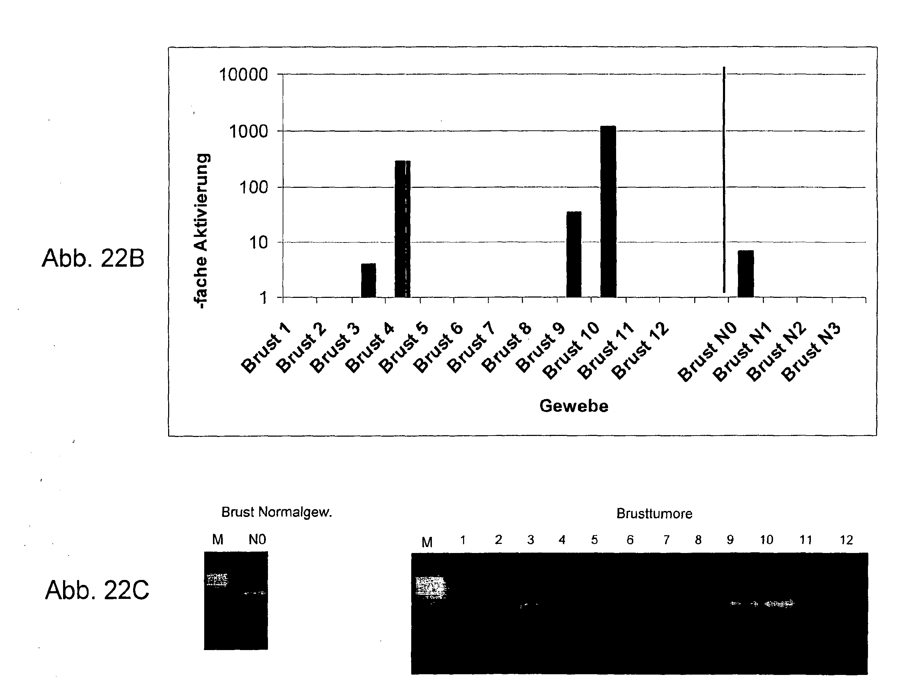

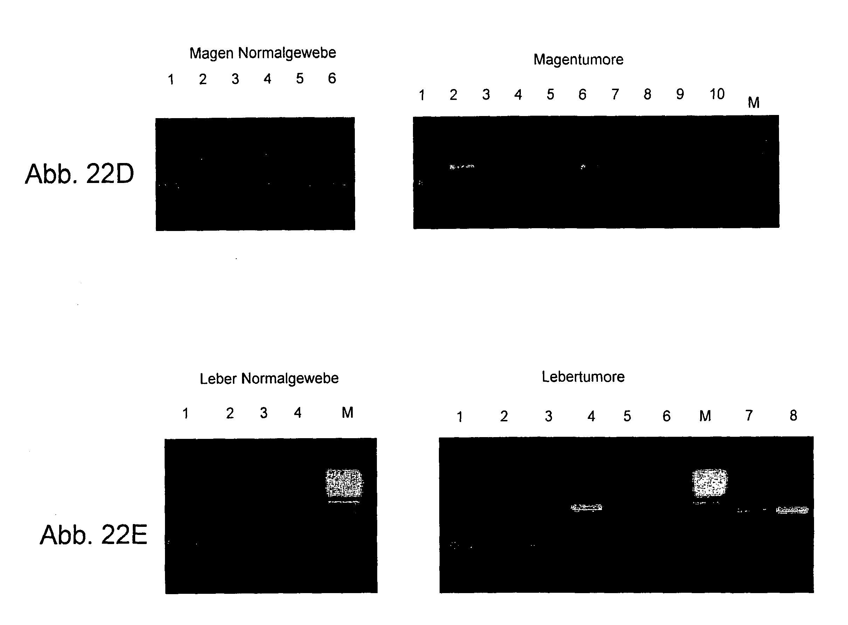

**Abb. 22****: Quantitative Expressionsanalyse von Claudin19**
   **A:** Quantitative Expressionsanalyse von Claudin19 in Normalgewebe (links) und in verschiedenen Tumoren (Pools bestehend aus jeweils 3-4 Einzelproben, rechts) in logarithmischer Darstellung der relativen Expression (-fache Aktivierung).
   **B:** Quantitative Expressionsanalyse von Claudin19 in verschiedenen Brusttumoren und zugehörigen Brust-Normalgeweben.
   **C:** Konventionelle RT-PCR mit Analyse von Claudin19 in verschiedenen Brusttumor-Proben sowie in einem Normalgewebe; M: DNA-Längenmarker.
   **D:** Konventionelle RT-PCR-Analyse von Claudin19 in verschiedenen Magen-Normalgeweben und Magentumoren.
   E: Konventionelle RT-PCR-Analyse von Claudin19 in verschiedenen Leber-Normalgeweben und Lebertumoren; M: DNA-Längenmarker.

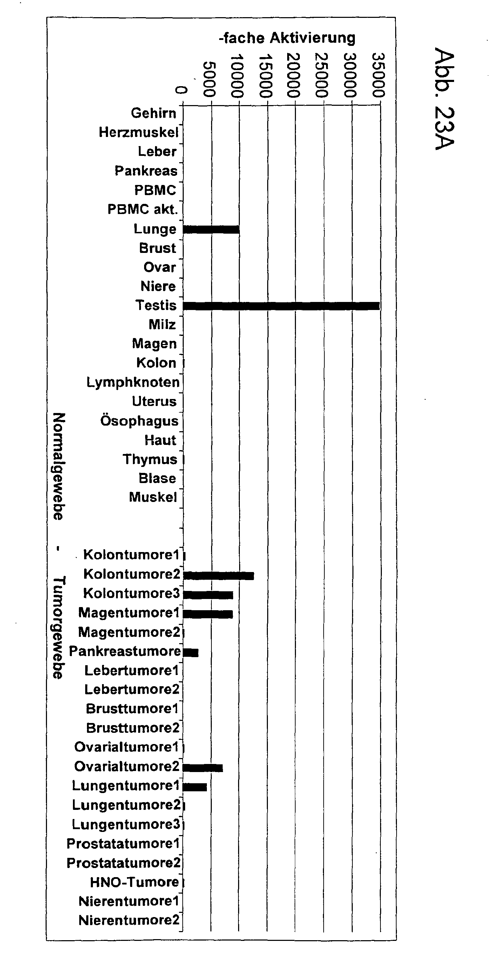

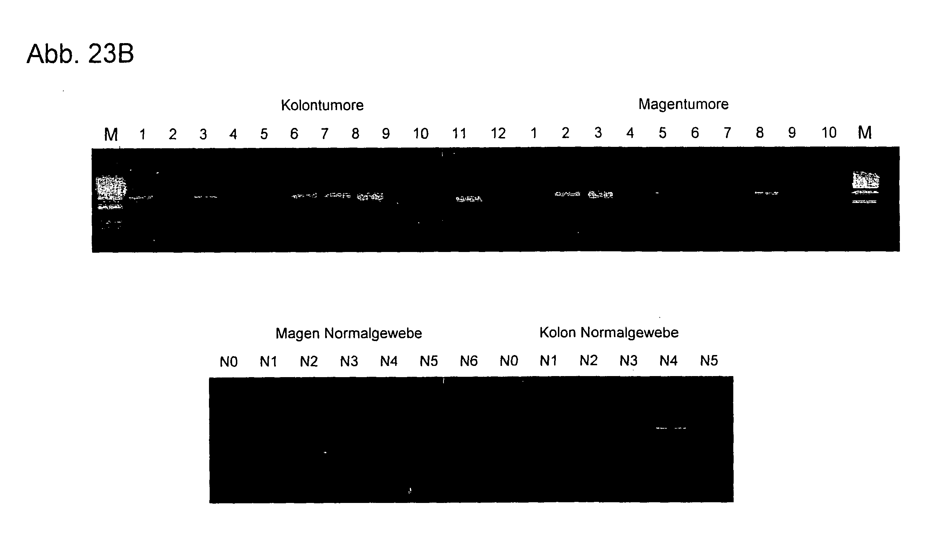

**Abb. 23****: qRT-PCR-Analyse von ALPPL2**
   **A:** Quantitative Expressionsanalyse von ALPPL2 in Normalgeweben (links) und in Tumoren (Pools bestehend aus jeweils 3-4 Einzelproben, rechts) in linearer Darstellung der relativen Expression (-fache Aktivierung).
   **B:** Gelbild einer konventionellen RT-PCR-Analyse von ALPPL2 in verschiedenen Kolon- und Magentumoren sowie den jeweils zugehörigen Normalgeweben nach gelelektrophoretischer Auftrennung; M: DNA-Längenmarker.

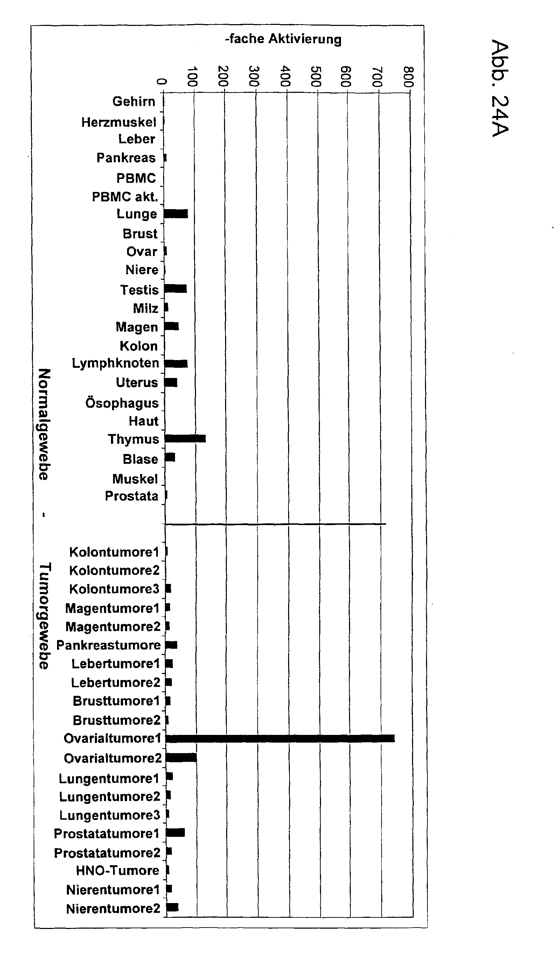

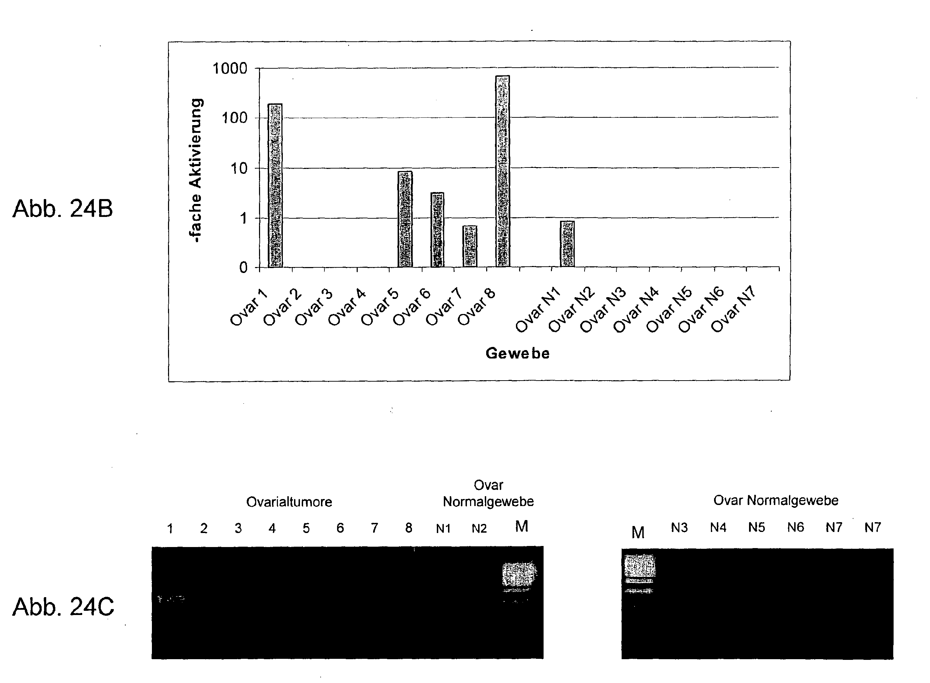

**Abb. 24****: Quantitative RT-PCR-Analyse des G-Protein-gekoppelten Rezeptors 64 (GPR64)**
   **A:** Quantitative Expressionsanalyse von GPR64 in Normalgeweben (links) und in Tumoren (Pools bestehend aus jeweils 3-4 Einzelproben, rechts) in linearer Darstellung der relativen Expression (-fache Aktivierung).
   **B:** Quantitative Expressionsanalyse von GPR64 in verschiedenen Ovarialtumoren und zugehörigen Ovarial-Normalgeweben.
   **C:** Gelbild einer RT-PCR-Analyse von GPR64 in verschiedenen Ovarialtumoren und Normalgeweben; M: DNA-Längenmarker.

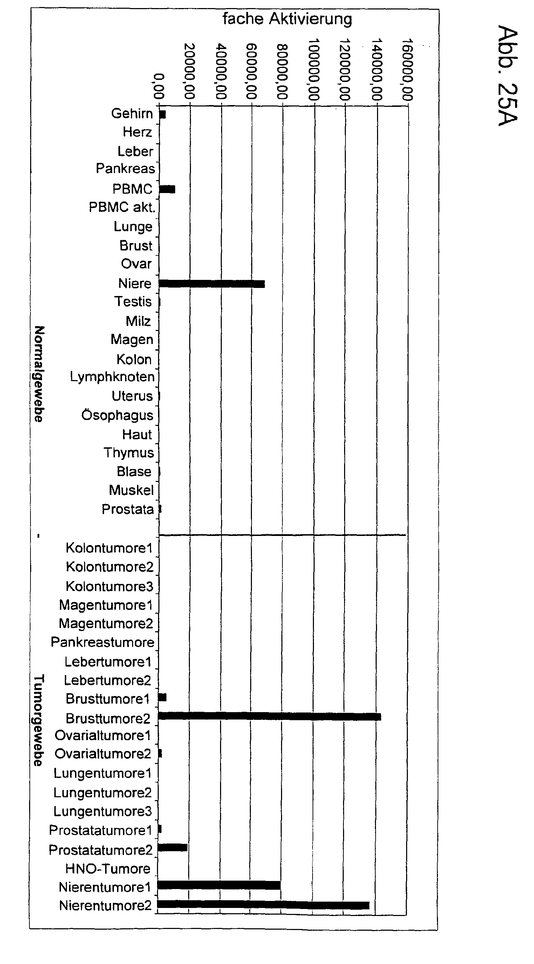

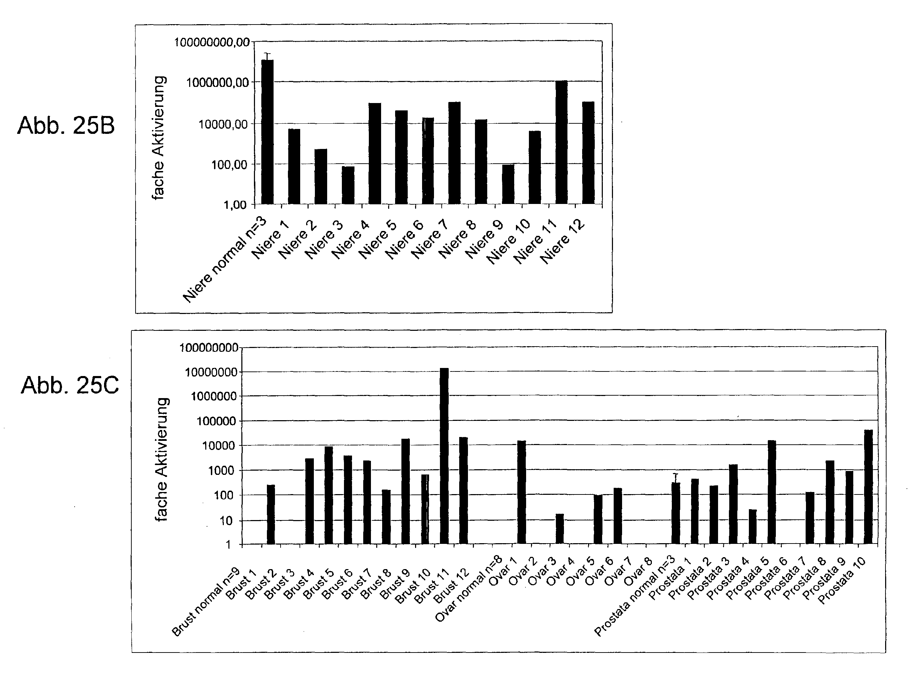

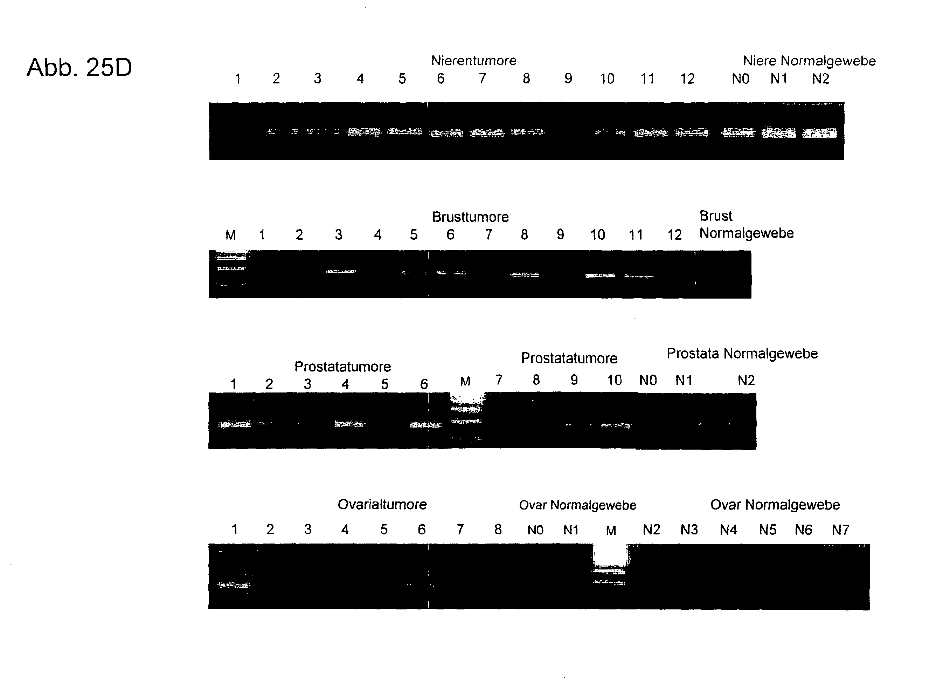

**Abb. 25****: Quantitative RT-PCR-Analyse von SLC12A1**
   **A:** Quantitative Expressionsanalyse von SLC12A1 in Normalgeweben (links) und in Tumoren (Pools bestehend aus jeweils 3-4 Einzelproben, rechts) in linearer Darstellung der relativen Expression (-fache Aktivierung).
   **B:** Quantitative Expressionsanalyse von SLC12A1 in 12 verschiedenen Nierentumoren im Vergleich zur Expression in der normalen Niere (n=3).
   **C:** Quantitative Expressionsanalyse von SLC12A1 in Brust-, Ovarial- und Prostatatumoren im Vergleich zur Expression in den zugehörigen Normalgeweben (Brust: n=9, Ovar: n=8, Prostata: n=3). Logarithmische Darstellungen.
   **D:** Konventionelle RT-PCR-Analyse von SLC12A1 in Nierentumoren, verschiedenen normalen Nieren und verschiedenen Tumortypen (Brust, Prostata, Ovar) mit den zugehörigen Normalgeweben.

### Beispiele

### Material und Methoden

Die Begriffe *"in silico* ", und "elektronisch" beziehen sich rein auf die Nutzung von auf Datenbanken beruhenden Verfahren, mit denen auch Labor-experimentelle Vorgänge simuliert werden können.

Alle anderen Begriffe und Termini sind, falls nicht explizit anders definiert, so verwendet, wie sie der Fachmann versteht. Die genannten Techniken und Verfahren erfolgen in an sich bekannter Weise und sind z.B. in Sambrook et al., Molecular Cloning: A Laboratory Manual, 2. Auflage (1989), Cold Spring Harbor Laboratory Press, Cold Spring Harbor, N.Y. beschrieben. Alle Verfahren, die die Verwendung von Kits und Reagenzien einschließen, sind entsprechend den Angaben der Hersteller durchgeführt.

### A. Datamining-basierte Strategie zur Identifizierung von Tumor-assoziierten Antigenen

Erfindungsgemäß wurden öffentliche humane Protein- und Nukleinsäuredatenbanken im Hinblick auf krebsspezifische Antigene untersucht, die auf der Zelloberfläche zugänglich sind. Die Definition der dafür notwendigen Filterkriterien zusammen mit einer Hochdurchsatz-Methodik zur Analyse möglichst aller Proteine bildet den zentralen Bestandteil dieser Strategie.

Den Ausgangspunkt bildeten die validierten Proteineinträge (NP-) bzw. die korrespondierenden mRNAs (NM-) in der "RefSeq" Datenbank (Pruitt et al., Trends Genet. Jan;16(1):44-47, 2000) des "National Center for Biotechnology Information" (NCBI). Dem Grundprinzip Gen zu mRNA zu Protein folgend wurden die Proteine zunächst auf das Vorhandensein einer oder mehrerer Transmembrandomänen hin untersucht. Hierbei kam das Proteinanalyseprogramm TMHMM Server v. 2.0 (Krogh et al., Journal of Molecular Biology 305(3):567-580, 2001) zum Einsatz, dessen Ergebnisse anschließend noch einmal mit dem Programm ALOM 2 (Nakai et al., Genomics 14:897-911, 1992) verifiziert wurden. Die Prädiktion weiterer Signalsequenzen, die die intrazelluläre Lokalisation von Proteinen beeinflussen, wurde mit den Programmen PSORT II (Horton et al., Intelligent Systems for Molecular Biology 4: 109-115, 1996) und iPSORT (Bannai et al., Bioinformatics, 18(2) 298-305, 2002) durchgeführt. Aus der humanen NP-Fraktion mit insgesamt 19.110 Einträgen wurden auf diese Weise 4.634 Proteine filtriert.

Die korrespondierende mRNA jedes dieser 4.634 Proteine wurde anschließend einer Homologiesuche in der EST-Datenbank (Boguski et al., Nat. Genet. 4(4):332-333, 1993) des NCBI mit Hilfe des "BLAST" Algorithmus (Altschul et al., Nucleic Acids Res.25:3389-3402, 1997) unterzogen. Die Filterkriterien wurden bei dieser Suche mit einem e-Value < 10e-20 und einer minimalen Sequenzidentität von 93% so eingestellt, dass die daraus resultierenden Treffer mit sehr hoher Wahrscheinlichkeit nur aus der jeweiligen mRNA und nicht aus homologen Transkripten hervorgegangen sein können. Fast alle mRNAs erzielten dabei mindestens einen Treffer in der EST-Datenbank, wobei die Anzahl der Treffer in Einzelfällen mehr als 4.000 betrug.

Für jeden Einzelnen dieser validen Treffer wurde anschließend die gewebsspezifische Herkunft der zugrunde liegenden cDNA-Bibliothek sowie der Name der Bibliothek ermittelt. Die daraus resultierenden Gewebe wurden in vier verschiedene Gruppen eingeteilt, die von dispensiblen Organen (Gruppe 3) bis hin zu absolut lebensnotwendigen Organen reichten (Gruppe 0). Eine weitere Gruppe 4 bildeten alle Proben, die aus Krebsgewebe gewonnen wurden. Die Verteilung der Treffer auf die fünf Gruppen wurde in einer Tabelle festgehalten, die nach dem besten Verhältnis der Summe der Gruppen 3 und 4 gegenüber der Summe der Gruppen 0-2 sortiert wurde. Dabei erreichten diejenigen mRNAs einen Spitzenplatz, deren EST-Treffer ausschließlich Krebsgewebe entstammten, gefolgt von denjenigen, die darüber hinaus noch in Geweben dispensibler Organe der Gruppe 3 zu finden sind. Ein weiteres Kriterium für die Aussagekraft dieser Verteilung bildete die Anzahl der unabhängigen cDNA-Bibliotheken, aus denen die ESTs gewonnen wurden, und wurde in einer eigenen Spalte in der Tabelle festgehalten.

Da es sich bei den im ersten Ansatz ermittelten Transkripten und den korrespondierenden Proteinen zunächst um hypothetische Konstrukte handelt, wurden noch weitere Filterkriterien hinzugezogen, die die reale Existenz der mRNAs und damit auch der Proteine belegen sollten. Dazu wurde jede mRNA mit Hilfe des Programms "Spidey" (Wheelan et al., Genome Res. 11(11):1952-1957, 2001) mit dem vorhergesagten Genlocus verglichen. Nur diejenigen Transkripte, die mindestens einen Spleißvorgang aufweisen, d.h. die sich auf mindestens 2 Exons verteilen, wurden für weitergehende Analysen verwendet.

Die sequenzielle Anwendung aller genannten Filter führte zu den erfindungsgemäßen Tumor-assoziierten Antigenen, die aufgrund einer vorhergesagten Transmembrandomäne und der damit verbundenen Topologie als von extrazellulär zugänglich anzusehen sind. Das aus den EST-Daten abgeleitete Expressionsprofil weist in allen Fällen auf eine krebsspezifische Expression hin, die sich höchstens noch auf dispensible Organe erstrecken kann.

### B. Validierungsstrategie der durch in silico-Analyse identifizierten Tumor-assoziierten Antigene

Zur Nutzung der Antigene für immuntherapeutische Zwecke (Antikörpertherapie mittels monoklonaler Antikörper, Vakzinierung, T-Zell-Rezeptor-vermittelte therapeutische Ansätze; vgl. EP-B-0 879 282) bei der Krebstherapie sowie für diagnostische Fragestellungen ist die Validierung der erfindungsgemäß identifizierten Antigene von zentraler Bedeutung. Die Validierung erfolgt dabei durch Expressionsanalyse sowohl auf RNA- als auch auf Proteinebene.

### 1. Untersuchung der RNA-Expression

Die erste Charakterisierung der identifizierten Tumorantigene erfolgt mit Hilfe von RNA, die aus verschiedenen Geweben bzw. aus gewebespezifischen Zelllinien gewonnen wird. Weil das differentielle Expressionsmuster aus gesundem Gewebe im Vergleich zu Tumorgewebe eine entscheidende Bedeutung für die spätere therapeutische Anwendung hat, erfolgt die Charakterisierung der Zielgene bevorzugt mit Hilfe dieser Gewebeproben.

Die Isolierung von Gesamt-RNA aus nativen Gewebeproben oder aus Tumorzelllinien erfolgt mit Verfahren, die in der Molekularbiologie Standard sind. Zum Beispiel kann die Isolierung mit Hilfe des RNeasy Maxi Kits (Qiagen, Kat. Nr. 75162) nach Vorschrift durch den Hersteller erfolgen. Dieses Isolierungsverfahren beruht auf der Verwendung von Guanidiniumisothiocyanat als chaotropes Reagenz. Alternativ kann die Isolierung mit saurem Phenol durchgeführt werden (Chomczynski & Sacchi, Anal. Biochem. 162: 156-159, 1987). Nach Aufarbeitung des Gewebes mittels Guanidiniumisothiocyanat wird die RNA mit saurem Phenol extrahiert, anschließend die RNA mit Isopropanol gefällt und in DEPC-behandeltem Wasser aufgenommen.

2-4 µg der so isolierten RNA werden anschließend z.B. mittels Superscript II (Invitrogen) entsprechend dem Protokoll des Herstellers in cDNA umgeschrieben. Das Priming der cDNA-Synthese erfolgt dabei mit Hilfe von zufälligen Hexameren (z.B. Roche Diagnostics) nach Standardprotokollen des jeweiligen Herstellers. Zur Qualitätskontrolle werden die cDNAs mit Primem in 30 Zyklen amplifiziert, die spezifisch für das nur gering exprimierte p53 Gen sind. Nur p53-positive cDNA-Proben werden für die weiteren Reaktionsschritte verwendet.

Zur detaillierten Antigenanalyse wird auf Basis eines eDNA-Archivs, das aus verschiedenen Normal- und Tumorgeweben sowie aus Tumorzelllinien isoliert wurde, eine Expressionsanalyse mittels PCR bzw. quantitativer PCR (qPCR) durchgeführt. Dazu werden 0,5 µl cDNA aus dem obigen Ansatz mit einer DNA-Polymerase (z.B. 1 U HotStarTaq DNA-Polymerase, Qiagen) analog den Protokollen des jeweiligen Herstellers amplifiziert (Gesamtvolumen des Ansatzes: 25-50 µl). Neben der Polymerase enthält der Amplifikationsansatz 0,3 mM dNTPs, Reaktionsbuffer (Endkonzentration 1 x, abhängig vom Hersteller der DNA-Polymerase) und je 0,3 mM des genspezifischen "sense"- und "antisense"-Primers.

Die spezifischen Primer des Zielgens werden, soweit möglich, so ausgewählt, dass sie in zwei unterschiedlichen Exons liegen und somit genomische Kontaminationen nicht zu falsch positiven Ergebnissen führen. Bei einer nicht quantitativen Endpunkt-PCR wird die cDNA typischerweise 15 Minuten bei 95°C inkubiert, um die DNA zu denaturieren und um das Hot-Start-Enzym zu aktivieren. Anschließend wird die DNA in 35 Zyklen amplifiziert (1 min 95°C, 1 min Primer-spezifische Hybridisierungstemperatur (ca. 55-65°C), 1 min 72°C zur Elongation der Amplifikate). 10 µl des PCR Ansatzes werden anschließend auf Agarosegelen aufgetragen und im elektrischen Feld aufgetrennt. Durch Färben mit Ethidiumbromid wird die DNA in den Gelen sichtbar gemacht und das Ergebnis der PCR durch ein Foto dokumentiert.

Alternativ zur konventionellen PCR kann die Expressionsanalyse eines Zielgens auch durch quantitative "real time"-PCR erfolgen. Zu dieser Analyse sind inzwischen verschiedene Analysesysteme erhältlich, die bekanntesten sind das ABI 7900HT Sequence detection system (Applied Biosystems), der iCycler (Biorad) sowie der Light cycler (Roche Diagnostics). Wie oben beschrieben wird ein spezifischer PCR-Ansatz einem Lauf in einem der "real time"-PCR-Geräte unterzogen. Durch Zusatz eines DNA-interkalierenden Farbstoffes (z.B. Ethidiumbromid, SYBRGreen) wird die neu synthetisierte DNA durch spezifische Lichtanregung (nach Angaben der Farbstoffhersteller) sichtbar gemacht. Durch eine Vielzahl von Messpunkten während der Amplifikation kann der gesamte Prozess verfolgt und eine quantitative Bestimmung der Nukleinsäurekonzentration des Zielgens durchgeführt werden. Die Normalisierung des PCR-Ansatzes erfolgt durch Messung eines "housekeeping Gens" (z.B. 18S RNA, β-Actin, GAPDH). Alternative Strategien über Fluoreszenz-markierte DNA-Sonden erlauben ebenfalls die quantitative Bestimmung des Zielgens aus einer spezifischen Gewebeprobe (siehe TaqMan-Applikationen der Fa. Applied Biosystems).

### 2. Klonierung

Die Klonierung des gesamten Zielgens, die für die weitere Charakterisierung des Tumorantigens notwendig ist, erfolgt nach gängigen molekularbiologischen Verfahren (z.B. in "Current Protocols in Molecular Biology", John Wiley & Sons Ltd., Wiley InterScience). Zur Klonierung bzw. Sequenzanalyse des Zielgens wird dieses zunächst mit einer DNA-Polymerase mit "proof reading-Funktion" (z.B. pfu, Roche Diagnostics) amplifiziert. Das Amplifikat wird anschließend mit Standardverfahren in einen Klonierungsvektor ligiert. Positive Klone werden durch Sequenzanalyse identifiziert und anschließend mit Hilfe von Prädiktionsprogrammen und bekannten Algorithmen charakterisiert.

### 3. Gewinnung von Antikörpern

Die Charakterisierung der erfindungsgemäß identifizierten Tumor-assoziierten Antigene erfolgt beispielsweise durch die Verwendung von Antikörpern. Ferner umfasst die Erfindung die diagnostische oder therapeutische Verwendung von Antikörpern. Dabei können Antikörper Proteine in nativem und/oder denaturiertem Zustand erkennen (Anderson et al., J. Immunol. 143: 1899-1904, 1989; Gardsvoll, J. Immunol. Methods 234: 107-116, 2000; Kayyem et al., Eur. J. Biochem. 208: 1-8, 1992; Spiller et al., J. Immunol. Methods 224: 51-60, 1999).

Antiseren, die spezifische Antikörper enthalten, die an das Zielprotein spezifisch binden, können über verschiedene Standardverfahren hergestellt werden; vgl. beispielsweise "Monoclonal Antibodies: A Practical Approach" von Philip Shepherd, Christopher Dean ISBN 0-19-963722-9, "Antibodies: A Laboratory Manual" von Ed Harlow, David Lane ISBN: 08796933142 und "Using Antibodies: A Laboratory Manual: Portable Protocol NO" von Edward Harlow, David Lane, Ed Harlow ISBN: 0879695447. Dabei ist auch möglich, affine und spezifische Antikörper zu generieren, die komplexe Membranproteine in ihrer nativen Form erkennen (Azorsa et al., J. Immunol. Methods 229: 35-48, 1999; Anderson et al., J. Immunol. 143: 1899-1904, 1989; Gardsvoll, J. Immunol. Methods. 234: 107-116, 2000). Dies ist vor allem für die Herstellung von therapeutischen Antikörpern von Bedeutung, hat aber auch für viele diagnostische Anwendungen eine hohe Bedeutung. Dazu kann sowohl mit dem gesamten Protein als auch mit extrazellulären Teilsequenzen immunisiert werden.

### Immunisierung und Gewinnung von polyklonalen Antikörpern

Verschiedenste Immunisierungsprotokolle sind publiziert. Eine Spezies (z.B. Kaninchen, Mäuse) wird durch eine erste Injektion des gewünschten Zielproteins immunisiert. Durch eine zweite oder dritte Immunisierung innerhalb eines definierten Zeitraums (ca. 2-4 Wochen nach der vorangegangenen Immunisierung) lässt sich die Immunantwort des Tieres gegen das Immunogen verstärken. Wiederum nach verschiedenen definierten Zeitabständen (1. Blutung nach 4 Wochen, anschließend alle 2-3 Wochen bis zu 5 Entnahmen) wird den Tieren Blut entnommen und Immunserum gewonnen. Die so entnommenen Immunseren enthalten polyklonale Antikörper, mit denen das Zielprotein im Western Blot, durch die Durchflusszytometrie, Immunfluoreszenz oder Immunhistochemie nachgewiesen und charakterisiert werden kann.

Die Immunisierung der Tiere erfolgt in der Regel über eines von vier gut etablierten Verfahren, wobei auch andere Verfahren existieren. Immunisiert werden kann dabei mit für das Zielprotein spezifischen Peptiden, dem gesamten Protein, mit extrazellulären Teilsequenzen eines Proteins, das experimentell oder über Prädiktionsprogramme identifiziert werden kann. Da die Prädiktionsprogramme nicht immer fehlerfrei arbeiten, wird u.U. auch mit zwei Domänen gearbeitet, die voneinander durch eine Transmembrandomäne getrennt sind. Eine der beiden Domänen muss dann extrazellulär sein, was dann experimentell belegt werden kann (siehe nachstehend). Die Durchführung einer Immunisierung wird als Service von verschiedenen Dienstleistern kommerziell angeboten.
(1) Im ersten Fall werden Peptide (Länge: 8-12 Aminosäuren) über *in vitro*-Verfahren synthetisiert (durch einen kommerziellen Service möglich) und diese Peptide zur Immunisierung verwendet. In der Regel erfolgen 3 Immunisierungen (z.B. mit einer Konzentration von 5-100 µg/Immunisierung).
(2) Alternativ kann die Immunisierung durch rekombinante Proteine erfolgen. Dazu wird die klonierte DNA des Zielgens in einen Expressionsvektor kloniert und das Zielprotein analog den Bedingungen des jeweiligen Herstellers (z.B. Roche Diagnostics, Invitrogen, Clontech, Qiagen) z.B. zellfrei *in vitro,* in Bakterien (z.B. E. *coli*), in Hefe (z.B. *S. pombe),* in Insektenzellen oder in Säugetierzellen synthetisiert. Dabei ist auch die Synthese des Zielproteins mit Hilfe von viralen Expressionssystemen möglich (z.B. Baculovirus, Vacciniavirus, Adenovirus). Nach Synthese in einem der Systeme wird das Zielprotein aufgereinigt. Die Aufreinigung erfolgt dabei in der Regel über chromatographische Verfahren. Dabei können auch Proteine für die Immunisierung verwendet werden, die über einen molekularen Anker als Hilfsmittel zur Reinigung verfügen (z.B. His-Tag, Qiagen; FLAG-Tag, Roche Diagnostics; GST-Fusionsproteine). Eine Vielzahl von Protokollen befindet sich z.B. in den "Current Protocols in Molecular Biology", John Wiley & Sons Ltd., Wiley InterScience. Nach Reinigung des Zielproteins erfolgt eine Immunisierung wie vorstehend beschrieben.
(3) Falls eine Zelllinie zur Verfügung steht, die das gewünschte Protein endogen synthetisiert, kann auch diese Zelllinie direkt zur Herstellung des spezifischen Antiserums verwendet werden. Die Immunisierung erfolgt dabei in 1-3 Injektionen mit jeweils ca. 1-5 x 10⁷ Zellen.
(4) Die Immunisierung kann auch durch Injektion von DNA (DNA-Immunisierung) erfolgen. Dazu wird das Zielgen zunächst in einen Expressionsvektor kloniert, so dass die Zielsequenz unter der Kontrolle eines starken eukaryontischen Promotors steht (z.B. CMV-Promotor). Anschließend wird DNA (z.B. 1-10 µg pro Injektion) als Immunogen mit einer "gene gun" in stark durchblutete, kapillare Bereiche eines Organismus transferiert (z.B. Maus, Kaninchen). Die transferierte DNA wird von Zellen des Tieres aufgenommen, das Zielgen wird exprimiert und das Tier entwickelt schließlich eine Immunantwort gegen das Zielprotein (Jung et al., Mol. Cells 12: 41-49,2001; Kasinrerk et al., Hybrid Hybridomics 21: 287-293, 2002).

### Gewinnung monoklonaler Antikörper

Monoklonale Antikörper werden traditionell mit Hilfe der Hybridoma-Technologie hergestellt (Technische Details: siehe "Monoclonal Antibodies: A Practical Approach" von Philip Shepherd, Christopher Dean ISBN 0-19-963722-9; "Antibodies: A Laboratory Manual" von Ed Harlow, David Lane ISBN: 0879693142, "Using Antibodies: A Laboratory Manual: Portable Protocol NO" von Edward Harlow, David Lane, Ed Harlow ISBN: 0879695447). Als ein neues Verfahren wird auch die so genannte "SLAM"-Technologie eingesetzt. Hierbei werden B-Zellen aus Vollblut isoliert und die Zellen monoklonalisiert. Anschließend wird der Überstand der vereinzelten B-Zelle auf ihre Antikörperspezifität hin analysiert. Im Gegensatz zur Hybridomatechnologie wird anschließend die variable Region des Antikörpergens durch eine Einzelzell-PCR amplifiziert und in einen geeigneten Vektor kloniert. Auf diese Art und Weise wird die Gewinnung von monoklonalen Antikörpern beschleunigt (de Wildt et al. J. Immunol. Methods 207:61-67, 1997).

### 4. Validierung der Targets mit proteinchemischen Verfahren unter Verwendung von Antikörpern

Mit den Antikörpern, die wie vorstehend beschrieben herstellbar sind, lassen sich eine Reihe von wichtigen Aussagen zu dem Zielprotein treffen. Im Einzelnen sind die nachstehenden Analysen zur Validierung des Zielproteins sinnvoll:

### Spezifität des Antikörpers

Um zu zeigen, dass ein Antikörper spezifisch nur an das gewünschte Zielprotein bindet, eignen sich am besten auf Zellkultur-basierende Tests mit anschließendem Western Blot (verschiedene Variationen sind z.B. in "Current Protocols in Proteinchemistry", John Wiley & Sons Ltd., Wiley InterScience, beschrieben). Für den Nachweis werden Zellen mit einer cDNA für das Zielprotein transfiziert, die unter Kontrolle eines starken eukaryontischen Promotors steht (z.B. Cytomegalievirus-Promotor; CMV). Zur Transfektion von Zelllinien mit DNA sind die verschiedensten Verfahren (z.B. Elektroporation, auf Liposomen basierende Transfektion, Kalziumphosphatpräzipitation) gut etabliert (z.B. Lemoine et al., Methods Mol. Biol. 75: 441-7, 1997). Alternativ können auch Zelllinien verwendet werden, die das Zielgen endogen exprimieren (Nachweis über Zielgen-spezifische RT-PCR). Zur Kontrolle werden im Experiment im Idealfall homologe Gene gleichzeitig transfiziert, um im folgenden Western Blot die Spezifität des analysierten Antikörpers nachweisen zu können.

Im anschließenden Western Blot werden Zellen aus Zellkultur oder Gewebeproben, die das Zielprotein enthalten könnten, in einer 1%igen SDS Lösung lysiert und die Proteine dabei denaturiert. Die Lysate werden auf 8-15%igen denaturierenden Polyacrylamidgelen (enthalten 1% SDS) der Größe nach elektrophoretisch aufgetrennt (SDS-Polyacrylamid Gelelektrophorese, SDS-PAGE). Anschließend werden die Proteine durch eines von mehreren Blotting-Verfahren (z.B. semi-dry Elektroblot; Biorad) auf eine spezifische Membran transferiert (z.B. Nitrozellulose, Schleicher & Schüll). Auf dieser Membran kann das gewünschte Protein sichtbar gemacht werden. Dazu wird die Membran zunächst mit dem Antikörper, der das Zielprotein erkennt (Verdünnung ca. 1:20-1:200, je nach Spezifität des Antikörpers), für 60 Minuten inkubiert. Nach einem Waschschritt wird die Membran mit einem zweiten, mit einem Marker (z.B. Enzyme wie Peroxidase oder alkalische Phosphatase) gekoppelten Antikörper inkubiert, der den ersten Antikörper erkennt. In einer Farb- oder chemilumineszenten Reaktion kann anschließend das Zielprotein auf der Membran sichtbar gemacht werden (z.B. ECL, Amersham Bioscience). Ein Antikörper mit einer hohen Spezifität für das Zielprotein sollte im Idealfall nur das gewünschte Protein selbst erkennen.

### Lokalisation des Zielproteins

Zur Bestätigung der im "*in silico*"-Ansatz identifizierten Membranlokalisation des Zielproteins werden verschiedene Verfahren verwendet. Ein wichtiges und gut etabliertes Verfahren unter Verwendung der vorstehend beschriebenen Antikörper ist die Immunfluoreszenz (IF). Dazu werden Zellen etablierter Zelllinien benutzt, die entweder das Zielprotein synthetisieren (Nachweis der RNA in der RT-PCR oder des Proteins im Western Blot) oder aber mit Plasmid-DNA transfiziert worden sind. Zur Transfektion von Zelllinien mit DNA sind die verschiedensten Verfahren (z.B. Elektroporation, auf Liposomen basierende Transfektion, Kalziumphosphatpräzipitation) gut etabliert (z.B. Lemoine et al., Methods Mol. Biol. 75: 441-7, 1997). Das transfizierte Plasmid kann bei der Immunfluoreszenz das unmodifizierte Protein kodieren oder aber auch unterschiedliche Aminosäuremarker an das Zielprotein koppeln. Die wichtigsten Marker sind z.B. das fluoreszierende "green fluorescent protein" (GFP) in seinen verschiedenen differentiell fluoreszierenden Formen, kurze Peptidsequenzen von 6-12 Aminosäuren, für die hoch affine und spezifische Antikörper zur Verfügung stehen, oder die kurze Aminosäuresequenz Cys-Cys-X-X-Cys-Cys, die über ihre Cysteine spezifische fluoreszierende Substanzen binden kann (Invitrogen). Zellen, die das Zielprotein synthetisieren, werden z.B. mit Paraformaldehyd oder Methanol fixiert. Anschließend können die Zellen bei Bedarf durch Inkubation mit Detergenzien (z.B. 0,2% Triton X-100) permeabilisiert werden. Anschließend werden die Zellen mit einem primären Antikörper inkubiert, der gegen das Zielprotein oder gegen einen der gekoppelten Marker gerichtet ist. Nach einem Waschschritt wird der Ansatz mit einem zweiten, mit einem fluoreszierenden Marker (z.B. Fluorescin, Texas Red; DAKO) gekoppelten Antikörper inkubiert, der an den ersten Antikörper bindet. Anschließend werden die so markierten Zellen mit Glycerin überschichtet und mit Hilfe eines Fluoreszenzmikroskops nach den Angaben des Herstellers analysiert. Spezifische Fluoreszenzemissionen werden dabei, abhängig von den eingesetzten Substanzen, durch spezifische Anregung erreicht. Die Analyse erlaubt in der Regel die sichere Lokalisation des Zielproteins, wobei zur Bestätigung der Antikörperqualität und des Zielproteins in Doppelfärbungen zusätzlich zum Zielprotein auch die gekoppelten Aminosäuremarker oder andere Markerproteine angefärbt werden, deren Lokalisation bereits in der Literatur beschrieben ist. Ein Sonderfall stellt das GFP und seine Derivate dar, die direkt angeregt werden können und selbst fluoreszieren. Die Membranpermeabilität, die durch den Einsatz von Detergenzien gesteuert werden kann, erlaubt in der Immunfluoreszenz die Demonstration, ob ein immunogenes Epitop innerhalb oder außerhalb der Zelle lokalisiert ist. Die Prädiktion der ausgewählten Proteine kann so experimentell untermauert werden. Alternativ kann der Nachweis von extrazellulären Domänen mittels Durchflusszytometrie erfolgen. Dazu werden Zellen unter nicht permeabilisierenden Bedingungen (z.B. mit PBS/Na-Azid/2% FCS/ 5 mM EDTA) fixiert und im Durchflusszytometer nach Angaben des Herstellers analysiert. Nur extrazelluläre Epitope können bei diesem Verfahren von dem zu analysierenden Antikörper erkannt werden. Im Unterschied zu Immunfluoreszenz kann durch Verwendung von z.B. Propidiumiodid oder Trypanblau zwischen toten und lebenden Zellen unterschieden werden und damit falsch positive Ergebnisse vermieden werden.

Ein weiterer wichtiger Nachweis erfolgt durch die Immunhistochemie (IHC) an spezifischen Gewebeproben. Ziel dieses Verfahrens ist es, die Lokalisation eines Proteins in einem funktionell intakten Gewebeverband zu identifizieren. Die IHC dient im einzelnen dazu, um (1) die Menge an Zielprotein in Tumor- und Normalgeweben abschätzen zu können, (2) zu analysieren, wie viele Zellen in Tumor- und gesundem Gewebe das Zielgen exprimieren, und (3) den Zelltyp in einem Gewebe (Tumor, gesunde Zellen) zu definieren, in dem das Zielprotein nachweisbar ist. Alternativ können die Proteinmengen eines Zielgens durch Gewebsimmunfluoreszenz mittels Digitalkamera und geeigneter Software (z.B. Tillvision, Till-photonics, Deutschland) quantifiziert werden. Die Technologie ist häufig publiziert worden, Details für Färbung und Mikroskopie sind daher z.B. "Diagnostic Immunohistochemistry" von David J., MD Dabbs ISBN: 0443065667 oder in "Microscopy, Immunohistochemistry, and Antigen Retrieval Methods: For Light and Electron Microscopy" ISBN: 0306467704 zu entnehmen. Zu beachten ist, dass aufgrund der Eigenschaften von Antikörpern unterschiedliche Protokolle verwendet werden müssen (nachstehend ist ein Beispiel beschrieben), um zu einem aussagekräftigen Ergebnis zu kommen.

In der Regel werden histologisch definierte Tumorgewebe und als Referenz vergleichbare gesunde Gewebe in der IHC eingesetzt. Als Positiv- und Negativkontrollen können dabei auch Zelllinien dienen, bei denen die Präsenz des Zielgens durch RT-PCR Analysen bekannt ist. Eine Hintergrundkontrolle ist immer mitzuführen.

Formalin-fixierte (ein anderes Fixierungsverfahren mit z.B. Methanol ist auch möglich) und in Paraffin eingebettete Gewebestücke mit einer Dicke von 4µm werden auf einem Glasträger aufgebracht und z.B. mit Xylol deparaffiniert. Die Proben werden mit TBS-T gewaschen und in Serum blockiert. Anschließend erfolgt die Inkubation mit dem ersten Antikörper (Verdünnung: 1:2 bis 1:2000) für 1-18 Stunden, wobei in der Regel affinitätsgereinigete Antikörper verwendet werden. Nach einem Waschschritt erfolgt eine ca. 30-60-minütige Inkubation mit einem zweiten Antikörper, der mit einer Alkalischen Phosphatase (alternativ: z.B. Peroxidase) gekoppelt und gegen den ersten Antikörper gerichtet ist. Anschließend erfolgt eine Farbreaktion unter Verwendung der Alkalischen Phosphatase (vgl. beispielsweise Shi et al., J. Histochem. Cytochem. 39: 741-748, 1991; Shin et al., Lab. Invest. 64: 693-702, 1991). Zum Nachweis der Antikörper-Spezifität kann die Reaktion durch vorherige Zugabe des Immunogens kompetitiert werden.

### Analyse von Proteinmodifikationen

Sekundäre Proteinmodifikationen wie zum Beispiel N- und O-Glykosylierungen oder Myristilierungen können die Zugänglichkeit von immunogenen Epitopen behindern oder sogar ganz verhindern und damit die Wirksamkeit von Antikörpertherapien in Frage stellen. Zudem konnte vielfach nachgewiesen werden, dass sich Art und Menge der sekundären Modifikationen in Normal- und Tumorgewebe unterscheiden (z.B. Durand & Seta, 2000; Clin. Chem. 46: 795-805; Hakomori, 1996; Cancer Res. 56: 5309-18). Die Analyse dieser Modifikationen ist daher essentiell für den Therapieerfolg eines Antikörpers. Potentielle Bindestellen lassen sich durch spezifische Algorithmen prädizieren.

Die Analyse von Proteinmodifikationen erfolgt in der Regel im Western Blot (siehe vorstehend). Vor allem Glykosylierungen, die in der Regel eine Größe von mehreren kDa haben, führen zu einer größeren Gesamtmasse des Zielproteins, die sich in der SDS-PAGE auftrennen lässt. Zum Nachweis von spezifischen O- und N-glykosidischen Bindungen werden Proteinlysate vor der Denaturierung durch SDS mit O- oder N-Glykosylasen inkubiert (nach Angaben des jeweiligen Herstellers, z.B. PNgase, Endoglykosidase F, Endoglykosidase H, Roche Diagnostics). Anschließend erfolgt ein Western Blot wie vorstehend beschrieben. Bei Verringerung der Größe eines Zielproteins kann so nach Inkubation mit einer Glykosidase eine spezifische Glykosylierung nachgewiesen und auf diesem Weg auch die Tumorspezifität einer Modifikation analysiert werden.

### Funktionsanalyse des Zielgens

Die Funktion des Zielmoleküls kann entscheidend für seinen therapeutischen Nutzen sein, so dass funktionelle Analysen ein wichtiger Baustein bei der Charakterisierung von therapeutisch nutzbaren Molekülen sind. Die Funktionsanalyse kann entweder in Zellen, in Zellkulturexperimenten oder aber in vivo mit Hilfe von Tiermodellen erfolgen. Dabei wird das Gen des Zielmoleküls entweder durch Mutation ausgeschaltet ("knockout") oder aber die Zielsequenz in die Zelle bzw. den Organismus eingefügt ("knockin"). Man kann so funktionelle Veränderungen im zellulären Kontext einerseits durch den Funktionsverlust des zu analysierenden Genes ("loss of function") analysieren. Im zweiten Fall lassen sich Veränderungen analysieren, die durch die Ergänzung des analysierten Genes verursacht werden ("gain of function").

### a. Funktionsanalyse in Zellen

Transfektion. Zur Analyse des "gain of function" muss das Gen des Zielmoleküls in die Zelle transferiert werden. Dazu werden Zellen mit einer DNA transfiziert, die die Synthese des Zielmoleküls erlauben. In der Regel steht das Gen des Zielmoleküls dabei unter Kontrolle eines starken eukaryontischen Promotors (z.B. Cytomegalievirus-Promotor; CMV). Zur Transfektion von Zelllinien mit DNA sind die verschiedensten Verfahren (z.B. Elektroporation, auf Liposomen basierende Transfektion, Kalziumphosphatpräzipitation) gut etabliert (z.B. Lemoine et al., Methods Mol. Biol. 75: 441-7, 1997). Das Gen kann dabei entweder ohne genomische Integration transient oder aber mit genomischer Integration nach Selektion mit z.B. Neomycin stabil synthetisiert werden.

RNA interference (siRNA). Eine Expressionsinhibition des Zielgens, die unter Umständen einen vollständigen Funktionsverlust des Zielmoleküls in Zellen induziert, kann durch die "RNA interference" (siRNA)-Technologie in Zellen erzeugt werden (Hannon, GJ. 2002. RNA interference. Nature 418: 244-51; Czauderna et al. 2003. Nucl. Acid Res. 31: 670-82). Dazu werden Zellen mit kurzen, ca. 20-25 Nukleotide langen, doppelsträngigen RNA-Molekülen transfiziert, die für das Zielmolekül spezifisch sind. Ein enzymatischer Prozess führt anschließend zum Abbau der spezifischen RNA des Zielgens, was in einer verminderten Expression des Zielproteins resultiert, und ermöglicht somit die funktionelle Analyse des Zielgens.

Zelllinien, die mittels Transfektion oder siRNA modifiziert wurden, können anschließend auf unterschiedliche Art und Weise analysiert werden. Nachstehend sind die geläufigsten Beispiele aufgeführt.

### 1. Proliferation

Eine Vielzahl von Verfahren ist zur Analyse der Zellproliferation etabliert und wird von verschiedenen Unternehmen kommerziell angeboten (z.B. Roche Diagnostics, Invitrogen, Details zu den Testverfahren sind in den zahlreichen Applikationsprotokollen beschrieben). Die Zellzahl in Zellkulturexperimenten lässt sich durch einfaches Auszählen oder durch kolometrische Tests ermitteln, die die metabolische Aktivität der Zellen messen (z.B. wst-1, Roche Diagnostics). Metabolische Testverfahren messen indirekt über enzymatische Marker die Zellzahl in einem Experiment. Direkt kann die Zellproliferation durch Analyse der DNA-Syntheserate z.B. durch Zugabe von Bromdesoxyuridin (BrdU) gemessen werden, der Nachweis des integrierten BrdU erfolgt über spezifische Antikörper kolometrisch.

### 2. Apoptose und Zytotoxizität

Eine große Anzahl von Testsystemen zum Nachweis von zellulärer Apoptose und von Zytotoxizität ist verfügbar. Ein entscheidendes Charakteristikum ist die spezifische, enzymabhängige Fragmentierung der genomischen DNA, die irreversibel ist und sicher zum Tod der Zelle führt. Verfahren zum Nachweis dieser spezifischen DNA-Fragmente sind kommerziell erhältlich. Als zusätzliches Verfahren steht der "TUNEL assay" zur Verfügung, der DNA-Einzelstrangbrüche auch in Gewebeschnitten nachweisen kann. Zytotoxizität wird vor allem über eine veränderte Zellpermeabilität nachgewiesen, die als Marker für den Vitalitätszustand von Zellen dient. Dazu werden entweder im Zellkulturüberstand Marker analysiert, die normalerweise intrazellulär zu finden sind. Alternativ kann auch die Aufnahmefähigkeit von Farbmarkern analysiert werden, die von intakten Zellen nicht aufgenommen werden. Die bekanntesten Beispiele für Farbmarker sind Trypanblau und Propidiumiodid, ein üblicher intrazellulärer Marker ist die Laktatdehydrogenase, die im Überstand enzymatisch nachgewiesen werden kann. Unterschiedliche Testsysteme stehen von verschiedenen kommerziellen Anbietern (z.B. Roche Diagnostics, Invitrogene) zur Verfügung.

### 3. Migrationstests

Die Fähigkeit von Zellen zur Migration wird in einem spezifischen Migrationstest vorzugsweise mit Hilfe einer Boyden-Kammer (Corning Costar) analysiert (Cinamon G., Alon R. J. Immunol. Methods. 2003 Feb; 273(1-2):53-62; Stockton et al. 2001. Mol. Biol. Cell. 12: 1937-56). Dazu werden Zellen auf einem Filter mit spezifischer Porengröße kultiviert. Zellen, die migrieren können, sind in der Lage, durch diesen Filter in ein weiteres darunter liegendes Kulturgefäß zu wandern. Eine anschließende mikroskopische Analyse erlaubt dann die Bestimmung eines möglicherweise veränderten Migrationsverhaltens, dass durch den "gain of function" bzw. "loss of function" des Zielmoleküls induziert wurde.

### b. Funktionsanalyse in Tiermodellen

Alternativ zu Zellkulturexperimenten bieten sich zur Analyse der Zielgenfunktion aufwendige in vivo-Experimente in Tiermodellen an. Diese Modelle haben im Vergleich zu den zellbasierenden Verfahren den Vorteil, dass sie Fehlentwicklungen bzw. Krankheiten nachweisen können, die erst im Kontext des gesamten Organismus nachweisbar sind. Eine Vielzahl von Modellen für humane Erkrankungen ist inzwischen verfügbar (Abate-Shen & Shen. 2002. Trends in Genetics S1-5; Matsusue et. al. 2003. J. Clin. Invest. 111:737-47). Verschiedene Tiermodelle wie zum Beispiel Hefe, Nematoden oder Zebrafische sind inzwischen intensiv charakterisiert worden. Bevorzugte Modelle sind aber im Vergleich zu anderen Spezies Tiermodelle wie zum Beispiel die Maus (Mus musculus), weil sie die biologischen Prozesse im humanen Kontext am besten abbilden können. Für Mäuse sind in den letzten Jahren sowohl transgene Verfahren etabliert worden, die neue Gene in das Mausgenom integrieren ("gain of function"; Jegstrup I. et al. 2003. Lab Anim. 2003 Jan.;37(1):1-9). Alternativ werden durch andere methodische Ansätze Gene im Mausgenom ausgeschaltet und so ein Funktionsverlust eines gewünschten Gens induziert (knockout-Modelle, "loss of function"; Zambrowicz BP & Sands AT. 2003. Nat. Rev. Drug Discov. 2003 Jan;2(1):38-51; Niwa H. 2001. Cell Struct. Funct. 2001 Jun;26(3):137-48.); technische Details sind vielfältig publiziert).

Nach Generierung der Mausmodelle können Veränderungen, die durch das Transgen bzw. durch den Funktionsverlust eines Gens induziert wurden, im Kontext des Gesamtorganismus analysiert werden (Balling R, 2001. Ann. Rev. Genomics Hum. Genet. 2:463-92). So sind zum Beispiel Verhaltenstests genauso wie biochemische Untersuchungen etablierter Blutparameter möglich. Histologische Analysen, Immunhistochemie oder die Elektronenmikroskopie ermöglichen die Charakterisierung von Veränderungen auf zellulärer Ebene. Das spezifische Expressionsmuster eines Genes kann durch eine in situ-Hybridisierung nachgewiesen werden (Peters et. al. 2003. Hum. Mol. Genet 12: 2109-20).

### Beispiel 1: Identifizierung des hypothetischen Proteins FLJ31461 als diagnostisches und therapeutisches Krebs-Target

FLJ31461 (SEQ ID NO: 1) mit der Genbank-Accession-Nummer NM_152454 wurde mittels Genprädiktionsprogrammen als putatives funktionell bisher nicht charakterisiertes Gen auf Chromosom 15 (15q25.3) ermittelt. Aus der in der Genbank hinterlegten Sequenz würden sich zwei mögliche offene Leserahmen ergeben. Der erste Leserahmen kodiert für ein Protein mit einer Länge von 136 Aminosäuren. Das Genprodukt (SEQ ID NO: 2), das in der RefSeq Datenbank des NCBI unter der Nummer NP_689667 abgelegt ist, hat somit ein kalkuliertes Molekulargewicht von ca. 15 kDa. Der zweite Leserahmen kodiert ein Protein mit einer Länge von 100 Aminosäuren (Nukleotidsequenz: SEQ ID NO: 69; Aminosäuresequenz: SEQ ID NO: 70).

Bei Sequenzanalysen des von uns klonierten FLJ31461-Gens stellten wir im Vergleich zu den in Datenbanken abgelegten Sequenzen überraschenderweise im kodierenden Bereich die Insertion eines Nukleotids fest. Dies resultiert in einer Leserahmenverschiebung. Zwei vollkommen neue offene Leseraster, die von den bereits in Sequenzdatenbanken hinterlegten Sequenzen nicht ableitbar sind, stellen sich dar. Dabei kodiert das neue Leseraster SEQ ID NO: 71 ein neues, 96 Aminosäure langes hypothetisches Protein (SEQ ID NO: 72). SEQ ID NO: 73 kodiert ein 133 Aminosäuren langes hypothetisches Protein (SEQ ID NO: 74). Da wir davon ausgehen müssen, dass die ursprünglichen Datenbankeingaben falsch sind, haben wir weitere Untersuchungen auf die SEQ ID NO: 71, SEQ ID NO: 72, SEQ ID NO: 73, SEQ ID NO: 74 fokussiert.

Erfindungsgemäß wurde nach der Etablierung von FLJ31461-spezifischen quantitativen RT-PCR (Primer mit den SEQ ID NO: 31, 32, 91, 92, 93, 94) die Menge der genspezifischen Transkripte in gesundem Gewebe und in Karzinomproben untersucht (Abb. 1). FLJ31461 lässt sich mit Ausnahme von Testis in keinem der von uns untersuchten Normalgewebe nachweisen (Abb. 1A). FLJ31461 ist demnach mit hoher Wahrscheinlichkeit ein streng keimzellspezifisches Genprodukt. Überraschenderweise fanden wir bei der Analyse von Tumoren, dass FLJ31461 in vielen Tumortypen angeschaltet ist, während es in den korrespondierenden Normalgeweben unter der Detektionsgrenze ist (Abb. 1 A-D). Dies betrifft nicht nur fast alle von uns untersuchten Brusttumoren (Abb. 1C), sowie eine Reihe von Lungentumoren und Karzinomen des Hals-Nasen-Rachenbereiches, sondern auch andere Neoplasien in unterschiedlicher Häufigkeit (Abb. 1D).

FLJ31461 ist somit ein hochsepezifischer molekularer Marker für Tumorgewebe, der sowohl diagnostisch als auch therapeutisch genutzt werden kann. Als typischer Vertreter der Klasse der sog. Cancer/Testis-Antigene, die aufgrund ihrer selektiven Gewebeverteilung als Markerstrukturen dienen, kann dieses Genprodukt z.B. eine gezielte Targetierung von Tumorzellen ohne Schädigung von Normalgeweben gewährleisten. Cancer/Testis-Gene werden als attraktive Zielstrukturen für targetierte Therapien angesehen und bereits für spezifische immuntherapeutische Ansätze bei Krebserkrankungen in Phase I/II Studien getestet (z.B. Scanlan MJ, Gure AO, Jungbluth AA, Old LJ, Chen YT. 2002. Immunol Rev. 2002 Oct; 188: 22-32).

Um diese Daten auf Proteinebene zu bestätigen, wurden spezifische Antikörper bzw. Immunseren durch Immunisierung von Tieren generiert. Über Analyse der Transmembrandomänen von SEQ ID NO: 72 und SEQ ID NO: 74 mit bioinformatischen Werkzeugen (TMHMM, TMPRED) wurde die Proteintopologie prädiziert. So werden z.B. für die SEQ ID NO: 72 zwei Transmembrandomänen vorausgesagt; N- und C-Terminus des Proteins sind extrazellulär.

Erfindungsgemäß wurden für die Immunisierung Peptidepitope ausgewählt; insbesondere extrazellulär gelegene, die spezifisch für beide Proteinvarianten sind.

Unter anderem wurden zur Immunisierung folgende Peptide für die Herstellung von Antikörpern ausgewählt: SEQ ID NO: 61, 62, 96, 97.

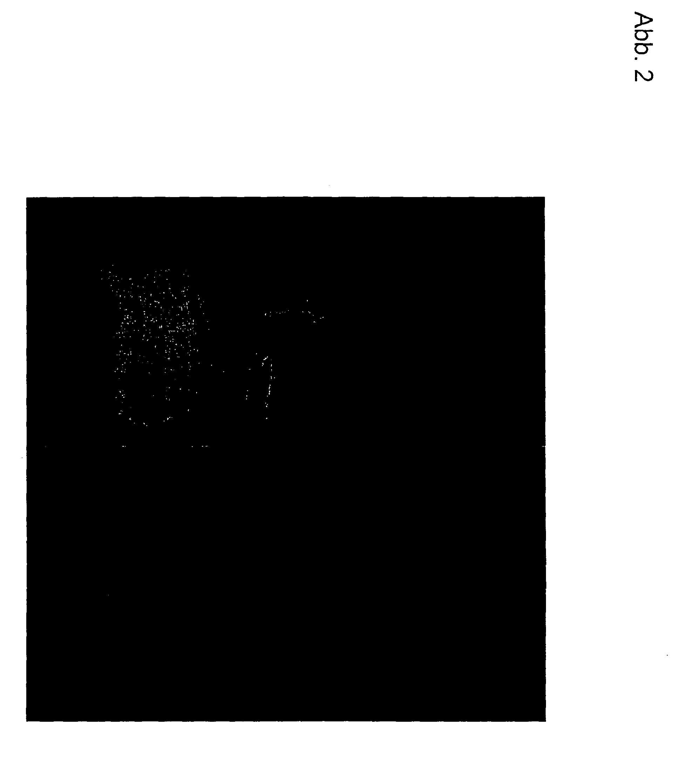

Beispielhaft werden die Daten für den Antikörper, der durch Immunisierung mit SEQ ID NO: 96 hergestellt wurde, dargestellt. Der spezifische Antikörper lässt sich unter verschiedenen Fixationsbedigungen für Immunfluoreszenz-Untersuchungen nutzen. Bei vergleichenden Färbungen von RT-PCR-positiven wie auch negativen Zelllinien ist das entsprechende Protein in gut nachweisbarer Menge spezifisch u.a. in den mittels quantitativer RT-PCR als positiv typisierten Brustkarzinomzellinien detektierbar (Abb. 2). Das endogene Protein stellt sich hier membranlokalisiert dar.

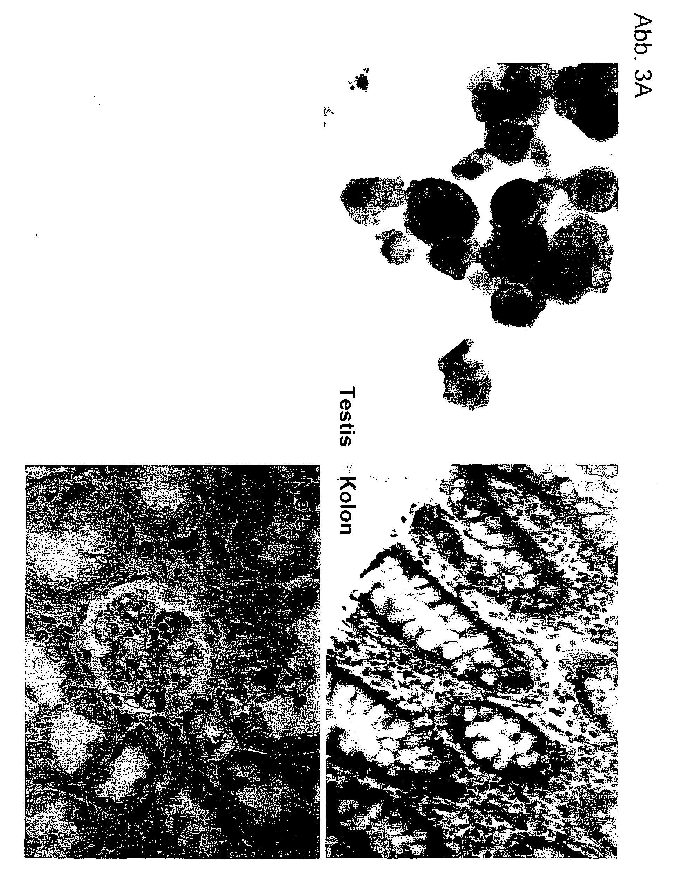

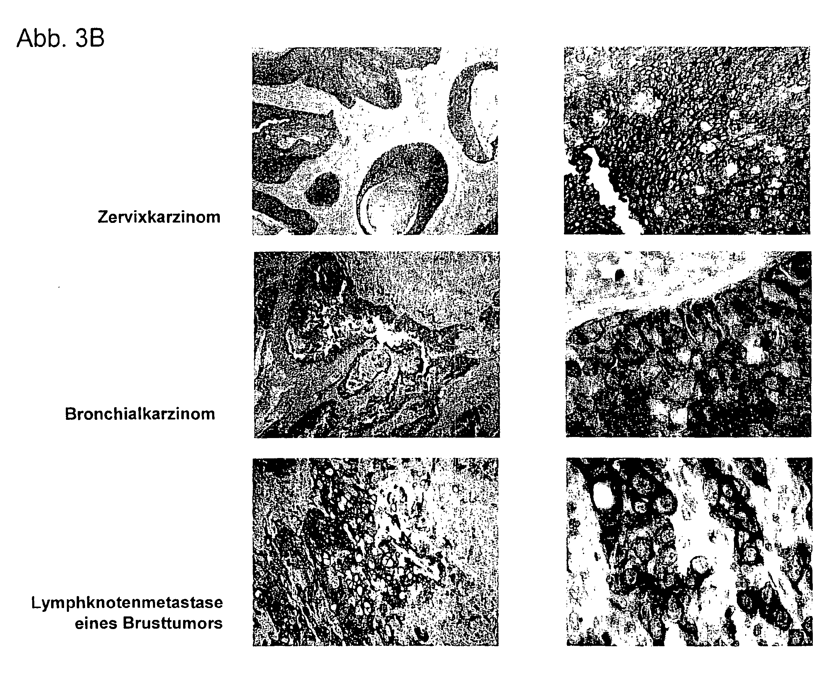

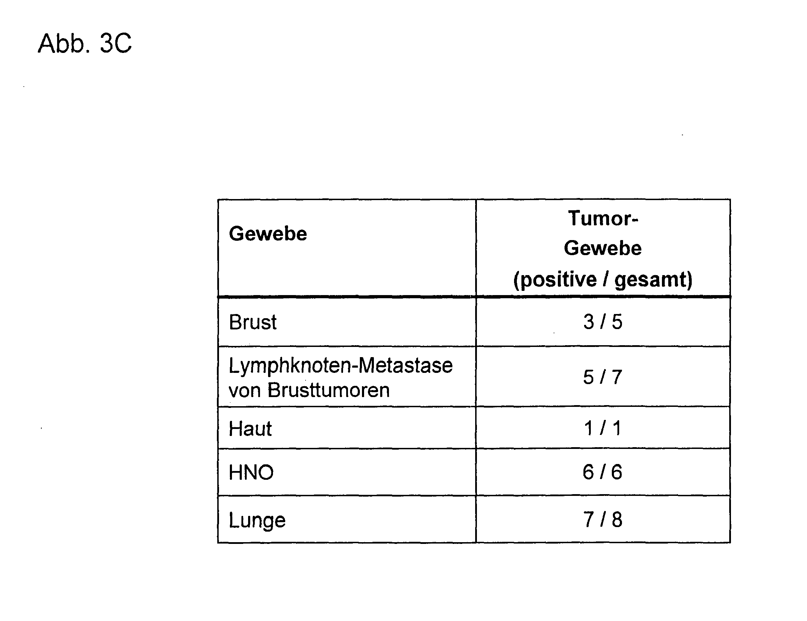

Solche Antikörper eigenen sich für die immunhistochemische Färbung von humanen Gewebeschnitten. Wir konnten in großen Teilen die auf Transkriptebene gefundene Gewebeverteilung bestätigen. Während wir außer in Testisgewebe kaum Reaktivität des Antikörpers in Normalgewebe feststellten (Abb. 3A), färben Antikörper gegen FLJ31461 verschiedenste menschliche Tumorpräparationen, unter diesen z.B. Brust- und Lungentumoren (Abb. 3B). Die Färbung der Zellen ist membranakzentuiert, was für die Lokalisierung des Proteins an der Zelloberfläche spricht. Überraschenderweise fanden wir, dass insbesondere Metastasen von Tumoren (Abb. 3B) besonders häufig und in einem hohen Anteil von Zellen dieses Protein exprimieren.

Diese Daten zeigen zum einen, dass dieses von uns gefundene Gen tatsächlich ein Protein bildet, dass dieses Protein hochspezifisch für humane Tumoren ist und sich auf der Oberflächenmembran solcher Tumorzellen befindet. Somit ist dieses Protein insbesondere für therapeutische Antikörper erreichbar. Unser Daten belegen ferner, dass spezifische Antikörper gegen dieses Protein hergestellt werden können. Diese Antikörper binden selektiv über den Marker FLJ31461 an Tumorzellen.
Erfindungsgemäß können derartige Antikörper für diagnostische Zwecke z.B. Immunhistologie genutzt werden. Insbesondere können solche Antikörper therapeutisch eingesetzt werden. Die hergestellten Antikörper können direkt auch zur Herstellung von chimären oder humanisierten rekombinanten Antikörpern verwendet werden. Dies kann auch direkt mit Antikörpern erfolgen, die aus Kaninchen gewonnen wurden (s. dazu J Biol Chem. 2000 May 5;275(18):13668-76 von Rader C, Ritter G, Nathan S, Elia M, Gout I, Jungbluth AA, Cohen LS, Welt S, Old LJ, Barbas CF 3rd. "The rabbit antibody repertoire as a novel source for the generation of therapeutic human antibodies"). Hierzu wurden von den immunisierten Tieren Lymphozyten asserviert. Auch für immuntherapeutische Verfahren wie Vakzinen bzw. den adoptiven Transfer von Antigen-spezifischen T-Lymphozyten stellt FLJ31461 ein hochattraktives Target dar.

### Beispiel 2: Identifizierung von DSG4 (Desmoglein 4) als diagnostisches und therapeutisches Krebs-Target

Das Gen DSG4 (Desmoglein4; SEQ ID NO: 75) mit seinem Translationsprodukt (SEQ ID NO: 76) ist ein Mitglied der desmosomalen Cadherin-Familie. Das Gen besteht aus 16 Exons und liegt auf Chromosom 18 (18q12). Die abgeleitete Aminosäuresequenz kodiert für ein Vorläuferprotein mit einer Länge von 1040 Aminosäuren. Das prozessierte Protein (N-terminal um 49 Aminosäuren abgespalten) hat eine Länge von 991 Aminosäuren und ohne Modifikationen ein Molekulargewicht von ca. 108 kDa. Es ist anzunehmen, dass DSG4 wie die anderen Desmogleine auch ein glykosyliertes Typ 1 Zelloberflächenprotein ist. DSG4 konnte als Konstituente von Desmosomen nachgewiesen werden (Kljuic et al. 2003. Cell 113: 249-260). Desmosomen sind komplexe interzelluläre Verbindungen, die epithelialen Geweben (wie z.B. der Epidermis) mechanische Stabilität verleihen. Autoantikörper gegen andere Mitglieder der Desmoglein-Familie scheinen durch Bindung an Desmosomen zum Verlust der Zell-Zell-Kontakte in der Epidermis beizutragen und an der Hauterkrankung Pemphigus Vulgaris beteiligt zu sein. Es ist beschrieben worden, dass DSG4 in den meisten gesunden Geweben nicht exprimiert wird. Signifikante Expression ist bisher nur für Speicheldrüse, Testis, Prostata und Haut berichtet (Whittock, Bower, 2003. J Invest Derm 120: 523-530). Ein Zusammenhang mit Tumorerkrankungen wurde bisher nicht hergestellt.

Erfindungsgemäß wurde mit DSG4-spezifischen Oligonukleotiden die Expression in gesunden Geweben und Tumoren untersucht. Erfindungsgemäß wurden mehrere DSG4-spezifische Primerpaare für RT-PCR-Untersuchungen eingesetzt. Dies sind: DSG4 Primerpaar SEQ ID NO: 77, 78 (Exon 10 und Exon 12), DSG4-Primerpaar SEQ ID NO: 83, 84 (Exon 1 und Exon 5), DSG4 Primerpaar SEQ ID NO: 89, 90 (Exon 5 und Exon 8) und DSG4 Primerpaar SEQ ID NO: 95, 78 (Exon 8 und Exon 12).

Die Untersuchung mit allen Primerpaaren bestätigte, dass DSG4 in den meisten Normalgeweben nicht exprimiert wird. Je nach Primerpaar stellten sich allerdings unterschiedliche Expressionsmuster dar (Abb. 4B). Mit den Primerpaaren SEQ ID NO: 95, 78 (Exon 8-12) fanden wir keine Expression in Normalgewebe, bis auf eine sehr schwache Expression in Prostata und Haut. Überraschenderweise lässt sich DSG4 mit diesem Primerpaar in einer Reihe von Tumoren nachweisen. Insbesondere sind dies Magentumoren, wie auch Karzinome des Mund-Nasen-Rachen-Raumes (Abb. 4A).

Mit den Primerpaaren SEQ ID NO: 77,78 (Exon 10-12) war selbst die Expression in den oben r genannten Normalgewebe Prostata und Haut schwächer. Überraschenderweise zeigt sich mit diesem Primerpaar eine noch ausgeprägtere Expression in Tumoren (Abb. 4A). Zum einen sind dies solche, die auch mit dem erstgenannten Primerpaar aufgefallen sind, wie Magentumoren, und Karzinome des Mund-Nasen-Rachen-Raumes, aber auch andere Krebsarten (Abb. 4B, C). Insbesondere fanden wir in allen Darmtumoren eine signifikante und hohe Expression, die wir mit dem erstgenannten Primepaar nicht detektieren konnten. Die Expression in den verschiedenen Tumoren lag mehr als ein vielfaches über der im höchstexprimierenden toxizitäts-relevanten Normalgewebe (Abb. 4B).

Auf der Grundlage dieser Untersuchungen ergibt sich, dass es neben dem Vollänge-Transkript SEQ ID NO: 75 und dem daraus abgeleiteten Protein (SEQ ID NO: 76) noch trunkierte Varianten von DSG4 gibt, denen Bereiche vor Exon 9 fehlen (Abb. 5).

Eine weitergehende Analyse des Genlocus von DSG4 zeigte uns, dass von verschiedenen Varianten des Moleküls mit einer Deletion vor Exon 9 auszugehen ist (Abb. 5). Dies sind die Transkripte SEQ ID NO: 85, 87, 108, 110 und 112 und deren veränderte Proteinprodukte SEQ ID NO: 86, 88, 109, 111 und 113. Auch das Vollänge-Transkript kann in Bereichen jenseits von Exon 10 verändert sein und zu varianten Transkripten SEQ ID NO: 102, 104, 106 und Proteinen SEQ ID NO: 103, 105, 107 führen.

Die vor Exon 9 trunkierten Varianten sind sogar tumor-selektiver als die Vollängevariante und in zusätzlichen Tumortypen wie dem Kolonkarzinom zu finden, in denen die Vollängevariante nicht exprimiert ist. Da die Transmembrandomäne sich in Exon12 befindet, ist der mit den Primern SEQ ID NO: 77, 78 amplifizierte Bereich extrazellulär und müsste z.B. mit Antikörpern angreifbar sein. Dieser trunkierte extrazelluläre Bereich enthält die DSG4 Genabschnitte Exon 10, 11 und 12. Somit sind Transkripte, die Exon 10, 11 und 12 (SEQ ID NO: 79) von DSG4 enthalten, besonders als diagnostische und therapeutische Krebstargets geeignet. Diese Bereiche von DSG4 kodieren für eine Domäne (SEQ ID NO: 81), welche extrazellulär liegt. Somit können DSG4-Polynukleotide, die Exon 10, 11, 12 umfassen (SEQ ID NO: 75, 79, 80, 85, 87. 106, 112) und die von ihnen kodierten Polypeptide (SEQ ID NO: 76, 81, 82, 86, 88, 107, 113) erfindungsgemäß besonders gut als Zielstruktur von monoklonalen Antikörpern genutzt werden.

Entsprechend haben wir Tiere mit Epitopen aus dem Bereich des Vollängemoleküls (SEQ ID NO: 75) bzw. aus dem extrazellulären Bereich des trunkierten Moleküls (SEQ ID NO: 81) immunisiert.

Wir konnten Antikörper generieren, die DSG4 auf der Oberfläche von Zellen, die mit DSG4 transfiziert sind, anfärben. Spezifische Antikörper können dieses Protein dann wie in der Immunfluoreszenz (Abb. 6A) und in der Flußzytometrie dargestellt (Abb. 6B) auf der Oberfläche spezifisch detektieren.

Die ausgeprägte Expression und hohe Inzidenz dieses Moleküls für die dargestellten Tumorindikationen machen dieses Protein, insbesondere seine trunkierte Variante, erfindungsgemäß zu einem hochinteressanten diagnostischen und therapeutischen Marker. Dies umfasst erfindungsgemäß den Nachweis disseminierter Tumorzellen im Serum, Knochenmark und Urin, sowie die Detektion von Metastasen in anderen Organen mittels RT-PCR.

Die extrazelluläre Domäne von DSG4, vor allem der zellmembrannahe Teil, kann erfindungsgemäß als Zielstruktur von monoklonalen Antikörpern für eine Therapie wie auch Immundiagnostik genutzt werden.

Des weiteren kann DSG4 erfindungsgemäß als Vakzine (RNA, DNA, Protein, Peptide) zur Induktion Tumor-spezischer Immunantworten (T-und B-Zell vermittelte Immunreaktionen) eingesetzt werden. Dies umfasst erfindungsgemäß auch die Entwicklung von sogenannten "small compunds", die die biologische Aktivität von DSG4 modulieren und zur Therapie von Tumoren eingesetzt werden können.

### Beispiel 3: Identifizierung von DSG3 (Desmoglein3) als diagnostisches und therapeutisches Krebs-Target

Das Gen DSG3 (Desmoglein3; SEQ ID NO: 3) und sein Translationsprodukt (SEQ ID NO: 4) ist ein Mitglied der desmosomalen Cadherin-Familie, dass am NCBI unter der Accession-Nummer NM_001944 (Nukleotidsequenz) bzw. NP_001935 (Proteinsequenz) publiziert ist. Das Gen besteht aus 15 Exons und liegt auf Chromosom 18 (18q12.1-q12.2). Die abgeleitete Aminosäuresequenz kodiert für ein Protein mit 999 Aminosäuren und einer hypothetischen Größe von ca. 130 kDa. DSG3 ist ein glykosyliertes Zelloberflächenprotein des Typ 1 und konnte in Desmosomen nachgewiesen werden (Silos et al. J. Biol. Chem. 271: 17504-17511, 1996). Desomosomen sind komplexe interzelluläre Verbindungen, die Keratinfilamente von angrenzenden Zellen verbinden, um epithelialen Geweben (wie z.B. der Epidermis) mechanische Stabilität zu verleihen. Die desmosomalen Cadherine Desmoglein und Desmocollin sind Kalzium-abhängige Adhäsionsmoleküle. Autoantikörper gegen Desmoglein3 und der damit verbundene Verlust der Zell-Zell-Kontakte in der Epidermis sind an der Hauterkrankung Pemphigus Vulgaris beteiligt (Amagai et al., 1991. Cell 67: 869-877). Dies konnte auch im Tiermodel nachgewiesen werden (Koch et al., 1997. J Cell Biol 5: 1091-1102).

Erfindungsgemäß wurde nach Etablierung einer DSG3-spezifischen quantitativen RT-PCR (Primerpaar SEQ ID NO: 33, 34) die Menge der genspezifischen Transkripte in gesundem Gewebe und in Karzinomproben untersucht (Abb. 7; Methodik: vgl. Material und Methoden, Absatz B.1.). Unsere Untersuchungen zeigten eine differentielle Verteilung der Expression in Normalgeweben. DSG3 Transkripte werden kaum in Normalgeweben gefunden. Die einzigen Normalgewebe, die signifikante Transkriptmengen exprimieren, sind Speiseröhre, Haut und Thymus (Abb. 7a). In allen anderen analysierten Geweben, insbesondere Gehirn, Herz, Leber, Pankreas, PBMC, Lunge, Mamma, Ovar, Niere, Milz, Kolon, Lymphknoten, Uterus, Harnblase und Prostata, ist eine Transkription sehr schwach oder nicht nachweisbar (Abb. 7A). Überraschenderweise haben wir in einigen Tumortypen eine signifikante bisher nicht beschriebene Expression von DSG3 nachgewiesen.

In quantitativen RT-PCR-Analysen von Tumoren konnten DSG3-spezifische Transkripte unter anderem in Tumoren des Hals-Nasen-Rachen Raumes ("Head Neck cancer") in einer Menge nachgewiesen werden, die über der im höchstexprimierenden Toxizität-relevanten Gewebe liegt (Abb. 7B). Aber auch weitere Tumoren, z.B. Karzinome der Speiseröhre (Abb. 7C), exprimieren dieses Protein.

Wir haben Schnitte menschlicher Gewebe mit DSG3-spezifischen Antikörpern gefärbt und konnten die in der PCR gefundene Tumor-Selektivität bestätigen (Abb. 8).

Die ausgeprägte Expression und hohe Inzidenz dieses Moleküls für die dargestellten Tumorindikationen machen dieses Protein erfindungsgemäß zu einem hochinteressanten diagnostischen und therapeutischen Marker. Dies umfasst erfindungsgemäß den Nachweis disseminierter Tumorzellen im Serum, Knochenmark und Urin, sowie die Detektion von Metastasen in anderen Organen mittels RT-PCR.

Die am N-Terminus lokalisierte extrazelluläre Domäne des Typ I Membranproteins Desmoglein3 (SEQ ID NO: 4, Aminosäuren 1-611) kann erfindungsgemäß als Zielstruktur von monoklonalen Antikörpern für eine Therapie wie auch Immundiagnostik genutzt werden. Des weiteren kann DSG3 erfindungsgemäß als Vakzine (RNA, DNA, Protein, Peptide) zur Induktion Tumor-spezischer Immunantworten (T-und B-Zell vermittelte Immunreaktionen) eingesetzt werden. Dies umfasst erfindungsgemäß auch die Entwicklung von sogenannten "small compunds", die die biologische Aktivität von DSG3 modulieren und zur Therapie von Tumoren eingesetzt werden können.

### Beispiel 4: Identifizierung des Transporters SLC6A3 (solute carrier family 6) als diagnostisches und therapeutisches Krebs-Target

Das Gen SLC6A3 (SEQ ID NO: 5) und sein Translationsprodukt (SEQ ID NO: 6) ist ein Mitglied der Natrium-Neurotransmitter Symporter Familie (SNF-Familie) und unter der Accession-Nummer NM_001044 (Nukleotidsequenz) bzw. NP_001035 (Proteinsequenz) abgelegt. Das Gen besteht aus 16 Exons und liegt auf Chromosom 5 (5p15.3). Das SLC6A3-Gen kodiert für ein Glykoprotein mit einer Länge von 620 Aminosäuren. SLC6A3 ist ein integrales Membranprotein mit insgesamt 12 Transmembrandomänen, das als Homooligomer Teil eines Ionentransporter-Komplexes ist (Hastrup et al., 2003. J Biol Chem 278: 45045-48). Erfindungsgemäß wurde nach Etablierung einer SLC6A3-spezifischen quantitativen RT-PCR (Primerpaar SEQ ID NO: 35, 36) die Verteilung von SLC6A3-spezifischen Transkripten in gesundem Gewebe und in Karzinomproben untersucht (Abb. 9; Methodik: vgl. Material und Methoden, Absatz B.1.). In den meisten Normalgeweben ist SLC6A3 sehr gering bis gar nicht exprimiert, eine moderate Expression von SLC6A3 war nur im Thymus, in der Milz, im Ovar, im Pankreas sowie in der Niere nachweisbar. Eine signifikante, ca. 100-fach erhöhte Überexpression von SLC6A3 war in Nierenkarzinomen nachweisbar (Abb. 9A). Eine Detailanalyse von verschiedenen Nierengeweben mittels quantitativer (Abb. 9B) und konventioneller RT-PCR (Abb. 9C) ergab, dass SLC6A3 in 7/12 Nierenzellkarzinomen exprimiert und in 5/12 Proben im Vergleich zu nicht tumorigenen Proben überexprimiert ist. Eine deutliche schwächere, aber nachweisbare SLC6A3-spezifische Expression konnte außerdem in einigen Tumorengeweben anderer Karzinome nachgewiesen werden. Insbesondere in einigen Mamma-, Ovar-, Bronchial-, und Prostatakarzinomen waren SLC6A3-spezifische Transkripte nachweisbar (Abb. 9D und 9E).

Die verschiedenen extrazellulären Domänen von SLC6A3 können erfindungsgemäß als Zielstrukttuen von monoklonalen therapeutischen Antikörpern genutzt werden. Die folgenden Sequenzbereiche werden, bezogen auf SEQ ID NO: 6, bei SLC6A3 als extrazellulär prädiziert (basierend auf einer Analyse mit der Software TMHMM2): Aminosäuren 89-97, 164-237, 288-310, 369-397, 470-478, 545-558. Zur Herstellung von SLC6A3-spezifischen Antikörpern wurden die unter SEQ ID NO: 63 und 64 aufgeführten Peptide verwendet.

### Beispiel 5: Identifizierung von GRM8 als diagnostisches und therapeutisches Krebs-Target

Das Gen GRM8/GluR8 oder "Metabotropher Glutamatrezeptor 8" (SEQ ID NO: 7) und sein Translationsprodukt (SEQ ID NO: 8) gehört zur Familie der Glutamatrezeptoren. Das Gen besteht aus 10 Exons und liegt auf Chromosom 7 (7q31.3-q32.1). Das vom GRM8 Gen kodierte Protein hat eine Länge von 908 Aminosäuren, das kalkulierte Molekulargewicht beträgt 102 kDa. Prädiktionsprogramme sagen 7 Transmembrandomänen voraus. Das Protein hat hohe Homologie (67 bis 70% Ähnlichkeit) zu GluR4 und GluR7 (Scherrer et al., 1996. Genomics 31: 230-233).

L-Glutamat ist ein wichtiger Neurotransmitter im Zentralen Nervensystem und aktiviert sowohl die ionotrophen und metabotrophen Glutamatrezeptoren. GRM8-spezifische Transkripte wurden bisher im Gehirn bzw. in Gliazellen nachgewiesen. Allerdings wurden bisher keine quantitativ vergleichenden Transkript- oder Proteinuntersuchungen über eine größere Anzahl von Geweben berichtet (Wu et al., 1998. Brain Res. 53: 88-97). Erfindungsgemäß wurde nach Etablierung einer GRM8-spezifischen quantitativen RT-PCR (Primerpaar SEQ ID NO: 37, 38) die Verteilung vom GRM8-spezifischen Transkripten in gesundem Gewebe und in Karzinomproben untersucht (Abb. 10; Methodik: vgl. Material und Methoden, Absatz B.1.). Unsere Untersuchungen zeigen eine differentielle Verteilung der Expression in unterschiedlichen Normalgeweben. GRM8-Transkripte fanden wir selektiv nicht nur in Hirn, sondern in etwas geringerer Menge auch in Magen-, Darm-, Blasen-, Ovarial-, Lungen- und Pankreasgeweben. In den meisten anderen Normalgeweben ist GRM8 deutlich geringer exprimiert bzw. nicht nachweisbar. In einigen Tumoren konnten wir eine deutliche, bisher nicht beschriebene Expression von GRM8 nachweisen. Insbesondere Kolon-, Zervix- und Nierenzellkarzinome zeigen eine mehr als 10-fache Überexpression im Vergleich zu allen anderen Normalgeweben und liegen auch deutlich über dem Expressionsniveau des Hirngewebes (Abb. 10A und 10B).

Die extrazellulären Domänen von GRM8 können erfindungsgemäß als Zielstrukturen von therapeutischen monoklonalen Antikörpern genutzt werden. Bezogen auf die SEQ ID NO: 8 sind die Aminosäuren 1-582, 644-652, 717-743 und 806-819 extrazellulär lokalisiert.

### Beispiel 6: Identifizierung von Cadherin 17 (CDH17) als diagnostisches und therapeutisches Krebs-Target

Das Gen CDH17 (SEQ ID NO: 9) und sein Translationsprodukt (SEQ ID NO: 10) ist ein Mitglied der Cadherin-Familie. Das Gen besteht aus 18 Exons und liegt auf Chromosom 8 (8q22.1). Es kodiert für ein Typ-1-Transmembranprotein mit einer Länge von 832 Aminosäuren, das ohne sekundäre Modifikationen ein kalkuliertes Molekulargewicht von 92,1 kDa hat, sowie eine Transniembrandomäne. Cadherin 17 wurde als protonenabhängiger Peptidtransporter von Dantzig et al. (Science 264: 430-433, 1994) kloniert. Das kalziumabhängige Glykoprotein Cadherin17 enthält 7 Cadherin-Domänen in der extrazellulären Region (Gessner et al., Ann N Y Acad Sci.;915:136-43, 2000). Die intrazelluläre Domäne zeigt keine Homologie zu anderen Cadherinen. Expressionsuntersuchungen lagen bisher lediglich nur vereinzelt vor und nicht als quantitativ vergleichenden Transkript- oder Proteinuntersuchungen über eine größere Anzahl von unterschiedlichen Geweben.

Erfindungsgemäß wurde nach Etablierung einer CDH17-spezifischen quantitativen RT-PCR (Primerpaar SEQ ID NO: 39, 40) die Verteilung von CDH17-spezifischen Transkripten in gesundem Gewebe und in Karzinomproben untersucht (Abb. 11; Methodik: vgl. Material und Methoden, Absatz B.1.). In den meisten Normalgeweben ist CDH17 gar nicht detektierbar (Abb. 11A). Signifikante Transkriptmengen fanden wir selektiv in Magen- und Darmgewebe exprimiert, weit geringere Expression in Blase, Milz, Lymphknoten, Thymus, Prostata und Ösophagus. Überraschenderweise detektierten wir in Tumoren eine deutliche, bisher nicht beschriebene CDH17-spezifische Expression. Für CDH17 war in Darmtumoren im Vergleich zu den Normalgeweben eine mindestens 2-10 fache Überexpression nachweisbar, außerdem ist CDH17 in Magen- und Ösophagustumoren stark exprimiert (Abb. 11B und 11C).

Die ausgeprägte Expression und hohe Inzidenz dieses Moleküls für die dargestellten Tumorindikationen machen dieses Protein erfindungsgemäß zu einem hochinteressanten diagnostischen und therapeutischen Marker. Dies umfasst erfindungsgemäß den Nachweis disseminierter Tumorzellen im Serum, Knochenmark und Urin, sowie die Detektion von Metastasen in anderen Organen mittels RT-PCR.

Die extrazelluläre Domäne von CDH17 kann erfindungsgemäß als Zielstruktur von monoklonalen Antikörpern für eine Therapie wie auch Immundiagnostik genutzt werden. Bezogen auf die SEQ ID NO: 10 sind die Aminosäuren 1-785 extrazellulär lokalisiert (Prädiktion erfolgte mit Hilfe der Software TMHMM2).

Des weiteren kann CDH17 erfindungsgemäß als Vakzine (RNA, DNA, Protein, Peptide) zur Induktion Tumor-spezischer Immunantworten (T-und B-Zell vermittelte Immunreaktionen) eingesetzt werden. Dies umfasst erfindungsgemäß auch die Entwicklung von sogenannten "small compunds", die die biologische Aktivität von CDH17 modulieren und zur Therapie von Tumoren eingesetzt werden können.

### Beispiel 7: Identifizierung von ABCC4 als diagnostisches und therapeutisches Krebs-Target

Das Gen ABCC4 (SEQ ID NO: 11) und sein Translationsprodukt (SEQ ID NO: 12) kodieren einen ABC-Transporter (ATP-binding-casette). Das Gen besteht aus 31 Exons und ist auf Chromosom 13 (13q31) lokalisiert. Es kodiert für ein Protein mit einer Länge von 1325 Aminosäuren, das ohne Modifikationen ein kalkuliertes Molekulargewicht von ca. 149 kDa hat. ABCC4 ist ein integrales Membranprotein. Die Topologie von ABCC4 ist noch nicht geklärt, Prädiktionsprogramme sagen 12-14 Transmembrandomänen voraus. ABC-Transporter befördern verschiedenste Moleküle über extra- und intrazelluläre Membranen. ABCC4 ist ein Mitglied der sog. MRP Familie, der "Multi-Drug-Resistance" Proteine. Die spezifische Funktion von ABCC4 ist bisher noch ungeklärt, allerdings scheint der Transporter eine Rolle bei der zellulären Detoxifikation zu spielen, die für die chemotherapeutische Resistenz vieler Tumoren verantwortlich gemacht wird.

Die Gewebeverteilung dieses Genproduktes über verschiedene Organe des menschlichen Körpers ist bisher nicht untersucht. Erfindungsgemäß wurden nach Etablierung einer ABCC4-spezifischen quantitativen RT-PCR (Primerpaar SEQ ID NO: 41, 42) spezifische Transkripte in gesundem Gewebe und in Karzinomproben untersucht (Abb. 12; Methodik: vgl. Material und Methoden, Abschnitt B.1.). Unsere vergleichenden Untersuchungen über alle Normalgewebe bestätigen die publizierte ubiquitäre Expression von ABCC4. ABCC4 konnten wir in allen getesteten Normalgeweben nachweisen. Überraschenderweise fanden wir allerdings, dass in einer Reihe von Tumoren eine über diese hinausgehende Überexpression des Transkriptes zu finden ist. So findet sich ABCC4 z.B. in Nieren-, Prostata- und Bronchialtumoren im Vergleich zu allen analysierten Normalgeweben in 2-15fach erhöhter Menge (Abb. 12).

Die ausgeprägte Expression und hohe Inzidenz dieses Moleküls für die dargestellten Tumorindikationen machen dieses Protein erfindungsgemäß zu einem hochinteressanten diagnostischen und therapeutischen Marker. Dies umfasst erfindungsgemäß den Nachweis disseminierter Tumorzellen im Serum, Knochenmark und Urin, sowie die Detektion von Metastasen in anderen Organen mittels RT-PCR.

Die extrazellulären Domänen von ABCC4 können erfindungsgemäß als Zielstrukturen von monoklonalen Antikörpern für Therapie wie auch Immundiagnostik genutzt werden. Die genaue Lokalisation der extrazellulären Domänen ist noch offen. Bezogen auf die SEQ ID NO: 12 prädiziert die Software TMHMM2 die Aminosäuren 114-132, 230-232, 347-350, 730-768, 879-946 und 999-1325 als extrazellulär.

Des weiteren kann ABCC4 erfindungsgemäß als Vakzine (RNA, DNA, Protein, Peptide) zur Induktion Tumor-spezischer Immunantworten (T-und B-Zell vermittelte Immunreaktionen) eingesetzt werden. Dies umfasst erfindungsgemäß auch die Entwicklung von sogenannten "small compunds", die die biologische Aktivität von ABCC4 modulieren und zur Therapie von Tumoren eingesetzt werden können.

### Beispiel 8: Identifizierung von VIL1 als diagnostisches und therapeutisches Krebs-Target

Das Gen VIL1 oder "Villin1" (SEQ ID NO: 13) und sein Translationsprodukt (SEQ ID NO: 14) werden von einem Gen aus 19 Exons auf Chromosom 2 (2q35-q36) kodiert. Das Gen kodiert für ein Protein mit 826 Aminosäuren, das ohne Modifikationen ein kalkuliertes Molekulargewicht von ca. 92 kDa hat Villin ist die strukturelle Hauptkomponente der Mikrovilli in Zellen der gastrointestinalen und urogenitalen Epithelien. Es ist ein kalziumreguliertes, aktinbindendes Protein.

Pringault et al. (EMBO J. 5: 3119-3124, 1986) klonierten Villin1 und konnten die Existenz zweier Transkripte (2,7 kb und 3,5 kb) nachweisen. Diese Varianten entstehen durch die Verwendung alternativer Polyadenylierungssignale im letzten Exon. VIL1-spezifische Transkripte wurden bisher in einer Vielzahl von Geweben wie Gehirn, Herz, Lunge, Darm, Niere und der Leber beschrieben. Allerdings wurden bisher keine umfassende quantitativ vergleichenden Transkript- oder Proteinuntersuchungen über eine größere Anzahl von Geweben durchgeführt, die Aufschluss über die Verwendbarkeit von VIL1 für therapeutische Zwecke geben könnte.

Erfindungsgemäß wurde nach Etablierung einer VIL1-spezifischen quantitativen RT-PCR (Primerpaar SEQ ID NO: 43, 44) die Verteilung der spezifischen Transkripte in gesundem Gewebe und in Karzinomproben untersucht (Abb. 13; Methodik: vgl. Material und Methoden, Abschnitt B.1.). Unsere vergleichenden Untersuchungen über alle Normalgewebe zeigen eine differentielle Verteilung der VIL1-spezifischen Expression. In fast allen Normalgeweben sind VIL1-spezifische Transkripte nicht nachweisbar (Abb. 13A). Insbesondere widerlegen unsere Daten die vorbeschriebene Expression in Gehirn, Herz, Brust, Ovar, Lymphknoten, Ösophagus, Haut, Thymus, Harnblase, Muskel. VIL1-Transkripte fanden wir lediglich in Magen und Darm, eine geringere Expression wiesen wir in Pankreas, Leber, PBMCs nach.

Überraschenderweise deckten wir allerdings auch in Tumoren eine deutliche bisher nicht beschriebene VIL1-spezifische Überexpression auf. So finden sich in Kolon- und Magenkarzinomen eine 5 bis 10-fache Überexpression im Vergleich zu allen analysierten Normalgeweben (Abb. 13A und 13B). Eine deutliche VIL1-spezifische Expression ist auch in Pankreas-, Magen-, Bronchial- und Lebertumoren nachweisbar.

Die ausgeprägte Expression und hohe Inzidenz dieses Moleküls für die dargestellten Tumorindikationen machen dieses Protein erfindungsgemäß zu einem hochinteressanten diagnostischen und therapeutischen Marker. Dies umfasst erfindungsgemäß den Nachweis disseminierter Tumorzellen im Serum, Knochenmark und Urin, sowie die Detektion von Metastasen in anderen Organen mittels RT-PCR.

Es kann erfindungsgemäß als Vakzine (RNA, DNA, Protein, Peptide) zur Induktion Tumor-spezischer Immunantworten (T-und B-Zell vermittelte Immunreaktionen) eingesetzt werden. Dies umfasst erfindungsgemäß auch die Entwicklung von sogenannten "small compunds", die die biologische Aktivität von VIL1 modulieren und zur Therapie von Tumoren eingesetzt werden können.

### Beispiel 9: Identifizierung von MGC34032 als diagnostisches und therapeutisches Krebs-Target

Das Translationsprodukt (SEQ ID NO: 16) des Gens MGC34032 (SEQ ID NO: 15) ist ein hypothetisches Protein mit bisher unbekannter Funktion. Das Gen besteht aus 28 Exons und liegt auf Chromosom 1 (1p31.1). Das Gen kodiert für ein Protein mit einer Länge von 719 Aminosäuren, das ein kalkuliertes Molekulargewicht von ca. 79 kDa hat. Prädiktionsprogramme sagen übereinstimmend 8 Transmembrandomänen voraus. Homologien sind nicht bekannt, Publikationen zu MGC34032 gibt es bisher nicht.

Erfindungsgemäß wurde nach Etablierung einer MGC34032-spezifischen quantitativen RT-PCR (Primerpaar SEQ ID NO: 45, 46) die Verteilung von spezifischen Transkripten in gesundem Gewebe und Karzinomproben untersucht (Abb. 14; Methodik: vgl. Material und Methoden, Abschnitt B.1.). MGC34032-Transkripte fanden wir in allen getesteten Normalgeweben. Der Vergleich der Transkriptmengen in Normalgeweben mit der in Tumoren zeigte allerdings unerwartet, dass verschiedene Tumortypen eine signifikante bisher nicht beschriebene 5-10-fache Überexpression dieses Genproduktes aufweisen. Dies sind insbesondere Ösophagus-, HNO-, Kolon-, Ovarial-, Lungen- und Nierenzellkarzinome (Abb. 14 A-D).

Zur Herstellung von MGC34032-spezifischen Antikörpern wurden die unter SEQ ID NO: 98 und 99 aufgeführten Peptide verwendet. Diese Antikörper konnte MGC34032 auf der Zelloberfläche anfärben (Abb. 15A).

Die ausgeprägte Expression und hohe Inzidenz dieses Moleküls für die dargestellten Tumorindikationen machen dieses Protein erfindungsgemäß zu einem hochinteressanten diagnostischen und therapeutischen Marker. Dies umfasst erfindungsgemäß den Nachweis disseminierter Tumorzellen im Serum, Knochenmark und Urin, sowie die Detektion von Metastasen in anderen Organen mittels RT-PCR.

Die extrazellulären Domänen von MCG34032 können erfindungsgemäß als Zielstrukturen von monoklonalen Antikörpern für eine Therapie wie auch Immundiagnostik genutzt werden. Bezogen auf die SEQ ID NO: 16 sind die Aminosäuren 62-240, 288-323, 395-461 und 633-646 extrazellulär lokalisiert (Prädiktion erfolgte mit Hilfe der Software TMHMM2).

Des weiteren kann MCG34032 erfindungsgemäß als Vakzine (RNA, DNA, Protein, Peptide) zur Induktion Tumor-spezischer Immunantworten (T-und B-Zell vermittelte Immunreaktionen) eingesetzt werden. Dies umfasst erfindungsgemäß auch die Entwicklung von sogenannten "small compunds", die die biologische Aktivität von MCG34032 modulieren und zur Therapie von Tumoren eingesetzt werden können.

### Beispiel 10: Identifizierung der Serinprotease PRSS7 (Enterokinase) als diagnostisches und therapeutisches Krebs-Target

Das Gen PRSS7 (SEQ ID NO: 17) und sein Translationsprodukt (SEQ ID NO: 18) gehört zur Familie der Serinprotasen. Das Gen besteht aus 25 Exons und liegt auf Chromosom 21 (21q21). Das Gen kodiert ein Protein mit einer Länge von 1019 Aminosäuren, das posttranslational weiter prozessiert wird. Das aktive Enzym besteht aus 2 durch eine Disulfidbrücke verbundenen Peptidketten, die durch eine proteolytische Spaltung aus einem gemeinsamen Vorläufermolekül entstanden sind. Die schwere Kette besteht aus 784 Aminosäuren. Die leichte Kette aus 235 Aminosäuren zeigt eine deutliche Homologie zu bekannten Serinproteasen. Prädiktionsprogramme sagen eine Transmembrandomäne für PRSS7 vorher. PRSS7 wird insbesondere in den apikalen Zellen und Enterozyten des Dünndarms gebildet und dient zur initialen Aktivierung der proteolytischen Enzyme des Pankreas (wie beispielsweise Trypsin, Chymotrypsin und Carboxypeptidase) (Imamura und Kitamoto, Am J Physiol Gastrointest Liver Physiol 285: G1235-G1241, 2003). Bisher wurde dieses Protein nicht mit humanen Tumoren in Zuammenhang gebracht.

Erfindungsgemäß wurde nach Etablierung einer PRSS7-spezifischen quantitativen RT-PCR (Primerpaar SEQ ID NO: 47, 48) die Verteilung spezifischer Transkripte in gesundem Gewebe und in Karzinomproben untersucht (Abb. 16; Methodik: vgl. Material und Methoden Abschnitt B.1.). In den meisten analysierten Geweben konnten wir eine PRSS7-spezifische Expression gar nicht oder nur in sehr geringem Ausmaß detektieren. (Abb. 16A). Relevante Expression fand sich lediglich im Duodenum (Abb. 16B).

PRSS7 wird von verschiedenen Tumortypen exprimiert. In einem Teil der untersuchten Magenkarzinome war im Vergleich zu normalem Magengewebe eine deutliche Überexpression von PRSS7 nachweisbar (Abb. 16B). Des Weiteren exprimieren Ösophagus-, Leber- sowie Pankreaskarzinome PRSS7, teilweise war das Gen in einigen Tumorproben im Vergleich zum entsprechenden Normalgewebe deutlich überexprimiert (Abb. 16B bis 16C).

Die ausgeprägte Expression und hohe Inzidenz dieses Moleküls für die dargestellten Tumorindikationen machen dieses Protein erfindungsgemäß zu einem hochinteressanten diagnostischen und therapeutischen Marker. Dies umfasst erfindungsgemäß den Nachweis disseminierter Tumorzellen im Serum, Knochenmark und Urin, sowie die Detektion von Metastasen in anderen Organen mittels RT-PCR.

Wir haben Zellen, die mit PRSS7 transfiziert worden sind, sowie Schnitte menschlicher Gewebe mit PRSS7-spezifischen Antikörpern gefärbt und konnten die prädezierte mebranständige Proteintopologie bestätigen (Abb. 17A und 17B).

Der extrazelluläre Teil von PRSS7 kann erfindungsgemäß als Zielstruktur von monoklonalen Antikörpern für Therapie wie auch Immundiagnostik genutzt werden. Bezogen auf die SEQ ID NO: 18 sind die Aminosäuren ab Aminosäurerest 50 extrazellulär lokalisiert. Des weiteren kann PRSS7 erfindungsgemäß als Vakzine (RNA, DNA, Protein, Peptide) zur Induktion Tumor-spezischer Immunantworten (T-und B-Zell vermittelte Immunreaktionen) eingesetzt werden. Dies umfasst erfindungsgemäß auch die Entwicklung von sogenannten "small compunds", die die biologische Aktivität von PRSS7 modulieren und zur Therapie von Tumoren eingesetzt werden können.

### Beispiel 11: Identifizierung von CLCA2 als diagnostisches und therapeutisches Krebs-Target

Das Gen CLCA2 oder "calcium activated chloride channel 2" (SEQ ID NO:19) gehört zur Familie der Chloridionen Transporter. Das Gen es besteht aus 14 Exons und liegt auf dem Chromosom 1 (lp31-p22). Das Gen kodiert für ein Protein' mit einer Länge von 943 Aminosäuren, das ein kalkuliertes Molekulargewicht von ca. 120 kDa hat. Experimentell konnten 5 Transmembrandomänen sowie eine große, N-terminal lokalisierte extrazelluläre Domäne nachgewiesen werden. CLCA2 ist ein Ionentransporter (Gruber, 1999. Am J Physiol 276, C1261-C1270).

CLCA2-Transkripte waren bisher in der Lunge, der Trachea und in der Brustdrüse (Gruber, 1999. Am J Physiol 276, C1261-C1270) sowie in Testis-, Prostata- und Uterusgewebe (Agnel, 1999. FEBS Letters 435, 295-301) beschrieben worden. Vergleichende Untersuchungen in einer umfassenden Sammlung von Geweben gab es bisher nicht.

Erfindungsgemäß wurde nach Etablierung einer CLCA2-spezifischen quantitativen RT-PCR (Primerpaar SEQ ID NO: 49, 50) die Verteilung spezifischer Transkripte in fast allen gesunden Gewebe des menschlichen Körpers und in Tumorproben untersucht (Abb. 18; Methodik: vgl. Material und Methoden, Abschnitt B.1.). Wir fanden eine differentielle Expression von CLCA2 in Normalgeweben. In den meisten analysierten Geweben ist eine Transkription nicht nachweisbar. Lediglich in Ösophagus, Haut, Pankreas, und deutlich geringer in Thymus, Blase, Kolon, Prostata konnten wir Expression nachweisen. Überraschenderweise fanden wir in einigen Tumortypen eine signifikante, bisher nicht beschriebene Expression von CLCA2. Insbesondere sind dies Tumoren des Hals-Nasen-Rachenraumes, sowie Bronchial-, Brust-, Speiseröhre-, Ovarial- und Pankreaskarzinome, die eine im Vergleich mit dem entsprechenden Normalgewebe eine um den Faktor 10-1000 erhöhte CLCA2-spezifische Expression aufweisen (Abb. 18).

Die ausgeprägte Expression und hohe Inzidenz dieses Moleküls für die dargestellten Tumorindikationen machen dieses Protein erfindungsgemäß zu einem hochinteressanten diagnostischen und therapeutischen Marker. Dies umfasst erfindungsgemäß den Nachweis disseminierter Tumorzellen im Serum, Knochenmark und Urin, sowie die Detektion von Metastasen in anderen Organen mittels RT-PCR.

Die beiden extrazellulären Domänen (bezogen auf SEQ ID NO: 20; Aminosäuren 1-235, 448-552 und 925-943) können erfindungsgemäß als Zielstrukturen von monoklonalen Antikörpern für Therapie wie auch Immundiagnostik genutzt werden.

Wir konnten durch Immunsierungen mit CLCA2-spezifischen Peptiden (SEQ ID NO: 100, SEQ ID NO: 101) Antikörper generieren, die CLCA2 auf Zelloberflächen anfärben. Zellen, die mit CLCA2 transfiziert wurden, exprimieren dieses Protein auf der Zellmembran (Abb. 19A). Die Tumorselektivität konnte durch den spezifischen Antikörper in der Immunfluoreszenz (Abb. 19B) bestätigt werden.

Des weiteren kann CLCA2 erfindungsgemäß als Vakzine (RNA, DNA, Protein, Peptide) zur Induktion Tumor-spezischer Immunantworten (T-und B-Zell vermittelte Immunreaktionen) eingesetzt werden. Dies umfasst erfindungsgemäß auch die Entwicklung von sogenannten "small compunds", die die biologische Aktivität von CLCA2 modulieren und zur Therapie von Tumoren eingesetzt werden können.

### Beispiel 12: Identifizierung von TM4SF4 ("transmembrane 4 superfamily member 4") als diagnostisches und therapeutisches Krebs-Target

Das Gen TM4SF4 (SEQ ID NO:21) mit seinem Translationsprodukt (SEQ ID NO: 22) ist ein Mitglied der Tetraspanin Familie (Hemler, 2001. J Cell Biol 155, 1103-07). Das Gen besteht aus 5 Exons und liegt auf dem Chromosom 3 (3q25).

Das Gen kodiert für ein Protein mit einer Länge von 202 Aminosäuren und einem kalkulierten Molekulargewicht von ca. 21,5 kDa. Prädiktionsprogramme sagen übereinstimmend 4 Transmembrandomänen für TM4SF4 voraus. Das Protein ist im Bereich der zweiten extrazellulären Domäne N-glykosyliert und in der Zellmembran lokalisiert. Es ist beschrieben, dass der Grad der N-Glykosylierung einen Einfluss auf die Regulation der Zellproliferation hat und diese mit zunehmender Glykosilierung inhibiert wird (Wice & Gordon, 1995. J Biol Chem 270, 21907-18). Tetraspanine bilden Komplexe mit verschiedenen Mitgliedern aus der Gruppe der Integrine. Diesen hochmolekularen Multi-Komplexen wird in der Zelle eine Vielzahl von wichtigen Funktionen zugeschrieben. So haben sie z.B. eine Funktion bei der Zell-Zell-Adhäsion und bei interzellulären Kontakten, bei der Signaltransduktion und bei der Zellmotilität (Bereditchevski, 2001. J Cell Sci 114, 4143-51).

TM4SF4-Transkripte sind im Periportalbereich der Leber sowie in spezifischen Darmabschnitten beschrieben, wurden jedoch in anderen Geweben und insbesondere in Tumoren bisher nicht analysiert (Wice & Gordon, 1995. J Biol Chem 270, 21907-18). Erfindungsgemäß wurde nach Etablierung einer TM4SF4-spezifischen quantitativen RT-PCR (Primerpaar SEQ ID NO: 51, 52) die Verteilung spezifischer Transkripte in gesundem Gewebe und in Karzinomproben untersucht (Abb. 20; Methodik: vgl. Material und Methoden, Abschnitt B.1.). Unsere Untersuchungen zeigen eine differentielle Verteilung der Expression in Normalgeweben. TM4SF4-spezifische Transkripte waren in erster Linie in normalen Lebergewebsproben nachweisbar. In mehreren anderen Normalgeweben (u.a. Pankreas) finden wir eine deutlich geringere (mindestens 10-fach) Expression. Nicht nachweisbar war die Expression in Gehirn, Herzmuskel, Skelettmuskel, Haut, Brustgewebe, Ovar, PBMC, Milz, Lymphknoten, Zervix. Entgegen der publizierten Vorhersage, dass TM4SF4 in Tumorgewebe herunterreguliert wird (Wice & Gordon, 1995. J Biol Chem 270, 21907-18), konnten wir eine zumindest vergleichbare TM4SF4-spezifische Expression in verschiedenen Tumoren nachweisen, teilweise war TM4SF4 in Tumoren überexprimiert (Abb. 20A). In einer detaillierten Expressionsanalyse konnten wir zudem nachweisen, das TM4SF4 entgegen der veröffentlichten Daten in Lebertumoren nicht suprimiert ist (Abb. 20B). Zudem war das Gen in 4/12 Kolontumorproben im Vergleich zu Kolon Normalgewebe überexprimiert (Abb. 20C).

Zur Herstellung von TM4SF4-spezifischen Antikörpern wurden die unter SEQ ID NO: 65 und 66 aufgeführten Peptide verwendet. Diese Antikörper konnten das TM4SF4-Protein in unterschiedlichen Größen, die putative Glykosilierungsmuster darstellen, erkennen (Abb. 21A). Des Weiteren konnte die Oberflächen-Lokalisation von TM4SF4 mittels Immunfluoreszenz (Abb. 21B) und die in der PCR gefundene Tumor-Selektivität mittels immunhistologischer Färbungen menschlicher Gewebe (Abb. 21 C) bestätigen werden. Zusammenfassend lässt sich TM4SF4 als ein Membranprotein charakterisieren, dessen Expression auf Zellsubpopulationen weniger ausgewählter Normalgewebe beschränkt ist. TM4SF4 ist vor allem in periportalen Hepatozyten in der Leber und in der apikalen Membran der Epithelien des Magendarmtraktes nachweisbar. Bei apikaler Proteinlokalisation ist das Protein in normalen Zellen durch Antikörper nicht erreichbar, weil dieses im Darmepithel lumenwärts gerichtet ist und keinen Bezug zum Gefäßsystem hat. Bei Darmtumoren allerdings sind solche im Normalgewebe nicht erreichbaren Moleküle durch die unkontrollierte Proliferation und die Neovaskularisierung des Tumors nicht mehr kompartimentiert, sondern für therapeutische Antikörper zugänglich.

Die beiden extrazellulären Domänen von TM4SF4 können somit erfindungsgemäß als Zielstrukturen von monoklonalen Antikörpern genutzt werden. Bezogen auf die SEQ ID NO: 22 sind die Aminosäuren 23-45 und 110-156 extrazellulär lokalisiert (Prädiktion erfolgte mit Hilfe der Software TMHMM2). Für die Peptide mit der SEQ ID NO: 65 und 66 konnten bereits erfolgreich polyklonale Antikörper generiert werden (Wice & Gordon, 1995. J Biol Chem 270, 21907-18). Für therapeutische Ansätze zur Entwicklung von tumorspezifischen Antikörpern eignen sich unter anderem die Peptide SEQ ID NO: 67 und SEQ ID NO: 68, die jeweils ein konserviertes Motiv "NXS/T" für posttranslationelle N-Glykosylierungen enthalten, wobei "X" für eine beliebige Aminosäure außer Prolin steht.

### Beispiel 13: Identifizierung von CLDN 19 als diagnostisches und therapeutisches Krebs-Target

Das Gen CLDN19 oder Claudin19 (SEQ ID NO:23) mit seinem Translationsprodukt (SEQ ID NO: 24) ist ein Mitglied der Claudin-Familie.

Das Gen kodiert für ein Protein mit einer Länge von 224 Aminosäuren, das ein kalkuliertes Molekulargewicht von ca. 21,5 kDa hat. Prädiktionsprogramme sagen übereinstimmend die für die Familie der Claudine charakteristischen 4 Transmembrandomänen für Claudin 19 voraus. Claudin 19 ist bisher selbst funktionell nicht näher charakterisiert. Für andere Mitglieder der Claudin-Familie sind Funktionen beschrieben. So spielen Claudine eine wichtige Rolle bei der Zell-Zell-Adhäsion und bei interzellulären Kontakten. Sie sind Teil von großen Molekülkomplexen und bilden so Membranporen ("tight junctions") für Zell-Zell-Kontakte.

Erfindungsgemäß wurde nach Etablierung einer CLDN19-spezifischen quantitativen RT-PCR (Primerpaar SEQ ID NO: 53, 54) die Verteilung spezifischer Transkripte in gesundem Gewebe und in Karzinomproben untersucht (Abb. 22; Methodik: vgl. Material und Methoden, Abschnitt B.1.). Überraschenderweise fanden wir eine differentielle Verteilung der Expression in Normalgeweben. In der Mehrzahl der Normalgewebe (insbesondere Gehirn, Herzmuskel, Skelettmuskel, Leber, Pankreas, PBMCs, Lunge, Brustgewebe, Ovar, Milz, Kolon, Magen, Lymphknoten, Ösophagus, Haut, Prostata) ist CLDN19 nicht nachweisbar. Lediglich in normalem Blasen-, Thymus- und Testisgewebe konnten wir CLDN19-Transkripte nachweisen. Die vergleichende Untersuchung von Tumorgeweben ergab überraschenderweise, dass CLDN19 von unterschiedlichen Tumoren exprimiert wird. Dies sind insbesondere Nieren-, Magen-, Leber- und Brustkarzinome, die im Vergleich zum korrespondierenden Normalgewebe eine bis zu 10fache Überexpression aufzeigen. Bisher war CLDN19 nicht im Zusammenhang mit humanen Tumoren beschrieben worden (Abb. 22A-22E).

Die ausgeprägte Expression und hohe Inzidenz dieses Moleküls für die dargestellten Tumorindikationen machen dieses Protein erfindungsgemäß zu einem hochinteressanten diagnostischen und therapeutischen Marker. Dies umfasst erfindungsgemäß den Nachweis disseminierter Tumorzellen im Serum, Knochenmark und Urin, sowie die Detektion von Metastasen in anderen Organen mittels RT-PCR.

Die beiden extrazellulären Domänen (Aminosäuren 28-76 und 142-160 bezogen auf SEQ ID NO: 24) von CLDN19 können erfindungsgemäß als Zielstrukturen von monoklonalen Antikörpern für eine Therapie wie auch Immundiagnostik genutzt werden.

Des weiteren kann CLDN19 erfindungsgemäß als Vakzine (RNA, DNA, Protein, Peptide) zur Induktion Tumor-spezischer Immunantworten (T-und B-Zell vermittelte Immunreaktionen) eingesetzt werden. Dies umfasst erfindungsgemäß auch die Entwicklung von sogenannten "small compunds", die die biologische Aktivität von CLDN19 modulieren und zur Therapie von Tumoren eingesetzt werden können.

### Beispiel 14: Identifizierung von ALPPL2 als diagnostisches und therapeutisches Krebs-Target

Das Gen ALPPL2 bzw. "Stammzell-spezifische Alkalische Phosphatase" oder GCAP (SEQ ID NO: 25) kodiert für ein Protein (SEQ ID NO: 26), das zur Familie der alkalischen Phosphatasen (AP) gehört. Diese besteht aus insgesamt vier sehr homologen Mitgliedern (Homologie: 90-98%). Das Gen kodiert für ein Transkript mit einer Länge von 2486 bp und besteht aus 11 Exons. ALPPL2 liegt auf dem Chromosom 2 (2q37.1) in der Nähe seiner nah verwandten Familienmitglieder ALPP und ALPI.

Das abgeleitete Protein hat eine Länge von 532 Aminosäuren und ein kalkuliertes Molekulargewicht von ca. 57,3 kDa. ALPPL2 ist glykosyliert und als Homodimer über einen GPI-Anker in der Plasmamembran lokalisiert. Die genaue physiologische Funktion des Enzyms ist nicht bekannt. Für Osteosarkome oder Paget's Disease wird die alkalische Phosphatase-Enzymaktivität als Tumormarker eingesetzt (Millán, 1995. Crit Rev Clin Lab Sci 32, 1-39). Allerdings ist diese Bestimmung unspezifisch und unabhängig von dem tatsächlich zugrunde liegenden Molekül. Es ist nicht klar, welche der drei obengenannten Phosphatasen, oder vielleicht noch weitere bisher nichtbekannte diese Aktivität ergeben.

ALPPL2 wurde bisher lediglich als diagnostischer Marker "in situ" für die Diagnose von Keimzelltumoren eingesetzt (Roelofs et al., 1999. J Pathol 189, 236-244).

Laut Literaturangaben, die sich auf ein beschränktes Ausgangs-Set von Gewebstypen beziehen, ist ALPPL2 in Testis und im Thymus sowie in einigen Stammzelltumoren exprimiert (LeDu, 2002. J Biol Chem 277, 49808-49814). Erfindungsgemäß wurde nach Etablierung einer ALPPL2-spezifischen quantitativen RT-PCR (Primerpaar SEQ ID NO: 55, 56) die Verteilung dieses Genproduktes in gesundem Gewebe und in Karzinomproben untersucht, wobei eine umfassende Diversität an Geweben, die u.a. auch alle Körpergewebe abbildeten, untersucht wurde (Abb. 23; Methodik: vgl. Material und Methoden B.1.). Wir detektierten in den meisten Normalgeweben (insbesondere Gehirn, Herzmuskel, Skelettmuskel, Leber, Pankreas, PBMCs, Brustgewebe, Ovar, Milz, Kolon, Magen, Lymphknoten, Ösophagus, Haut, Prostata) kein Protein. Wir wiesen Expression lediglich in normalem Testis- und Lungengewebe, und sehr schwach in Thymus und Kolon nach. Die vergleichende Untersuchung von Tumoren ergab allerdings überraschenderweise, dass ALPPL2 in signifikanter Menge von verschiedenen. Tumortypen exprimiert wird, insbesondere in den Karzinomen von Kolon, Magen, Pankreas, Ovar und Lunge, aber auch Karzinomen des Hals-Nasen-Rachenbereiches (Abb. 23A und 23B).

Die ausgeprägte Expression und hohe Inzidenz dieses Moleküls für die dargestellten Tumorindikationen machen dieses Protein erfindungsgemäß zu einem hochinteressanten diagnostischen und therapeutischen Marker. Dies umfasst erfindungsgemäß den Nachweis disseminierter Tumorzellen im Serum, Knochenmark und Urin, sowie die Detektion von Metastasen in anderen Organen mittels RT-PCR.

Das gesamte ALPPL2-Protein (SEQ ID NO: 26) ist extrazellulär lokalisiert und kann somit erfindungsgemäß als -Zielstruktur zur Entwicklung von monoklonalen Antikörpern für eine Therapie wie auch Immundiagnostik genutzt werden.

Des weiteren kann ALPPL2 erfindungsgemäß als Vakzine (RNA, DNA, Protein, Peptide) zur Induktion Tumor-spezischer Immunantworten (T-und B-Zell vermittelte Immunreaktionen) eingesetzt werden. Dies umfasst erfindungsgemäß auch die Entwicklung von sogenannten "small compunds", die die biologische Aktivität von ALPPL2 modulieren und zur Therapie von Tumoren eingesetzt werden können.

### Beispiel 15: Identifizierung von GPR64 als diagnostisches und therapeutisches Krebs-Target

Das Gen GPR64 bzw. "G-Protein gekoppelter Rezeptor 64" (SEQ ID NO: 27) und sein Translationsprodukt (SEQ ID NO: 28) gehört zur großen Gruppe der 7-Transmembran-Rezeptoren. Das Gen kodiert für ein Transkript mit einer Länge von 3045 bp und besteht aus 27 Exons. GPR64 liegt auf dem Chromosom X (Xp22). Das Gen kodiert für ein Protein mit einer Länge von 987 Aminosäuren, das ein kalkuliertes Molekulargewicht von ca. 108 kDa hat. Der N-terminale Bereich stellt eine extrazelluläre Domäne dar, die stark glykosyliert ist. Die genaue physiologische Funktion des Proteins ist nicht bekannt.

GPR64 ist bisher nur in einer kleinen Auswahl von Normalgeweben untersucht worden, von denen lediglich Nebenhodengewebe als exprimierend beschrieben wurde (Osterhoff, 1997. DNA Cell Biol 16, 379-389). Erfindungsgemäß haben wir eine GPR64-spezifische RT-PCR etabliert (Primerpaar SEQ ID NO: 57, 58) und die Verteilung dieses Genproduktes in einer umfassenden Sammlung gesunder Gewebe untersucht (Abb. 24; Methodik: vgl. Material und Methoden, Abschnitt B.1.). In vielen Normalgeweben ist GPR64 gar nicht detektierbar, einige weisen eine geringe Expression auf. Überraschenderweise ergab die Untersuchung dieses Proteins in Tumoren eine Überexpression, die über ein vielfaches relevanter Normalgewebe lag. So fanden wir in fast der Hälfte der Ovarialkarzinome eine deutliche Überexpression (Abb. 24A- bis 24C).

Die ausgeprägte Expression und hohe Inzidenz dieses Moleküls für die dargestellten Tumorindikationen machen dieses Protein erfindungsgemäß zu einem hochinteressanten diagnostischen und therapeutischen Marker. Dies umfasst erfindungsgemäß den Nachweis disseminierter Tumorzellen im Serum, Knochenmark und Urin, sowie die Detektion von Metastasen in anderen Organen mittels RT-PCR.

Die vier extrazellulären Domänen von GPR64 können erfindungsgemäß als Zielstrukturen von monoklonalen Antikörpern für eine Therapie wie auch Immundiagnostik genutzt werden. Bezogen auf die SEQ ID NO: 28 sind die Aminosäuren 1-625, 684-695, 754-784 und 854-856 extrazellulär lokalisiert.

Des weiteren kann GPR64 erfindungsgemäß als Vakzine (RNA, DNA, Protein, Peptide) zur Induktion Tumor-spezischer Immunantworten (T-und B-Zell vermittelte Immunreaktionen) eingesetzt werden. Dies umfasst erfindungsgemäß auch die Entwicklung von sogenannten "small compunds", die die biologische Aktivität von GPR64 modulieren und zur Therapie von Tumoren eingesetzt werden können.

### Beispiel 16: Identifizierung des Natrium/Kalium/Chloridtransporters SLC12A1 (solute carrier family 12) als diagnostisches und therapeutisches Krebs-Target

Das Gen SLC12A1 (SEQ ID NO: 29) kodiert für das Translationsprodukt (SEQ ID NO: 30) und gehört zur Familie der Natrium-Kalium-Chlorid-Kotransporter. Das Gen besteht aus 26 Exons und liegt auf Chromosom 15 (15q15-q21.1). Es kodiert für ein Protein mit einer Länge von 1099 Aminosäuren, das ohne sekundäre Modifikationen ein kalkuliertes Molekulargewicht von ca. 120 kDa hat. SLC12A1 ist ein integrales Membranprotein mit 10 Transmembrandomänen. SLC12A1 vermittelt die Reabsorption von Natriumchlorid in der Henle-Schleife und ist der Ansatzpunkt vieler klinisch relevanter Diuretika (Quaggin et al., Mammalian Genome 6: 557-561, 1995). Entsprechend ist dieses Molekül prinzipiell als Zielstruktur für Medikamente erreich- und angehbar, d.h. es ist "drugable". Erfindungsgemäß wurde nach Etablierung einer SLC12A1-spezifischen quantitativen RT-PCR (Primerpaar SEQ ID NO: 59, 60) die Verteilung spezifischer Transkripte in gesundem Gewebe und in Karzinomproben untersucht (Abb. 25). Wir bestätigten, dass sich in Normalgeweben die Expression von SLC12A1 in erster Linie, wie auch in der Literatur beschrieben, auf normales Nierengewebe beschränkt. In allen anderen Normalgeweben sind SLC12A1-spezifische Transkipte in sehr geringen Mengen oder gar nicht nachzuweisen (Abb. 25A). Überraschenderweise fanden wir bei der vergleichenden Analyse von Tumoren eine Expression von SLC12A1. Vor allem in Nieren-, Brust-, Ovarial- und Prostatakarzinomen (Abb. 25A bis 25C) fanden wir unerwartet eine bis zu 1.000.000-fache Überexpression im Vergleich zu den jeweils dazugehörigen Normalgeweben (Abb. 25B bis 25D). Bisher wurde SLC12A1 nicht im Zusammenhang mit Tumorerkrankungen beschrieben.

Die ausgeprägte Expression und hohe Inzidenz dieses Moleküls für die dargestellten Tumorindikationen machen dieses Protein erfindungsgemäß zu einem hochinteressanten diagnostischen und therapeutischen Marker. Dies umfasst erfindungsgemäß den Nachweis disseminierter Tumorzellen im Serum, Knochenmark und Urin, sowie die Detektion von Metastasen in anderen Organen mittels RT-PCR. Die extrazellulären Domänen von SLC12A1 können erfindungsgemäß als Zielstrukturen von monoklonalen Antikörpern für Therapie wie auch Immundiagnostik genutzt werden. Bezogen auf die SEQ ID NO: 30 sind die Aminosäuren 1-181, 234-257, 319-327, 402-415, 562-564 und 630-1099 extrazellulär lokalisiert.

Des weiteren kann SLC12A1 erfindungsgemäß als Vakzine (RNA, DNA, Protein, Peptide) zur Induktion Tumor-spezischer Immunantworten (T-und B-Zell vermittelte Immunreaktionen) eingesetzt werden. Dies umfasst erfindungsgemäß auch die Entwicklung von sogenannten "small compunds", die die biologische Aktivität von SLC12A1 modulieren und zur Therapie von Tumoren eingesetzt werden können.

## Patentansprüche

1. Pharmazeutische Zusammensetzung umfassend einen oder mehrer Bestandteile, die aus der Gruppe ausgewählt sind, bestehend aus:
(i) einem Tumor-assoziierten Antigen oder einem Teil davon,
(ii) einer Nukleinsäure, die für ein Tumor-assoziiertes Antigen oder einen Teil davon kodiert,
(iii) einem Antikörper, der an ein Tumor-assoziiertes Antigen oder einen Teil davon bindet,
(iv) einer Antisense-Nukleinsäure, die spezifisch mit einer Nukleinsäure, die für ein Tumor-assoziiertes Antigen kodiert, hybridisiert,
(v) einer Wirtszelle, die ein Tumor-assoziiertes Antigen oder einen Teil davon exprimiert, und
(vi) isolierten Komplexen zwischen einem Tumor-assoziierten Antigen oder einem Teil davon und einem HLA-Molekül,
wobei das Tumor-assoziierte Antigen eine Sequenz aufweist, die von einer Nukleinsäure kodiert wird, die aus der Gruppe ausgewählt ist, bestehend aus:
(a) einer Nukleinsäure, die eine Nukleinsäuresequenz umfasst, die aus der Gruppe bestehend aus SEQ ID NOs: 3, 1, 5, 7, 9, 11, 13, 15, 17, 19, 21, 23, 25, 27, 29, 69, 71, 73, 75, 79, 80, 85, 87, 102, 104, 106, 108, 110, 112, einem Teil oder Derivat davon ausgewählt ist,
(b) einer Nukleinsäure, die unter stringenten Bedingungen mit der Nukleinsäure unter (a) hybridisiert,
(c) einer Nukleinsäure, die in Bezug auf die Nukleinsäure unter (a) oder (b) degeneriert ist und
(d) einer Nukleinsäure, die zu der Nukleinsäure unter (a), (b) oder (c) komplementär ist.

2. Pharmazeutische Zusammensetzung nach Anspruch 1, wobei die Wirtszelle das Tumor-assoziierte Antigen oder den Teil davon sekretiert, auf der Oberfläche exprimiert oder ein HLA-Molekül exprimiert, das an das Tumor-assoziierte Antigen oder den Teil davon bindet.

3. Pharmazeutische Zusammensetzung nach Anspruch 1 oder 2 zur Behandlung einer Erkrankung, die sich durch die Expression oder abnormale Expression eines Tumor-assoziierten Antigens auszeichnet.

4. Verfahren zur Diagnose einer Erkrankung, die sich durch die Expression oder abnormale Expression eines Tumor-assoziierten Antigens auszeichnet, umfassend
(i) den Nachweis einer Nukleinsäure, die für das Tumor-assoziierte Antigen kodiert, oder eines Teils davon, und/oder
(ii) den Nachweis des Tumor-assoziierten Antigens oder eines Teils davon, und/oder
(iii) den Nachweis eines Antikörpers gegen das Tumor-assoziierte Antigen oder eines Teils davon und/oder
(iv) den Nachweis von cytotoxischen oder Helfer-T-Lymphozyten, die für das Tumor-assoziierte Antigen oder einen Teil davon spezifisch sind,
in einer aus einem Patienten isolierten biologischen Probe,
wobei das Tumor-assoziierte Antigen eine Sequenz aufweist, die von einer Nukleinsäure kodiert wird, die aus der Gruppe ausgewählt ist, bestehend aus:
(a) einer Nukleinsäure, die eine Nukleinsäuresequenz umfasst, die aus der Gruppe bestehend aus SEQ ID NOs: 3, 1, 5, 7, 9, 11, 13, 15, 17, 19, 21, 23, 25, 27, 29, 69, 71, 73, 75, 79, 80, 85, 87, 102, 104, 106, 108, 110, 112, einem Teil oder Derivat davon ausgewählt ist,
(b) einer Nukleinsäure, die unter stringenten Bedingungen mit der Nukleinsäure unter (a) hybridisiert,
(c) einer Nukleinsäure, die in Bezug auf die Nukleinsäure unter (a) oder (b) degeneriert ist und
(d) einer Nukleinsäure, die zu der Nukleinsäure unter (a), (b) oder (c) komplementär ist.

5. Verfahren zur Bestimmung der Regression, des Verlaufs oder des Ausbruchs einer Erkrankung, die sich durch die Expression oder abnormale Expression eines Tumor-assoziierten Antigens auszeichnet, umfassend die Überwachung einer Probe aus einem Patienten, der die Erkrankung aufweist oder in Verdacht steht, an der Erkrankung zu erkranken, in Bezug auf einen oder mehrere Parameter, ausgewählt aus der Gruppe, bestehend aus:
(i) der Menge der Nukleinsäure, die für das Tumor-assoziierte Antigen kodiert, oder eines Teils davon,
(ii) der Menge des Tumor-assoziierten Antigens oder eines Teils davon,
(iii) der Menge an Antikörpern, die an das Tumor-assoziierte Antigen oder einen Teil davon binden, und
(iv) der Menge an cytolytischen oder Cytokin-ausschüttenden T-Zellen, die für einen Komplex zwischen dem Tumor-assoziierten Antigen oder einem Teil davon und einem MHC-Molekül spezifisch sind,
wobei das Tumor-assoziierte Antigen eine Sequenz aufweist, die von einer Nukleinsäure kodiert wird, die aus der Gruppe ausgewählt ist, bestehend aus:
(a) einer Nukleinsäure, die eine Nukleinsäuresequenz umfasst, die aus der Gruppe bestehend aus SEQ ID NOs: 3, 1, 5, 7, 9, 11, 13, 15, 17, 19, 21, 23, 25, 27, 29, 69, 71, 73, 75, 79, 80, 85, 87, 102, 104, 106, 108, 110, 112, einem Teil oder Derivat davon ausgewählt ist,
(b) einer Nukleinsäure, die unter stringenten Bedingungen mit der Nukleinsäure unter (a) hybridisiert,
(c) einer Nukleinsäure, die in Bezug auf die Nukleinsäure unter (a) oder (b) degeneriert ist und
(d) einer Nukleinsäure, die zu der Nukleinsäure unter (a), (b) oder (c) komplementär ist.

6. Antikörper, der spezifisch an ein Protein oder Polypeptid oder an einen Teil davon bindet, wobei das Protein oder Polypeptid von einer Nukleinsäure kodiert wird, die aus der Gruppe ausgewählt ist, bestehend aus:
(a) einer Nukleinsäure, die eine Nukleinsäuresequenz umfasst, die aus der Gruppe bestehend aus SEQ ID NOs: 3, 1, 5, 7, 9, 11, 13, 15, 17, 19, 21, 23, 25, 27, 29, 69, 71, 73, 75, 79, 80, 85, 87, 102, 104, 106, 108, 110, 112, einem Teil oder Derivat davon ausgewählt ist,
(b) einer Nukleinsäure, die unter stringenten Bedingungen mit der Nukleinsäure unter (a) hybridisiert,
(c) einer Nukleinsäure, die in Bezug auf die Nukleinsäure unter (a) oder (b) degeneriert ist und
(d) einer Nukleinsäure, die zu der Nukleinsäure unter (a), (b) oder (c) komplementär ist.

7. Pharmazeutische Zusammensetzung nach Anspruch 1 oder Antikörper nach Anspruch 6, wobei der Antikörper mit einem therapeutischen oder diagnostischen Mittel gekoppelt ist.

8. Antikörper nach Anspruch 7 zur Verwendung in einem Verfahren zur Behandlung, Diagnose oder Überwachung einer Erkrankung, die sich durch die Expression oder abnormale Expression eines Tumor-assoziierten Antigens auszeichnet, wobei das Verfahren die Verabreichung des Antikörpers umfasst und das Tumor-assoziierte Antigen eine Sequenz aufweist, die von einer Nukleinsäure kodiert wird, die aus der Gruppe ausgewählt ist, bestehend aus:
(a) einer Nukleinsäure, die eine Nukleinsäuresequenz umfasst, die aus der Gruppe bestehend aus SEQ ID NOs: 3, 1, 5, 7, 9, 11, 13, 15, 17, 19, 21, 23, 25, 27, 29, 69, 71, 73, 75, 79, 80, 85, 87, 102, 104, 106, 108, 110, 112, einem Teil oder Derivat davon ausgewählt ist,
(b) einer Nukleinsäure, die unter stringenten Bedingungen mit der Nukleinsäure unter (a) hybridisiert,
(c) einer Nukleinsäure, die in Bezug auf die Nukleinsäure unter (a) oder (b) degeneriert ist und
(d) einer Nukleinsäure, die zu der Nukleinsäure unter (a), (b) oder (c) komplementär ist.

9. Pharmazeutische Zusammensetzung nach Anspruch 1 oder 7 oder Antikörper nach einem der Ansprüche 6 bis 8, wobei der Antikörper ein monoklonaler, chimärer oder humanisierter Antikörper oder ein Fragment eines Antikörpers ist.

10. Cytolytische oder Cytokine-ausschüttende T-Zellen zur Verwendung in einem Verfahren zur Behandlung eines Patienten mit einer Erkrankung, die sich durch die Expression oder abnormale Expression eines Tumor-assoziierten Antigens auszeichnet, umfassend:
(i) die Entfernung einer Probe mit immunreaktiven Zellen aus dem Patienten,
(ii) die Kontaktierung der Probe mit einer Wirtszelle, die das Tumor-assoziierte Antigen oder einen Teil davon exprimiert, unter Bedingungen, die eine Produktion cytolytischer oder Cytokine-ausschüttender T-Zellen gegen das Tumor-assoziierte Antigen oder einen Teil davon begünstigen, und
(iii) das Einbringen der cytolytischen oder Cytokine-ausschüttenden T-Zellen in den Patienten in einer Menge, die geeignet ist, Zellen zu lysieren, die das Tumor-assoziierte Antigen oder einen Teil davon exprimieren, wobei das Tumor-assoziierte Antigen eine Sequenz aufweist, die von einer Nukleinsäure kodiert wird, die aus der Gruppe ausgewählt ist, bestehend aus:
(a) einer Nukleinsäure, die eine Nukleinsäuresequenz umfasst, die aus der Gruppe bestehend aus SEQ ID NOs: 3, 1, 5, 7, 9, 11, 13, 15, 17, 19, 21, 23, 25, 27, 29, 69, 71, 73, 75, 79, 80, 85, 87, 102, 104, 106, 108, 110, 112, einem Teil oder Derivat davon ausgewählt ist,
(b) einer Nukleinsäure, die unter stringenten Bedingungen mit der Nukleinsäure unter (a) hybridisiert,
(c) einer Nukleinsäure, die in Bezug auf die Nukleinsäure unter (a) oder (b) degeneriert ist und
(d) einer Nukleinsäure, die zu der Nukleinsäure unter (a), (b) oder (c) komplementär ist.

11. Cytolytische oder Cytokine-ausschüttende T-Zellen nach Anspruch 10, wobei die Wirtszelle ein HLA-Molekül rekombinant oder endogen exprimiert, das an das Tumor-assoziierte Antigen oder einen Teil davon bindet.

12. Pharmazeutische Zusammensetzung nach Anspruch 3, Verfahren nach Anspruch 4 oder 5, Antikörper nach Anspruch 8 oder 9, oder cytolytische oder Cytokine-ausschüttende T-Zellen nach Anspruch 10 oder 11, wobei die Erkrankung Krebs ist.

13. Pharmazeutische Zusammensetzung nach Anspruch 3, Verfahren nach Anspruch 4 oder 5, Antikörper nach einem der Ansprüche 8 oder 9, oder cytolytische oder Cytokine-ausschüttende T-Zellen nach Anspruch 10 oder 11, wobei die Erkrankung ein Lungentumor, ein Brusttumor, ein Prostatatumor, ein Melanom, ein Colontumor, eine Metastase eines Colontumors, ein Nierenzellkarzinom, ein Zervixkarzinom, ein Coloncarcinom oder ein Mammacarcinom ist.

14. Pharmazeutische Zusammensetzung nach einem der Ansprüche 1 bis 3, 7, 9, 12 und 13, Verfahren nach einem der Ansprüche 4, 5, 12 und 13, Antikörper nach einem der Ansprüche 6 bis 9, 12 und 13, oder cytolytische oder Cytokine-ausschüttende T-Zellen nach einem der Ansprüche 10 bis 13, wobei das Protein oder Polypeptid oder das Tumor-assoziierte Antigen eine Aminosäuresequenz umfasst, die aus der Gruppe bestehend aus SEQ ID NOs: 4, 2, 6, 8, 10, 12, 14, 16, 18, 20, 22, 24, 26, 28, 30, 61 bis 68, 70, 72, 74, 76, 81, 82, 86, 88, 96 bis 101, 103, 105, 107, 109, 111, 113, einem Teil oder Derivat davon ausgewählt ist.

15. Nukleinsäure, die für ein Protein oder Polypeptid kodiert, das eine Aminosäuresequenz umfasst, ausgewählt aus der Gruppe, bestehend aus SEQ ID NOs: 4, 61 bis 68, 70, 72, 74, 81, 82, 86, 88, 96 bis 101, 103, 105, 107, 109, 111, 113, einem Teil oder Derivat davon.

16. Nukleinsäure nach Anspruch 15, die aus der Gruppe ausgewählt ist, bestehend aus:
(a) einer Nukleinsäure, die eine Nukleinsäuresequenz umfasst, die aus der Gruppe bestehend aus SEQ ID NOs: 3, 69, 71, 73, 79, 80, 85, 87, 102, 104, 106, 108, 110, 112, einem Teil oder Derivat davon ausgewählt ist,
(b) einer Nukleinsäure, die unter stringenten Bedingungen mit der Nukleinsäure unter (a) hybridisiert,
(c) einer Nukleinsäure, die in Bezug auf die Nukleinsäure unter (a) oder (b) degeneriert ist und
(d) einer Nukleinsäure, die zu der Nukleinsäure unter (a), (b) oder (c) komplementär ist.

17. Wirtszelle, die eine Nukleinsäure nach Anspruch 15 oder 16 umfasst.

18. Protein oder Polypeptid, das von einer Nukleinsäure nach Anspruch 15 oder 16 kodiert wird, oder ein immunogenes Fragment des Proteins oder Polypeptids.

19. Protein oder Polypeptid, das eine Aminosäuresequenz umfasst, ausgewählt aus der Gruppe, bestehend aus SEQ ID NOs: 4, 61 bis 68, 70, 72, 74, 81, 82, 86, 88, 96 bis 101, 103, 105, 107, 109, 111, 113, einem Teil oder Derivat davon, oder ein immunogenes Fragment des Proteins oder Polypeptids.

20. Kit zum Nachweis der Expression oder abnormalen Expression eines Tumor-assoziierten Antigens, umfassend Mittel zum Nachweis
(i) der Nukleinsäure, die für das Tumor-assoziierte Antigen kodiert, oder eines Teils davon,
(ii) des Tumor-assoziierten Antigens oder eines Teils davon,
(iii) von Antikörpern, die an das Tumor-assoziierte Antigen oder einen Teil davon binden, und/oder
(iv) von T-Zellen, die für einen Komplex zwischen dem Tumor-assoziierten Antigen oder einem Teil davon und einem MHC-Molekül spezifisch sind,
wobei das Tumor-assoziierte Antigen eine Sequenz aufweist, die von einer Nukleinsäure kodiert wird, die aus der Gruppe ausgewählt ist, bestehend aus:
(a) einer Nukleinsäure, die eine Nukleinsäuresequenz umfasst, die aus der Gruppe bestehend aus SEQ ID NOs: 3, 1, 5, 7, 9, 11, 13, 15, 17, 19, 21, 23, 25, 27, 29, 69, 71, 73, 75, 79, 80, 85, 87, 102, 104, 106, 108, 110, 112, einem Teil oder Derivat davon ausgewählt ist,
(b) einer Nukleinsäure, die unter stringenten Bedingungen mit der Nukleinsäure unter (a) hybridisiert,
(c) einer Nukleinsäure, die in Bezug auf die Nukleinsäure unter (a) oder (b) degeneriert ist und
(d) einer Nukleinsäure, die zu der Nukleinsäure unter (a), (b) oder (c) komplementär ist.


SEQUENZPROTOKOLL

<110> Ganymed Pharmaceuticals AG

<120> Identifizierung von Oberflächen-assoziierten Antigenen für die
Tumordiagnose und -therapie

<130> 342-20 EPT8

<150> DE 10 2004 023 187.7
<151> 2004-05-11

<160> 113

<170> PatentIn version 3.3

<210> 1
<211> 1785
<212> DNA
<213> Homo sapiens

<220>
<221> CDS
<222> (204)..(614)

<400> 1
actcagctct ctcaccatgc gattgccctg caacaccttg gaactctgca gagagtcccc 60

agcaggaagg tctcacctga ggtgaacctt cgaccttgga cttctcagcc tccagaatga 120

agaatggcaa ccatcaaatc aagaaattgg cccaaagccc tacagtctgc aaacatcata 180

acaattcatc ctgaagtttc tcc atg aat tgt gat gct ttg cta cat cat tct 233
Met Asn Cys Asp Ala Leu Leu His His Ser
1               5                   10

gca atc cca gaa gat ttt ttg cat att ttt ttg cta tta cag aaa atc 281
Ala Ile Pro Glu Asp Phe Leu His Ile Phe Leu Leu Leu Gln Lys Ile
                15                  20                  25

tca gtc tcc ctc cct ctc tct ctc tct caa tct gtg tgt ctc ttt tac 329
Ser Val Ser Leu Pro Leu Ser Leu Ser Gln Ser Val Cys Leu Phe Tyr
            30                  35                  40

tcc ata tct ctg tgt gtg tct ctt tta ctc cat atc tct ctg tgt gtg 377
Ser Ile Ser Leu Cys Val Ser Leu Leu Leu His Ile Ser Leu Cys Val
        45                  50                  55

tct gtt tat gtc tct ctc tct ctc tca tcc ttc cca tgt ttc tct ctc 425
Ser Val Tyr Val Ser Leu Ser Leu Ser Ser Phe Pro Cys Phe Ser Leu
    60                  65                  70

aca cac aca cac act cat tca cag ctt tca aaa gac acg tct gtc ctt 473
Thr His Thr His Thr His Ser Gln Leu Ser Lys Asp Thr Ser Val Leu
75                  80                  85                  90

acc ttc act ttt tgt ttt aaa cag cac act cac ttt act ctg aac tat 521
Thr Phe Thr Phe Cys Phe Lys Gln His Thr His Phe Thr Leu Asn Tyr
                95                  100                 105

acc tca cat gca cac gag ctt tct gct cca tct gtt cat ccc aca tgt 569
Thr Ser His Ala His Glu Leu Ser Ala Pro Ser Val His Pro Thr Cys
            110                 115                 120

gtc ttc aca ttc aaa gca gca cct tcc cca aga cca gct acc taa 614
Val Phe Thr Phe Lys Ala Ala Pro Ser Pro Arg Pro Ala Thr
        125                 130                 135

ccacctccca cctccacccc atccctagtc agaggaaggc ctggttccca cctgaattca 674

gctttgtcaa agagcctcct ggaaagctgt catcttcagt tagtagggat aatgggatta 734

ttctatctgt gtaataataa catgttcaat ttaaagaaaa aaatctgaag ccacttaaaa 794

gctactgttt ggcaccgata cattattcca gtaatgaata atcattaaag atattattct 854

ggatgcagtt accatgcagt gatgtgaata aaatgcatta gatggaaaat tgtatttcaa 914

gtaaatatat gcactggtag aaatgtatta ccacccacta atatgtatta attcaaaacc 974

aaatgccaac tggagttcgc ctacacgggt ttgaatggca ggcagtgatt tggaagtggg 1034

aggaaatagg tttggatttg gtcaaataga ctgagaagtg atagtggggg cgggggttta 1094

tgactcaaac tttaacaggt gagaagacta tgccatggac agaacaggca tgaggggctc 1154

ccctcctacg cctctttaag agatttttat ctctgactaa ggattactgg tagttgttga 1214

catttctgaa gcagtggatc tttttccttt ttcactatct gcatcttcaa atattctttt 1274

ctgaagaaag ttaaaaggaa gcctgtacat tttttgctaa ggtaaatgcc ttgccatctt 1334

atttcatttt ctcatttttt tcttcagtgc acaacataag caactgtcct ccttgtcata 1394

ctcaagatga gcttggcata tctgaaatct gcagggattt tctcattagc acagggttcc 1454

aagcccaaac cgtgaagatg gagttttcat ttttaaatgg cacatcttca agttcttgcc 1514

ctgtcctcac tttagtatgc cccagaggaa gtcaaagata tggacactct aagactcaga 1574

agaactttct caggcattca ttttcctatc tattttgagc cattttattt aaaaggttac 1634

aattttaaac ctctctttaa ttaaaagata ccagagttac aatgcaatac tatttggcaa 1694

tcaaaactaa tgaagcacag atgcatgcta caacacgaat gaactttgaa acgttgtgtt 1754

aagtgaaata aaccagttat tatacaaggc c 1785

<210> 2
<211> 136
<212> PRT
<213> Homo sapiens

<400> 2

Met Asn Cys Asp Ala Leu Leu His His Ser Ala Ile Pro Glu Asp Phe
1               5                   10                  15

Leu His Ile Phe Leu Leu Leu Gln Lys Ile Ser Val Ser Leu Pro Leu
            20                  25                  30

Ser Leu Ser Gln Ser Val Cys Leu Phe Tyr Ser Ile Ser Leu Cys Val
        35                  40                  45

Ser Leu Leu Leu His Ile Ser Leu Cys Val Ser Val Tyr Val Ser Leu
    50                  55                  60

Ser Leu Ser Ser Phe Pro Cys Phe Ser Leu Thr His Thr His Thr His
65                  70                  75                  80

Ser Gln Leu Ser Lys Asp Thr Ser Val Leu Thr Phe Thr Phe Cys Phe
                85                  90                  95

Lys Gln His Thr His Phe Thr Leu Asn Tyr Thr Ser His Ala His Glu
            100                 105                 110

Leu Ser Ala Pro Ser Val His Pro Thr Cys Val Phe Thr Phe Lys Ala
        115                 120                 125

Ala Pro Ser Pro Arg Pro Ala Thr
    130                 135

<210>  3
<211>  3336
<212>  DNA
<213>  Homo sapiens

<220>
<221>  CDS
<222>  (84)..(3083)

<400>  3
ttttcttaga cattaactgc agacggctgg caggatagaa gcagcggctc acttggactt      60

tttcaccagg gaaatcagag aca atg atg ggg ctc ttc ccc aga act aca ggg     113
                          Met Met Gly Leu Phe Pro Arg Thr Thr Gly
                          1               5                   10

gct ctg gcc atc ttc gtg gtg gtc ata ttg gtt cat gga gaa ttg cga     161
Ala Leu Ala Ile Phe Val Val Val Ile Leu Val His Gly Glu Leu Arg
                15                  20                  25

ata gag act aaa ggt caa tat gat gaa gaa gag atg act atg caa caa     209
Ile Glu Thr Lys Gly Gln Tyr Asp Glu Glu Glu Met Thr Met Gln Gln
            30                  35                  40

gct aaa aga agg caa aaa cgt gaa tgg gtg aaa ttt gcc aaa ccc tgc     257
Ala Lys Arg Arg Gln Lys Arg Glu Trp Val Lys Phe Ala Lys Pro Cys
        45                  50                  55

aga gaa gga gaa gat aac tca aaa aga aac cca att gcc aag att act     305
Arg Glu Gly Glu Asp Asn Ser Lys Arg Asn Pro Ile Ala Lys Ile Thr
    60                  65                  70

tca gat tac caa gca acc cag aaa atc acc tac cga atc tct gga gtg     353
Ser Asp Tyr Gln Ala Thr Gln Lys Ile Thr Tyr Arg Ile Ser Gly Val
75                  80                  85                  90

gga atc gat cag ccg cct ttt gga atc ttt gtt gtt gac aaa aac act     401
Gly Ile Asp Gln Pro Pro Phe Gly Ile Phe Val Val Asp Lys Asn Thr
                95                  100                 105

gga gat att aac ata aca gct ata gtc gac cgg gag gaa act cca agc     449
Gly Asp Ile Asn Ile Thr Ala Ile Val Asp Arg Glu Glu Thr Pro Ser
            110                 115                 120

ttc ctg atc aca tgt cgg gct cta aat gcc caa gga cta gat gta gag     497
Phe Leu Ile Thr Cys Arg Ala Leu Asn Ala Gln Gly Leu Asp Val Glu
        125                 130                 135

aaa cca ctt ata cta acg gtt aaa att ttg gat att aat gat aat cct     545
Lys Pro Leu Ile Leu Thr Val Lys Ile Leu Asp Ile Asn Asp Asn Pro

140                 145                 150

cca gta ttt tca caa caa att ttc atg ggt gaa att gaa gaa aat agt     593
Pro Val Phe Ser Gln Gln Ile Phe Met Gly Glu Ile Glu Glu Asn Ser
155                 160                 165                 170

gcc tca aac tca ctg gtg atg ata cta aat gcc aca gat gca gat gaa     641
Ala Ser Asn Ser Leu Val Met Ile Leu Asn Ala Thr Asp Ala Asp Glu
                175                 180                 185

cca aac cac ttg aat tct aaa att gcc ttc aaa att gtc tct cag gaa     689
Pro Asn His Leu Asn Ser Lys Ile Ala Phe Lys Ile Val Ser Gln Glu
            190                 195                 200

cca gca ggc aca ccc atg ttc ctc cta agc aga aac act ggg gaa gtc     737
Pro Ala Gly Thr Pro Met Phe Leu Leu Ser Arg Asn Thr Gly Glu Val
        205                 210                 215

cgt act ttg acc aat tct ctt gac cga gag caa gct agc agc tat cgt     785
Arg Thr Leu Thr Asn Ser Leu Asp Arg Glu Gln Ala Ser Ser Tyr Arg
    220                 225                 230

ctg gtt gtg agt ggt gca gac aaa gat gga gaa gga cta tca act caa     833
Leu Val Val Ser Gly Ala Asp Lys Asp Gly Glu Gly Leu Ser Thr Gln
235                 240                 245                 250

tgt gaa tgt aat att aaa gtg aaa gat gtc aac gat aac ttc cca atg     881
Cys Glu Cys Asn Ile Lys Val Lys Asp Val Asn Asp Asn Phe Pro Met
                255                 260                 265

ttt aga gac tct cag tat tca gca cgt att gaa gaa aat att tta agt     929
Phe Arg Asp Ser Gln Tyr Ser Ala Arg Ile Glu Glu Asn Ile Leu Ser
            270                 275                 280

tct gaa tta ctt cga ttt caa gta aca gat ttg gat gaa gag tac aca     977
Ser Glu Leu Leu Arg Phe Gln Val Thr Asp Leu Asp Glu Glu Tyr Thr
        285                 290                 295

gat aat tgg ctt gca gta tat ttc ttt acc tct ggg aat gaa gga aat     1025
Asp Asn Trp Leu Ala Val Tyr Phe Phe Thr Ser Gly Asn Glu Gly Asn
    300                 305                 310

tgg ttt gaa ata caa act gat cct aga act aat gaa ggc atc ctg aaa     1073
Trp Phe Glu Ile Gln Thr Asp Pro Arg Thr Asn Glu Gly Ile Leu Lys
315                 320                 325                 330

gtg gtg aag gct cta gat tat gaa caa cta caa agc gtg aaa ctt agt     1121
Val Val Lys Ala Leu Asp Tyr Glu Gln Leu Gln Ser Val Lys Leu Ser
                335                 340                 345

att gct gtc aaa aac aaa gct gaa ttt cac caa tca gtt atc tct cga     1169
Ile Ala Val Lys Asn Lys Ala Glu Phe His Gln Ser Val Ile Ser Arg
            350                 355                 360

tac cga gtt cag tca acc cca gtc aca att cag gta ata aat gta aga     1217
Tyr Arg Val Gln Ser Thr Pro Val Thr Ile Gln Val Ile Asn Val Arg
        365                 370                 375

gaa gga att gca ttc cgt cct gct tcc aag aca ttt act gtg caa aaa     1265
Glu Gly Ile Ala Phe Arg Pro Ala Ser Lys Thr Phe Thr Val Gln Lys
    380                 385                 390

ggc ata agt agc aaa aaa ttg gtg gat tat atc ctg gga aca tat caa     1313
Gly Ile Ser Ser Lys Lys Leu Val Asp Tyr Ile Leu Gly Thr Tyr Gln
395                 400                 405                 410

gcc atc gat gag gac act aac aaa gct gcc tca aat gtc aaa tat gtc     1361
Ala Ile Asp Glu Asp Thr Asn Lys Ala Ala Ser Asn Val Lys Tyr Val

                415                 420                 425

atg gga cgt aac gat ggt gga tac cta atg att gat tca aaa act gct 1409
Met Gly Arg Asn Asp Gly Gly Tyr Leu Met Ile Asp Ser Lys Thr Ala
            430                 435                 440

gaa atc aaa ttt gtc aaa aat atg aac cga gat tct act ttc ata gtt 1457
Glu Ile Lys Phe Val Lys Asn Met Asn Arg Asp Ser Thr Phe Ile Val
        445                 450                 455

aac aaa aca atc aca gct gag gtt ctg gcc ata gat gaa tac acg ggt 1505
Asn Lys Thr Ile Thr Ala Glu Val Leu Ala Ile Asp Glu Tyr Thr Gly
    460                 465                 470

aaa act tct aca ggc acg gta tat gtt aga gta ccc gat ttc aat gac 1553
Lys Thr Ser Thr Gly Thr Val Tyr Val Arg Val Pro Asp Phe Asn Asp
475                 480                 485                 490

aat tgt cca aca gct gtc ctc gaa aaa gat gca gtt tgc agt tct tca 1601
Asn Cys Pro Thr Ala Val Leu Glu Lys Asp Ala Val Cys Ser Ser Ser
                495                 500                 505

cct tcc gtg gtt gtc tcc gct aga aca ctg aat aat aga tac act ggc 1649
Pro Ser Val Val Val Ser Ala Arg Thr Leu Asn Asn Arg Tyr Thr Gly
            510                 515                 520

ccc tat aca ttt gca ctg gaa gat caa cct gta aag ttg cct gcc gta 1697
Pro Tyr Thr Phe Ala Leu Glu Asp Gln Pro Val Lys Leu Pro Ala Val
        525                 530                 535

tgg agt atc aca acc ctc aat gct acc tcg gcc ctc ctc aga gcc cag 1745
Trp Ser Ile Thr Thr Leu Asn Ala Thr Ser Ala Leu Leu Arg Ala Gln
    540                 545                 550

gaa cag ata cct cct gga gta tac cac atc tcc ctg gta ctt aca gac 1793
Glu Gln Ile Pro Pro Gly Val Tyr His Ile Ser Leu Val Leu Thr Asp
555                 560                 565                 570

agt cag aac aat cgg tgt gag atg cca cgc agc ttg aca ctg gaa gtc 1841
Ser Gln Asn Asn Arg Cys Glu Met Pro Arg Ser Leu Thr Leu Glu Val
                575                 580                 585

tgt cag tgt gac aac agg ggc atc tgt gga act tct tac cca acc aca 1889
Cys Gln Cys Asp Asn Arg Gly Ile Cys Gly Thr Ser Tyr Pro Thr Thr
            590                 595                 600

agc cct ggg acc agg tat ggc agg ccg cac tca ggg agg ctg ggg cct 1937
Ser Pro Gly Thr Arg Tyr Gly Arg Pro His Ser Gly Arg Leu Gly Pro
        605                 610                 615

gcc gcc atc ggc ctg ctg ctc ctt ggt ctc ctg ctg ctg ctg ttg gcc 1985
Ala Ala Ile Gly Leu Leu Leu Leu Gly Leu Leu Leu Leu Leu Leu Ala
    620                 625                 630

ccc ctt ctg ctg ttg acc tgt gac tgt ggg gca ggt tct act ggg gga 2033
Pro Leu Leu Leu Leu Thr Cys Asp Cys Gly Ala Gly Ser Thr Gly Gly
635                 640                 645                 650

gtg aca ggt ggt ttt atc cca gtt cct gat ggc tca gaa gga aca att 2081
Val Thr Gly Gly Phe Ile Pro Val Pro Asp Gly Ser Glu Gly Thr Ile
                655                 660                 665

cat cag tgg gga att gaa gga gcc cat cct gaa gac aag gaa atc aca 2129
His Gln Trp Gly Ile Glu Gly Ala His Pro Glu Asp Lys Glu Ile Thr
            670                 675                 680

aat att tgt gtg cct cct gta aca gcc aat gga gcc gat ttc atg gaa 2177
Asn Ile Cys Val Pro Pro Val Thr Ala Asn Gly Ala Asp Phe Met Glu

        685                 690                 695

agt tct gaa gtt tgt aca aat acg tat gcc aga ggc aca gcg gtg gaa 2225
Ser Ser Glu Val Cys Thr Asn Thr Tyr Ala Arg Gly Thr Ala Val Glu
    700                 705                 710

ggc act tca gga atg gaa atg acc act aag ctt gga gca gcc act gaa 2273
Gly Thr Ser Gly Met Glu Met Thr Thr Lys Leu Gly Ala Ala Thr Glu
715                 720                 725                 730

tct gga ggt gct gca ggc ttt gca aca ggg aca gtg tca gga gct gct 2321
Ser Gly Gly Ala Ala Gly Phe Ala Thr Gly Thr Val Ser Gly Ala Ala
                735                 740                 745

tca gga ttc gga gca gcc act gga gtt ggc atc tgt tcc tca ggg cag 2369
Ser Gly Phe Gly Ala Ala Thr Gly Val Gly Ile Cys Ser Ser Gly Gln
            750                 755                 760

tct gga acc atg aga aca agg cat tcc act gga gga acc aat aag gac 2417
Ser Gly Thr Met Arg Thr Arg His Ser Thr Gly Gly Thr Asn Lys Asp
        765                 770                 775

tac gct gat ggg gcg ata agc atg aat ttt ctg gac tcc tac ttt tct 2465
Tyr Ala Asp Gly Ala Ile Ser Met Asn Phe Leu Asp Ser Tyr Phe Ser
    780                 785                 790

cag aaa gca ttt gcc tgt gcg gag gaa gac gat ggc cag gaa gca aat 2513
Gln Lys Ala Phe Ala Cys Ala Glu Glu Asp Asp Gly Gln Glu Ala Asn
795                 800                 805                 810

gac tgc ttg ttg atc tat gat aat gaa ggc gca gat gcc act ggt tct 2561
Asp Cys Leu Leu Ile Tyr Asp Asn Glu Gly Ala Asp Ala Thr Gly Ser
                815                 820                 825

cct gtg ggc tcc gtg ggt tgt tgc agt ttt att gct gat gac ctg gat 2609
Pro Val Gly Ser Val Gly Cys Cys Ser Phe Ile Ala Asp Asp Leu Asp
            830                 835                 840

gac agc ttc ttg gac tca ctt gga ccc aaa ttt aaa aaa ctt gca gag 2657
Asp Ser Phe Leu Asp Ser Leu Gly Pro Lys Phe Lys Lys Leu Ala Glu
        845                 850                 855

ata agc ctt ggt gtt gat ggt gaa ggc aaa gaa gtt cag cca ccc tct 2705
Ile Ser Leu Gly Val Asp Gly Glu Gly Lys Glu Val Gln Pro Pro Ser
    860                 865                 870

aaa gac agc ggt tat ggg att gaa tcc tgt ggc cat ccc ata gaa gtc 2753
Lys Asp Ser Gly Tyr Gly Ile Glu Ser Cys Gly His Pro Ile Glu Val
875                 880                 885                 890

cag cag aca gga ttt gtt aag tgc cag act ttg tca gga agt caa gga 2801
Gln Gln Thr Gly Phe Val Lys Cys Gln Thr Leu Ser Gly Ser Gln Gly
                895                 900                 905

gct tct gct ttg tcc gcc tct ggg tct gtc cag cca gct gtt tcc atc 2849
Ala Ser Ala Leu Ser Ala Ser Gly Ser Val Gln Pro Ala Val Ser Ile
            910                 915                 920

cct gac cct ctg cag cat ggt aac tat tta gta acg gag act tac tcg 2897
Pro Asp Pro Leu Gln His Gly Asn Tyr Leu Val Thr Glu Thr Tyr Ser
        925                 930                 935

gct tct ggt tcc ctc gtg caa cct tcc act gca ggc ttt gat cca ctt 2945
Ala Ser Gly Ser Leu Val Gln Pro Ser Thr Ala Gly Phe Asp Pro Leu
    940                 945                 950

ctc aca caa aat gtg ata gtg aca gaa agg gtg atc tgt ccc att tcc 2993
Leu Thr Gln Asn Val Ile Val Thr Glu Arg Val Ile Cys Pro Ile Ser

955                 960                 965                 970

agt gtt cct ggc aac cta gct ggc cca acg cag cta cga ggg tca cat     3041
Ser Val Pro Gly Asn Leu Ala Gly Pro Thr Gln Leu Arg Gly Ser His
                975                 980                 985

act atg ctc tgt aca gag gat cct tgc tcc cgt cta ata tga             3083
Thr Met Leu Cys Thr Glu Asp Pro Cys Ser Arg Leu Ile
            990                 995

ccagaatgag ctggaatacc acactgacca aatctggatc tttggactaa agtattcaaa   3143

atagcatagc aaagctcact gtattgggct aataatttgg cacttattag cttctctcat   3203

aaactgatca cgattataaa ttaaatgttt gggttcatac cccaaaagca atatgttgtc   3263

actcctaatt ctcaagtact attcaaattg tagtaaatct taaagttttt caaaacccta   3323

aaatcatatt cgc                                                      3336

<210>  4
<211>  999
<212>  PRT
<213>  Homo sapiens

<400>  4

Met Met Gly Leu Phe Pro Arg Thr Thr Gly Ala Leu Ala Ile Phe Val
1               5                   10                  15

Val Val Ile Leu Val His Gly Glu Leu Arg Ile Glu Thr Lys Gly Gln
            20                  25                  30

Tyr Asp Glu Glu Glu Met Thr Met Gln Gln Ala Lys Arg Arg Gln Lys
        35                  40                  45

Arg Glu Trp Val Lys Phe Ala Lys Pro Cys Arg Glu Gly Glu Asp Asn
    50                  55                  60

Ser Lys Arg Asn Pro Ile Ala Lys Ile Thr Ser Asp Tyr Gln Ala Thr
65                  70                  75                  80

Gln Lys Ile Thr Tyr Arg Ile Ser Gly Val Gly Ile Asp Gln Pro Pro
                85                  90                  95

Phe Gly Ile Phe Val Val Asp Lys Asn Thr Gly Asp Ile Asn Ile Thr
            100                 105                 110

Ala Ile Val Asp Arg Glu Glu Thr Pro Ser Phe Leu Ile Thr Cys Arg
        115                 120                 125

Ala Leu Asn Ala Gln Gly Leu Asp Val Glu Lys Pro Leu Ile Leu Thr
    130                 135                 140

Val Lys Ile Leu Asp Ile Asn Asp Asn Pro Pro Val Phe Ser Gln Gln
145                 150                 155                 160

Ile Phe Met Gly Glu Ile Glu Glu Asn Ser Ala Ser Asn Ser Leu Val
                165                 170                 175

Met Ile Leu Asn Ala Thr Asp Ala Asp Glu Pro Asn His Leu Asn Ser
            180                 185                 190

Lys Ile Ala Phe Lys Ile Val Ser Gln Glu Pro Ala Gly Thr Pro Met
        195                 200                 205

Phe Leu Leu Ser Arg Asn Thr Gly Glu Val Arg Thr Leu Thr Asn Ser
    210                 215                 220

Leu Asp Arg Glu Gln Ala Ser Ser Tyr Arg Leu Val Val Ser Gly Ala
225                 230                 235                 240

Asp Lys Asp Gly Glu Gly Leu Ser Thr Gln Cys Glu Cys Asn Ile Lys
                245                 250                 255

Val Lys Asp Val Asn Asp Asn Phe Pro Met Phe Arg Asp Ser Gln Tyr
            260                 265                 270

Ser Ala Arg Ile Glu Glu Asn Ile Leu Ser Ser Glu Leu Leu Arg Phe
        275                 280                 285

Gln Val Thr Asp Leu Asp Glu Glu Tyr Thr Asp Asn Trp Leu Ala Val
    290                 295                 300

Tyr Phe Phe Thr Ser Gly Asn Glu Gly Asn Trp Phe Glu Ile Gln Thr
305                 310                 315                 320

Asp Pro Arg Thr Asn Glu Gly Ile Leu Lys Val Val Lys Ala Leu Asp
                325                 330                 335

Tyr Glu Gln Leu Gln Ser Val Lys Leu Ser Ile Ala Val Lys Asn Lys
            340                 345                 350

Ala Glu Phe His Gln Ser Val Ile Ser Arg Tyr Arg Val Gln Ser Thr
        355                 360                 365

Pro Val Thr Ile Gln Val Ile Asn Val Arg Glu Gly Ile Ala Phe Arg
    370                 375                 380

Pro Ala Ser Lys Thr Phe Thr Val Gln Lys Gly Ile Ser Ser Lys Lys
385                 390                 395                 400

Leu Val Asp Tyr Ile Leu Gly Thr Tyr Gln Ala Ile Asp Glu Asp Thr
                405                 410                 415

Asn Lys Ala Ala Ser Asn Val Lys Tyr Val Met Gly Arg Asn Asp Gly
            420                 425                 430

Gly Tyr Leu Met Ile Asp Ser Lys Thr Ala Glu Ile Lys Phe Val Lys
        435                 440                 445

Asn Met Asn Arg Asp Ser Thr Phe Ile Val Asn Lys Thr Ile Thr Ala
    450                 455                 460

Glu Val Leu Ala Ile Asp Glu Tyr Thr Gly Lys Thr Ser Thr Gly Thr
465                 470                 475                 480

Val Tyr Val Arg Val Pro Asp Phe Asn Asp Asn Cys Pro Thr Ala Val
                485                 490                 495

Leu Glu Lys Asp Ala Val Cys Ser Ser Ser Pro Ser Val Val Val Ser
            500                 505                 510

Ala Arg Thr Leu Asn Asn Arg Tyr Thr Gly Pro Tyr Thr Phe Ala Leu
        515                 520                 525

Glu Asp Gln Pro Val Lys Leu Pro Ala Val Trp Ser Ile Thr Thr Leu
    530                 535                 540

Asn Ala Thr Ser Ala Leu Leu Arg Ala Gln Glu Gln Ile Pro Pro Gly
545                 550                 555                 560

Val Tyr His Ile Ser Leu Val Leu Thr Asp Ser Gln Asn Asn Arg Cys
                565                 570                 575

Glu Met Pro Arg Ser Leu Thr Leu Glu Val Cys Gln Cys Asp Asn Arg
            580                 585                 590

Gly Ile Cys Gly Thr Ser Tyr Pro Thr Thr Ser Pro Gly Thr Arg Tyr
        595                 600                 605

Gly Arg Pro His Ser Gly Arg Leu Gly Pro Ala Ala Ile Gly Leu Leu
    610                 615                 620

Leu Leu Gly Leu Leu Leu Leu Leu Leu Ala Pro Leu Leu Leu Leu Thr
625                 630                 635                 640

Cys Asp Cys Gly Ala Gly Ser Thr Gly Gly Val Thr Gly Gly Phe Ile
                645                 650                 655

Pro Val Pro Asp Gly Ser Glu Gly Thr Ile His Gln Trp Gly Ile Glu
            660                 665                 670

Gly Ala His Pro Glu Asp Lys Glu Ile Thr Asn Ile Cys Val Pro Pro
        675                 680                 685

Val Thr Ala Asn Gly Ala Asp Phe Met Glu Ser Ser Glu Val Cys Thr
    690                 695                 700

Asn Thr Tyr Ala Arg Gly Thr Ala Val Glu Gly Thr Ser Gly Met Glu
705                 710                 715                 720

Met Thr Thr Lys Leu Gly Ala Ala Thr Glu Ser Gly Gly Ala Ala Gly
                725                 730                 735

Phe Ala Thr Gly Thr Val Ser Gly Ala Ala Ser Gly Phe Gly Ala Ala
            740                 745                 750

Thr Gly Val Gly Ile Cys Ser Ser Gly Gln Ser Gly Thr Met Arg Thr
        755                 760                 765

Arg His Ser Thr Gly Gly Thr Asn Lys Asp Tyr Ala Asp Gly Ala Ile
    770                 775                 780

Ser Met Asn Phe Leu Asp Ser Tyr Phe Ser Gln Lys Ala Phe Ala Cys
785                 790                 795                 800

Ala Glu Glu Asp Asp Gly Gln Glu Ala Asn Asp Cys Leu Leu Ile Tyr
                805                 810                 815

Asp Asn Glu Gly Ala Asp Ala Thr Gly Ser Pro Val Gly Ser Val Gly
            820                 825                 830

Cys Cys Ser Phe Ile Ala Asp Asp Leu Asp Asp Ser Phe Leu Asp Ser
        835                 840                 845

Leu Gly Pro Lys Phe Lys Lys Leu Ala Glu Ile Ser Leu Gly Val Asp
    850                 855                 860

Gly Glu Gly Lys Glu Val Gln Pro Pro Ser Lys Asp Ser Gly Tyr Gly
865                 870                 875                 880

Ile Glu Ser Cys Gly His Pro Ile Glu Val Gln Gln Thr Gly Phe Val
                885                 890                 895

Lys Cys Gln Thr Leu Ser Gly Ser Gln Gly Ala Ser Ala Leu Ser Ala
            900                 905                 910

Ser Gly Ser Val Gln Pro Ala Val Ser Ile Pro Asp Pro Leu Gln His
        915                 920                 925

Gly Asn Tyr Leu Val Thr Glu Thr Tyr Ser Ala Ser Gly Ser Leu Val
    930                 935                 940

Gln Pro Ser Thr Ala Gly Phe Asp Pro Leu Leu Thr Gln Asn Val Ile
945                 950                 955                 960

Val Thr Glu Arg Val Ile Cys Pro Ile Ser Ser Val Pro Gly Asn Leu
                965                 970                 975

Ala Gly Pro Thr Gln Leu Arg Gly Ser His Thr Met Leu Cys Thr Glu
            980                 985                 990

Asp Pro Cys Ser Arg Leu Ile
        995

<210>  5
<211>  3946
<212>  DNA
<213>  Homo sapiens

<220>
<221>  CDS
<222>  (129)..(1991)

<400>  5
accgctccgg agcgggaggg gaggcttcgc ggaacgctct cggcgccagg actcgcgtgc      60
aaagcccagg cccgggcggc cagaccaaga gggaagaagc acagaattcc tcaactccca     120
gtgtgccc atg agt aag agc aaa tgc tcc gtg gga ctc atg tct tcc gtg      170
gtg gcc ccg gct aag gag ccc aat gcc gtg ggc ccg aag gag gtg gag      218
ctc atc ctt gtc aag gag cag aac gga gtg cag ctc acc agc tcc acc      266
ctc acc aac ccg cgg cag agc ccc gtg gag gcc cag gat cgg gag acc      314
tgg ggc aag aag atc gac ttt ctc ctg tcc gtc att ggc ttt gct gtg      362
gac ctg gcc aac gtc tgg cgg ttc ccc tac ctg tgc tac aaa aat ggt      410
ggc ggt gcc ttc ctg gtc ccc tac ctg ctc ttc atg gtc att gct ggg      458
atg cca ctt ttc tac atg gag ctg gcc ctc ggc cag ttc aac agg gaa      506
ggg gcc gct ggt gtc tgg aag atc tgc ccc ata ctg aaa ggt gtg ggc      554
ttc acg gtc atc ctc atc tca ctg tat gtc ggc ttc ttc tac aac gtc      602
atc atc gcc tgg gcg ctg cac tat ctc ttc tcc tcc ttc acc acg gag      650
ctc ccc tgg atc cac tgc aac aac tcc tgg aac agc ccc aac tgc tcg      698
gat gcc cat cct ggt gac tcc agt gga gac agc tcg ggc ctc aac gac     746
act ttt ggg acc aca cct gct gcc gag tac ttt gaa cgt ggc gtg ctg     794
cac ctc cac cag agc cat ggc atc gac gac ctg ggg cct ccg cgg tgg     842
cag ctc aca gcc tgc ctg gtg ctg gtc atc gtg ctg ctc tac ttc agc     890
ctc tgg aag ggc gtg aag acc tca ggg aag gtg gta tgg atc aca gcc     938
acc atg cca tac gtg gtc ctc act gcc ctg ctc ctg cgt ggg gtc acc     986
ctc cct gga gcc ata gac ggc atc aga gca tac ctg agc gtt gac ttc     1034
tac cgg ctc tgc gag gcg tct gtt tgg att gac gcg gcc acc cag gtg     1082
tgc ttc tcc ctg ggc gtg ggg ttc ggg gtg ctg atc gcc ttc tcc agc     1130
tac aac aag ttc acc aac aac tgc tac agg gac gcg att gtc acc acc     1178
tcc atc aac tcc ctg acg agc ttc tcc tcc ggc ttc gtc gtc ttc tcc     1226
ttc ctg ggg tac atg gca cag aag cac agt gtg ccc atc ggg gac gtg     1274
gcc aag gac ggg cca ggg ctg atc ttc atc atc tac ccg gaa gcc atc     1322
gcc acg ctc cct ctg tcc tca gcc tgg gcc gtg gtc ttc ttc atc atg     1370
ctg ctc acc ctg ggt atc gac agc gcc atg ggt ggt atg gag tca gtg     1418
atc acc ggg ctc atc gat gag ttc cag ctg ctg cac aga cac cgt gag     1466
ctc ttc acg ctc ttc atc gtc ctg gcg acc ttc ctc ctg tcc ctg ttc     1514
tgc gtc acc aac ggt ggc atc tac gtc ttc acg ctc ctg gac cat ttt      1562
gca gcc ggc acg tcc atc ctc ttt gga gtg ctc atc gaa gcc atc gga      1610
gtg gcc tgg ttc tat ggt gtt ggg cag ttc agc gac gac atc cag cag      1658
atg acc ggg cag cgg ccc agc ctg tac tgg cgg ctg tgc tgg aag ctg      1706
gtc agc ccc tgc ttt ctc ctg ttc gtg gtc gtg gtc agc att gtg acc      1754
ttc aga ccc ccc cac tac gga gcc tac atc ttc ccc gac tgg gcc aac      1802
gcg ctg ggc tgg gtc atc gcc aca tcc tcc atg gcc atg gtg ccc atc      1850
tat gcg gcc tac aag ttc tgc agc ctg cct ggg tcc ttt cga gag aaa      1898
ctg gcc tac gcc att gca ccc gag aag gac cgt gag ctg gtg gac aga      1946
ggg gag gtg cgc cag ttc acg ctc cgc cac tgg ctc aag gtg tag          1991
agggagcaga gacgaagacc ccaggaagtc atcctgcaat gggagagaca cgaacaaacc    2051
aaggaaatct aagtttcgag agaaaggagg gcaacttcta ctcttcaacc tctactgaaa    2111
acacaaacaa caaagcagaa gactcctctc ttctgactgt ttacaccttt ccgtgccggg    2171
agcgcacctc gccgtgtctt gtgttgctgt aataacgacg tagatctgtg cagcgaggtc    2231
caccccgttg ttgtccctgc agggcagaaa aacgtctaac ttcatgctgt ctgtgtgagg    2291
ctccctccct ccctgctccc tgctcccggc tctgaggctg ccccaggggc actgtgttct    2351
caggcgggga tcacgatcct tgtagacgca cctgctgaga atccccgtgc tcacagtagc    2411
ttcctagacc atttactttg cccatattaa aaagccaagt gtcctgcttg gtttagctgt    2471
gcagaaggtg aaatggagga aaccacaaat tcatgcaaag tcctttcccg atgcgtggct    2531
cccagcagag gccgtaaatt gagcgttcag ttgacacatt gcacacacag tctgttcaga    2591
ggcattggag gatgggggtc ctggtatgtc tcaccaggaa attctgttta tgttcttgca    2651
gcagagagaa ataaaactcc ttgaaaccag ctcaggctac tgccactcag gcagcctgtg    2711
ggtccttgtg gtgtagggaa cggcctgaga ggagcgtgtc ctatccccgg acgcatgcag    2771
ggcccccaca ggagcgtgtc ctatccccgg acgcatgcag ggcccccaca ggagcatgtc 2831
ctatccctgg acgcatgcag ggcccccaca ggagcgtgta ctaccccaga acgcatgcag 2891
ggcccccaca ggagcgtgta ctaccccagg acgcatgcag ggcccccact ggagcgtgta 2951
ctaccccagg acgcatgcag ggcccccaca ggagcgtgtc ctatccccgg accggacgca 3011
tgcagggccc ccacaggagc gtgtactacc ccaggacgca tgcagggccc ccacaggagc 3071
gtgtactacc ccaggatgca tgcagggccc ccacaggagc gtgtactacc ccaggacgca 3131
tgcagggccc ccatgcaggc agcctgcaga ccaacactct gcctggcctt gagccgtgac 3191
ctccaggaag ggaccccact ggaattttat ttctctcagg tgcgtgccac atcaataaca 3251
acagttttta tgtttgcgaa tggcttttta aaatcatatt tacctgtgaa tcaaaacaaa 3311
ttcaagaatg cagtatccgc gagcctgctt gctgatattg cagtttttgt ttacaagaat 3371
aattagcaat actgagtgaa ggatgttggc caaaagctgc tttccatggc acactgccct 3431
ctgccactga caggaaagtg gatgccatag tttgaattca tgcctcaagt cggtgggcct 3491
gcctacgtgc tgcccgaggg caggggccgt gcagggccag tcatggctgt cccctgcaag 3551
tggacgtggg ctccagggac tggagtgtaa tgctcggtgg gagccgtcag cctgtgaact 3611
gccaggcagc tgcagttagc acagaggatg gcttccccat tgccttctgg ggagggacac 3671
agaggacggc ttccccatcg ccttctggcc gctgcagtca gcacagagag cggcttcccc 3731
attgccttct ggggagggac acagaggaca gtttccccat cgccttctgg ttgttgaaga 3791
cagcacagag agcggcttcc ccatcgcctt ctggggaggg gctccgtgta gcaacccagg 3851
tgttgtccgt gtctgttgac caatctctat tcagcatcgt gtgggtccct aagcacaata 3911
aaagacatcc acaatggaaa aaaaaaaagg aattc 3946

<210> 6
<211> 620
<212> PRT
<213> Homo sapiens

<400> 6

<210> 7
<211> 3321
<212> DNA
<213> Homo sapiens

<220>
<221> CDS
<222> (58)..(2784)

<400> 7
tgctgtgttg caagaataaa ctttgggtct tggattgcaa taccacctgt ggagaaa 57

atg gta tgc gag gga aag cga tca gcc tct tgc cct tgt ttc ttc ctc 105
Met Val Cys Glu Gly Lys Arg Ser Ala Ser Cys Pro Cys Phe Phe Leu
1               5                   10                  15

ttg acc gcc aag ttc tac tgg atc ctc aca atg atg caa aga act cac 153
Leu Thr Ala Lys Phe Tyr Trp Ile Leu Thr Met Met Gln Arg Thr His
            20                  25                  30

agc cag gag tat gcc cat tcc ata cgg gtg gat ggg gac att att ttg 201
Ser Gln Glu Tyr Ala His Ser Ile Arg Val Asp Gly Asp Ile Ile Leu
        35                  40                  45

ggg ggt ctc ttc cct gtc cac gca aag gga gag aga ggg gtg cct tgt 249
Gly Gly Leu Phe Pro Val His Ala Lys Gly Glu Arg Gly Val Pro Cys
    50                  55                  60

ggg gag ctg aag aag gaa aag ggg att cac aga ctg gag gcc atg ctt 297
Gly Glu Leu Lys Lys Glu Lys Gly Ile His Arg Leu Glu Ala Met Leu
65                  70                  75                  80

tat gca att gac cag att aac aag gac cct gat ctc ctt tcc aac atc 345
Tyr Ala Ile Asp Gln Ile Asn Lys Asp Pro Asp Leu Leu Ser Asn Ile
                85                  90                  95

act ctg ggt gtc cgc atc ctc gac acg tgc tct agg gac acc tat gct 393
Thr Leu Gly Val Arg Ile Leu Asp Thr Cys Ser Arg Asp Thr Tyr Ala
            100                 105                 110

ttg gag cag tct cta aca ttc gtg cag gca tta ata gag aaa gat gct 441
Leu Glu Gln Ser Leu Thr Phe Val Gln Ala Leu Ile Glu Lys Asp Ala
        115                 120                 125

tcg gat gtg aag tgt gct aat gga gat cca ccc att ttc acc aag ccc 489
Ser Asp Val Lys Cys Ala Asn Gly Asp Pro Pro Ile Phe Thr Lys Pro
    130                 135                 140

gac aag att tct ggc gtc ata ggt gct gca gca agc tcc gtg tcc atc 537
Asp Lys Ile Ser Gly Val Ile Gly Ala Ala Ala Ser Ser Val Ser Ile
145                 150                 155                 160

atg gtt gct aac att tta aga ctt ttt aag ata cct caa atc agc tat 585
Met Val Ala Asn Ile Leu Arg Leu Phe Lys Ile Pro Gln Ile Ser Tyr
                165                 170                 175

gca tcc aca gcc cca gag cta agt gat aac acc agg tat gac ttt ttc 633
Ala Ser Thr Ala Pro Glu Leu Ser Asp Asn Thr Arg Tyr Asp Phe Phe
            180                 185                 190

tct cga gtg gtt ccg cct gac tcc tac caa gcc caa gcc atg gtg gac 681
Ser Arg Val Val Pro Pro Asp Ser Tyr Gln Ala Gln Ala Met Val Asp
        195                 200                 205

atc gtg aca gca ctg gga tgg aat tat gtt tcg aca ctg gct tct gag 729
Ile Val Thr Ala Leu Gly Trp Asn Tyr Val Ser Thr Leu Ala Ser Glu

210                 215                 220

ggg aac tat ggt gag agc ggt gtg gag gcc ttc acc cag atc tcg agg     777
Gly Asn Tyr Gly Glu Ser Gly Val Glu Ala Phe Thr Gln Ile Ser Arg
225                 230                 235                 240

gag att ggt ggt gtt tgc att gct cag tca cag aaa atc cca cgt gaa     825
Glu Ile Gly Gly Val Cys Ile Ala Gln Ser Gln Lys Ile Pro Arg Glu
                245                 250                 255

cca aga cct gga gaa ttt gaa aaa att atc aaa cgc ctg cta gaa aca     873
Pro Arg Pro Gly Glu Phe Glu Lys Ile Ile Lys Arg Leu Leu Glu Thr
            260                 265                 270

cct aat gct cga gca gtg att atg ttt gcc aat gag gat gac atc agg     921
Pro Asn Ala Arg Ala Val Ile Met Phe Ala Asn Glu Asp Asp Ile Arg
        275                 280                 285

agg ata ttg gaa gca gca aaa aaa cta aac caa agt ggg cat ttt ctc     969
Arg Ile Leu Glu Ala Ala Lys Lys Leu Asn Gln Ser Gly His Phe Leu
    290                 295                 300

tgg att ggc tca gat agt tgg gga tcc aaa ata gca cct gtc tat cag     1017
Trp Ile Gly Ser Asp Ser Trp Gly Ser Lys Ile Ala Pro Val Tyr Gln
305                 310                 315                 320

caa gag gag att gca gaa ggg gct gtg aca att ttg ccc aaa cga gca     1065
Gln Glu Glu Ile Ala Glu Gly Ala Val Thr Ile Leu Pro Lys Arg Ala
                325                 330                 335

tca att gat gga ttt gat cga tac ttt aga agc cga act ctt gcc aat     1113
Ser Ile Asp Gly Phe Asp Arg Tyr Phe Arg Ser Arg Thr Leu Ala Asn
            340                 345                 350

aat cga aga aat gtg tgg ttt gca gaa ttc tgg gag gag aat ttt ggc     1161
Asn Arg Arg Asn Val Trp Phe Ala Glu Phe Trp Glu Glu Asn Phe Gly
        355                 360                 365

tgc aag tta gga tca cat ggg aaa agg aac agt cat ata aag aaa tgc     1209
Cys Lys Leu Gly Ser His Gly Lys Arg Asn Ser His Ile Lys Lys Cys
    370                 375                 380

aca ggg ctg gag cga att gct cgg gat tca tct tat gaa cag gaa gga     1257
Thr Gly Leu Glu Arg Ile Ala Arg Asp Ser Ser Tyr Glu Gln Glu Gly
385                 390                 395                 400

aag gtc caa ttt gta att gat gct gta tat tcc atg gct tac gcc ctg     1305
Lys Val Gln Phe Val Ile Asp Ala Val Tyr Ser Met Ala Tyr Ala Leu
                405                 410                 415

cac aat atg cac aaa gat ctc tgc cct gga tac att ggc ctt tgt cca     1353
His Asn Met His Lys Asp Leu Cys Pro Gly Tyr Ile Gly Leu Cys Pro
            420                 425                 430

cga atg agt acc att gat ggg aaa gag cta ctt ggt tat att cgg gct     1401
Arg Met Ser Thr Ile Asp Gly Lys Glu Leu Leu Gly Tyr Ile Arg Ala
        435                 440                 445

gta aat ttt aat ggc agt gct ggc act cct gtc act ttt aat gaa aac     1449
Val Asn Phe Asn Gly Ser Ala Gly Thr Pro Val Thr Phe Asn Glu Asn
    450                 455                 460

gga gat gct cct gga cgt tat gat atc ttc cag tat caa ata acc aac     1497
Gly Asp Ala Pro Gly Arg Tyr Asp Ile Phe Gln Tyr Gln Ile Thr Asn
465                 470                 475                 480

aaa agc aca gag tac aaa gtc atc ggc cac tgg acc aat cag ctt cat     1545
Lys Ser Thr Glu Tyr Lys Val Ile Gly His Trp Thr Asn Gln Leu His

                485                 490                 495

cta aaa gtg gaa gac atg cag tgg gct cat aga gaa cat act cac ccg 1593
Leu Lys Val Glu Asp Met Gln Trp Ala His Arg Glu His Thr His Pro
            500                 505                 510

gcg tct gtc tgc agc ctg ccg tgt aag cca ggg gag agg aag aaa acg 1641
Ala Ser Val Cys Ser Leu Pro Cys Lys Pro Gly Glu Arg Lys Lys Thr
        515                 520                 525

gtg aaa ggg gtc cct tgc tgc tgg cac tgt gaa cgc tgt gaa ggt tac 1689
Val Lys Gly Val Pro Cys Cys Trp His Cys Glu Arg Cys Glu Gly Tyr
    530                 535                 540

aac tac cag gtg gat gag ctg tcc tgt gaa ctt tgc cct ctg gat cag 1737
Asn Tyr Gln Val Asp Glu Leu Ser Cys Glu Leu Cys Pro Leu Asp Gln
545                 550                 555                 560

aga ccc aac atg aac cgc aca ggc tgc cag ctt atc ccc atc atc aaa 1785
Arg Pro Asn Met Asn Arg Thr Gly Cys Gln Leu Ile Pro Ile Ile Lys
                565                 570                 575

ttg gag tgg cat tct ccc tgg gct gtg gtg cct gtg ttt gtt gca ata 1833
Leu Glu Trp His Ser Pro Trp Ala Val Val Pro Val Phe Val Ala Ile
            580                 585                 590

ttg gga atc atc gcc acc acc ttt gtg atc gtg acc ttt gtc cgc tat 1881
Leu Gly Ile Ile Ala Thr Thr Phe Val Ile Val Thr Phe Val Arg Tyr
        595                 600                 605

aat gac aca cct atc gtg agg gct tca gga cgc gaa ctt agt tac gtg 1929
Asn Asp Thr Pro Ile Val Arg Ala Ser Gly Arg Glu Leu Ser Tyr Val
    610                 615                 620

ctc cta acg ggg att ttt ctc tgt tat tca atc acg ttt tta atg att 1977
Leu Leu Thr Gly Ile Phe Leu Cys Tyr Ser Ile Thr Phe Leu Met Ile
625                 630                 635                 640

gca gca cca gat aca atc ata tgc tcc ttc cga cgg gtc ttc cta gga 2025
Ala Ala Pro Asp Thr Ile Ile Cys Ser Phe Arg Arg Val Phe Leu Gly
                645                 650                 655

ctt ggc atg tgt ttc agc tat gca gcc ctt ctg acc aaa aca aac cgt 2073
Leu Gly Met Cys Phe Ser Tyr Ala Ala Leu Leu Thr Lys Thr Asn Arg
            660                 665                 670

atc cac cga ata ttt gag cag ggg aag aaa tct gtc aca gcg ccc aag 2121
Ile His Arg Ile Phe Glu Gln Gly Lys Lys Ser Val Thr Ala Pro Lys
        675                 680                 685

ttc att agt cca gca tct cag ctg gtg atc acc ttc agc ctc atc tcc 2169
Phe Ile Ser Pro Ala Ser Gln Leu Val Ile Thr Phe Ser Leu Ile Ser
    690                 695                 700

gtc cag ctc ctt gga gtg ttt gtc tgg ttt gtt gtg gat ccc ccc cac 2217
Val Gln Leu Leu Gly Val Phe Val Trp Phe Val Val Asp Pro Pro His
705                 710                 715                 720

atc atc att gac tat gga gag cag cgg aca cta gat cca gag aag gcc 2265
Ile Ile Ile Asp Tyr Gly Glu Gln Arg Thr Leu Asp Pro Glu Lys Ala
                725                 730                 735

agg gga gtg ctc aag tgt gac att tct gat ctc tca ctc att tgt tca 2313
Arg Gly Val Leu Lys Cys Asp Ile Ser Asp Leu Ser Leu Ile Cys Ser
            740                 745                 750

ctt gga tac agt atc ctc ttg atg gtc act tgt act gtt tat gcc aat 2361
Leu Gly Tyr Ser Ile Leu Leu Met Val Thr Cys Thr Val Tyr Ala Asn

755                 760                 765

aaa acg aga ggt gtc cca gag act ttc aat gaa gcc aaa cct att gga      2409
Lys Thr Arg Gly Val Pro Glu Thr Phe Asn Glu Ala Lys Pro Ile Gly
    770                 775                 780

ttt acc atg tat acc acc tgc atc att tgg tta gct ttc atc ccc atc      2457
Phe Thr Met Tyr Thr Thr Cys Ile Ile Trp Leu Ala Phe Ile Pro Ile
785                 790                 795                 800

ttt ttt ggt aca gcc cag tca gca gaa aag atg tac atc cag aca aca      2505
Phe Phe Gly Thr Ala Gln Ser Ala Glu Lys Met Tyr Ile Gln Thr Thr
                805                 810                 815

aca ctt act gtc tcc atg agt tta agt gct tca gta tct ctg ggc atg      2553
Thr Leu Thr Val Ser Met Ser Leu Ser Ala Ser Val Ser Leu Gly Met
            820                 825                 830

ctc tat atg ccc aag gtt tat att ata att ttt cat cca gaa cag aat      2601
Leu Tyr Met Pro Lys Val Tyr Ile Ile Ile Phe His Pro Glu Gln Asn
        835                 840                 845

gtt caa aaa cgc aag agg agc ttc aag gct gtg gtg aca gct gcc acc      2649
Val Gln Lys Arg Lys Arg Ser Phe Lys Ala Val Val Thr Ala Ala Thr
    850                 855                 860

atg caa agc aaa ctg atc caa aaa gga aat gac aga cca aat ggc gag      2697
Met Gln Ser Lys Leu Ile Gln Lys Gly Asn Asp Arg Pro Asn Gly Glu
865                 870                 875                 880

gtg aaa agt gaa ctc tgt gag agt ctt gaa acc aac act tcc tct acc      2745
Val Lys Ser Glu Leu Cys Glu Ser Leu Glu Thr Asn Thr Ser Ser Thr
                885                 890                 895

aag aca aca tat atc agt tac agc aat cat tca atc tga aacagggaaa      2794
Lys Thr Thr Tyr Ile Ser Tyr Ser Asn His Ser Ile
            900                 905

tggcacaatc tgaagagacg tggtatatga tcttaaatga tgaacatgag accgcaaaaa      2854

ttcactcctg gagatctccg tagactacaa tcaatcaaat caatagtcag tcttgtaagg      2914

aacaaaaatt agccatgagc caaaagtatc aataaacggg gagtgaagaa acccgtttta      2974

tacaataaaa ccaatgagtg tcaagctaaa gtattgctta ttcatgagca gttaaaacaa      3034

atcacaaaag gaaaactaat gttagctcgt gaaaaaaatg ctgttgaaat aaataatgtc      3094

tgatgttatt cttgtatttt tctgtgattg tgagaactcc cgttcctgtc ccacattgtt      3154

taacttgtat aagacaatga gtctgtttct tgtaatggct gaccagattg aagccctggg      3214

ttgtgctaaa aataaatgca atgattgatg catgcaattt tttatacaaa taatttattt      3274

ctaataataa aggaatgttt tgcaaaaaaa aaaaaaaaaa actcgag                  3321

<210>  8
<211>  908
<212>  PRT
<213>  Homo sapiens

<400>  8

Met Val Cys Glu Gly Lys Arg Ser Ala Ser Cys Pro Cys Phe Phe Leu
1               5                   10                  15

Leu Thr Ala Lys Phe Tyr Trp Ile Leu Thr Met Met Gln Arg Thr His
            20                  25                  30

Ser Gln Glu Tyr Ala His Ser Ile Arg Val Asp Gly Asp Ile Ile Leu
        35                  40                  45

Gly Gly Leu Phe Pro Val His Ala Lys Gly Glu Arg Gly Val Pro Cys
    50                  55                  60

Gly Glu Leu Lys Lys Glu Lys Gly Ile His Arg Leu Glu Ala Met Leu
65                  70                  75                  80

Tyr Ala Ile Asp Gln Ile Asn Lys Asp Pro Asp Leu Leu Ser Asn Ile
                85                  90                  95

Thr Leu Gly Val Arg Ile Leu Asp Thr Cys Ser Arg Asp Thr Tyr Ala
            100                 105                 110

Leu Glu Gln Ser Leu Thr Phe Val Gln Ala Leu Ile Glu Lys Asp Ala
        115                 120                 125

Ser Asp Val Lys Cys Ala Asn Gly Asp Pro Pro Ile Phe Thr Lys Pro
    130                 135                 140

Asp Lys Ile Ser Gly Val Ile Gly Ala Ala Ala Ser Ser Val Ser Ile
145                 150                 155                 160

Met Val Ala Asn Ile Leu Arg Leu Phe Lys Ile Pro Gln Ile Ser Tyr
                165                 170                 175

Ala Ser Thr Ala Pro Glu Leu Ser Asp Asn Thr Arg Tyr Asp Phe Phe
            180                 185                 190

Ser Arg Val Val Pro Pro Asp Ser Tyr Gln Ala Gln Ala Met Val Asp
        195                 200                 205

Ile Val Thr Ala Leu Gly Trp Asn Tyr Val Ser Thr Leu Ala Ser Glu
    210                 215                 220

Gly Asn Tyr Gly Glu Ser Gly Val Glu Ala Phe Thr Gln Ile Ser Arg
225                 230                 235                 240

Glu Ile Gly Gly Val Cys Ile Ala Gln Ser Gln Lys Ile Pro Arg Glu
                245                 250                 255

Pro Arg Pro Gly Glu Phe Glu Lys Ile Ile Lys Arg Leu Leu Glu Thr
            260                 265                 270

Pro Asn Ala Arg Ala Val Ile Met Phe Ala Asn Glu Asp Asp Ile Arg
        275                 280                 285

Arg Ile Leu Glu Ala Ala Lys Lys Leu Asn Gln Ser Gly His Phe Leu
    290                 295                 300

Trp Ile Gly Ser Asp Ser Trp Gly Ser Lys Ile Ala Pro Val Tyr Gln
305                 310                 315                 320

Gln Glu Glu Ile Ala Glu Gly Ala Val Thr Ile Leu Pro Lys Arg Ala
                325                 330                 335

Ser Ile Asp Gly Phe Asp Arg Tyr Phe Arg Ser Arg Thr Leu Ala Asn
            340                 345                 350

Asn Arg Arg Asn Val Trp Phe Ala Glu Phe Trp Glu Glu Asn Phe Gly
        355                 360                 365

Cys Lys Leu Gly Ser His Gly Lys Arg Asn Ser His Ile Lys Lys Cys
    370                 375                 380

Thr Gly Leu Glu Arg Ile Ala Arg Asp Ser Ser Tyr Glu Gln Glu Gly
385                 390                 395                 400

Lys Val Gln Phe Val Ile Asp Ala Val Tyr Ser Met Ala Tyr Ala Leu
                405                 410                 415

His Asn Met His Lys Asp Leu Cys Pro Gly Tyr Ile Gly Leu Cys Pro
            420                 425                 430

Arg Met Ser Thr Ile Asp Gly Lys Glu Leu Leu Gly Tyr Ile Arg Ala
        435                 440                 445

Val Asn Phe Asn Gly Ser Ala Gly Thr Pro Val Thr Phe Asn Glu Asn
    450                 455                 460

Gly Asp Ala Pro Gly Arg Tyr Asp Ile Phe Gln Tyr Gln Ile Thr Asn
465                 470                 475                 480

Lys Ser Thr Glu Tyr Lys Val Ile Gly His Trp Thr Asn Gln Leu His
                485                 490                 495

Leu Lys Val Glu Asp Met Gln Trp Ala His Arg Glu His Thr His Pro
            500                 505                 510

Ala Ser Val Cys Ser Leu Pro Cys Lys Pro Gly Glu Arg Lys Lys Thr
        515                 520                 525

Val Lys Gly Val Pro Cys Cys Trp His Cys Glu Arg Cys Glu Gly Tyr
    530                 535                 540

Asn Tyr Gln Val Asp Glu Leu Ser Cys Glu Leu Cys Pro Leu Asp Gln
545                 550                 555                 560

Arg Pro Asn Met Asn Arg Thr Gly Cys Gln Leu Ile Pro Ile Ile Lys
                565                 570                 575

Leu Glu Trp His Ser Pro Trp Ala Val Val Pro Val Phe Val Ala Ile
            580                 585                 590

Leu Gly Ile Ile Ala Thr Thr Phe Val Ile Val Thr Phe Val Arg Tyr
        595                 600                 605

Asn Asp Thr Pro Ile Val Arg Ala Ser Gly Arg Glu Leu Ser Tyr Val
    610                 615                 620

Leu Leu Thr Gly Ile Phe Leu Cys Tyr Ser Ile Thr Phe Leu Met Ile
625                 630                 635                 640

Ala Ala Pro Asp Thr Ile Ile Cys Ser Phe Arg Arg Val Phe Leu Gly
                645                 650                 655

Leu Gly Met Cys Phe Ser Tyr Ala Ala Leu Leu Thr Lys Thr Asn Arg
            660                 665                 670

Ile His Arg Ile Phe Glu Gln Gly Lys Lys Ser Val Thr Ala Pro Lys
        675                 680                 685

Phe Ile Ser Pro Ala Ser Gln Leu Val Ile Thr Phe Ser Leu Ile Ser
    690                 695                 700

Val Gln Leu Leu Gly Val Phe Val Trp Phe Val Val Asp Pro Pro His
705                 710                 715                 720

Ile Ile Ile Asp Tyr Gly Glu Gln Arg Thr Leu Asp Pro Glu Lys Ala
                725                 730                 735

Arg Gly Val Leu Lys Cys Asp Ile Ser Asp Leu Ser Leu Ile Cys Ser
            740                 745                 750

Leu Gly Tyr Ser Ile Leu Leu Met Val Thr Cys Thr Val Tyr Ala Asn
        755                 760                 765

Lys Thr Arg Gly Val Pro Glu Thr Phe Asn Glu Ala Lys Pro Ile Gly
    770                 775                 780

Phe Thr Met Tyr Thr Thr Cys Ile Ile Trp Leu Ala Phe Ile Pro Ile
785                 790                 795                 800

Phe Phe Gly Thr Ala Gln Ser Ala Glu Lys Met Tyr Ile Gln Thr Thr
                805                 810                 815

Thr Leu Thr Val Ser Met Ser Leu Ser Ala Ser Val Ser Leu Gly Met
            820                 825                 830

Leu Tyr Met Pro Lys Val Tyr Ile Ile Ile Phe His Pro Glu Gln Asn
        835                 840                 845

Val Gln Lys Arg Lys Arg Ser Phe Lys Ala Val Val Thr Ala Ala Thr
    850                 855                 860

Met Gln Ser Lys Leu Ile Gln Lys Gly Asn Asp Arg Pro Asn Gly Glu
865                 870                 875                 880

Val Lys Ser Glu Leu Cys Glu Ser Leu Glu Thr Asn Thr Ser Ser Thr
                885                 890                 895

Lys Thr Thr Tyr Ile Ser Tyr Ser Asn His Ser Ile
            900                 905

<210>  9
<211>  3697
<212>  DNA
<213>  Homo sapiens

<220>
<221>  CDS
<222>  (121)..(2619)

<400>  9
agggagtgtt cccgggggag atactccagt cgtagcaaga gtctcgacca ctgaatggaa      60

gaaaaggact tttaaccacc attttgtgac ttacagaaag gaatttgaat aaagaaaact     120

atg ata ctt cag gcc cat ctt cac tcc ctg tgt ctt ctt atg ctt tat      168
Met Ile Leu Gln Ala His Leu His Ser Leu Cys Leu Leu Met Leu Tyr
1               5                   10                  15

ttg gca act gga tat ggc caa gag ggg aag ttt agt gga ccc ctg aaa      216
Leu Ala Thr Gly Tyr Gly Gln Glu Gly Lys Phe Ser Gly Pro Leu Lys
            20                  25                  30

ccc atg aca ttt tct att tat gaa ggc caa gaa ccg agt caa att ata      264
Pro Met Thr Phe Ser Ile Tyr Glu Gly Gln Glu Pro Ser Gln Ile Ile
        35                  40                  45

ttc cag ttt aag gcc aat cct cct gct gtg act ttt gaa cta act ggg      312
Phe Gln Phe Lys Ala Asn Pro Pro Ala Val Thr Phe Glu Leu Thr Gly
    50                  55                  60

gag aca gac aac ata ttt gtg ata gaa cgg gag gga ctt ctg tat tac      360
Glu Thr Asp Asn Ile Phe Val Ile Glu Arg Glu Gly Leu Leu Tyr Tyr
65                  70                  75                  80

aac aga gcc ttg gac agg gaa aca aga tct act cac aat ctc cag gtt      408
Asn Arg Ala Leu Asp Arg Glu Thr Arg Ser Thr His Asn Leu Gln Val
                85                  90                  95

gca gcc ctg gac gct aat gga att ata gtg gag ggt cca gtc cct atc      456
Ala Ala Leu Asp Ala Asn Gly Ile Ile Val Glu Gly Pro Val Pro Ile
            100                 105                 110

acc ata gaa gtg aag gac atc aac gac aat cga ccc acg ttt ctc cag      504
Thr Ile Glu Val Lys Asp Ile Asn Asp Asn Arg Pro Thr Phe Leu Gln
        115                 120                 125

tca aag tac gaa ggc tca gta agg cag aac tct cgc cca gga aag ccc      552

Ser Lys Tyr Glu Gly Ser Val Arg Gln Asn Ser Arg Pro Gly Lys Pro
130                 135                 140

ttc ttg tat gtc aat gcc aca gac ctg gat gat ccg gcc act ccc aat      600
Phe Leu Tyr Val Asn Ala Thr Asp Leu Asp Asp Pro Ala Thr Pro Asn
145                 150                 155                 160

ggc cag ctt tat tac cag att gtc atc cag ctt ccc atg atc aac aat      648
Gly Gln Leu Tyr Tyr Gln Ile Val Ile Gln Leu Pro Met Ile Asn Asn
                165                 170                 175

gtc atg tac ttt cag atc aac aac aaa acg gga gcc atc tct ctt acc      696
Val Met Tyr Phe Gln Ile Asn Asn Lys Thr Gly Ala Ile Ser Leu Thr
            180                 185                 190

cga gag gga tct cag gaa ttg aat cct gct aag aat cct tcc tat aat      744
Arg Glu Gly Ser Gln Glu Leu Asn Pro Ala Lys Asn Pro Ser Tyr Asn
        195                 200                 205

ctg gtg atc tca gtg aag gac atg gga ggc cag agt gag aat tcc ttc      792
Leu Val Ile Ser Val Lys Asp Met Gly Gly Gln Ser Glu Asn Ser Phe
    210                 215                 220

agt gat acc aca tct gtg gat atc ata gtg aca gag aat att tgg aaa      840
Ser Asp Thr Thr Ser Val Asp Ile Ile Val Thr Glu Asn Ile Trp Lys
225                 230                 235                 240

gca cca aaa cct gtg gag atg gtg gaa aac tca act gat cct cac ccc      888
Ala Pro Lys Pro Val Glu Met Val Glu Asn Ser Thr Asp Pro His Pro
                245                 250                 255

atc aaa atc act cag gtg cgg tgg aat gat ccc ggt gca caa tat tcc      936
Ile Lys Ile Thr Gln Val Arg Trp Asn Asp Pro Gly Ala Gln Tyr Ser
            260                 265                 270

tta gtt gac aaa gag aag ctg cca aga ttc cca ttt tca att gac cag      984
Leu Val Asp Lys Glu Lys Leu Pro Arg Phe Pro Phe Ser Ile Asp Gln
        275                 280                 285

gaa gga gat att tac gtg act cag ccc ttg gac cga gaa gaa aag gat     1032
Glu Gly Asp Ile Tyr Val Thr Gln Pro Leu Asp Arg Glu Glu Lys Asp
    290                 295                 300

gca tat gtt ttt tat gca gtt gca aag gat gag tac gga aaa cca ctt     1080
Ala Tyr Val Phe Tyr Ala Val Ala Lys Asp Glu Tyr Gly Lys Pro Leu
305                 310                 315                 320

tca tat ccg ctg gaa att cat gta aaa gtt aaa gat att aat gat aat     1128
Ser Tyr Pro Leu Glu Ile His Val Lys Val Lys Asp Ile Asn Asp Asn
                325                 330                 335

cca cct aca tgt ccg tca cca gta acc gta ttt gag gtc cag gag aat     1176
Pro Pro Thr Cys Pro Ser Pro Val Thr Val Phe Glu Val Gln Glu Asn
            340                 345                 350

gaa cga ctg ggt aac agt atc ggg acc ctt act gca cat gac agg gat     1224
Glu Arg Leu Gly Asn Ser Ile Gly Thr Leu Thr Ala His Asp Arg Asp
        355                 360                 365

gaa gaa aat act gcc aac agt ttt cta aac tac agg att gtg gag caa     1272
Glu Glu Asn Thr Ala Asn Ser Phe Leu Asn Tyr Arg Ile Val Glu Gln
    370                 375                 380

act ccc aaa ctt ccc atg gat gga ctc ttc cta atc caa acc tat gct     1320
Thr Pro Lys Leu Pro Met Asp Gly Leu Phe Leu Ile Gln Thr Tyr Ala
385                 390                 395                 400

gga atg tta cag tta gct aaa cag tcc ttg aag aag caa gat act cct     1368

Gly Met Leu Gln Leu Ala Lys Gln Ser Leu Lys Lys Gln Asp Thr Pro
                405                 410                 415

cag tac aac tta acg ata gag gtg tct gac aaa gat ttc aag acc ctt 1416
Gln Tyr Asn Leu Thr Ile Glu Val Ser Asp Lys Asp Phe Lys Thr Leu
            420                 425                 430

tgt ttt gtg caa atc aac gtt att gat atc aat gat cag atc ccc atc 1464
Cys Phe Val Gln Ile Asn Val Ile Asp Ile Asn Asp Gln Ile Pro Ile
        435                 440                 445

ttt gaa aaa tca gat tat gga aac ctg act ctt gct gaa gac aca aac 1512
Phe Glu Lys Ser Asp Tyr Gly Asn Leu Thr Leu Ala Glu Asp Thr Asn
    450                 455                 460

att ggg tcc acc atc tta acc atc cag gcc act gat gct gat gag cca 1560
Ile Gly Ser Thr Ile Leu Thr Ile Gln Ala Thr Asp Ala Asp Glu Pro
465                 470                 475                 480

ttt act ggg agt tct aaa att ctg tat cat atc ata aag gga gac agt 1608
Phe Thr Gly Ser Ser Lys Ile Leu Tyr His Ile Ile Lys Gly Asp Ser
                485                 490                 495

gag gga cgc ctg ggg gtt gac aca gat ccc cat acc aac acc gga tat 1656
Glu Gly Arg Leu Gly Val Asp Thr Asp Pro His Thr Asn Thr Gly Tyr
            500                 505                 510

gtc ata att aaa aag cct ctt gat ttt gaa aca gca gct gtt tcc aac 1704
Val Ile Ile Lys Lys Pro Leu Asp Phe Glu Thr Ala Ala Val Ser Asn
        515                 520                 525

att gtg ttc aaa gca gaa aat cct gag cct cta gtg ttt ggt gtg aag 1752
Ile Val Phe Lys Ala Glu Asn Pro Glu Pro Leu Val Phe Gly Val Lys
    530                 535                 540

tac aat gca agt tct ttt gcc aag ttc acg ctt att gtg aca gat gtg 1800
Tyr Asn Ala Ser Ser Phe Ala Lys Phe Thr Leu Ile Val Thr Asp Val
545                 550                 555                 560

aat gaa gca cct caa ttt tcc caa cac gta ttc caa gcg aaa gtc agt 1848
Asn Glu Ala Pro Gln Phe Ser Gln His Val Phe Gln Ala Lys Val Ser
                565                 570                 575

gag gat gta gct ata ggc act aaa gtg ggc aat gtg act gcc aag gat 1896
Glu Asp Val Ala Ile Gly Thr Lys Val Gly Asn Val Thr Ala Lys Asp
            580                 585                 590

cca gaa ggt ctg gac ata agc tat tca ctg agg gga gac aca aga ggt 1944
Pro Glu Gly Leu Asp Ile Ser Tyr Ser Leu Arg Gly Asp Thr Arg Gly
        595                 600                 605

tgg ctt aaa att gac cac gtg act ggt gag atc ttt agt gtg gct cca 1992
Trp Leu Lys Ile Asp His Val Thr Gly Glu Ile Phe Ser Val Ala Pro
    610                 615                 620

ttg gac aga gaa gcc gga agt cca tat cgg gta caa gtg gtg gcc aca 2040
Leu Asp Arg Glu Ala Gly Ser Pro Tyr Arg Val Gln Val Val Ala Thr
625                 630                 635                 640

gaa gta ggg ggg tct tcc ttg agc tct gtg tca gag ttc cac ctg atc 2088
Glu Val Gly Gly Ser Ser Leu Ser Ser Val Ser Glu Phe His Leu Ile
                645                 650                 655

ctt atg gat gtg aat gac aac cct ccc agg cta gcc aag gac tac acg 2136
Leu Met Asp Val Asn Asp Asn Pro Pro Arg Leu Ala Lys Asp Tyr Thr
            660                 665                 670

ggc ttg ttc ttc tgc cat ccc ctc agt gca cct gga agt ctc att ttc 2184

Gly Leu Phe Phe Cys His Pro Leu Ser Ala Pro Gly Ser Leu Ile Phe
        675                 680                 685

gag gct act gat gat gat cag cac tta ttt cgg ggt ccc cat ttt aca      2232
Glu Ala Thr Asp Asp Asp Gln His Leu Phe Arg Gly Pro His Phe Thr
    690                 695                 700

ttt tcc ctc ggc agt gga agc tta caa aac gac tgg gaa gtt tcc aaa      2280
Phe Ser Leu Gly Ser Gly Ser Leu Gln Asn Asp Trp Glu Val Ser Lys
705                 710                 715                 720

atc aat ggt act cat gcc cga ctg tct acc agg cac aca gag ttt gag      2328
Ile Asn Gly Thr His Ala Arg Leu Ser Thr Arg His Thr Glu Phe Glu
                725                 730                 735

gag agg gag tat gtc gtc ttg atc cgc atc aat gat ggg ggt cgg cca      2376
Glu Arg Glu Tyr Val Val Leu Ile Arg Ile Asn Asp Gly Gly Arg Pro
            740                 745                 750

ccc ttg gaa ggc att gtt tct tta cca gtt aca ttc tgc agt tgt gtg      2424
Pro Leu Glu Gly Ile Val Ser Leu Pro Val Thr Phe Cys Ser Cys Val
        755                 760                 765

gaa gga agt tgt ttc cgg cca gca ggt cac cag act ggg ata ccc act      2472
Glu Gly Ser Cys Phe Arg Pro Ala Gly His Gln Thr Gly Ile Pro Thr
    770                 775                 780

gtg ggc atg gca gtt ggt ata ctg ctg acc acc ctt ctg gtg att ggt      2520
Val Gly Met Ala Val Gly Ile Leu Leu Thr Thr Leu Leu Val Ile Gly
785                 790                 795                 800

ata att tta gca gtt gtg ttt atc cgc ata aag aag gat aaa ggc aaa      2568
Ile Ile Leu Ala Val Val Phe Ile Arg Ile Lys Lys Asp Lys Gly Lys
                805                 810                 815

gat aat gtt gaa agt gct caa gca tct gaa gtc aaa cct ctg aga agc      2616
Asp Asn Val Glu Ser Ala Gln Ala Ser Glu Val Lys Pro Leu Arg Ser
            820                 825                 830

tga atttgaaaag gaatgtttga atttatatag caagtgctat ttcagcaaca      2669

accatctcat cctattactt ttcatctaac gtgcattata attttttaaa cagatattcc      2729

ctcttgtcct ttaatatttg ctaaatattt cttttttgag gtggagtctt gctctgtcgc      2789

ccaggctgga gtacagtggt gtgatcccag ctcactgcaa cctccgcctc ctgggttcac      2849

atgattctcc tgcctcagct tcctaagtag ctgggtttac aggcacccac caccatgccc      2909

agctaatttt tgtattttta atagagacgg ggtttcgcca tttggccagg ctggtcttga      2969

actcctgacg tcaagtgatc tgcctgcctt ggtctcccaa tacaggcatg aaccactgca      3029

cccacctact tagatatttc atgtgctata gacattagag agatttttca tttttccatg      3089

acatttttcc tctctgcaaa tggcttagct acttgtgttt ttcccttttg gggcaagaca      3149

gactcattaa atattctgta cattttttct ttatcaagga gatatatcag tgttgtctca      3209

tagaactgcc tggattccat ttatgttttt tctgattcca tcctgtgtcc ccttcatcct      3269

tgactccttt ggtatttcac tgaatttcaa acatttgtca gagaagaaaa acgtgaggac      3329

tcaggaaaaa taaataaata aaagaacagc cttttccctt agtattaaca gaaatgtttc      3389

tgtgtcatta accatcttta atcaatgtga catgttgctc tttggctgaa attcttcaac      3449

ttggaaatga cacagaccca cagaaggtgt tcaaacacaa cctactctgc aaaccttggt      3509

aaaggaacca gtcagctggc cagatttcct cactacctgc catgcataca tgctgcgcat 3569
gttttcttca ttcgtatgtt agtaaagttt tggttattat atatttaaca tgtggaagaa 3629
aacaagacat gaaaagagtg gtgacaaatc aagaataaac actggttgta gtcagttttg 3689
tttgttaa 3697

<210> 10
<211> 832
<212> PRT
<213> Homo sapiens

<400> 10

Met Ile Leu Gln Ala His Leu His Ser Leu Cys Leu Leu Met Leu Tyr
1               5                   10                  15

Leu Ala Thr Gly Tyr Gly Gln Glu Gly Lys Phe Ser Gly Pro Leu Lys
            20                  25                  30

Pro Met Thr Phe Ser Ile Tyr Glu Gly Gln Glu Pro Ser Gln Ile Ile
        35                  40                  45

Phe Gln Phe Lys Ala Asn Pro Pro Ala Val Thr Phe Glu Leu Thr Gly
    50                  55                  60

Glu Thr Asp Asn Ile Phe Val Ile Glu Arg Glu Gly Leu Leu Tyr Tyr
65                  70                  75                  80

Asn Arg Ala Leu Asp Arg Glu Thr Arg Ser Thr His Asn Leu Gln Val
                85                  90                  95

Ala Ala Leu Asp Ala Asn Gly Ile Ile Val Glu Gly Pro Val Pro Ile
            100                 105                 110

Thr Ile Glu Val Lys Asp Ile Asn Asp Asn Arg Pro Thr Phe Leu Gln
        115                 120                 125

Ser Lys Tyr Glu Gly Ser Val Arg Gln Asn Ser Arg Pro Gly Lys Pro
    130                 135                 140

Phe Leu Tyr Val Asn Ala Thr Asp Leu Asp Asp Pro Ala Thr Pro Asn
145                 150                 155                 160

Gly Gln Leu Tyr Tyr Gln Ile Val Ile Gln Leu Pro Met Ile Asn Asn
                165                 170                 175

Val Met Tyr Phe Gln Ile Asn Asn Lys Thr Gly Ala Ile Ser Leu Thr
            180                 185                 190

Arg Glu Gly Ser Gln Glu Leu Asn Pro Ala Lys Asn Pro Ser Tyr Asn
        195                 200                 205

Leu Val Ile Ser Val Lys Asp Met Gly Gly Gln Ser Glu Asn Ser Phe
    210                 215                 220

Ser Asp Thr Thr Ser Val Asp Ile Ile Val Thr Glu Asn Ile Trp Lys
225                 230                 235                 240

Ala Pro Lys Pro Val Glu Met Val Glu Asn Ser Thr Asp Pro His Pro
                245                 250                 255

Ile Lys Ile Thr Gln Val Arg Trp Asn Asp Pro Gly Ala Gln Tyr Ser
            260                 265                 270

Leu Val Asp Lys Glu Lys Leu Pro Arg Phe Pro Phe Ser Ile Asp Gln
        275                 280                 285

Glu Gly Asp Ile Tyr Val Thr Gln Pro Leu Asp Arg Glu Glu Lys Asp
    290                 295                 300

Ala Tyr Val Phe Tyr Ala Val Ala Lys Asp Glu Tyr Gly Lys Pro Leu
305                 310                 315                 320

Ser Tyr Pro Leu Glu Ile His Val Lys Val Lys Asp Ile Asn Asp Asn
                325                 330                 335

Pro Pro Thr Cys Pro Ser Pro Val Thr Val Phe Glu Val Gln Glu Asn
            340                 345                 350

Glu Arg Leu Gly Asn Ser Ile Gly Thr Leu Thr Ala His Asp Arg Asp
        355                 360                 365

Glu Glu Asn Thr Ala Asn Ser Phe Leu Asn Tyr Arg Ile Val Glu Gln
    370                 375                 380

Thr Pro Lys Leu Pro Met Asp Gly Leu Phe Leu Ile Gln Thr Tyr Ala
385                 390                 395                 400

Gly Met Leu Gln Leu Ala Lys Gln Ser Leu Lys Lys Gln Asp Thr Pro
                405                 410                 415

Gln Tyr Asn Leu Thr Ile Glu Val Ser Asp Lys Asp Phe Lys Thr Leu
            420                 425                 430

Cys Phe Val Gln Ile Asn Val Ile Asp Ile Asn Asp Gln Ile Pro Ile
        435                 440                 445

Phe Glu Lys Ser Asp Tyr Gly Asn Leu Thr Leu Ala Glu Asp Thr Asn
    450                 455                 460

Ile Gly Ser Thr Ile Leu Thr Ile Gln Ala Thr Asp Ala Asp Glu Pro
465                 470                 475                 480

Phe Thr Gly Ser Ser Lys Ile Leu Tyr His Ile Ile Lys Gly Asp Ser
                485                 490                 495

Glu Gly Arg Leu Gly Val Asp Thr Asp Pro His Thr Asn Thr Gly Tyr
            500                 505                 510

Val Ile Ile Lys Lys Pro Leu Asp Phe Glu Thr Ala Ala Val Ser Asn
        515                 520                 525

Ile Val Phe Lys Ala Glu Asn Pro Glu Pro Leu Val Phe Gly Val Lys
    530                 535                 540

Tyr Asn Ala Ser Ser Phe Ala Lys Phe Thr Leu Ile Val Thr Asp Val
545                 550                 555                 560

Asn Glu Ala Pro Gln Phe Ser Gln His Val Phe Gln Ala Lys Val Ser
                565                 570                 575

Glu Asp Val Ala Ile Gly Thr Lys Val Gly Asn Val Thr Ala Lys Asp
            580                 585                 590

Pro Glu Gly Leu Asp Ile Ser Tyr Ser Leu Arg Gly Asp Thr Arg Gly
        595                 600                 605

Trp Leu Lys Ile Asp His Val Thr Gly Glu Ile Phe Ser Val Ala Pro
    610                 615                 620

Leu Asp Arg Glu Ala Gly Ser Pro Tyr Arg Val Gln Val Val Ala Thr
625                 630                 635                 640

Glu Val Gly Gly Ser Ser Leu Ser Ser Val Ser Glu Phe His Leu Ile
                645                 650                 655

Leu Met Asp Val Asn Asp Asn Pro Pro Arg Leu Ala Lys Asp Tyr Thr
            660                 665                 670

Gly Leu Phe Phe Cys His Pro Leu Ser Ala Pro Gly Ser Leu Ile Phe
        675                 680                 685

Glu Ala Thr Asp Asp Asp Gln His Leu Phe Arg Gly Pro His Phe Thr
    690                 695                 700

Phe Ser Leu Gly Ser Gly Ser Leu Gln Asn Asp Trp Glu Val Ser Lys
705                 710                 715                 720

Ile Asn Gly Thr His Ala Arg Leu Ser Thr Arg His Thr Glu Phe Glu
                725                 730                 735

Glu Arg Glu Tyr Val Val Leu Ile Arg Ile Asn Asp Gly Gly Arg Pro
            740                 745                 750

Pro Leu Glu Gly Ile Val Ser Leu Pro Val Thr Phe Cys Ser Cys Val
        755                 760                 765

Glu Gly Ser Cys Phe Arg Pro Ala Gly His Gln Thr Gly Ile Pro Thr
    770                 775                 780

Val Gly Met Ala Val Gly Ile Leu Leu Thr Thr Leu Leu Val Ile Gly
785                 790                 795                 800

Ile Ile Leu Ala Val Val Phe Ile Arg Ile Lys Lys Asp Lys Gly Lys
                805                 810                 815

Asp Asn Val Glu Ser Ala Gln Ala Ser Glu Val Lys Pro Leu Arg Ser
            820                 825                 830

<210> 11
<211> 4231
<212> DNA
<213> Homo sapiens

<220>
<221> CDS
<222> (116)..(4093)

<400> 11
ggacaggcgt ggcggccgga gccccagcat ccctgcttga ggtccaggag cggagcccgc 60

ggccaccgcc gcctgatcag cgcgaccccg gcccgcgccc gccccgcccg gcaag atg 118
Met
1

ctg ccc gtg tac cag gag gtg aag ccc aac ccg ctg cag gac gcg aac 166
Leu Pro Val Tyr Gln Glu Val Lys Pro Asn Pro Leu Gln Asp Ala Asn
            5                   10                  15

atc tgc tca cgc gtg ttc ttc tgg tgg ctc aat ccc ttg ttt aaa att 214
Ile Cys Ser Arg Val Phe Phe Trp Trp Leu Asn Pro Leu Phe Lys Ile
        20                  25                  30

ggc cat aaa cgg aga tta gag gaa gat gat atg tat tca gtg ctg cca 262
Gly His Lys Arg Arg Leu Glu Glu Asp Asp Met Tyr Ser Val Leu Pro
    35                  40                  45

gaa gac cgc tca cag cac ctt gga gag gag ttg caa ggg ttc tgg gat 310
Glu Asp Arg Ser Gln His Leu Gly Glu Glu Leu Gln Gly Phe Trp Asp
50                  55                  60                  65

aaa gaa gtt tta aga gct gag aat gac gca cag aag cct tct tta aca 358
Lys Glu Val Leu Arg Ala Glu Asn Asp Ala Gln Lys Pro Ser Leu Thr
                70                  75                  80

aga gca atc ata aag tgt tac tgg aaa tct tat tta gtt ttg gga att 406
Arg Ala Ile Ile Lys Cys Tyr Trp Lys Ser Tyr Leu Val Leu Gly Ile
            85                  90                  95

ttt acg tta att gag gaa agt gcc aaa gta atc cag ccc ata ttt ttg 454
Phe Thr Leu Ile Glu Glu Ser Ala Lys Val Ile Gln Pro Ile Phe Leu
        100                 105                 110

gga aaa att att aat tat ttt gaa aat tat gat ccc atg gat tct gtg 502
Gly Lys Ile Ile Asn Tyr Phe Glu Asn Tyr Asp Pro Met Asp Ser Val

115                    120                    125

gct ttg aac aca gcg tac gcc tat gcc acg gtg ctg act ttt tgc acg          550
Ala Leu Asn Thr Ala Tyr Ala Tyr Ala Thr Val Leu Thr Phe Cys Thr
130                 135                 140                 145

ctc att ttg gct ata ctg cat cac tta tat ttt tat cac gtt cag tgt          598
Leu Ile Leu Ala Ile Leu His His Leu Tyr Phe Tyr His Val Gln Cys
                150                 155                 160

gct ggg atg agg tta cga gta gcc atg tgc cat atg att tat cgg aag          646
Ala Gly Met Arg Leu Arg Val Ala Met Cys His Met Ile Tyr Arg Lys
            165                 170                 175

gca ctt cgt ctt agt aac atg gcc atg ggg aag aca acc aca ggc cag          694
Ala Leu Arg Leu Ser Asn Met Ala Met Gly Lys Thr Thr Thr Gly Gln
        180                 185                 190

ata gtc aat ctg ctg tcc aat gat gtg aac aag ttt gat cag gtg aca          742
Ile Val Asn Leu Leu Ser Asn Asp Val Asn Lys Phe Asp Gln Val Thr
    195                 200                 205

gtg ttc tta cac ttc ctg tgg gca gga cca ctg cag gcg atc gca gtg          790
Val Phe Leu His Phe Leu Trp Ala Gly Pro Leu Gln Ala Ile Ala Val
210                 215                 220                 225

act gcc cta ctc tgg atg gag ata gga ata tcg tgc ctt gct ggg atg          838
Thr Ala Leu Leu Trp Met Glu Ile Gly Ile Ser Cys Leu Ala Gly Met
                230                 235                 240

gca gtt cta atc att ctc ctg ccc ttg caa agc tgt ttt ggg aag ttg          886
Ala Val Leu Ile Ile Leu Leu Pro Leu Gln Ser Cys Phe Gly Lys Leu
            245                 250                 255

ttc tca tca ctg agg agt aaa act gca act ttc acg gat gcc agg atc          934
Phe Ser Ser Leu Arg Ser Lys Thr Ala Thr Phe Thr Asp Ala Arg Ile
        260                 265                 270

agg acc atg aat gaa gtt ata act ggt ata agg ata ata aaa atg tac          982
Arg Thr Met Asn Glu Val Ile Thr Gly Ile Arg Ile Ile Lys Met Tyr
    275                 280                 285

gcc tgg gaa aag tca ttt tca aat ctt att acc aat ttg aga aag aag          1030
Ala Trp Glu Lys Ser Phe Ser Asn Leu Ile Thr Asn Leu Arg Lys Lys
290                 295                 300                 305

gag att tcc aag att ctg aga agt tcc tgc ctc agg ggg atg aat ttg          1078
Glu Ile Ser Lys Ile Leu Arg Ser Ser Cys Leu Arg Gly Met Asn Leu
                310                 315                 320

gct tcg ttt ttc agt gca agc aaa atc atc gtg ttt gtg acc ttc acc          1126
Ala Ser Phe Phe Ser Ala Ser Lys Ile Ile Val Phe Val Thr Phe Thr
            325                 330                 335

acc tac gtg ctc ctc ggc agt gtg atc aca gcc agc cgc gtg ttc gtg          1174
Thr Tyr Val Leu Leu Gly Ser Val Ile Thr Ala Ser Arg Val Phe Val
        340                 345                 350

gca gtg acg ctg tat ggg gct gtg cgg ctg acg gtt acc ctc ttc ttc          1222
Ala Val Thr Leu Tyr Gly Ala Val Arg Leu Thr Val Thr Leu Phe Phe
    355                 360                 365

ccc tca gcc att gag agg gtg tca gag gca atc gtc agc atc cga aga          1270
Pro Ser Ala Ile Glu Arg Val Ser Glu Ala Ile Val Ser Ile Arg Arg
370                 375                 380                 385

atc cag acc ttt ttg cta ctt gat gag ata tca cag cgc aac cgt cag          1318
Ile Gln Thr Phe Leu Leu Leu Asp Glu Ile Ser Gln Arg Asn Arg Gln

                390                 395                 400

ctg ccg tca gat ggt aaa aag atg gtg cat gtg cag gat ttt act gct 1366
Leu Pro Ser Asp Gly Lys Lys Met Val His Val Gln Asp Phe Thr Ala
            405                 410                 415

ttt tgg gat aag gca tca gag acc cca act cta caa ggc ctt tcc ttt 1414
Phe Trp Asp Lys Ala Ser Glu Thr Pro Thr Leu Gln Gly Leu Ser Phe
        420                 425                 430

act gtc aga cct ggc gaa ttg tta gct gtg gtc ggc ccc gtg gga gca 1462
Thr Val Arg Pro Gly Glu Leu Leu Ala Val Val Gly Pro Val Gly Ala
    435                 440                 445

ggg aag tca tca ctg tta agt gcc gtg ctc ggg gaa ttg gcc cca agt 1510
Gly Lys Ser Ser Leu Leu Ser Ala Val Leu Gly Glu Leu Ala Pro Ser
450                 455                 460                 465

cac ggg ctg gtc agc gtg cat gga aga att gcc tat gtg tct cag cag 1558
His Gly Leu Val Ser Val His Gly Arg Ile Ala Tyr Val Ser Gln Gln
                470                 475                 480

ccc tgg gtg ttc tcg gga act ctg agg agt aat att tta ttt ggg aag 1606
Pro Trp Val Phe Ser Gly Thr Leu Arg Ser Asn Ile Leu Phe Gly Lys
            485                 490                 495

aaa tat gaa aag gaa cga tat gaa aaa gtc ata aag gct tgt gct ctg 1654
Lys Tyr Glu Lys Glu Arg Tyr Glu Lys Val Ile Lys Ala Cys Ala Leu
        500                 505                 510

aaa aag gat tta cag ctg ttg gag gat ggt gat ctg act gtg ata gga 1702
Lys Lys Asp Leu Gln Leu Leu Glu Asp Gly Asp Leu Thr Val Ile Gly
    515                 520                 525

gat cgg gga acc acg ctg agt gga ggg cag aaa gca cgg gta aac ctt 1750
Asp Arg Gly Thr Thr Leu Ser Gly Gly Gln Lys Ala Arg Val Asn Leu
530                 535                 540                 545

gca aga gca gtg tat caa gat gct gac atc tat ctc ctg gac gat cct 1798
Ala Arg Ala Val Tyr Gln Asp Ala Asp Ile Tyr Leu Leu Asp Asp Pro
                550                 555                 560

ctc agt gca gta gat gcg gaa gtt agc aga cac ttg ttc gaa ctg tgt 1846
Leu Ser Ala Val Asp Ala Glu Val Ser Arg His Leu Phe Glu Leu Cys
            565                 570                 575

att tgt caa att ttg cat gag aag atc aca att tta gtg act cat cag 1894
Ile Cys Gln Ile Leu His Glu Lys Ile Thr Ile Leu Val Thr His Gln
        580                 585                 590

ttg cag tac ctc aaa gct gca agt cag att ctg ata ttg aaa gat ggt 1942
Leu Gln Tyr Leu Lys Ala Ala Ser Gln Ile Leu Ile Leu Lys Asp Gly
    595                 600                 605

aaa atg gtg cag aag ggg act tac act gag ttc cta aaa tct ggt ata 1990
Lys Met Val Gln Lys Gly Thr Tyr Thr Glu Phe Leu Lys Ser Gly Ile
610                 615                 620                 625

gat ttt ggc tcc ctt tta aag aag gat aat gag gaa agt gaa caa cct 2038
Asp Phe Gly Ser Leu Leu Lys Lys Asp Asn Glu Glu Ser Glu Gln Pro
                630                 635                 640

cca gtt cca gga act ccc aca cta agg aat cgt acc ttc tca gag tct 2086
Pro Val Pro Gly Thr Pro Thr Leu Arg Asn Arg Thr Phe Ser Glu Ser
            645                 650                 655

tcg gtt tgg tct caa caa tct tct aga ccc tcc ttg aaa gat ggt gct 2134
Ser Val Trp Ser Gln Gln Ser Ser Arg Pro Ser Leu Lys Asp Gly Ala

660                 665                 670

ctg gag agc caa gat aca gag aat gtc cca gtt aca cta tca gag gag      2182
Leu Glu Ser Gln Asp Thr Glu Asn Val Pro Val Thr Leu Ser Glu Glu
    675                 680                 685

aac cgt tct gaa gga aaa gtt ggt ttt cag gcc tat aag aat tac ttc      2230
Asn Arg Ser Glu Gly Lys Val Gly Phe Gln Ala Tyr Lys Asn Tyr Phe
690                 695                 700                 705

aga gct ggt gct cac tgg att gtc ttc att ttc ctt att ctc cta aac      2278
Arg Ala Gly Ala His Trp Ile Val Phe Ile Phe Leu Ile Leu Leu Asn
                710                 715                 720

act gca gct cag gtt gcc tat gtg ctt caa gat tgg tgg ctt tca tac      2326
Thr Ala Ala Gln Val Ala Tyr Val Leu Gln Asp Trp Trp Leu Ser Tyr
            725                 730                 735

tgg gca aac aaa caa agt atg cta aat gtc act gta aat gga gga gga      2374
Trp Ala Asn Lys Gln Ser Met Leu Asn Val Thr Val Asn Gly Gly Gly
        740                 745                 750

aat gta acc gag aag cta gat ctt aac tgg tac tta gga att tat tca      2422
Asn Val Thr Glu Lys Leu Asp Leu Asn Trp Tyr Leu Gly Ile Tyr Ser
    755                 760                 765

ggt tta act gta gct acc gtt ctt ttt ggc ata gca aga tct cta ttg      2470
Gly Leu Thr Val Ala Thr Val Leu Phe Gly Ile Ala Arg Ser Leu Leu
770                 775                 780                 785

gta ttc tac gtc ctt gtt aac tct tca caa act ttg cac aac aaa atg      2518
Val Phe Tyr Val Leu Val Asn Ser Ser Gln Thr Leu His Asn Lys Met
                790                 795                 800

ttt gag tca att ctg aaa gct ccg gta tta ttc ttt gat aga aat cca      2566
Phe Glu Ser Ile Leu Lys Ala Pro Val Leu Phe Phe Asp Arg Asn Pro
            805                 810                 815

ata gga aga att tta aat cgt ttc tcc aaa gac att gga cac ttg gat      2614
Ile Gly Arg Ile Leu Asn Arg Phe Ser Lys Asp Ile Gly His Leu Asp
        820                 825                 830

gat ttg ctg ccg ctg acg ttt tta gat ttc atc cag aca ttg cta caa      2662
Asp Leu Leu Pro Leu Thr Phe Leu Asp Phe Ile Gln Thr Leu Leu Gln
    835                 840                 845

gtg gtt ggt gtg gtc tct gtg gct gtg gcc gtg att cct tgg atc gca      2710
Val Val Gly Val Val Ser Val Ala Val Ala Val Ile Pro Trp Ile Ala
850                 855                 860                 865

ata ccc ttg gtt ccc ctt gga atc att ttc att ttt ctt cgg cga tat      2758
Ile Pro Leu Val Pro Leu Gly Ile Ile Phe Ile Phe Leu Arg Arg Tyr
                870                 875                 880

ttt ttg gaa acg tca aga gat gtg aag cgc ctg gaa tct aca act cgg      2806
Phe Leu Glu Thr Ser Arg Asp Val Lys Arg Leu Glu Ser Thr Thr Arg
            885                 890                 895

agt cca gtg ttt tcc cac ttg tca tct tct ctc cag ggg ctc tgg acc      2854
Ser Pro Val Phe Ser His Leu Ser Ser Ser Leu Gln Gly Leu Trp Thr
        900                 905                 910

atc cgg gca tac aaa gca gaa gag agg tgt cag gaa ctg ttt gat gca      2902
Ile Arg Ala Tyr Lys Ala Glu Glu Arg Cys Gln Glu Leu Phe Asp Ala
    915                 920                 925

cac cag gat tta cat tca gag gct tgg ttc ttg ttt ttg aca acg tcc      2950
His Gln Asp Leu His Ser Glu Ala Trp Phe Leu Phe Leu Thr Thr Ser

930                 935                 940                 945

cgc tgg ttc gcc gtc cgt ctg gat gcc atc tgt gcc atg ttt gtc atc     2998
Arg Trp Phe Ala Val Arg Leu Asp Ala Ile Cys Ala Met Phe Val Ile
                950                 955                 960

atc gtt gcc ttt ggg tcc ctg att ctg gca aaa act ctg gat gcc ggg     3046
Ile Val Ala Phe Gly Ser Leu Ile Leu Ala Lys Thr Leu Asp Ala Gly
            965                 970                 975

cag gtt ggt ttg gca ctg tcc tat gcc ctc acg ctc atg ggg atg ttt     3094
Gln Val Gly Leu Ala Leu Ser Tyr Ala Leu Thr Leu Met Gly Met Phe
        980                 985                 990

cag tgg tgt gtt cga caa agt  gct gaa gtt gag aat  atg atg atc tca     3142
Gln Trp Cys Val Arg Gln Ser  Ala Glu Val Glu Asn  Met Met Ile Ser
    995                 1000                 1005

gta  gaa agg gtc att gaa  tac aca gac ctt gaa  aaa gaa gca cct     3187
Val  Glu Arg Val Ile Glu  Tyr Thr Asp Leu Glu  Lys Glu Ala Pro
1010                 1015                 1020

tgg  gaa tat cag aaa cgc  cca cca cca gcc tgg  ccc cat gaa gga     3232
Trp  Glu Tyr Gln Lys Arg  Pro Pro Pro Ala Trp  Pro His Glu Gly
1025                 1030                 1035

gtg  ata atc ttt gac aat  gtg aac ttc atg tac  agt cca ggt ggg     3277
Val  Ile Ile Phe Asp Asn  Val Asn Phe Met Tyr  Ser Pro Gly Gly
1040                 1045                 1050

cct  ctg gta ctg aag cat  ctg aca gca ctc att  aaa tca caa gaa     3322
Pro  Leu Val Leu Lys His  Leu Thr Ala Leu Ile  Lys Ser Gln Glu
1055                 1060                 1065

aag  gtt ggc att gtg gga  aga acc gga gct gga  aaa agt tcc ctc     3367
Lys  Val Gly Ile Val Gly  Arg Thr Gly Ala Gly  Lys Ser Ser Leu
1070                 1075                 1080

atc  tca gcc ctt ttt aga  ttg tca gaa ccc gaa  ggt aaa att tgg     3412
Ile  Ser Ala Leu Phe Arg  Leu Ser Glu Pro Glu  Gly Lys Ile Trp
1085                 1090                 1095

att  gat aag atc ttg aca  act gaa att gga ctt  cac gat tta agg     3457
Ile  Asp Lys Ile Leu Thr  Thr Glu Ile Gly Leu  His Asp Leu Arg
1100                 1105                 1110

aag  aaa atg tca atc ata  cct cag gaa cct gtt  ttg ttc act gga     3502
Lys  Lys Met Ser Ile Ile  Pro Gln Glu Pro Val  Leu Phe Thr Gly
1115                 1120                 1125

aca  atg agg aaa aac ctg  gat ccc ttt aag gag  cac acg gat gag     3547
Thr  Met Arg Lys Asn Leu  Asp Pro Phe Lys Glu  His Thr Asp Glu
1130                 1135                 1140

gaa  ctg tgg aat gcc tta  caa gag gta caa ctt  aaa gaa acc att     3592
Glu  Leu Trp Asn Ala Leu  Gln Glu Val Gln Leu  Lys Glu Thr Ile
1145                 1150                 1155

gaa  gat ctt cct ggt aaa  atg gat act gaa tta  gca gaa tca gga     3637
Glu  Asp Leu Pro Gly Lys  Met Asp Thr Glu Leu  Ala Glu Ser Gly
1160                 1165                 1170

tcc  aat ttt agt gtt gga  caa aga caa ctg gtg  tgc ctt gcc agg     3682
Ser  Asn Phe Ser Val Gly  Gln Arg Gln Leu Val  Cys Leu Ala Arg
1175                 1180                 1185

gca  att ctc agg aaa aat  cag ata ttg att att  gat gaa gcg acg     3727
Ala  Ile Leu Arg Lys Asn  Gln Ile Leu Ile Ile  Asp Glu Ala Thr

1190                1195                1200

gca aat gtg gat cca aga act gat gag tta ata caa aaa aaa atc      3772
Ala Asn Val Asp Pro Arg Thr Asp Glu Leu Ile Gln Lys Lys Ile
1205                1210                1215

cgg gag aaa ttt gcc cac tgc acc gtg cta acc att gca cac aga      3817
Arg Glu Lys Phe Ala His Cys Thr Val Leu Thr Ile Ala His Arg
1220                1225                1230

ttg aac acc att att gac agc gac aag ata atg gtt tta gat tca      3862
Leu Asn Thr Ile Ile Asp Ser Asp Lys Ile Met Val Leu Asp Ser
1235                1240                1245

gga aga ctg aaa gaa tat gat gag ccg tat gtt ttg ctg caa aat      3907
Gly Arg Leu Lys Glu Tyr Asp Glu Pro Tyr Val Leu Leu Gln Asn
1250                1255                1260

aaa gag agc cta ttt tac aag atg gtg caa caa ctg ggc aag gca      3952
Lys Glu Ser Leu Phe Tyr Lys Met Val Gln Gln Leu Gly Lys Ala
1265                1270                1275

gaa gcc gct gcc ctc act gaa aca gca aaa cag gta tac ttc aaa      3997
Glu Ala Ala Ala Leu Thr Glu Thr Ala Lys Gln Val Tyr Phe Lys
1280                1285                1290

aga aat tat cca cat att ggt cac act gac cac atg gtt aca aac      4042
Arg Asn Tyr Pro His Ile Gly His Thr Asp His Met Val Thr Asn
1295                1300                1305

act tcc aat gga cag ccc tcg acc tta act att ttc gag aca gca      4087
Thr Ser Asn Gly Gln Pro Ser Thr Leu Thr Ile Phe Glu Thr Ala
1310                1315                1320

ctg tga atccaaccaa aatgtcaagt ccgttccgaa ggcattttcc actagttttt      4143
Leu
1325

ggactatgta aaccacattg tacttttttt tactttggca acaaatattt atacatacaa      4203

gatgctagtt catttgaata tttctccc                                        4231

<210>  12
<211>  1325
<212>  PRT
<213>  Homo sapiens

<400>  12

Met Leu Pro Val Tyr Gln Glu Val Lys Pro Asn Pro Leu Gln Asp Ala
1               5                   10                  15

Asn Ile Cys Ser Arg Val Phe Phe Trp Trp Leu Asn Pro Leu Phe Lys
            20                  25                  30

Ile Gly His Lys Arg Arg Leu Glu Glu Asp Asp Met Tyr Ser Val Leu
        35                  40                  45

Pro Glu Asp Arg Ser Gln His Leu Gly Glu Glu Leu Gln Gly Phe Trp
    50                  55                  60

Asp Lys Glu Val Leu Arg Ala Glu Asn Asp Ala Gln Lys Pro Ser Leu
65                  70                  75                  80

Thr Arg Ala Ile Ile Lys Cys Tyr Trp Lys Ser Tyr Leu Val Leu Gly
                85                  90                  95

Ile Phe Thr Leu Ile Glu Glu Ser Ala Lys Val Ile Gln Pro Ile Phe
            100                 105                 110

Leu Gly Lys Ile Ile Asn Tyr Phe Glu Asn Tyr Asp Pro Met Asp Ser
        115                 120                 125

Val Ala Leu Asn Thr Ala Tyr Ala Tyr Ala Thr Val Leu Thr Phe Cys
    130                 135                 140

Thr Leu Ile Leu Ala Ile Leu His His Leu Tyr Phe Tyr His Val Gln
145                 150                 155                 160

Cys Ala Gly Met Arg Leu Arg Val Ala Met Cys His Met Ile Tyr Arg
                165                 170                 175

Lys Ala Leu Arg Leu Ser Asn Met Ala Met Gly Lys Thr Thr Thr Gly
            180                 185                 190

Gln Ile Val Asn Leu Leu Ser Asn Asp Val Asn Lys Phe Asp Gln Val
        195                 200                 205

Thr Val Phe Leu His Phe Leu Trp Ala Gly Pro Leu Gln Ala Ile Ala
    210                 215                 220

Val Thr Ala Leu Leu Trp Met Glu Ile Gly Ile Ser Cys Leu Ala Gly
225                 230                 235                 240

Met Ala Val Leu Ile Ile Leu Leu Pro Leu Gln Ser Cys Phe Gly Lys
                245                 250                 255

Leu Phe Ser Ser Leu Arg Ser Lys Thr Ala Thr Phe Thr Asp Ala Arg
            260                 265                 270

Ile Arg Thr Met Asn Glu Val Ile Thr Gly Ile Arg Ile Ile Lys Met
        275                 280                 285

Tyr Ala Trp Glu Lys Ser Phe Ser Asn Leu Ile Thr Asn Leu Arg Lys
    290                 295                 300

Lys Glu Ile Ser Lys Ile Leu Arg Ser Ser Cys Leu Arg Gly Met Asn
305                 310                 315                 320

Leu Ala Ser Phe Phe Ser Ala Ser Lys Ile Ile Val Phe Val Thr Phe
                325                 330                 335

Thr Thr Tyr Val Leu Leu Gly Ser Val Ile Thr Ala Ser Arg Val Phe
            340                 345                 350

Val Ala Val Thr Leu Tyr Gly Ala Val Arg Leu Thr Val Thr Leu Phe
        355                 360                 365

Phe Pro Ser Ala Ile Glu Arg Val Ser Glu Ala Ile Val Ser Ile Arg
    370                 375                 380

Arg Ile Gln Thr Phe Leu Leu Leu Asp Glu Ile Ser Gln Arg Asn Arg
385                 390                 395                 400

Gln Leu Pro Ser Asp Gly Lys Lys Met Val His Val Gln Asp Phe Thr
                405                 410                 415

Ala Phe Trp Asp Lys Ala Ser Glu Thr Pro Thr Leu Gln Gly Leu Ser
            420                 425                 430

Phe Thr Val Arg Pro Gly Glu Leu Leu Ala Val Val Gly Pro Val Gly
        435                 440                 445

Ala Gly Lys Ser Ser Leu Leu Ser Ala Val Leu Gly Glu Leu Ala Pro
    450                 455                 460

Ser His Gly Leu Val Ser Val His Gly Arg Ile Ala Tyr Val Ser Gln
465                 470                 475                 480

Gln Pro Trp Val Phe Ser Gly Thr Leu Arg Ser Asn Ile Leu Phe Gly
                485                 490                 495

Lys Lys Tyr Glu Lys Glu Arg Tyr Glu Lys Val Ile Lys Ala Cys Ala
            500                 505                 510

Leu Lys Lys Asp Leu Gln Leu Leu Glu Asp Gly Asp Leu Thr Val Ile
        515                 520                 525

Gly Asp Arg Gly Thr Thr Leu Ser Gly Gly Gln Lys Ala Arg Val Asn
    530                 535                 540

Leu Ala Arg Ala Val Tyr Gln Asp Ala Asp Ile Tyr Leu Leu Asp Asp
545                 550                 555                 560

Pro Leu Ser Ala Val Asp Ala Glu Val Ser Arg His Leu Phe Glu Leu
                565                 570                 575

Cys Ile Cys Gln Ile Leu His Glu Lys Ile Thr Ile Leu Val Thr His
            580                 585                 590

Gln Leu Gln Tyr Leu Lys Ala Ala Ser Gln Ile Leu Ile Leu Lys Asp
        595                 600                 605

Gly Lys Met Val Gln Lys Gly Thr Tyr Thr Glu Phe Leu Lys Ser Gly
    610                 615                 620

Ile Asp Phe Gly Ser Leu Leu Lys Lys Asp Asn Glu Glu Ser Glu Gln
625                 630                 635                 640

Pro Pro Val Pro Gly Thr Pro Thr Leu Arg Asn Arg Thr Phe Ser Glu
                645                 650                 655

Ser Ser Val Trp Ser Gln Gln Ser Ser Arg Pro Ser Leu Lys Asp Gly
            660                 665                 670

Ala Leu Glu Ser Gln Asp Thr Glu Asn Val Pro Val Thr Leu Ser Glu
        675                 680                 685

Glu Asn Arg Ser Glu Gly Lys Val Gly Phe Gln Ala Tyr Lys Asn Tyr
    690                 695                 700

Phe Arg Ala Gly Ala His Trp Ile Val Phe Ile Phe Leu Ile Leu Leu
705                 710                 715                 720

Asn Thr Ala Ala Gln Val Ala Tyr Val Leu Gln Asp Trp Trp Leu Ser
                725                 730                 735

Tyr Trp Ala Asn Lys Gln Ser Met Leu Asn Val Thr Val Asn Gly Gly
            740                 745                 750

Gly Asn Val Thr Glu Lys Leu Asp Leu Asn Trp Tyr Leu Gly Ile Tyr
        755                 760                 765

Ser Gly Leu Thr Val Ala Thr Val Leu Phe Gly Ile Ala Arg Ser Leu
    770                 775                 780

Leu Val Phe Tyr Val Leu Val Asn Ser Ser Gln Thr Leu His Asn Lys
785                 790                 795                 800

Met Phe Glu Ser Ile Leu Lys Ala Pro Val Leu Phe Phe Asp Arg Asn
                805                 810                 815

Pro Ile Gly Arg Ile Leu Asn Arg Phe Ser Lys Asp Ile Gly His Leu
            820                 825                 830

Asp Asp Leu Leu Pro Leu Thr Phe Leu Asp Phe Ile Gln Thr Leu Leu
        835                 840                 845

Gln Val Val Gly Val Val Ser Val Ala Val Ala Val Ile Pro Trp Ile
    850                 855                 860

Ala Ile Pro Leu Val Pro Leu Gly Ile Ile Phe Ile Phe Leu Arg Arg
865                 870                 875                 880

Tyr Phe Leu Glu Thr Ser Arg Asp Val Lys Arg Leu Glu Ser Thr Thr
                885                 890                 895

Arg Ser Pro Val Phe Ser His Leu Ser Ser Ser Leu Gln Gly Leu Trp
            900                 905                 910

Thr Ile Arg Ala Tyr Lys Ala Glu Glu Arg Cys Gln Glu Leu Phe Asp
        915                 920                 925

Ala His Gln Asp Leu His Ser Glu Ala Trp Phe Leu Phe Leu Thr Thr
    930                 935                 940

Ser Arg Trp Phe Ala Val Arg Leu Asp Ala Ile Cys Ala Met Phe Val
945                 950                 955                 960

Ile Ile Val Ala Phe Gly Ser Leu Ile Leu Ala Lys Thr Leu Asp Ala
                965                 970                 975

Gly Gln Val Gly Leu Ala Leu Ser Tyr Ala Leu Thr Leu Met Gly Met
            980                 985                 990

Phe Gln Trp Cys Val Arg Gln Ser  Ala Glu Val Glu Asn  Met Met Ile
        995                 1000                 1005

Ser Val  Glu Arg Val Ile Glu  Tyr Thr Asp Leu Glu  Lys Glu Ala
    1010                 1015                 1020

Pro Trp  Glu Tyr Gln Lys Arg  Pro Pro Pro Ala Trp  Pro His Glu
    1025                 1030                 1035

Gly Val  Ile Ile Phe Asp Asn  Val Asn Phe Met Tyr  Ser Pro Gly
    1040                 1045                 1050

Gly Pro  Leu Val Leu Lys His  Leu Thr Ala Leu Ile  Lys Ser Gln
    1055                 1060                 1065

Glu Lys  Val Gly Ile Val Gly  Arg Thr Gly Ala Gly  Lys Ser Ser
    1070                 1075                 1080

Leu Ile  Ser Ala Leu Phe Arg  Leu Ser Glu Pro Glu  Gly Lys Ile
    1085                 1090                 1095

Trp Ile  Asp Lys Ile Leu Thr  Thr Glu Ile Gly Leu  His Asp Leu
    1100                 1105                 1110

Arg Lys  Lys Met Ser Ile Ile  Pro Gln Glu Pro Val  Leu Phe Thr
    1115                 1120                 1125

Gly Thr  Met Arg Lys Asn Leu  Asp Pro Phe Lys Glu  His Thr Asp
    1130                 1135                 1140

Glu Glu  Leu Trp Asn Ala Leu  Gln Glu Val Gln Leu  Lys Glu Thr
    1145                 1150                 1155

Ile Glu Asp Leu Pro Gly Lys Met Asp Thr Glu Leu Ala Glu Ser
    1160                1165                1170

Gly Ser Asn Phe Ser Val Gly Gln Arg Gln Leu Val Cys Leu Ala
    1175                1180                1185

Arg Ala Ile Leu Arg Lys Asn Gln Ile Leu Ile Ile Asp Glu Ala
    1190                1195                1200

Thr Ala Asn Val Asp Pro Arg Thr Asp Glu Leu Ile Gln Lys Lys
    1205                1210                1215

Ile Arg Glu Lys Phe Ala His Cys Thr Val Leu Thr Ile Ala His
    1220                1225                1230

Arg Leu Asn Thr Ile Ile Asp Ser Asp Lys Ile Met Val Leu Asp
    1235                1240                1245

Ser Gly Arg Leu Lys Glu Tyr Asp Glu Pro Tyr Val Leu Leu Gln
    1250                1255                1260

Asn Lys Glu Ser Leu Phe Tyr Lys Met Val Gln Gln Leu Gly Lys
    1265                1270                1275

Ala Glu Ala Ala Ala Leu Thr Glu Thr Ala Lys Gln Val Tyr Phe
    1280                1285                1290

Lys Arg Asn Tyr Pro His Ile Gly His Thr Asp His Met Val Thr
    1295                1300                1305

Asn Thr Ser Asn Gly Gln Pro Ser Thr Leu Thr Ile Phe Glu Thr
    1310                1315                1320

Ala Leu
    1325

<210> 13
<211> 2702
<212> DNA
<213> Homo sapiens

<220>
<221> CDS
<222> (25)..(2508)

<400> 13
cattctcccc caggctcact cacc atg acc aag ctg agc gcc caa gtc aaa 51
Met Thr Lys Leu Ser Ala Gln Val Lys
1               5

ggc tct ctc aac atc acc acc ccg ggg ctg cag ata tgg agg atc gag 99
Gly Ser Leu Asn Ile Thr Thr Pro Gly Leu Gln Ile Trp Arg Ile Glu
10                  15                  20                  25

gcc atg cag atg gtg cct gtt cct tcc agc acc ttt gga agc ttc ttc 147
Ala Met Gln Met Val Pro Val Pro Ser Ser Thr Phe Gly Ser Phe Phe
                30                  35                  40

gat ggt gac tgc tac atc atc ctg gct atc cac aag aca gcc agc agc 195
Asp Gly Asp Cys Tyr Ile Ile Leu Ala Ile His Lys Thr Ala Ser Ser
            45                  50                  55

ctg tcc tat gac atc cac tac tgg att ggc cag gac tca tcc ctg gat 243
Leu Ser Tyr Asp Ile His Tyr Trp Ile Gly Gln Asp Ser Ser Leu Asp
        60                  65                  70

gag cag ggg gca gct gcc atc tac acc aca cag atg gat gac ttc ctg 291
Glu Gln Gly Ala Ala Ala Ile Tyr Thr Thr Gln Met Asp Asp Phe Leu
    75                  80                  85

aag ggc cgg gct gtg cag cac cgc gag gtc cag ggc aac gag agc gag 339
Lys Gly Arg Ala Val Gln His Arg Glu Val Gln Gly Asn Glu Ser Glu
90                  95                  100                 105

gcc ttc cga ggc tac ttc aag caa ggc ctt gtg atc cgg aaa ggg ggc 387
Ala Phe Arg Gly Tyr Phe Lys Gln Gly Leu Val Ile Arg Lys Gly Gly
                110                 115                 120

gtg gct tct ggc atg aag cac gtg gag acc aac tcc tat gac gtc cag 435
Val Ala Ser Gly Met Lys His Val Glu Thr Asn Ser Tyr Asp Val Gln
            125                 130                 135

agg ctg ctg cat gtc aag ggc aag agg aac gtg gta gct gga gag gta 483
Arg Leu Leu His Val Lys Gly Lys Arg Asn Val Val Ala Gly Glu Val
        140                 145                 150

gag atg tcc tgg aag agt ttc aac cga ggg gat gtt ttc ctc ctg gac 531
Glu Met Ser Trp Lys Ser Phe Asn Arg Gly Asp Val Phe Leu Leu Asp
    155                 160                 165

ctt ggg aag ctt atc atc cag tgg aat gga ccg gaa agc acc cgt atg 579
Leu Gly Lys Leu Ile Ile Gln Trp Asn Gly Pro Glu Ser Thr Arg Met
170                 175                 180                 185

gag aga ctc agg ggc atg act ctg gcc aag gag atc cga gac cag gag 627
Glu Arg Leu Arg Gly Met Thr Leu Ala Lys Glu Ile Arg Asp Gln Glu
                190                 195                 200

cgg gga ggg cgc acc tat gta ggc gtg gtg gac gga gag aat gaa ttg 675
Arg Gly Gly Arg Thr Tyr Val Gly Val Val Asp Gly Glu Asn Glu Leu
            205                 210                 215

gca tcc ccg aag ctg atg gag gtg atg aac cac gtg ctg ggc aag cgc 723
Ala Ser Pro Lys Leu Met Glu Val Met Asn His Val Leu Gly Lys Arg
        220                 225                 230

agg gag ctg aag gcg gcc gtg ccc gac acg gtg gtg gag ccg gca ctc 771
Arg Glu Leu Lys Ala Ala Val Pro Asp Thr Val Val Glu Pro Ala Leu
    235                 240                 245

aag gct gca ctc aaa ctg tac cat gtg tct gac tcc gag ggg aat ctg 819
Lys Ala Ala Leu Lys Leu Tyr His Val Ser Asp Ser Glu Gly Asn Leu
250                 255                 260                 265

gtg gtg agg gaa gtc gcc aca cgg cca ctg aca cag gac ctg ctc agt 867
Val Val Arg Glu Val Ala Thr Arg Pro Leu Thr Gln Asp Leu Leu Ser
                270                 275                 280

cac gag gac tgt tac atc ctg gac cag ggg ggc ctg aag atc tac gtg 915
His Glu Asp Cys Tyr Ile Leu Asp Gln Gly Gly Leu Lys Ile Tyr Val
            285                 290                 295

tgg aaa ggg aag aaa gcc aat gag cag gag aag aag gga gcc atg agc     963
Trp Lys Gly Lys Lys Ala Asn Glu Gln Glu Lys Lys Gly Ala Met Ser
    300                 305                 310

cat gcg ctg aac ttc atc aaa gcc aag cag tac cca cca agc aca cag     1011
His Ala Leu Asn Phe Ile Lys Ala Lys Gln Tyr Pro Pro Ser Thr Gln
    315                 320                 325

gtg gag gtg cag aat gat ggg gct gag tcg gcc gtc ttt cag cag ctc     1059
Val Glu Val Gln Asn Asp Gly Ala Glu Ser Ala Val Phe Gln Gln Leu
330                 335                 340                 345

ttc cag aag tgg aca gcg tcc aac cgg acc tca ggc cta ggc aaa acc     1107
Phe Gln Lys Trp Thr Ala Ser Asn Arg Thr Ser Gly Leu Gly Lys Thr
                350                 355                 360

cac act gtg ggc tcc gtg gcc aaa gtg gaa cag gtg aag ttc gat gcc     1155
His Thr Val Gly Ser Val Ala Lys Val Glu Gln Val Lys Phe Asp Ala
            365                 370                 375

aca tcc atg cat gtc aag cct cag gtg gct gcc cag cag aag atg gta     1203
Thr Ser Met His Val Lys Pro Gln Val Ala Ala Gln Gln Lys Met Val
        380                 385                 390

gat gat ggg agt ggg gaa gtg cag gtg tgg cgc att gag aac cta gag     1251
Asp Asp Gly Ser Gly Glu Val Gln Val Trp Arg Ile Glu Asn Leu Glu
    395                 400                 405

ctg gta cct gtg gat tcc aag tgg cta ggc cac ttc tat ggg ggc gac     1299
Leu Val Pro Val Asp Ser Lys Trp Leu Gly His Phe Tyr Gly Gly Asp
410                 415                 420                 425

tgc tac ctg ctg ctc tac acc tac ctc atc ggc gag aag cag cat tac     1347
Cys Tyr Leu Leu Leu Tyr Thr Tyr Leu Ile Gly Glu Lys Gln His Tyr
                430                 435                 440

ctg ctc tac gtt tgg cag ggc agc cag gcc agc caa gat gaa att aca     1395
Leu Leu Tyr Val Trp Gln Gly Ser Gln Ala Ser Gln Asp Glu Ile Thr
            445                 450                 455

gca tca gct tat caa gcc gtc atc ctg gac cag aag tac aat ggt gaa     1443
Ala Ser Ala Tyr Gln Ala Val Ile Leu Asp Gln Lys Tyr Asn Gly Glu
        460                 465                 470

cca gtc cag atc cgg gtc cca atg ggc aag gag cca cct cat ctt atg     1491
Pro Val Gln Ile Arg Val Pro Met Gly Lys Glu Pro Pro His Leu Met
    475                 480                 485

tcc atc ttc aag gga cgc atg gtg gtc tac cag gga ggc acc tcc cga     1539
Ser Ile Phe Lys Gly Arg Met Val Val Tyr Gln Gly Gly Thr Ser Arg
490                 495                 500                 505

act aac aac ttg gag acc ggg ccc tcc aca cgg ctg ttc cag gtc cag     1587
Thr Asn Asn Leu Glu Thr Gly Pro Ser Thr Arg Leu Phe Gln Val Gln
                510                 515                 520

gga act ggc gcc aac aac acc aag gcc ttt gag gtc cca gcg cgg gcc     1635
Gly Thr Gly Ala Asn Asn Thr Lys Ala Phe Glu Val Pro Ala Arg Ala
            525                 530                 535

aat ttc ctc aat tcc aat gat gtc ttt gtc ctc aag acc cag tct tgc     1683
Asn Phe Leu Asn Ser Asn Asp Val Phe Val Leu Lys Thr Gln Ser Cys
        540                 545                 550

tgc tat cta tgg tgt ggg aag ggt tgt agc ggg gac gag cgg gag atg     1731
Cys Tyr Leu Trp Cys Gly Lys Gly Cys Ser Gly Asp Glu Arg Glu Met
    555                 560                 565

gcc aag atg gtt gct gac acc atc tcc cgg acg gag aag caa gtg gtg 1779
Ala Lys Met Val Ala Asp Thr Ile Ser Arg Thr Glu Lys Gln Val Val
570                 575                 580                 585

gtg gaa ggg cag gag cca gcc aac ttc tgg atg gcc ctg ggt ggg aag 1827
Val Glu Gly Gln Glu Pro Ala Asn Phe Trp Met Ala Leu Gly Gly Lys
                590                 595                 600

gcc ccc tat gcc aac acc aag aga cta cag gaa gaa aac ctg gtc atc 1875
Ala Pro Tyr Ala Asn Thr Lys Arg Leu Gln Glu Glu Asn Leu Val Ile
            605                 610                 615

acc ccc cgg ctc ttt gag tgt tcc aac aag act ggg cgc ttc ctg gcc 1923
Thr Pro Arg Leu Phe Glu Cys Ser Asn Lys Thr Gly Arg Phe Leu Ala
        620                 625                 630

aca gag atc cct gac ttc aat cag gat gac ttg gaa gag gat gat gtg 1971
Thr Glu Ile Pro Asp Phe Asn Gln Asp Asp Leu Glu Glu Asp Asp Val
    635                 640                 645

ttc cta cta gat gtc tgg gac cag gtc ttc ttc tgg att ggg aaa cat 2019
Phe Leu Leu Asp Val Trp Asp Gln Val Phe Phe Trp Ile Gly Lys His
650                 655                 660                 665

gcc aac gag gag gag aag aag gcc gca gca acc act gca cag gaa tac 2067
Ala Asn Glu Glu Glu Lys Lys Ala Ala Ala Thr Thr Ala Gln Glu Tyr
                670                 675                 680

ctc aag acc cat ccc agc ggg cgt gac cct gag acc ccc atc att gtg 2115
Leu Lys Thr His Pro Ser Gly Arg Asp Pro Glu Thr Pro Ile Ile Val
            685                 690                 695

gtg aag cag gga cac gag ccc ccc acc ttc aca ggc tgg ttc ctg gct 2163
Val Lys Gln Gly His Glu Pro Pro Thr Phe Thr Gly Trp Phe Leu Ala
        700                 705                 710

tgg gat ccc ttc aag tgg agt aac acc aaa tcc tat gag gac ctg aag 2211
Trp Asp Pro Phe Lys Trp Ser Asn Thr Lys Ser Tyr Glu Asp Leu Lys
    715                 720                 725

gcg gag tct ggc aac ctt agg gac tgg agc cag atc act gct gag gtc 2259
Ala Glu Ser Gly Asn Leu Arg Asp Trp Ser Gln Ile Thr Ala Glu Val
730                 735                 740                 745

aca agc ccc aaa gtg gac gtg ttc aat gct aac agc aac ctc agt tct 2307
Thr Ser Pro Lys Val Asp Val Phe Asn Ala Asn Ser Asn Leu Ser Ser
                750                 755                 760

ggg cct ctg ccc atc ttc ccc ctg gag cag cta gtg aac aag cct gta 2355
Gly Pro Leu Pro Ile Phe Pro Leu Glu Gln Leu Val Asn Lys Pro Val
            765                 770                 775

gag gag ctc ccc gag ggt gtg gac ccc agc agg aag gag gaa cac ctg 2403
Glu Glu Leu Pro Glu Gly Val Asp Pro Ser Arg Lys Glu Glu His Leu
        780                 785                 790

tcc att gaa gat ttc act cag gcc ttt ggg atg act cca gct gcc ttc 2451
Ser Ile Glu Asp Phe Thr Gln Ala Phe Gly Met Thr Pro Ala Ala Phe
    795                 800                 805

tct gct ctg cct cga tgg aag caa caa aac ctc aag aaa gaa aaa gga 2499
Ser Ala Leu Pro Arg Trp Lys Gln Gln Asn Leu Lys Lys Glu Lys Gly
810                 815                 820                 825

cta ttt tga gaagagtagc tgtggttgta aagcagtacc ctaccctgat 2548
Leu Phe

tgtagggtct cattttctca ccgatattag tcctacacca attgaagtga aattttgcag 2608

atgtgcctat gagcacaaac ttctgtggca aatgccagtt ttgtttaata atgtacctat 2668

tccttcagaa agatgatacc ccaaaaaaaa aaaa 2702

<210> 14
<211> 827
<212> PRT
<213> Homo sapiens

<400> 14

Met Thr Lys Leu Ser Ala Gln Val Lys Gly Ser Leu Asn Ile Thr Thr
1               5                   10                  15

Pro Gly Leu Gln Ile Trp Arg Ile Glu Ala Met Gln Met Val Pro Val
            20                  25                  30

Pro Ser Ser Thr Phe Gly Ser Phe Phe Asp Gly Asp Cys Tyr Ile Ile
        35                  40                  45

Leu Ala Ile His Lys Thr Ala Ser Ser Leu Ser Tyr Asp Ile His Tyr
    50                  55                  60

Trp Ile Gly Gln Asp Ser Ser Leu Asp Glu Gln Gly Ala Ala Ala Ile
65                  70                  75                  80

Tyr Thr Thr Gln Met Asp Asp Phe Leu Lys Gly Arg Ala Val Gln His
                85                  90                  95

Arg Glu Val Gln Gly Asn Glu Ser Glu Ala Phe Arg Gly Tyr Phe Lys
            100                 105                 110

Gln Gly Leu Val Ile Arg Lys Gly Gly Val Ala Ser Gly Met Lys His
        115                 120                 125

Val Glu Thr Asn Ser Tyr Asp Val Gln Arg Leu Leu His Val Lys Gly
    130                 135                 140

Lys Arg Asn Val Val Ala Gly Glu Val Glu Met Ser Trp Lys Ser Phe
145                 150                 155                 160

Asn Arg Gly Asp Val Phe Leu Leu Asp Leu Gly Lys Leu Ile Ile Gln
                165                 170                 175

Trp Asn Gly Pro Glu Ser Thr Arg Met Glu Arg Leu Arg Gly Met Thr
            180                 185                 190

Leu Ala Lys Glu Ile Arg Asp Gln Glu Arg Gly Gly Arg Thr Tyr Val
        195                 200                 205

Gly Val Val Asp Gly Glu Asn Glu Leu Ala Ser Pro Lys Leu Met Glu

210                 215                 220

Val Met Asn His Val Leu Gly Lys Arg Arg Glu Leu Lys Ala Ala Val
225                 230                 235                 240

Pro Asp Thr Val Val Glu Pro Ala Leu Lys Ala Ala Leu Lys Leu Tyr
                245                 250                 255

His Val Ser Asp Ser Glu Gly Asn Leu Val Val Arg Glu Val Ala Thr
            260                 265                 270

Arg Pro Leu Thr Gln Asp Leu Leu Ser His Glu Asp Cys Tyr Ile Leu
        275                 280                 285

Asp Gln Gly Gly Leu Lys Ile Tyr Val Trp Lys Gly Lys Lys Ala Asn
    290                 295                 300

Glu Gln Glu Lys Lys Gly Ala Met Ser His Ala Leu Asn Phe Ile Lys
305                 310                 315                 320

Ala Lys Gln Tyr Pro Pro Ser Thr Gln Val Glu Val Gln Asn Asp Gly
                325                 330                 335

Ala Glu Ser Ala Val Phe Gln Gln Leu Phe Gln Lys Trp Thr Ala Ser
            340                 345                 350

Asn Arg Thr Ser Gly Leu Gly Lys Thr His Thr Val Gly Ser Val Ala
        355                 360                 365

Lys Val Glu Gln Val Lys Phe Asp Ala Thr Ser Met His Val Lys Pro
    370                 375                 380

Gln Val Ala Ala Gln Gln Lys Met Val Asp Asp Gly Ser Gly Glu Val
385                 390                 395                 400

Gln Val Trp Arg Ile Glu Asn Leu Glu Leu Val Pro Val Asp Ser Lys
                405                 410                 415

Trp Leu Gly His Phe Tyr Gly Gly Asp Cys Tyr Leu Leu Leu Tyr Thr
            420                 425                 430

Tyr Leu Ile Gly Glu Lys Gln His Tyr Leu Leu Tyr Val Trp Gln Gly
        435                 440                 445

Ser Gln Ala Ser Gln Asp Glu Ile Thr Ala Ser Ala Tyr Gln Ala Val
    450                 455                 460

Ile Leu Asp Gln Lys Tyr Asn Gly Glu Pro Val Gln Ile Arg Val Pro
465                 470                 475                 480

Met Gly Lys Glu Pro Pro His Leu Met Ser Ile Phe Lys Gly Arg Met

                485                 490                 495

Val Val Tyr Gln Gly Gly Thr Ser Arg Thr Asn Asn Leu Glu Thr Gly
            500                 505                 510

Pro Ser Thr Arg Leu Phe Gln Val Gln Gly Thr Gly Ala Asn Asn Thr
        515                 520                 525

Lys Ala Phe Glu Val Pro Ala Arg Ala Asn Phe Leu Asn Ser Asn Asp
    530                 535                 540

Val Phe Val Leu Lys Thr Gln Ser Cys Cys Tyr Leu Trp Cys Gly Lys
545                 550                 555                 560

Gly Cys Ser Gly Asp Glu Arg Glu Met Ala Lys Met Val Ala Asp Thr
                565                 570                 575

Ile Ser Arg Thr Glu Lys Gln Val Val Val Glu Gly Gln Glu Pro Ala
            580                 585                 590

Asn Phe Trp Met Ala Leu Gly Gly Lys Ala Pro Tyr Ala Asn Thr Lys
        595                 600                 605

Arg Leu Gln Glu Glu Asn Leu Val Ile Thr Pro Arg Leu Phe Glu Cys
    610                 615                 620

Ser Asn Lys Thr Gly Arg Phe Leu Ala Thr Glu Ile Pro Asp Phe Asn
625                 630                 635                 640

Gln Asp Asp Leu Glu Glu Asp Asp Val Phe Leu Leu Asp Val Trp Asp
                645                 650                 655

Gln Val Phe Phe Trp Ile Gly Lys His Ala Asn Glu Glu Glu Lys Lys
            660                 665                 670

Ala Ala Ala Thr Thr Ala Gln Glu Tyr Leu Lys Thr His Pro Ser Gly
        675                 680                 685

Arg Asp Pro Glu Thr Pro Ile Ile Val Val Lys Gln Gly His Glu Pro
    690                 695                 700

Pro Thr Phe Thr Gly Trp Phe Leu Ala Trp Asp Pro Phe Lys Trp Ser
705                 710                 715                 720

Asn Thr Lys Ser Tyr Glu Asp Leu Lys Ala Glu Ser Gly Asn Leu Arg
                725                 730                 735

Asp Trp Ser Gln Ile Thr Ala Glu Val Thr Ser Pro Lys Val Asp Val
            740                 745                 750

Phe Asn Ala Asn Ser Asn Leu Ser Ser Gly Pro Leu Pro Ile Phe Pro

755                 760                 765

Leu Glu Gln Leu Val Asn Lys Pro Val Glu Glu Leu Pro Glu Gly Val
    770                 775                 780

Asp Pro Ser Arg Lys Glu Glu His Leu Ser Ile Glu Asp Phe Thr Gln
785                 790                 795                 800

Ala Phe Gly Met Thr Pro Ala Ala Phe Ser Ala Leu Pro Arg Trp Lys
                805                 810                 815

Gln Gln Asn Leu Lys Lys Glu Lys Gly Leu Phe
            820                 825

<210>  15
<211>  2307
<212>  DNA
<213>  Homo sapiens

<220>
<221>  CDS
<222>  (123)..(2282)

<400>  15
agcgggggag gttcagtgtt gatggagtta ttgaagaaat gatggagtaa gagactcttt      60

tctaagcaac tcaagtttgc agtgattcag gcctacttct gaagagacag ccttttatct     120

ca atg aat gac aca gaa aaa cca gca gat act ccc tct gag gaa gag      167
   Met Asn Asp Thr Glu Lys Pro Ala Asp Thr Pro Ser Glu Glu Glu
   1               5                   10                  15

gac ttt ggt gat cca agg aca tat gac cca gat ttc aag ggg cct gtt      215
Asp Phe Gly Asp Pro Arg Thr Tyr Asp Pro Asp Phe Lys Gly Pro Val
                20                  25                  30

gcc aac agg agt tgt aca gat gtt ctg tgc tgt atg atc ttc cta ctg      263
Ala Asn Arg Ser Cys Thr Asp Val Leu Cys Cys Met Ile Phe Leu Leu
            35                  40                  45

tgt att att ggc tac att gtt tta gga ctt gtg gcc tgg gta cat ggg      311
Cys Ile Ile Gly Tyr Ile Val Leu Gly Leu Val Ala Trp Val His Gly
        50                  55                  60

gac ccc aga aga gca gcc tat cct aca gac agc cag ggc cac ttt tgt      359
Asp Pro Arg Arg Ala Ala Tyr Pro Thr Asp Ser Gln Gly His Phe Cys
    65                  70                  75

ggc cag aag ggc act ccc aat gag aac aag acc att ttg ttt tac ttt      407
Gly Gln Lys Gly Thr Pro Asn Glu Asn Lys Thr Ile Leu Phe Tyr Phe
80                  85                  90                  95

aac ctg tta cgc tgt acc agt ccc tcc gta ttg cta aac cta cag tgc      455
Asn Leu Leu Arg Cys Thr Ser Pro Ser Val Leu Leu Asn Leu Gln Cys
                100                 105                 110

cct acc aca cag atc tgt gtc tcc aag tgc cca gaa aaa ttt tta acc      503
Pro Thr Thr Gln Ile Cys Val Ser Lys Cys Pro Glu Lys Phe Leu Thr
            115                 120                 125

tat gtg gaa atg caa ctt ttg tac aca aaa gac aaa agc tac tgg gaa      551
Tyr Val Glu Met Gln Leu Leu Tyr Thr Lys Asp Lys Ser Tyr Trp Glu

        130                 135                 140

gac tac cgt cag ttc tgt aag acc act gct aag cct gtg aag tct ctc 599
Asp Tyr Arg Gln Phe Cys Lys Thr Thr Ala Lys Pro Val Lys Ser Leu
    145                 150                 155

aca cag ctt tta ctg gat gat gat tgt cca aca gcg att ttt ccc agc 647
Thr Gln Leu Leu Leu Asp Asp Asp Cys Pro Thr Ala Ile Phe Pro Ser
160                 165                 170                 175

aaa cct ttt ctc cag aga tgt ttc cct gac ttc tct acc aaa aat ggc 695
Lys Pro Phe Leu Gln Arg Cys Phe Pro Asp Phe Ser Thr Lys Asn Gly
                180                 185                 190

act tta aca ata gga agt aag atg atg ttt caa gat gga aat gga ggg 743
Thr Leu Thr Ile Gly Ser Lys Met Met Phe Gln Asp Gly Asn Gly Gly
            195                 200                 205

aca aga agt gtt gta gaa ctc ggg att gct gca aat ggt atc aat aaa 791
Thr Arg Ser Val Val Glu Leu Gly Ile Ala Ala Asn Gly Ile Asn Lys
        210                 215                 220

ctt ctt gat gca aag tca ctt gga ttg aaa gtg ttt gaa gac tat gca 839
Leu Leu Asp Ala Lys Ser Leu Gly Leu Lys Val Phe Glu Asp Tyr Ala
    225                 230                 235

aga act tgg tat tgg att ctc att ggc ctg acg att gcc atg gtc ctt 887
Arg Thr Trp Tyr Trp Ile Leu Ile Gly Leu Thr Ile Ala Met Val Leu
240                 245                 250                 255

agt tgg ata ttt ttg ata ctt ctg agg ttc ata gct gga tgc ctc ttc 935
Ser Trp Ile Phe Leu Ile Leu Leu Arg Phe Ile Ala Gly Cys Leu Phe
                260                 265                 270

tgg gtc ttc atg att ggt gtg att gga att ata ggt tat gga ata tgg 983
Trp Val Phe Met Ile Gly Val Ile Gly Ile Ile Gly Tyr Gly Ile Trp
            275                 280                 285

cac tgt tac cag cag tac acc aat ctt cag gaa cgc cca agt tct gta 1031
His Cys Tyr Gln Gln Tyr Thr Asn Leu Gln Glu Arg Pro Ser Ser Val
        290                 295                 300

tta act atc tat gac atc ggg att cag act aac ata agc atg tac ttt 1079
Leu Thr Ile Tyr Asp Ile Gly Ile Gln Thr Asn Ile Ser Met Tyr Phe
    305                 310                 315

gaa ctg caa caa aca tgg ttc aca ttt atg ata ata ctc tgc atc att 1127
Glu Leu Gln Gln Thr Trp Phe Thr Phe Met Ile Ile Leu Cys Ile Ile
320                 325                 330                 335

gaa gtg att gtc atc ctc atg ctg atc ttc ctc agg aat cga atc cga 1175
Glu Val Ile Val Ile Leu Met Leu Ile Phe Leu Arg Asn Arg Ile Arg
                340                 345                 350

gtc gcc att atc ctg ctg aag gaa gga agc aaa gcc att gga tat gtt 1223
Val Ala Ile Ile Leu Leu Lys Glu Gly Ser Lys Ala Ile Gly Tyr Val
            355                 360                 365

cct agt aca tta gtc tat cca gct tta act ttc att ttg ctc tca atc 1271
Pro Ser Thr Leu Val Tyr Pro Ala Leu Thr Phe Ile Leu Leu Ser Ile
        370                 375                 380

tgc att tgc tac tgg gtc gtg aca gca gtt ttc ttg gcg aca tcg ggg 1319
Cys Ile Cys Tyr Trp Val Val Thr Ala Val Phe Leu Ala Thr Ser Gly
    385                 390                 395

gta cct gta tac aaa gtc ata gct cca ggg ggg cat tgt ata cat gaa 1367
Val Pro Val Tyr Lys Val Ile Ala Pro Gly Gly His Cys Ile His Glu

400                 405                 410                 415

aat caa acc tgt gac cca gag att ttt aat aca act gaa att gcc aaa      1415
Asn Gln Thr Cys Asp Pro Glu Ile Phe Asn Thr Thr Glu Ile Ala Lys
                420                 425                 430

gct tgc cct ggg gct ctg tgt aac ttt gct ttc tat ggt gga aag agc      1463
Ala Cys Pro Gly Ala Leu Cys Asn Phe Ala Phe Tyr Gly Gly Lys Ser
            435                 440                 445

ttg tac cat cag tac atc cct acc ttc cat gta tac aac tta ttt gtc      1511
Leu Tyr His Gln Tyr Ile Pro Thr Phe His Val Tyr Asn Leu Phe Val
        450                 455                 460

ttt ctc tgg ctt ata aac ttc gtc att gca tta ggt cag tgc gcc ctt      1559
Phe Leu Trp Leu Ile Asn Phe Val Ile Ala Leu Gly Gln Cys Ala Leu
    465                 470                 475

gct ggt gca ttc gct act tat tac tgg gcc atg aaa aaa cct gat gac      1607
Ala Gly Ala Phe Ala Thr Tyr Tyr Trp Ala Met Lys Lys Pro Asp Asp
480                 485                 490                 495

atc cca cga tat cca ctt ttt act gca ttt gga cga gcc ata cga tat      1655
Ile Pro Arg Tyr Pro Leu Phe Thr Ala Phe Gly Arg Ala Ile Arg Tyr
                500                 505                 510

cac aca gga tcc cta gca ttt gga tct tta att att gca tta att caa      1703
His Thr Gly Ser Leu Ala Phe Gly Ser Leu Ile Ile Ala Leu Ile Gln
            515                 520                 525

atg ttt aaa att gta cta gaa tac ttg gac cac cgt ctt aaa cgt acc      1751
Met Phe Lys Ile Val Leu Glu Tyr Leu Asp His Arg Leu Lys Arg Thr
        530                 535                 540

cag aac aca ttg tct aaa ttc cta cag tgc tgc ctg aga tgc tgc ttc      1799
Gln Asn Thr Leu Ser Lys Phe Leu Gln Cys Cys Leu Arg Cys Cys Phe
    545                 550                 555

tgg tgt ttg gaa aat gca ata aag ttt tta aac aga aat gcc tat att      1847
Trp Cys Leu Glu Asn Ala Ile Lys Phe Leu Asn Arg Asn Ala Tyr Ile
560                 565                 570                 575

atg att gca ata tat ggc aga aac ttc tgc agg tca gca aaa gat gct      1895
Met Ile Ala Ile Tyr Gly Arg Asn Phe Cys Arg Ser Ala Lys Asp Ala
                580                 585                 590

ttc aat ctg ctg atg aga aat gtt ttg aaa gtt gca gtt aca gat gaa      1943
Phe Asn Leu Leu Met Arg Asn Val Leu Lys Val Ala Val Thr Asp Glu
            595                 600                 605

gtt aca tac ttt gta tta ttc ctg ggg aaa ctt cta gtt gct gga agt      1991
Val Thr Tyr Phe Val Leu Phe Leu Gly Lys Leu Leu Val Ala Gly Ser
        610                 615                 620

ata ggt gtt ctg gcc ttc cta ttc ttc aca caa aga ctg cca gtg att      2039
Ile Gly Val Leu Ala Phe Leu Phe Phe Thr Gln Arg Leu Pro Val Ile
    625                 630                 635

gca caa gga cca gca tct tta tat tac tac tgg gta cct ttg ctg aca      2087
Ala Gln Gly Pro Ala Ser Leu Tyr Tyr Tyr Trp Val Pro Leu Leu Thr
640                 645                 650                 655

gtc att ttt ggg tct tac ctg att gca cat ggg ttc ttc agc gtc tat      2135
Val Ile Phe Gly Ser Tyr Leu Ile Ala His Gly Phe Phe Ser Val Tyr
                660                 665                 670

gca atg tgt gtt gaa aca att ttc atc tgc ttc tgt gaa gat ctg gaa      2183
Ala Met Cys Val Glu Thr Ile Phe Ile Cys Phe Cys Glu Asp Leu Glu

675                    680                    685

aga aat gat gga tcc aca gaa aaa ccc tac ttc gta acc cct aac ctg      2231
Arg Asn Asp Gly Ser Thr Glu Lys Pro Tyr Phe Val Thr Pro Asn Leu
        690                 695                 700

cat gga att ctg atc aag aag caa cta gtt ccc cag aag cag aaa gag      2279
His Gly Ile Leu Ile Lys Lys Gln Leu Val Pro Gln Lys Gln Lys Glu
    705                 710                 715

tag aaaagctcca aaaaaaaaaa aaaaa                                      2307

<210>  16
<211>  719
<212>  PRT
<213>  Homo sapiens

<400>  16

Met Asn Asp Thr Glu Lys Pro Ala Asp Thr Pro Ser Glu Glu Glu Asp
1               5                   10                  15

Phe Gly Asp Pro Arg Thr Tyr Asp Pro Asp Phe Lys Gly Pro Val Ala
            20                  25                  30

Asn Arg Ser Cys Thr Asp Val Leu Cys Cys Met Ile Phe Leu Leu Cys
        35                  40                  45

Ile Ile Gly Tyr Ile Val Leu Gly Leu Val Ala Trp Val His Gly Asp
    50                  55                  60

Pro Arg Arg Ala Ala Tyr Pro Thr Asp Ser Gln Gly His Phe Cys Gly
65                  70                  75                  80

Gln Lys Gly Thr Pro Asn Glu Asn Lys Thr Ile Leu Phe Tyr Phe Asn
                85                  90                  95

Leu Leu Arg Cys Thr Ser Pro Ser Val Leu Leu Asn Leu Gln Cys Pro
            100                 105                 110

Thr Thr Gln Ile Cys Val Ser Lys Cys Pro Glu Lys Phe Leu Thr Tyr
        115                 120                 125

Val Glu Met Gln Leu Leu Tyr Thr Lys Asp Lys Ser Tyr Trp Glu Asp
    130                 135                 140

Tyr Arg Gln Phe Cys Lys Thr Thr Ala Lys Pro Val Lys Ser Leu Thr
145                 150                 155                 160

Gln Leu Leu Leu Asp Asp Asp Cys Pro Thr Ala Ile Phe Pro Ser Lys
                165                 170                 175

Pro Phe Leu Gln Arg Cys Phe Pro Asp Phe Ser Thr Lys Asn Gly Thr
            180                 185                 190

Leu Thr Ile Gly Ser Lys Met Met Phe Gln Asp Gly Asn Gly Gly Thr
        195                 200                 205

Arg Ser Val Val Glu Leu Gly Ile Ala Ala Asn Gly Ile Asn Lys Leu
    210                 215                 220

Leu Asp Ala Lys Ser Leu Gly Leu Lys Val Phe Glu Asp Tyr Ala Arg
225                 230                 235                 240

Thr Trp Tyr Trp Ile Leu Ile Gly Leu Thr Ile Ala Met Val Leu Ser
                245                 250                 255

Trp Ile Phe Leu Ile Leu Leu Arg Phe Ile Ala Gly Cys Leu Phe Trp
            260                 265                 270

Val Phe Met Ile Gly Val Ile Gly Ile Ile Gly Tyr Gly Ile Trp His
        275                 280                 285

Cys Tyr Gln Gln Tyr Thr Asn Leu Gln Glu Arg Pro Ser Ser Val Leu
    290                 295                 300

Thr Ile Tyr Asp Ile Gly Ile Gln Thr Asn Ile Ser Met Tyr Phe Glu
305                 310                 315                 320

Leu Gln Gln Thr Trp Phe Thr Phe Met Ile Ile Leu Cys Ile Ile Glu
                325                 330                 335

Val Ile Val Ile Leu Met Leu Ile Phe Leu Arg Asn Arg Ile Arg Val
            340                 345                 350

Ala Ile Ile Leu Leu Lys Glu Gly Ser Lys Ala Ile Gly Tyr Val Pro
        355                 360                 365

Ser Thr Leu Val Tyr Pro Ala Leu Thr Phe Ile Leu Leu Ser Ile Cys
    370                 375                 380

Ile Cys Tyr Trp Val Val Thr Ala Val Phe Leu Ala Thr Ser Gly Val
385                 390                 395                 400

Pro Val Tyr Lys Val Ile Ala Pro Gly Gly His Cys Ile His Glu Asn
                405                 410                 415

Gln Thr Cys Asp Pro Glu Ile Phe Asn Thr Thr Glu Ile Ala Lys Ala
            420                 425                 430

Cys Pro Gly Ala Leu Cys Asn Phe Ala Phe Tyr Gly Gly Lys Ser Leu
        435                 440                 445

Tyr His Gln Tyr Ile Pro Thr Phe His Val Tyr Asn Leu Phe Val Phe
    450                 455                 460

Leu Trp Leu Ile Asn Phe Val Ile Ala Leu Gly Gln Cys Ala Leu Ala
465                 470                 475                 480

Gly Ala Phe Ala Thr Tyr Tyr Trp Ala Met Lys Lys Pro Asp Asp Ile
                485                 490                 495

Pro Arg Tyr Pro Leu Phe Thr Ala Phe Gly Arg Ala Ile Arg Tyr His
            500                 505                 510

Thr Gly Ser Leu Ala Phe Gly Ser Leu Ile Ile Ala Leu Ile Gln Met
        515                 520                 525

Phe Lys Ile Val Leu Glu Tyr Leu Asp His Arg Leu Lys Arg Thr Gln
    530                 535                 540

Asn Thr Leu Ser Lys Phe Leu Gln Cys Cys Leu Arg Cys Cys Phe Trp
545                 550                 555                 560

Cys Leu Glu Asn Ala Ile Lys Phe Leu Asn Arg Asn Ala Tyr Ile Met
                565                 570                 575

Ile Ala Ile Tyr Gly Arg Asn Phe Cys Arg Ser Ala Lys Asp Ala Phe
            580                 585                 590

Asn Leu Leu Met Arg Asn Val Leu Lys Val Ala Val Thr Asp Glu Val
        595                 600                 605

Thr Tyr Phe Val Leu Phe Leu Gly Lys Leu Leu Val Ala Gly Ser Ile
    610                 615                 620

Gly Val Leu Ala Phe Leu Phe Phe Thr Gln Arg Leu Pro Val Ile Ala
625                 630                 635                 640

Gln Gly Pro Ala Ser Leu Tyr Tyr Tyr Trp Val Pro Leu Leu Thr Val
                645                 650                 655

Ile Phe Gly Ser Tyr Leu Ile Ala His Gly Phe Phe Ser Val Tyr Ala
            660                 665                 670

Met Cys Val Glu Thr Ile Phe Ile Cys Phe Cys Glu Asp Leu Glu Arg
        675                 680                 685

Asn Asp Gly Ser Thr Glu Lys Pro Tyr Phe Val Thr Pro Asn Leu His
    690                 695                 700

Gly Ile Leu Ile Lys Lys Gln Leu Val Pro Gln Lys Gln Lys Glu
705                 710                 715

<210>  17
<211>  3696
<212>  DNA
<213>  Homo sapiens

<220>
<221> CDS
<222> (41)..(3100)

<400> 17
accagacagt tcttaaatta gcaagccttc aaaaccaaaa atg ggg tcg aaa aga 55
Met Gly Ser Lys Arg
1               5

ggc ata tct tct agg cat cat tct ctc agc tcc tat gaa atc atg ttt 103
Gly Ile Ser Ser Arg His His Ser Leu Ser Ser Tyr Glu Ile Met Phe
                10                  15                  20

gca gct ctc ttt gcc ata ttg gta gtg ctc tgt gct gga tta att gca 151
Ala Ala Leu Phe Ala Ile Leu Val Val Leu Cys Ala Gly Leu Ile Ala
            25                  30                  35

gta tcc tgc ctg aca atc aag gaa tcc caa cga ggt gca gca ctt gga 199
Val Ser Cys Leu Thr Ile Lys Glu Ser Gln Arg Gly Ala Ala Leu Gly
        40                  45                  50

cag agt cat gaa gcc aga gcg aca ttt aaa ata aca tcc gga gtt aca 247
Gln Ser His Glu Ala Arg Ala Thr Phe Lys Ile Thr Ser Gly Val Thr
    55                  60                  65

tat aat cct aat ttg caa gac aaa ctc tca gtg gat ttc aaa gtt ctt 295
Tyr Asn Pro Asn Leu Gln Asp Lys Leu Ser Val Asp Phe Lys Val Leu
70                  75                  80                  85

gct ttt gac ctt cag caa atg ata gat gag atc ttt cta tca agc aat 343
Ala Phe Asp Leu Gln Gln Met Ile Asp Glu Ile Phe Leu Ser Ser Asn
                90                  95                  100

ctg aag aat gaa tat aag aac tca aga gtt tta caa ttt gaa aat ggc 391
Leu Lys Asn Glu Tyr Lys Asn Ser Arg Val Leu Gln Phe Glu Asn Gly
            105                 110                 115

agc att ata gtc gta ttt gac ctt ttc ttt gcc cag tgg gtg tca gat 439
Ser Ile Ile Val Val Phe Asp Leu Phe Phe Ala Gln Trp Val Ser Asp
        120                 125                 130

caa aat gta aaa gaa gaa ctg att caa ggc ctt gaa gca aat aaa tcc 487
Gln Asn Val Lys Glu Glu Leu Ile Gln Gly Leu Glu Ala Asn Lys Ser
    135                 140                 145

agc caa ctg gtc act ttc cat att gat ttg aac agc gtt gat atc cta 535
Ser Gln Leu Val Thr Phe His Ile Asp Leu Asn Ser Val Asp Ile Leu
150                 155                 160                 165

gac aag cta aca acc acc agt cat ctg gca act cca gga aat gtc tca 583
Asp Lys Leu Thr Thr Thr Ser His Leu Ala Thr Pro Gly Asn Val Ser
                170                 175                 180

ata gag tgc ctg cct ggt tca agt cct tgt act gat gct cta acg tgt 631
Ile Glu Cys Leu Pro Gly Ser Ser Pro Cys Thr Asp Ala Leu Thr Cys
            185                 190                 195

ata aaa gct gat tta ttt tgt gat gga gaa gta aac tgt cca gat ggt 679
Ile Lys Ala Asp Leu Phe Cys Asp Gly Glu Val Asn Cys Pro Asp Gly
        200                 205                 210

tct gac gaa gac aat aaa atg tgt gcc aca gtt tgt gat gga aga ttt 727
Ser Asp Glu Asp Asn Lys Met Cys Ala Thr Val Cys Asp Gly Arg Phe
    215                 220                 225

ttg tta act gga tca tct ggg tct ttc cag gct act cat tat cca aaa 775

Leu Leu Thr Gly Ser Ser Gly Ser Phe Gln Ala Thr His Tyr Pro Lys
230                 235                 240                 245

cct tct gaa aca agt gtt gtc tgc cag tgg atc ata cgt gta aac caa     823
Pro Ser Glu Thr Ser Val Val Cys Gln Trp Ile Ile Arg Val Asn Gln
                250                 255                 260

gga ctt tcc att aaa ctg agc ttc gat gat ttt aat aca tat tat aca     871
Gly Leu Ser Ile Lys Leu Ser Phe Asp Asp Phe Asn Thr Tyr Tyr Thr
            265                 270                 275

gat ata tta gat att tat gaa ggt gta gga tca agc aag att tta aga     919
Asp Ile Leu Asp Ile Tyr Glu Gly Val Gly Ser Ser Lys Ile Leu Arg
        280                 285                 290

gct tct att tgg gaa act aat cct ggc aca ata aga att ttt tcc aac     967
Ala Ser Ile Trp Glu Thr Asn Pro Gly Thr Ile Arg Ile Phe Ser Asn
    295                 300                 305

caa gtt act gcc acc ttt ctt ata gaa tct gat gaa agt gat tat gtt     1015
Gln Val Thr Ala Thr Phe Leu Ile Glu Ser Asp Glu Ser Asp Tyr Val
310                 315                 320                 325

ggc ttt aat gca aca tat act gca ttt aac agc agt gag ctt aat aat     1063
Gly Phe Asn Ala Thr Tyr Thr Ala Phe Asn Ser Ser Glu Leu Asn Asn
                330                 335                 340

tat gag aaa att aat tgt aac ttt gag gat ggc ttt tgt ttc tgg gtc     1111
Tyr Glu Lys Ile Asn Cys Asn Phe Glu Asp Gly Phe Cys Phe Trp Val
            345                 350                 355

cag gat cta aat gat gat aat gaa tgg gaa agg att cag gga agc acc     1159
Gln Asp Leu Asn Asp Asp Asn Glu Trp Glu Arg Ile Gln Gly Ser Thr
        360                 365                 370

ttt tct cct ttt act gga ccc aat ttt gac cac act ttt ggc aat gct     1207
Phe Ser Pro Phe Thr Gly Pro Asn Phe Asp His Thr Phe Gly Asn Ala
    375                 380                 385

tca gga ttt tac att tct acc cca act gga cca gga ggg aga caa gaa     1255
Ser Gly Phe Tyr Ile Ser Thr Pro Thr Gly Pro Gly Gly Arg Gln Glu
390                 395                 400                 405

cga gtg ggg ctt tta agc ctc cct ttg gac ccc act ttg gag cca gct     1303
Arg Val Gly Leu Leu Ser Leu Pro Leu Asp Pro Thr Leu Glu Pro Ala
                410                 415                 420

tgc ctt agt ttc tgg tat cat atg tat ggt gaa aat gtc cat aaa tta     1351
Cys Leu Ser Phe Trp Tyr His Met Tyr Gly Glu Asn Val His Lys Leu
            425                 430                 435

agc att aat atc agc aat gac caa aat atg gag aag aca gtt ttc caa     1399
Ser Ile Asn Ile Ser Asn Asp Gln Asn Met Glu Lys Thr Val Phe Gln
        440                 445                 450

aag gaa gga aat tat gga gac aat tgg aat tat gga caa gta acc cta     1447
Lys Glu Gly Asn Tyr Gly Asp Asn Trp Asn Tyr Gly Gln Val Thr Leu
    455                 460                 465

aat gaa aca gtt aaa ttt aag gtt gct ttt aat gct ttt aaa aac aag     1495
Asn Glu Thr Val Lys Phe Lys Val Ala Phe Asn Ala Phe Lys Asn Lys
470                 475                 480                 485

atc ctg agt gat att gcg ttg gat gac att agc cta aca tat ggg att     1543
Ile Leu Ser Asp Ile Ala Leu Asp Asp Ile Ser Leu Thr Tyr Gly Ile
                490                 495                 500

tgc aat ggg agt ctt tat cca gaa cca act ttg gtg cca act cct cca     1591

Cys Asn Gly Ser Leu Tyr Pro Glu Pro Thr Leu Val Pro Thr Pro Pro
            505                 510                 515

cca gaa ctt cct acg gac tgt gga gga cct ttt gag ctg tgg gag cca 1639
Pro Glu Leu Pro Thr Asp Cys Gly Gly Pro Phe Glu Leu Trp Glu Pro
        520                 525                 530

aat aca aca ttc agt tct acg aac ttt cca aac agc tac cct aat ctg 1687
Asn Thr Thr Phe Ser Ser Thr Asn Phe Pro Asn Ser Tyr Pro Asn Leu
    535                 540                 545

gct ttc tgt gtt tgg att tta aat gca caa aaa gga aag aat ata caa 1735
Ala Phe Cys Val Trp Ile Leu Asn Ala Gln Lys Gly Lys Asn Ile Gln
550                 555                 560                 565

ctt cat ttt caa gaa ttt gac tta gaa aat att aac gat gta gtt gaa 1783
Leu His Phe Gln Glu Phe Asp Leu Glu Asn Ile Asn Asp Val Val Glu
                570                 575                 580

ata aga gat ggt gaa gaa gct gat tcc ttg ctc tta gct gtg tac aca 1831
Ile Arg Asp Gly Glu Glu Ala Asp Ser Leu Leu Leu Ala Val Tyr Thr
            585                 590                 595

ggg cct ggc cca gta aag gat gtg ttc tct acc acc aac aga atg act 1879
Gly Pro Gly Pro Val Lys Asp Val Phe Ser Thr Thr Asn Arg Met Thr
        600                 605                 610

gtg ctt ctc atc act aac gat gtg ttg gca aga gga ggg ttt aaa gca 1927
Val Leu Leu Ile Thr Asn Asp Val Leu Ala Arg Gly Gly Phe Lys Ala
    615                 620                 625

aac ttt act act ggc tat cac ttg ggg att cca gag cca tgc aag gca 1975
Asn Phe Thr Thr Gly Tyr His Leu Gly Ile Pro Glu Pro Cys Lys Ala
630                 635                 640                 645

gac cat ttt caa tgt aaa aat gga gag tgt gtt cca ctg gtg aat ctc 2023
Asp His Phe Gln Cys Lys Asn Gly Glu Cys Val Pro Leu Val Asn Leu
                650                 655                 660

tgt gac ggt cat ctg cac tgt gag gat ggc tca gat gaa gca gat tgt 2071
Cys Asp Gly His Leu His Cys Glu Asp Gly Ser Asp Glu Ala Asp Cys
            665                 670                 675

gtg cgt ttt ttc aat ggc aca acg aac aac aat ggt tta gtg cgg ttc 2119
Val Arg Phe Phe Asn Gly Thr Thr Asn Asn Asn Gly Leu Val Arg Phe
        680                 685                 690

aga atc cag agc ata tgg cat aca gct tgt gct gag aac tgg acc acc 2167
Arg Ile Gln Ser Ile Trp His Thr Ala Cys Ala Glu Asn Trp Thr Thr
    695                 700                 705

cag att tca aat gat gtt tgt caa ctg ctg gga cta ggg agt gga aac 2215
Gln Ile Ser Asn Asp Val Cys Gln Leu Leu Gly Leu Gly Ser Gly Asn
710                 715                 720                 725

tca tca aag cca atc ttc tct acc gat ggt gga cca ttt gtc aaa tta 2263
Ser Ser Lys Pro Ile Phe Ser Thr Asp Gly Gly Pro Phe Val Lys Leu
                730                 735                 740

aac aca gca cct gat ggc cac tta ata cta aca ccc agt caa cag tgt 2311
Asn Thr Ala Pro Asp Gly His Leu Ile Leu Thr Pro Ser Gln Gln Cys
            745                 750                 755

tta cag gat tcc ttg att cgg tta cag tgt aac cat aaa tct tgt gga 2359
Leu Gln Asp Ser Leu Ile Arg Leu Gln Cys Asn His Lys Ser Cys Gly
        760                 765                 770

aaa aaa ctg gca gct caa gac atc acc cca aag att gtt gga gga agt 2407

Lys Lys Leu Ala Ala Gln Asp Ile Thr Pro Lys Ile Val Gly Gly Ser
    775                 780                 785

aat gcc aaa gaa ggg gcc tgg ccc tgg gtt gtg ggt ctg tat tat ggc      2455
Asn Ala Lys Glu Gly Ala Trp Pro Trp Val Val Gly Leu Tyr Tyr Gly
790                 795                 800                 805

ggc cga ctg ctc tgc ggc gca tct ctc gtc agc agt gac tgg ctg gtg      2503
Gly Arg Leu Leu Cys Gly Ala Ser Leu Val Ser Ser Asp Trp Leu Val
                810                 815                 820

tcc gcc gca cac tgc gtg tat ggg aga aac tta gag cca tcc aag tgg      2551
Ser Ala Ala His Cys Val Tyr Gly Arg Asn Leu Glu Pro Ser Lys Trp
            825                 830                 835

aca gca atc cta ggc ctg cat atg aaa tca aat ctg acc tct cct caa      2599
Thr Ala Ile Leu Gly Leu His Met Lys Ser Asn Leu Thr Ser Pro Gln
        840                 845                 850

aca gtc cct cga tta ata gat gaa att gtc ata aac cct cat tac aat      2647
Thr Val Pro Arg Leu Ile Asp Glu Ile Val Ile Asn Pro His Tyr Asn
    855                 860                 865

agg cga aga aag gac aac gac att gcc atg atg cat ctg gaa ttt aaa      2695
Arg Arg Arg Lys Asp Asn Asp Ile Ala Met Met His Leu Glu Phe Lys
870                 875                 880                 885

gtg aat tac aca gat tac ata caa cct att tgt tta ccg gaa gaa aat      2743
Val Asn Tyr Thr Asp Tyr Ile Gln Pro Ile Cys Leu Pro Glu Glu Asn
                890                 895                 900

caa gtt ttt cct cca gga aga aat tgt tct att gct ggt tgg ggg acg      2791
Gln Val Phe Pro Pro Gly Arg Asn Cys Ser Ile Ala Gly Trp Gly Thr
            905                 910                 915

gtt gta tat caa ggt act act gca aac ata ttg caa gaa gct gat gtt      2839
Val Val Tyr Gln Gly Thr Thr Ala Asn Ile Leu Gln Glu Ala Asp Val
        920                 925                 930

cct ctt cta tca aat gag aga tgc caa cag cag atg cca gaa tat aac      2887
Pro Leu Leu Ser Asn Glu Arg Cys Gln Gln Gln Met Pro Glu Tyr Asn
    935                 940                 945

att act gaa aat atg ata tgt gca ggc tat gaa gaa gga gga ata gat      2935
Ile Thr Glu Asn Met Ile Cys Ala Gly Tyr Glu Glu Gly Gly Ile Asp
950                 955                 960                 965

tct tgt cag ggg gat tca gga gga cca tta atg tgc caa gaa aac aac      2983
Ser Cys Gln Gly Asp Ser Gly Gly Pro Leu Met Cys Gln Glu Asn Asn
                970                 975                 980

agg tgg ttc ctt gct ggt gtg acc tca ttt gga tac aag tgt gcc ctg      3031
Arg Trp Phe Leu Ala Gly Val Thr Ser Phe Gly Tyr Lys Cys Ala Leu
            985                 990                 995

cct aat cgc  ccc gga gtg tat gcc  agg gtc tca agg ttt  acc gaa      3076
Pro Asn Arg  Pro Gly Val Tyr Ala  Arg Val Ser Arg Phe  Thr Glu
        1000                1005                1010

tgg ata caa  agt ttt cta cat tag cgcatttctt aaactaaaca ggaaagtcgc      3130
Trp Ile Gln  Ser Phe Leu His
        1015

attattttcc cattctactc tagaaagcat ggaaattaag tgtttcgtac aaaaatttta      3190

aaaagttacc aaaggttttt attcttacct atgtcaatga aatgctaggg ggccagggaa      3250

acaaaatttt aaaaataata aaattcacca tagcaataca gaataacttt aaaataccat      3310

taaatacatt tgtatttcat tgtgaacagg tatttcttca cagatctcat ttttaaaatt 3370

cttaatgatt atttttatta cttactgttg tttaaaggga tgttatttta aagcatatac 3430

catacactta agaaatttga gcagaattta aaaaagaaag aaaataaatt gtttttccca 3490

aagtatgtca ctgttggaaa taaactgcca taaattttct agttccagtt tagtttgctg 3550

ctattagcag aaactcaatt gtttctctgt cttttctatc aaaattttca acatatgcat 3610

aaccttagta ttttcccaac caatagaaac tatttattgt aagcttatgt cacaggcctg 3670

gactaaattg attttacgtt cctctt 3696

<210> 18
<211> 1019
<212> PRT
<213> Homo sapiens

<400> 18

Met Gly Ser Lys Arg Gly Ile Ser Ser Arg His His Ser Leu Ser Ser
1               5                   10                  15

Tyr Glu Ile Met Phe Ala Ala Leu Phe Ala Ile Leu Val Val Leu Cys
            20                  25                  30

Ala Gly Leu Ile Ala Val Ser Cys Leu Thr Ile Lys Glu Ser Gln Arg
        35                  40                  45

Gly Ala Ala Leu Gly Gln Ser His Glu Ala Arg Ala Thr Phe Lys Ile
    50                  55                  60

Thr Ser Gly Val Thr Tyr Asn Pro Asn Leu Gln Asp Lys Leu Ser Val
65                  70                  75                  80

Asp Phe Lys Val Leu Ala Phe Asp Leu Gln Gln Met Ile Asp Glu Ile
                85                  90                  95

Phe Leu Ser Ser Asn Leu Lys Asn Glu Tyr Lys Asn Ser Arg Val Leu
            100                 105                 110

Gln Phe Glu Asn Gly Ser Ile Ile Val Val Phe Asp Leu Phe Phe Ala
        115                 120                 125

Gln Trp Val Ser Asp Gln Asn Val Lys Glu Glu Leu Ile Gln Gly Leu
    130                 135                 140

Glu Ala Asn Lys Ser Ser Gln Leu Val Thr Phe His Ile Asp Leu Asn
145                 150                 155                 160

Ser Val Asp Ile Leu Asp Lys Leu Thr Thr Thr Ser His Leu Ala Thr
                165                 170                 175

Pro Gly Asn Val Ser Ile Glu Cys Leu Pro Gly Ser Ser Pro Cys Thr

            180                 185                 190

Asp Ala Leu Thr Cys Ile Lys Ala Asp Leu Phe Cys Asp Gly Glu Val
        195                 200                 205

Asn Cys Pro Asp Gly Ser Asp Glu Asp Asn Lys Met Cys Ala Thr Val
    210                 215                 220

Cys Asp Gly Arg Phe Leu Leu Thr Gly Ser Ser Gly Ser Phe Gln Ala
225                 230                 235                 240

Thr His Tyr Pro Lys Pro Ser Glu Thr Ser Val Val Cys Gln Trp Ile
                245                 250                 255

Ile Arg Val Asn Gln Gly Leu Ser Ile Lys Leu Ser Phe Asp Asp Phe
            260                 265                 270

Asn Thr Tyr Tyr Thr Asp Ile Leu Asp Ile Tyr Glu Gly Val Gly Ser
        275                 280                 285

Ser Lys Ile Leu Arg Ala Ser Ile Trp Glu Thr Asn Pro Gly Thr Ile
    290                 295                 300

Arg Ile Phe Ser Asn Gln Val Thr Ala Thr Phe Leu Ile Glu Ser Asp
305                 310                 315                 320

Glu Ser Asp Tyr Val Gly Phe Asn Ala Thr Tyr Thr Ala Phe Asn Ser
                325                 330                 335

Ser Glu Leu Asn Asn Tyr Glu Lys Ile Asn Cys Asn Phe Glu Asp Gly
            340                 345                 350

Phe Cys Phe Trp Val Gln Asp Leu Asn Asp Asp Asn Glu Trp Glu Arg
        355                 360                 365

Ile Gln Gly Ser Thr Phe Ser Pro Phe Thr Gly Pro Asn Phe Asp His
    370                 375                 380

Thr Phe Gly Asn Ala Ser Gly Phe Tyr Ile Ser Thr Pro Thr Gly Pro
385                 390                 395                 400

Gly Gly Arg Gln Glu Arg Val Gly Leu Leu Ser Leu Pro Leu Asp Pro
                405                 410                 415

Thr Leu Glu Pro Ala Cys Leu Ser Phe Trp Tyr His Met Tyr Gly Glu
            420                 425                 430

Asn Val His Lys Leu Ser Ile Asn Ile Ser Asn Asp Gln Asn Met Glu
        435                 440                 445

Lys Thr Val Phe Gln Lys Glu Gly Asn Tyr Gly Asp Asn Trp Asn Tyr

450                 455                 460

Gly Gln Val Thr Leu Asn Glu Thr Val Lys Phe Lys Val Ala Phe Asn
465                 470                 475                 480

Ala Phe Lys Asn Lys Ile Leu Ser Asp Ile Ala Leu Asp Asp Ile Ser
                485                 490                 495

Leu Thr Tyr Gly Ile Cys Asn Gly Ser Leu Tyr Pro Glu Pro Thr Leu
            500                 505                 510

Val Pro Thr Pro Pro Pro Glu Leu Pro Thr Asp Cys Gly Gly Pro Phe
        515                 520                 525

Glu Leu Trp Glu Pro Asn Thr Thr Phe Ser Ser Thr Asn Phe Pro Asn
    530                 535                 540

Ser Tyr Pro Asn Leu Ala Phe Cys Val Trp Ile Leu Asn Ala Gln Lys
545                 550                 555                 560

Gly Lys Asn Ile Gln Leu His Phe Gln Glu Phe Asp Leu Glu Asn Ile
                565                 570                 575

Asn Asp Val Val Glu Ile Arg Asp Gly Glu Glu Ala Asp Ser Leu Leu
            580                 585                 590

Leu Ala Val Tyr Thr Gly Pro Gly Pro Val Lys Asp Val Phe Ser Thr
        595                 600                 605

Thr Asn Arg Met Thr Val Leu Leu Ile Thr Asn Asp Val Leu Ala Arg
    610                 615                 620

Gly Gly Phe Lys Ala Asn Phe Thr Thr Gly Tyr His Leu Gly Ile Pro
625                 630                 635                 640

Glu Pro Cys Lys Ala Asp His Phe Gln Cys Lys Asn Gly Glu Cys Val
                645                 650                 655

Pro Leu Val Asn Leu Cys Asp Gly His Leu His Cys Glu Asp Gly Ser
            660                 665                 670

Asp Glu Ala Asp Cys Val Arg Phe Phe Asn Gly Thr Thr Asn Asn Asn
        675                 680                 685

Gly Leu Val Arg Phe Arg Ile Gln Ser Ile Trp His Thr Ala Cys Ala
    690                 695                 700

Glu Asn Trp Thr Thr Gln Ile Ser Asn Asp Val Cys Gln Leu Leu Gly
705                 710                 715                 720

Leu Gly Ser Gly Asn Ser Ser Lys Pro Ile Phe Ser Thr Asp Gly Gly

725                 730                 735

Pro Phe Val Lys Leu Asn Thr Ala Pro Asp Gly His Leu Ile Leu Thr
            740                 745                 750

Pro Ser Gln Gln Cys Leu Gln Asp Ser Leu Ile Arg Leu Gln Cys Asn
        755                 760                 765

His Lys Ser Cys Gly Lys Lys Leu Ala Ala Gln Asp Ile Thr Pro Lys
    770                 775                 780

Ile Val Gly Gly Ser Asn Ala Lys Glu Gly Ala Trp Pro Trp Val Val
785                 790                 795                 800

Gly Leu Tyr Tyr Gly Gly Arg Leu Leu Cys Gly Ala Ser Leu Val Ser
                805                 810                 815

Ser Asp Trp Leu Val Ser Ala Ala His Cys Val Tyr Gly Arg Asn Leu
            820                 825                 830

Glu Pro Ser Lys Trp Thr Ala Ile Leu Gly Leu His Met Lys Ser Asn
        835                 840                 845

Leu Thr Ser Pro Gln Thr Val Pro Arg Leu Ile Asp Glu Ile Val Ile
    850                 855                 860

Asn Pro His Tyr Asn Arg Arg Arg Lys Asp Asn Asp Ile Ala Met Met
865                 870                 875                 880

His Leu Glu Phe Lys Val Asn Tyr Thr Asp Tyr Ile Gln Pro Ile Cys
                885                 890                 895

Leu Pro Glu Glu Asn Gln Val Phe Pro Pro Gly Arg Asn Cys Ser Ile
            900                 905                 910

Ala Gly Trp Gly Thr Val Val Tyr Gln Gly Thr Thr Ala Asn Ile Leu
        915                 920                 925

Gln Glu Ala Asp Val Pro Leu Leu Ser Asn Glu Arg Cys Gln Gln Gln
    930                 935                 940

Met Pro Glu Tyr Asn Ile Thr Glu Asn Met Ile Cys Ala Gly Tyr Glu
945                 950                 955                 960

Glu Gly Gly Ile Asp Ser Cys Gln Gly Asp Ser Gly Gly Pro Leu Met
                965                 970                 975

Cys Gln Glu Asn Asn Arg Trp Phe Leu Ala Gly Val Thr Ser Phe Gly
            980                 985                 990

Tyr Lys Cys Ala Leu Pro Asn Arg  Pro Gly Val Tyr Ala  Arg Val Ser

995                 1000                1005

Arg Phe Thr Glu Trp Ile Gln Ser Phe Leu His
    1010                1015

<210>  19
<211>  3669
<212>  DNA
<213>  Homo sapiens

<220>
<221>  CDS
<222>  (109)..(2940)

<400>  19
acctaaaacc ttgcaagttc aggaagaaac catctgcatc catattgaaa acctgacaca      60

atgtatgcag caggctcagt gtgagtgaac tggaggcttc tctacaac atg acc caa      117
                                                     Met Thr Gln
                                                     1

agg agc att gca ggt cct att tgc aac ctg aag ttt gtg act ctc ctg      165
Arg Ser Ile Ala Gly Pro Ile Cys Asn Leu Lys Phe Val Thr Leu Leu
    5                   10                  15

gtt gcc tta agt tca gaa ctc cca ttc ctg gga gct gga gta cag ctt      213
Val Ala Leu Ser Ser Glu Leu Pro Phe Leu Gly Ala Gly Val Gln Leu
20                  25                  30                  35

caa gac aat ggg tat aat gga ttg ctc att gca att aat cct cag gta      261
Gln Asp Asn Gly Tyr Asn Gly Leu Leu Ile Ala Ile Asn Pro Gln Val
                40                  45                  50

cct gag aat cag aac ctc atc tca aac att aag gaa atg ata act gaa      309
Pro Glu Asn Gln Asn Leu Ile Ser Asn Ile Lys Glu Met Ile Thr Glu
            55                  60                  65

gct tca ttt tac cta ttt aat gct acc aag aga aga gta ttt ttc aga      357
Ala Ser Phe Tyr Leu Phe Asn Ala Thr Lys Arg Arg Val Phe Phe Arg
        70                  75                  80

aat ata aag att tta ata cct gcc aca tgg aaa gct aat aat aac agc      405
Asn Ile Lys Ile Leu Ile Pro Ala Thr Trp Lys Ala Asn Asn Asn Ser
    85                  90                  95

aaa ata aaa caa gaa tca tat gaa aag gca aat gtc ata gtg act gac      453
Lys Ile Lys Gln Glu Ser Tyr Glu Lys Ala Asn Val Ile Val Thr Asp
100                 105                 110                 115

tgg tat ggg gca cat gga gat gat cca tac acc cta caa tac aga ggg      501
Trp Tyr Gly Ala His Gly Asp Asp Pro Tyr Thr Leu Gln Tyr Arg Gly
                120                 125                 130

tgt gga aaa gag gga aaa tac att cat ttc aca cct aat ttc cta ctg      549
Cys Gly Lys Glu Gly Lys Tyr Ile His Phe Thr Pro Asn Phe Leu Leu
            135                 140                 145

aat gat aac tta aca gct ggc tac gga tca cga ggc cga gtg ttt gtc      597
Asn Asp Asn Leu Thr Ala Gly Tyr Gly Ser Arg Gly Arg Val Phe Val
        150                 155                 160

cat gaa tgg gcc cac ctc cgt tgg ggt gtg ttc gat gag tat aac aat      645
His Glu Trp Ala His Leu Arg Trp Gly Val Phe Asp Glu Tyr Asn Asn
    165                 170                 175

gac aaa cct ttc tac ata aat ggg caa aat caa att aaa gtg aca agg 693
Asp Lys Pro Phe Tyr Ile Asn Gly Gln Asn Gln Ile Lys Val Thr Arg
180                 185                 190                 195

tgt tca tct gac atc aca ggc att ttt gtg tgt gaa aaa ggt cct tgc 741
Cys Ser Ser Asp Ile Thr Gly Ile Phe Val Cys Glu Lys Gly Pro Cys
                200                 205                 210

ccc caa gaa aac tgt att att agt aag ctt ttt aaa gaa gga tgc acc 789
Pro Gln Glu Asn Cys Ile Ile Ser Lys Leu Phe Lys Glu Gly Cys Thr
            215                 220                 225

ttt atc tac aat agc acc caa aat gca act gca tca ata atg ttc atg 837
Phe Ile Tyr Asn Ser Thr Gln Asn Ala Thr Ala Ser Ile Met Phe Met
        230                 235                 240

caa agt tta tct tct gtg gtt gaa ttt tgt aat gca agt acc cac aac 885
Gln Ser Leu Ser Ser Val Val Glu Phe Cys Asn Ala Ser Thr His Asn
    245                 250                 255

caa gaa gca cca aac cta cag aac cag atg tgc agc ctc aga agt gca 933
Gln Glu Ala Pro Asn Leu Gln Asn Gln Met Cys Ser Leu Arg Ser Ala
260                 265                 270                 275

tgg gat gta atc aca gac tct gct gac ttt cac cac agc ttt ccc atg 981
Trp Asp Val Ile Thr Asp Ser Ala Asp Phe His His Ser Phe Pro Met
                280                 285                 290

aat ggg act gag ctt cca cct cct ccc aca ttc tcg ctt gta cag gct 1029
Asn Gly Thr Glu Leu Pro Pro Pro Pro Thr Phe Ser Leu Val Gln Ala
            295                 300                 305

ggt gac aaa gtg gtc tgt tta gtg ctg gat gtg tcc agc aag atg gca 1077
Gly Asp Lys Val Val Cys Leu Val Leu Asp Val Ser Ser Lys Met Ala
        310                 315                 320

gag gct gac aga ctc ctt caa cta caa caa gcc gca gaa ttt tat ttg 1125
Glu Ala Asp Arg Leu Leu Gln Leu Gln Gln Ala Ala Glu Phe Tyr Leu
    325                 330                 335

atg cag att gtt gaa att cat acc ttc gtg ggc att gcc agt ttc gac 1173
Met Gln Ile Val Glu Ile His Thr Phe Val Gly Ile Ala Ser Phe Asp
340                 345                 350                 355

agc aaa gga gag atc aga gcc cag cta cac caa att aac agc aat gat 1221
Ser Lys Gly Glu Ile Arg Ala Gln Leu His Gln Ile Asn Ser Asn Asp
                360                 365                 370

gat cga aag ttg ctg gtt tca tat ctg ccc acc act gta tca gct aaa 1269
Asp Arg Lys Leu Leu Val Ser Tyr Leu Pro Thr Thr Val Ser Ala Lys
            375                 380                 385

aca gac atc agc att tgt tca ggg ctt aag aaa gga ttt gag gtg gtt 1317
Thr Asp Ile Ser Ile Cys Ser Gly Leu Lys Lys Gly Phe Glu Val Val
        390                 395                 400

gaa aaa ctg aat gga aaa gct tat ggc tct gtg atg ata tta gtg acc 1365
Glu Lys Leu Asn Gly Lys Ala Tyr Gly Ser Val Met Ile Leu Val Thr
    405                 410                 415

agc gga gat gat aag ctt ctt ggc aat tgc tta ccc act gtg ctc agc 1413
Ser Gly Asp Asp Lys Leu Leu Gly Asn Cys Leu Pro Thr Val Leu Ser
420                 425                 430                 435

agt ggt tca aca att cac tcc att gcc ctg ggt tca tct gca gcc cca 1461
Ser Gly Ser Thr Ile His Ser Ile Ala Leu Gly Ser Ser Ala Ala Pro
                440                 445                 450

aat ctg gag gaa tta tca cgt ctt aca gga ggt tta aag ttc ttt gtt 1509
Asn Leu Glu Glu Leu Ser Arg Leu Thr Gly Gly Leu Lys Phe Phe Val
            455                 460                 465

cca gat ata tca aac tcc aat agc atg att gat gct ttc agt aga att 1557
Pro Asp Ile Ser Asn Ser Asn Ser Met Ile Asp Ala Phe Ser Arg Ile
        470                 475                 480

tcc tct gga act gga gac att ttc cag caa cat att cag ctt gaa agt 1605
Ser Ser Gly Thr Gly Asp Ile Phe Gln Gln His Ile Gln Leu Glu Ser
    485                 490                 495

aca ggt gaa aat gtc aaa cct cac cat caa ttg aaa aac aca gtg act 1653
Thr Gly Glu Asn Val Lys Pro His His Gln Leu Lys Asn Thr Val Thr
500                 505                 510                 515

gtg gat aat act gtg ggc aac gac act atg ttt cta gtt acg tgg cag 1701
Val Asp Asn Thr Val Gly Asn Asp Thr Met Phe Leu Val Thr Trp Gln
                520                 525                 530

gcc agt ggt cct cct gag att ata tta ttt gat cct gat gga cga aaa 1749
Ala Ser Gly Pro Pro Glu Ile Ile Leu Phe Asp Pro Asp Gly Arg Lys
            535                 540                 545

tac tac aca aat aat ttt atc acc aat cta act ttt cgg aca gct agt 1797
Tyr Tyr Thr Asn Asn Phe Ile Thr Asn Leu Thr Phe Arg Thr Ala Ser
        550                 555                 560

ctt tgg att cca gga aca gct aag cct ggg cac tgg act tac acc ctg 1845
Leu Trp Ile Pro Gly Thr Ala Lys Pro Gly His Trp Thr Tyr Thr Leu
    565                 570                 575

aac aat acc cat cat tct ctg caa gcc ctg aaa gtg aca gtg acc tct 1893
Asn Asn Thr His His Ser Leu Gln Ala Leu Lys Val Thr Val Thr Ser
580                 585                 590                 595

cgc gcc tcc aac tca gct gtg ccc cca gcc act gtg gaa gcc ttt gtg 1941
Arg Ala Ser Asn Ser Ala Val Pro Pro Ala Thr Val Glu Ala Phe Val
                600                 605                 610

gaa aga gac agc ctc cat ttt cct cat cct gtg atg att tat gcc aat 1989
Glu Arg Asp Ser Leu His Phe Pro His Pro Val Met Ile Tyr Ala Asn
            615                 620                 625

gtg aaa cag gga ttt tat ccc att ctt aat gcc act gtc act gcc aca 2037
Val Lys Gln Gly Phe Tyr Pro Ile Leu Asn Ala Thr Val Thr Ala Thr
        630                 635                 640

gtt gag cca gag act gga gat cct gtt acg ctg aga ctc ctt gat gat 2085
Val Glu Pro Glu Thr Gly Asp Pro Val Thr Leu Arg Leu Leu Asp Asp
    645                 650                 655

gga gca ggt gct gat gtt ata aaa aat gat gga att tac tcg agg tat 2133
Gly Ala Gly Ala Asp Val Ile Lys Asn Asp Gly Ile Tyr Ser Arg Tyr
660                 665                 670                 675

ttt ttc tcc ttt gct gca aat ggt aga tat agc ttg aaa gtg cat gtc 2181
Phe Phe Ser Phe Ala Ala Asn Gly Arg Tyr Ser Leu Lys Val His Val
                680                 685                 690

aat cac tct ccc agc ata agc acc cca gcc cac tct att cca ggg agt 2229
Asn His Ser Pro Ser Ile Ser Thr Pro Ala His Ser Ile Pro Gly Ser
            695                 700                 705

cat gct atg tat gta cca ggt tac aca gca aac ggt aat att cag atg 2277
His Ala Met Tyr Val Pro Gly Tyr Thr Ala Asn Gly Asn Ile Gln Met
        710                 715                 720

aat gct cca agg aaa tca gta ggc aga aat gag gag gag cga aag tgg 2325
Asn Ala Pro Arg Lys Ser Val Gly Arg Asn Glu Glu Glu Arg Lys Trp
    725                 730                 735

ggc ttt agc cga gtc agc tca gga ggc tcc ttt tca gtg ctg gga gtt 2373
Gly Phe Ser Arg Val Ser Ser Gly Gly Ser Phe Ser Val Leu Gly Val
740                 745                 750                 755

cca gct ggc ccc cac cct gat gtg ttt cca cca tgc aaa att att gac 2421
Pro Ala Gly Pro His Pro Asp Val Phe Pro Pro Cys Lys Ile Ile Asp
                760                 765                 770

ctg gaa gct gta aaa gta gaa gag gaa ttg acc cta tct tgg aca gca 2469
Leu Glu Ala Val Lys Val Glu Glu Glu Leu Thr Leu Ser Trp Thr Ala
            775                 780                 785

cct gga gaa gac ttt gat cag ggc cag gct aca agc tat gaa ata aga 2517
Pro Gly Glu Asp Phe Asp Gln Gly Gln Ala Thr Ser Tyr Glu Ile Arg
        790                 795                 800

atg agt aaa agt cta cag aat atc caa gat gac ttt aac aat gct att 2565
Met Ser Lys Ser Leu Gln Asn Ile Gln Asp Asp Phe Asn Asn Ala Ile
    805                 810                 815

tta gta aat aca tca aag cga aat cct cag caa gct ggc atc agg gag 2613
Leu Val Asn Thr Ser Lys Arg Asn Pro Gln Gln Ala Gly Ile Arg Glu
820                 825                 830                 835

ata ttt acg ttc tca ccc cag att tcc acg aat gga cct gaa cat cag 2661
Ile Phe Thr Phe Ser Pro Gln Ile Ser Thr Asn Gly Pro Glu His Gln
                840                 845                 850

cca aat gga gaa aca cat gaa agc cac aga att tat gtt gca ata cga 2709
Pro Asn Gly Glu Thr His Glu Ser His Arg Ile Tyr Val Ala Ile Arg
            855                 860                 865

gca atg gat agg aac tcc tta cag tct gct gta tct aac att gcc cag 2757
Ala Met Asp Arg Asn Ser Leu Gln Ser Ala Val Ser Asn Ile Ala Gln
        870                 875                 880

gcg cct ctg ttt att ccc ccc aat tct gat cct gta cct gcc aga gat 2805
Ala Pro Leu Phe Ile Pro Pro Asn Ser Asp Pro Val Pro Ala Arg Asp
    885                 890                 895

tat ctt ata ttg aaa gga gtt tta aca gca atg ggt ttg ata gga atc 2853
Tyr Leu Ile Leu Lys Gly Val Leu Thr Ala Met Gly Leu Ile Gly Ile
900                 905                 910                 915

att tgc ctt att ata gtt gtg aca cat cat act tta agc agg aaa aag 2901
Ile Cys Leu Ile Ile Val Val Thr His His Thr Leu Ser Arg Lys Lys
                920                 925                 930

aga gca gac aag aaa gag aat gga aca aaa tta tta taa ataaatatcc 2950
Arg Ala Asp Lys Lys Glu Asn Gly Thr Lys Leu Leu
            935                 940

aaagtgtctt ccttcttaga tataagaccc atggccttcg actacaaaaa catactaaca 3010

aagtcaaatt aacatcaaaa ctgtattaaa atgcattgag tttttgtaca atacagataa 3070

gatttttaca tggtagatca acaattcttt ttgggggtag attagaaaac ccttacactt 3130

tggctatgaa caaataataa aaattattct ttaaagtaat gtctttaaag gcaaagggaa 3190

gggtaaagtc ggaccagtgt caaggaaagt ttgttttatt gaggtggaaa aatagcccca 3250

agcagagaaa aggagggtag gtctgcatta taactgtctg tgtgaagcaa tcatttagtt 3310

actttgatta atttttcttt tctccttatc tgtgcagaac aggttgcttg tttacaactg 3370

aagatcatgc tatattttat atatgaagcc cctaatgcaa agctctttac ctcttgctat 3430

tttgttatat atattacaga tgaaatctca ctgctaatgc tcagagatct tttttcactg 3490

taagaggtaa cctttaacaa tatgggtatt acctttgtct cttcataccg gttttatgac 3550

aaaggtctat tgaatttatt tgtttgtaag tttctactcc catcaaagca gctttctaag 3610

ttattgcctt ggttattatg gatgatagtt atagccctta taatgcctta cctaggaaa 3669

<210> 20
<211> 943
<212> PRT
<213> Homo sapiens

<400> 20

Met Thr Gln Arg Ser Ile Ala Gly Pro Ile Cys Asn Leu Lys Phe Val
1               5                   10                  15

Thr Leu Leu Val Ala Leu Ser Ser Glu Leu Pro Phe Leu Gly Ala Gly
            20                  25                  30

Val Gln Leu Gln Asp Asn Gly Tyr Asn Gly Leu Leu Ile Ala Ile Asn
        35                  40                  45

Pro Gln Val Pro Glu Asn Gln Asn Leu Ile Ser Asn Ile Lys Glu Met
    50                  55                  60

Ile Thr Glu Ala Ser Phe Tyr Leu Phe Asn Ala Thr Lys Arg Arg Val
65                  70                  75                  80

Phe Phe Arg Asn Ile Lys Ile Leu Ile Pro Ala Thr Trp Lys Ala Asn
                85                  90                  95

Asn Asn Ser Lys Ile Lys Gln Glu Ser Tyr Glu Lys Ala Asn Val Ile
            100                 105                 110

Val Thr Asp Trp Tyr Gly Ala His Gly Asp Asp Pro Tyr Thr Leu Gln
        115                 120                 125

Tyr Arg Gly Cys Gly Lys Glu Gly Lys Tyr Ile His Phe Thr Pro Asn
    130                 135                 140

Phe Leu Leu Asn Asp Asn Leu Thr Ala Gly Tyr Gly Ser Arg Gly Arg
145                 150                 155                 160

Val Phe Val His Glu Trp Ala His Leu Arg Trp Gly Val Phe Asp Glu
                165                 170                 175

Tyr Asn Asn Asp Lys Pro Phe Tyr Ile Asn Gly Gln Asn Gln Ile Lys
            180                 185                 190

Val Thr Arg Cys Ser Ser Asp Ile Thr Gly Ile Phe Val Cys Glu Lys
        195                 200                 205

Gly Pro Cys Pro Gln Glu Asn Cys Ile Ile Ser Lys Leu Phe Lys Glu
    210                 215                 220

Gly Cys Thr Phe Ile Tyr Asn Ser Thr Gln Asn Ala Thr Ala Ser Ile
225                 230                 235                 240

Met Phe Met Gln Ser Leu Ser Ser Val Val Glu Phe Cys Asn Ala Ser
                245                 250                 255

Thr His Asn Gln Glu Ala Pro Asn Leu Gln Asn Gln Met Cys Ser Leu
            260                 265                 270

Arg Ser Ala Trp Asp Val Ile Thr Asp Ser Ala Asp Phe His His Ser
        275                 280                 285

Phe Pro Met Asn Gly Thr Glu Leu Pro Pro Pro Pro Thr Phe Ser Leu
    290                 295                 300

Val Gln Ala Gly Asp Lys Val Val Cys Leu Val Leu Asp Val Ser Ser
305                 310                 315                 320

Lys Met Ala Glu Ala Asp Arg Leu Leu Gln Leu Gln Gln Ala Ala Glu
                325                 330                 335

Phe Tyr Leu Met Gln Ile Val Glu Ile His Thr Phe Val Gly Ile Ala
            340                 345                 350

Ser Phe Asp Ser Lys Gly Glu Ile Arg Ala Gln Leu His Gln Ile Asn
        355                 360                 365

Ser Asn Asp Asp Arg Lys Leu Leu Val Ser Tyr Leu Pro Thr Thr Val
    370                 375                 380

Ser Ala Lys Thr Asp Ile Ser Ile Cys Ser Gly Leu Lys Lys Gly Phe
385                 390                 395                 400

Glu Val Val Glu Lys Leu Asn Gly Lys Ala Tyr Gly Ser Val Met Ile
                405                 410                 415

Leu Val Thr Ser Gly Asp Asp Lys Leu Leu Gly Asn Cys Leu Pro Thr
            420                 425                 430

Val Leu Ser Ser Gly Ser Thr Ile His Ser Ile Ala Leu Gly Ser Ser
        435                 440                 445

Ala Ala Pro Asn Leu Glu Glu Leu Ser Arg Leu Thr Gly Gly Leu Lys
    450                 455                 460

Phe Phe Val Pro Asp Ile Ser Asn Ser Asn Ser Met Ile Asp Ala Phe
465                 470                 475                 480

Ser Arg Ile Ser Ser Gly Thr Gly Asp Ile Phe Gln Gln His Ile Gln
                485                 490                 495

Leu Glu Ser Thr Gly Glu Asn Val Lys Pro His His Gln Leu Lys Asn
            500                 505                 510

Thr Val Thr Val Asp Asn Thr Val Gly Asn Asp Thr Met Phe Leu Val
        515                 520                 525

Thr Trp Gln Ala Ser Gly Pro Pro Glu Ile Ile Leu Phe Asp Pro Asp
    530                 535                 540

Gly Arg Lys Tyr Tyr Thr Asn Asn Phe Ile Thr Asn Leu Thr Phe Arg
545                 550                 555                 560

Thr Ala Ser Leu Trp Ile Pro Gly Thr Ala Lys Pro Gly His Trp Thr
                565                 570                 575

Tyr Thr Leu Asn Asn Thr His His Ser Leu Gln Ala Leu Lys Val Thr
            580                 585                 590

Val Thr Ser Arg Ala Ser Asn Ser Ala Val Pro Pro Ala Thr Val Glu
        595                 600                 605

Ala Phe Val Glu Arg Asp Ser Leu His Phe Pro His Pro Val Met Ile
    610                 615                 620

Tyr Ala Asn Val Lys Gln Gly Phe Tyr Pro Ile Leu Asn Ala Thr Val
625                 630                 635                 640

Thr Ala Thr Val Glu Pro Glu Thr Gly Asp Pro Val Thr Leu Arg Leu
                645                 650                 655

Leu Asp Asp Gly Ala Gly Ala Asp Val Ile Lys Asn Asp Gly Ile Tyr
            660                 665                 670

Ser Arg Tyr Phe Phe Ser Phe Ala Ala Asn Gly Arg Tyr Ser Leu Lys
        675                 680                 685

Val His Val Asn His Ser Pro Ser Ile Ser Thr Pro Ala His Ser Ile
    690                 695                 700

Pro Gly Ser His Ala Met Tyr Val Pro Gly Tyr Thr Ala Asn Gly Asn
705                 710                 715                 720

Ile Gln Met Asn Ala Pro Arg Lys Ser Val Gly Arg Asn Glu Glu Glu
                725                 730                 735

Arg Lys Trp Gly Phe Ser Arg Val Ser Ser Gly Gly Ser Phe Ser Val
            740                 745                 750

Leu Gly Val Pro Ala Gly Pro His Pro Asp Val Phe Pro Pro Cys Lys
        755                 760                 765

Ile Ile Asp Leu Glu Ala Val Lys Val Glu Glu Glu Leu Thr Leu Ser
    770                 775                 780

Trp Thr Ala Pro Gly Glu Asp Phe Asp Gln Gly Gln Ala Thr Ser Tyr
785                 790                 795                 800

Glu Ile Arg Met Ser Lys Ser Leu Gln Asn Ile Gln Asp Asp Phe Asn
                805                 810                 815

Asn Ala Ile Leu Val Asn Thr Ser Lys Arg Asn Pro Gln Gln Ala Gly
            820                 825                 830

Ile Arg Glu Ile Phe Thr Phe Ser Pro Gln Ile Ser Thr Asn Gly Pro
        835                 840                 845

Glu His Gln Pro Asn Gly Glu Thr His Glu Ser His Arg Ile Tyr Val
    850                 855                 860

Ala Ile Arg Ala Met Asp Arg Asn Ser Leu Gln Ser Ala Val Ser Asn
865                 870                 875                 880

Ile Ala Gln Ala Pro Leu Phe Ile Pro Pro Asn Ser Asp Pro Val Pro
                885                 890                 895

Ala Arg Asp Tyr Leu Ile Leu Lys Gly Val Leu Thr Ala Met Gly Leu
            900                 905                 910

Ile Gly Ile Ile Cys Leu Ile Ile Val Val Thr His His Thr Leu Ser
        915                 920                 925

Arg Lys Lys Arg Ala Asp Lys Lys Glu Asn Gly Thr Lys Leu Leu
    930                 935                 940

<210>  21
<211>  1428
<212>  DNA
<213>  Homo sapiens

<220>
<221>  CDS
<222>  (232)..(840)

<400>  21
   cttcaggtca gggagaatgt ataaatgtcc attgccatcg aggttctgct atttttgaga      60
   agctgaagca actccaagga cacagttcac agaaatttgg ttctcagccc caaaatactg     120
   attgaattgg agacaattac aaggactctc tggccaaaaa cccttgaaga ggccccgtga     180
   aggaggcagt gaggagcttt tgattgctga cctgtgtcgt accaccccag a atg tgc 237
   Met Cys
   1
   act ggg ggc tgt gcc aga tgc ctg ggg ggg acc ctc att ccc ctt gct 285
   Thr Gly Gly Cys Ala Arg Cys Leu Gly Gly Thr Leu Ile Pro Leu Ala
   5 10 15
   ttt ttt ggc ttc ctg gct aac atc ctg tta ttt ttt cct gga gga aaa 333
   Phe Phe Gly Phe Leu Ala Asn Ile Leu Leu Phe Phe Pro Gly Gly Lys
   20 25 30
   gtg ata gat gac aac gac cac ctt tcc caa gag atc tgg ttt ttc gga 381
   Val Ile Asp Asp Asn Asp His Leu Ser Gln Glu Ile Trp Phe Phe Gly
   35 40 45 50
   gga ata tta gga agc ggt gtc ttg atg atc ttc cct gcg ctg gtg ttc 429
   Gly Ile Leu Gly Ser Gly Val Leu Met Ile Phe Pro Ala Leu Val Phe
   55 60 65
   ttg ggc ctg aag aac aat gac tgc tgt ggg tgc tgc ggc aac gag ggc 477
   Leu Gly Leu Lys Asn Asn Asp Cys Cys Gly Cys Cys Gly Asn Glu Gly
   70 75 80
   tgt ggg aag cga ttt gcg atg ttc acc tcc acg ata ttt gct gtg gtt 525
   Cys Gly Lys Arg Phe Ala Met Phe Thr Ser Thr Ile Phe Ala Val Val
   85 90 95
   gga ttc ttg gga gct gga tac tcg ttt atc atc tca gcc att tca atc 573
   Gly Phe Leu Gly Ala Gly Tyr Ser Phe Ile Ile Ser Ala Ile Ser Ile
   100 105 110
   aac aag ggt cct aaa tgc ctc atg gcc aat agt aca tgg ggc tac ccc 621
   Asn Lys Gly Pro Lys Cys Leu Met Ala Asn Ser Thr Trp Gly Tyr Pro
   115 120 125 130
   ttc cac gac ggg gat tat ctc aat gat gag gcc tta tgg aac aag tgc 669
   Phe His Asp Gly Asp Tyr Leu Asn Asp Glu Ala Leu Trp Asn Lys Cys
   135 140 145
   cga gag cct ctc aat gtg gtt ccc tgg aat ctg acc ctc ttc tcc atc 717
   Arg Glu Pro Leu Asn Val Val Pro Trp Asn Leu Thr Leu Phe Ser Ile
   150 155 160
   ctg ctg gtc gta gga gga atc cag atg gtt ctc tgc gcc atc cag gtg 765
   Leu Leu Val Val Gly Gly Ile Gln Met Val Leu Cys Ala Ile Gln Val
   165 170 175
   gtc aat ggc ctc ctg ggg acc ctc tgt ggg gac tgc cag tgt tgt ggc 813
   Val Asn Gly Leu Leu Gly Thr Leu Cys Gly Asp Cys Gln Cys Cys Gly
   180 185 190
   tgc tgt ggg gga gat gga ccc gtt taa acctccgaga tgagctgctc 860
   Cys Cys Gly Gly Asp Gly Pro Val
   195 200
   agactctaca gcatgacgac tacaatttct tttcataaaa cttcttctct tcttggaatt 920
   attaattcct atctgcttcc tagctgataa agcttagaaa aggcagttat tccttctttc 980
   caaccagctt tgctcgagtt agaattttgt tattttcaaa taaaaaatag tttggccact 1040
   taacaaattt gatttataaa tctttcaaat tagttccttt ttagaattta ccaacaggtt 1100
   caaagcatac ttttcatgat ttttttatta caaatgtaaa atgtataaag tcacatgtac 1160
   tgccatacta cttctttgta tataaagatg tttatatctt tggaagtttt acataaatca 1220
   aaggaagaaa gcacatttaa aatgagaaac taagaccaat ttctgttttt aagaggaaaa 1280
   agaatgattg atgtatccta agtattgtta tttgttgtct ttttttgctg ccttgcttga 1340
   gttgcttgtg actgatcttt tgaggctgtc atcatggcta gggttctttt atgtatgtta 1400
   aattaaaacc tgaattcaga ggtaacgt 1428

<210> 22
<211> 202
<212> PRT
<213> Homo sapiens

<400> 22

Met Cys Thr Gly Gly Cys Ala Arg Cys Leu Gly Gly Thr Leu Ile Pro
1               5                   10                  15

Leu Ala Phe Phe Gly Phe Leu Ala Asn Ile Leu Leu Phe Phe Pro Gly
            20                  25                  30

Gly Lys Val Ile Asp Asp Asn Asp His Leu Ser Gln Glu Ile Trp Phe
        35                  40                  45

Phe Gly Gly Ile Leu Gly Ser Gly Val Leu Met Ile Phe Pro Ala Leu
    50                  55                  60

Val Phe Leu Gly Leu Lys Asn Asn Asp Cys Cys Gly Cys Cys Gly Asn
65                  70                  75                  80

Glu Gly Cys Gly Lys Arg Phe Ala Met Phe Thr Ser Thr Ile Phe Ala
                85                  90                  95

Val Val Gly Phe Leu Gly Ala Gly Tyr Ser Phe Ile Ile Ser Ala Ile
            100                 105                 110

Ser Ile Asn Lys Gly Pro Lys Cys Leu Met Ala Asn Ser Thr Trp Gly
        115                 120                 125

Tyr Pro Phe His Asp Gly Asp Tyr Leu Asn Asp Glu Ala Leu Trp Asn
    130                 135                 140

Lys Cys Arg Glu Pro Leu Asn Val Val Pro Trp Asn Leu Thr Leu Phe
145                 150                 155                 160

Ser Ile Leu Leu Val Val Gly Gly Ile Gln Met Val Leu Cys Ala Ile
                165                 170                 175

Gln Val Val Asn Gly Leu Leu Gly Thr Leu Cys Gly Asp Cys Gln Cys
            180                 185                 190

Cys Gly Cys Cys Gly Gly Asp Gly Pro Val
        195                 200

<210>  23
<211>  2859
<212>  DNA
<213>  Homo sapiens

<220>
<221>  CDS
<222>  (192)..(866)

<400>  23
attctggagt ccagagccac tgcctttgct ccagccgctg ccgccgcacc acctctcctt      60

ctctgcctct gaccctcctt ctcgctgctc cccctgccca gctgctcctc ccacctggcc     120

atgaccaaag cccctgctgg caccctggcc cagctctgag tcctgggacc ctcggtcctc     180

tctcctgggc c atg gcc aac tca ggc ctc cag ctc ctg ggc tac ttc ttg      230
             Met Ala Asn Ser Gly Leu Gln Leu Leu Gly Tyr Phe Leu
             1               5                   10

gcc ctg ggt ggc tgg gtg ggc atc att gct agc aca gcc ctg cca cag      278
Ala Leu Gly Gly Trp Val Gly Ile Ile Ala Ser Thr Ala Leu Pro Gln
    15                  20                  25

tgg aag cag tct tcc tac gca ggc gac gcc atc atc act gcc gtg ggc      326
Trp Lys Gln Ser Ser Tyr Ala Gly Asp Ala Ile Ile Thr Ala Val Gly
30                  35                  40                  45

ccc tat gaa ggg ctc tgg atg tcc tgc gcc tcc cag agc act ggg caa      374
Pro Tyr Glu Gly Leu Trp Met Ser Cys Ala Ser Gln Ser Thr Gly Gln
                50                  55                  60

gtg cag tgc aag ctc tac gac tcg ctg ctc gcc ctg gac ggt cac atc      422
Val Gln Cys Lys Leu Tyr Asp Ser Leu Leu Ala Leu Asp Gly His Ile
            65                  70                  75

caa tca gcg cgg gcc ctg atg gtg gtg gcc gtg ctc ctg ggc ttc gtg      470
Gln Ser Ala Arg Ala Leu Met Val Val Ala Val Leu Leu Gly Phe Val
        80                  85                  90

gcc atg gtc ctc agc gta gtt ggc atg aag tgt acg cgg gtg gga gac      518
Ala Met Val Leu Ser Val Val Gly Met Lys Cys Thr Arg Val Gly Asp
    95                  100                 105

agc aac ccc att gcc aag ggc cgt gtt gcc atc gcc ggg gga gcc ctc      566
Ser Asn Pro Ile Ala Lys Gly Arg Val Ala Ile Ala Gly Gly Ala Leu
110                 115                 120                 125

ttc atc ctg gca ggc ctc tgc act ttg act gct gtc tcg tgg tat gcc      614
Phe Ile Leu Ala Gly Leu Cys Thr Leu Thr Ala Val Ser Trp Tyr Ala
                130                 135                 140

acc ctg gtg acc cag gag ttc ttc aac cca agc aca cct gtc aat gcc      662
Thr Leu Val Thr Gln Glu Phe Phe Asn Pro Ser Thr Pro Val Asn Ala
            145                 150                 155

agg tat gaa ttt ggc cca gcc ctg ttc gtg ggc tgg gcc tca gct ggc      710
Arg Tyr Glu Phe Gly Pro Ala Leu Phe Val Gly Trp Ala Ser Ala Gly
        160                 165                 170

ctg gcc gtg ctg ggc ggc tcc ttc ctc tgc tgc aca tgc ccg gag cca      758
Leu Ala Val Leu Gly Gly Ser Phe Leu Cys Cys Thr Cys Pro Glu Pro
    175                 180                 185

gag aga ccc aac agc agc cca cag ccc tat cgg cct gga ccc tct gct      806
Glu Arg Pro Asn Ser Ser Pro Gln Pro Tyr Arg Pro Gly Pro Ser Ala

190                 195                 200                 205

gct gcc cga gaa cca gtt gtt aaa ttg ccc gcc tcc gcc aag ggc ccc     854
Ala Ala Arg Glu Pro Val Val Lys Leu Pro Ala Ser Ala Lys Gly Pro
                210                 215                 220

ctg ggt gtg taa tgtccagtcc ccagccaggc tctgtcccct gccataccta         906
Leu Gly Val

gactgtgtgt ttcatatttt tttggaaaga gaagtgaaca tccagcccca atcatggtat   966

cattcggtct gtcctcagcg tggcttggac ggggcctgtg tcagagtggt cagtgctgac   1026

ccctggggct cttgggcaga aagatgagga gacagaggtc cagggtgggt tacatagcac   1086

atccagggct gagcaagaaa taattcagag gtcctaccct ctgtctaggg acccccctcc   1146

caagcctggc cttggccttg gcacaaagtc ctccttgata ggagatccca ctcactcctg   1206

gaggctgccc ctgaggcttg gcccagctct aggagcagtc cccagggtca gggagcccct   1266

ggtgtggaaa gaggccccaa ggtagtaaac cctgcccctg ttactgtgct ccagagacct   1326

cctaagggaa gggacagttc ctggaaggcc ctccagctgg atgctgggga tcagcgatag   1386

gtgaggggac acagtgtagg agctccccat gtagaaaagg gaatgtgggg agggcgttag   1446

gagcttgcag gcattaggac tgtcctgagc aaggtctgca gcccccagct ctgctcaccc   1506

cgaatcctgc cccttgtttc cacacctacc attcctcctc tcctgatccc cagcatccag   1566

ctgaggtcca aggtctttgt cctagaatca gagtggggag gggacagcct ggggctgccc   1626

agagactgtg ggtggagctg cctgctgcac tcagcagtgc ggtcagagaa gggcttttgg   1686

tcttgaagtc caggtaccat ccccccttag catacagggg gaagggcctg agaggaatgt   1746

aaggaaacca gcccagatca gtcccaaggc cagagtcctt tgtcctacat ctccctgaac   1806

cagagtgtgc cctgcccctc atgctcagac ctctcccacc ccaaaccctc tcccgggact   1866

cagtctccct ggccactgcg tatcaggctt ctggggaaag catccatcac agaacctccc   1926

cttccctgcc acgcaccttc cttggccagc tccattctgg cctcctccac cacctgcctt   1986

gtgaccacat ctcccaccac gtccccagat ctcaagaacg cagctcagct tctccttcga   2046

gcttgactct gagagggaaa gtgacggaaa ccaagtcaga tgagatgact gccatgtaca   2106

ctgcagtcaa gggcagggag gggaggaatg acacaaatgg cagggagctg ctgggggact   2166

gacccctcgg cgcctggcct ggccggtgct gcacatccac cggggcacaa cagggacttg   2226

tccagcctct ggtcagagga tgtggccacc tgaccctaaa taggttcccc agagtcctgc   2286

ccctctaatg aatgagaact gcaggagttt ctcctctggg tgcctgaagc tatagtgcaa   2346

tggttcccaa ccctgcatgc acattcgaat cacctggggg cacaatgcct aggctccaac   2406

cccagacact cttatttcat tggtctgggt ggacctggca tcagaagtca tgtagctcct   2466

caggggactg tagtgtgtgg tcagcactga gggctcctct atgaggcctc aagcccaggt   2526

gactctgtga ggtctgcaga gggagaaaag aacccacaag ggaagaggtg gaggtcaggc   2586

acggtggctc acgcttgtaa tcccagcact ttgggaggcc gaggtgggta gatacctgaa   2646

gtcaggagtt cgagactagc ctggccaata tggtaaaacc ccgtgtctat taaaaataca    2706

aaaattagct ggctgtggtg gtgggcacct gtaatcccag ctactcggga ggctgaggca    2766

ggagaatcgc ttgaactcgg gaggtggagg ttgcagtcag ccaagatcgt gccactgcac    2826

accatcctgg atgacagagc aagactccat cac                                 2859

<210>  24
<211>  224
<212>  PRT
<213>  Homo sapiens

<400>  24

Met Ala Asn Ser Gly Leu Gln Leu Leu Gly Tyr Phe Leu Ala Leu Gly
1               5                   10                  15

Gly Trp Val Gly Ile Ile Ala Ser Thr Ala Leu Pro Gln Trp Lys Gln
            20                  25                  30

Ser Ser Tyr Ala Gly Asp Ala Ile Ile Thr Ala Val Gly Pro Tyr Glu
        35                  40                  45

Gly Leu Trp Met Ser Cys Ala Ser Gln Ser Thr Gly Gln Val Gln Cys
    50                  55                  60

Lys Leu Tyr Asp Ser Leu Leu Ala Leu Asp Gly His Ile Gln Ser Ala
65                  70                  75                  80

Arg Ala Leu Met Val Val Ala Val Leu Leu Gly Phe Val Ala Met Val
                85                  90                  95

Leu Ser Val Val Gly Met Lys Cys Thr Arg Val Gly Asp Ser Asn Pro
            100                 105                 110

Ile Ala Lys Gly Arg Val Ala Ile Ala Gly Gly Ala Leu Phe Ile Leu
        115                 120                 125

Ala Gly Leu Cys Thr Leu Thr Ala Val Ser Trp Tyr Ala Thr Leu Val
    130                 135                 140

Thr Gln Glu Phe Phe Asn Pro Ser Thr Pro Val Asn Ala Arg Tyr Glu
145                 150                 155                 160

Phe Gly Pro Ala Leu Phe Val Gly Trp Ala Ser Ala Gly Leu Ala Val
                165                 170                 175

Leu Gly Gly Ser Phe Leu Cys Cys Thr Cys Pro Glu Pro Glu Arg Pro
            180                 185                 190

Asn Ser Ser Pro Gln Pro Tyr Arg Pro Gly Pro Ser Ala Ala Ala Arg
        195                 200                 205

Glu Pro Val Val Lys Leu Pro Ala Ser Ala Lys Gly Pro Leu Gly Val
210                 215                 220

<210>  25
<211>  2485
<212>  DNA
<213>  Homo sapiens

<220>
<221>  CDS
<222>  (53)..(1651)

<400>  25
catactccat acctgggatt tccgcctcgc cgctctccga ctgcttccag ac atg cag      58
                                                          Met Gln
                                                          1

ggg ccc tgg gtg ctg ctc ctg ctg ggc ctg agg cta cag ctc tcc ctg      106
Gly Pro Trp Val Leu Leu Leu Leu Gly Leu Arg Leu Gln Leu Ser Leu
        5                   10                  15

ggc atc atc cca gtt gag gag gag aac ccg gac ttc tgg aac cgc cag      154
Gly Ile Ile Pro Val Glu Glu Glu Asn Pro Asp Phe Trp Asn Arg Gln
    20                  25                  30

gca gcc gag gcc ctg ggt gcc gcc aag aag ctg cag cct gca cag aca      202
Ala Ala Glu Ala Leu Gly Ala Ala Lys Lys Leu Gln Pro Ala Gln Thr
35                  40                  45                  50

gcc gcc aag aac ctc atc atc ttc ctg ggt gac ggg atg ggg gtg tct      250
Ala Ala Lys Asn Leu Ile Ile Phe Leu Gly Asp Gly Met Gly Val Ser
                55                  60                  65

acg gtg aca gct gcc agg atc cta aaa ggg cag aag aag gac aaa ctg      298
Thr Val Thr Ala Ala Arg Ile Leu Lys Gly Gln Lys Lys Asp Lys Leu
            70                  75                  80

ggg cct gag acc ttc ctg gcc atg gac cgc ttc ccg tac gtg gct ctg      346
Gly Pro Glu Thr Phe Leu Ala Met Asp Arg Phe Pro Tyr Val Ala Leu
        85                  90                  95

tcc aag aca tac agt gta gac aag cat gtg cca gac agt gga gcc aca      394
Ser Lys Thr Tyr Ser Val Asp Lys His Val Pro Asp Ser Gly Ala Thr
    100                 105                 110

gcc acg gcc tac ctg tgc ggg gtc aag ggc aac ttc cag acc att ggc      442
Ala Thr Ala Tyr Leu Cys Gly Val Lys Gly Asn Phe Gln Thr Ile Gly
115                 120                 125                 130

ttg agt gca gcc gcc cgc ttt aac cag tgc aac acg aca cgc ggc aac      490
Leu Ser Ala Ala Ala Arg Phe Asn Gln Cys Asn Thr Thr Arg Gly Asn
                135                 140                 145

gag gtc atc tcc gtg gtg aat cgg gcc aag aaa gca gga aag tca gtg      538
Glu Val Ile Ser Val Val Asn Arg Ala Lys Lys Ala Gly Lys Ser Val
            150                 155                 160

gga gtg gta acc acc aca cgg gtg cag cat gcc tcg cca gcc ggc acc      586
Gly Val Val Thr Thr Thr Arg Val Gln His Ala Ser Pro Ala Gly Thr
        165                 170                 175

tac gcc cac acg gtg aac cgc aac tgg tac tcg gat gcc gac gtg cct      634
Tyr Ala His Thr Val Asn Arg Asn Trp Tyr Ser Asp Ala Asp Val Pro
    180                 185                 190

gcc tcg gcc cgc cag gag ggg tgc cag gac atc gcc acg cag ctc atc      682

Ala Ser Ala Arg Gln Glu Gly Cys Gln Asp Ile Ala Thr Gln Leu Ile
195                 200                 205                 210

tcc aac atg gac att gat gtg atc cta ggt gga ggc cga aag tac atg      730
Ser Asn Met Asp Ile Asp Val Ile Leu Gly Gly Gly Arg Lys Tyr Met
                215                 220                 225

ttt ccc atg ggg acc cca gac cct gag tac cca gat gac tac agc caa      778
Phe Pro Met Gly Thr Pro Asp Pro Glu Tyr Pro Asp Asp Tyr Ser Gln
            230                 235                 240

ggt ggg acc agg ctg gac ggg aag aat ctg gtg cag gaa tgg ctg gcg      826
Gly Gly Thr Arg Leu Asp Gly Lys Asn Leu Val Gln Glu Trp Leu Ala
        245                 250                 255

aag cac cag ggt gcc cgg tac gtg tgg aac cgc act gag ctc ctg cag      874
Lys His Gln Gly Ala Arg Tyr Val Trp Asn Arg Thr Glu Leu Leu Gln
    260                 265                 270

gct tcc ctg gac ccg tct gtg acc cat ctc atg ggt ctc ttt gag cct      922
Ala Ser Leu Asp Pro Ser Val Thr His Leu Met Gly Leu Phe Glu Pro
275                 280                 285                 290

gga gac atg aaa tac gag atc cac cga gac tcc aca ctg gac ccc tcc      970
Gly Asp Met Lys Tyr Glu Ile His Arg Asp Ser Thr Leu Asp Pro Ser
                295                 300                 305

ctg atg gag atg aca gag gct gcc ctg cgc ctg ctg agc agg aac ccc      1018
Leu Met Glu Met Thr Glu Ala Ala Leu Arg Leu Leu Ser Arg Asn Pro
            310                 315                 320

cgc ggc ttc ttc ctc ttc gtg gag ggt ggt cgc atc gac cat ggt cat      1066
Arg Gly Phe Phe Leu Phe Val Glu Gly Gly Arg Ile Asp His Gly His
        325                 330                 335

cat gaa agc agg gct tac cgg gca ctg act gag acg atc atg ttc gac      1114
His Glu Ser Arg Ala Tyr Arg Ala Leu Thr Glu Thr Ile Met Phe Asp
    340                 345                 350

gac gcc att gag agg gcg ggc cag ctc acc agc gag gag gac acg ctg      1162
Asp Ala Ile Glu Arg Ala Gly Gln Leu Thr Ser Glu Glu Asp Thr Leu
355                 360                 365                 370

agc ctc gtc act gcc gac cac tcc cac gtc ttc tcc ttc gga ggc tac      1210
Ser Leu Val Thr Ala Asp His Ser His Val Phe Ser Phe Gly Gly Tyr
                375                 380                 385

ccc ctg cga ggg agc tcc atc ttc ggg ctg gcc cct ggc aag gcc cgg      1258
Pro Leu Arg Gly Ser Ser Ile Phe Gly Leu Ala Pro Gly Lys Ala Arg
            390                 395                 400

gac agg aag gcc tac acg gtc ctc cta tac gga aac ggt cca ggc tat      1306
Asp Arg Lys Ala Tyr Thr Val Leu Leu Tyr Gly Asn Gly Pro Gly Tyr
        405                 410                 415

gtg ctc aag gac ggc gcc cgg ccg gat gtt acc gag agc gag agc ggg      1354
Val Leu Lys Asp Gly Ala Arg Pro Asp Val Thr Glu Ser Glu Ser Gly
    420                 425                 430

agc ccc gag tat cgg cag cag tca gca gtg ccc ctg gac gga gag acc      1402
Ser Pro Glu Tyr Arg Gln Gln Ser Ala Val Pro Leu Asp Gly Glu Thr
435                 440                 445                 450

cac gca ggc gag gac gtg gcg gtg ttc gcg cgc ggc ccg cag gcg cac      1450
His Ala Gly Glu Asp Val Ala Val Phe Ala Arg Gly Pro Gln Ala His
                455                 460                 465

ctg gtt cac ggc gtg cag gag cag acc ttc ata gcg cac gtc atg gcc      1498

Leu Val His Gly Val Gln Glu Gln Thr Phe Ile Ala His Val Met Ala
            470                 475                 480

ttc gcc gcc tgc ctg gag ccc tac acc gcc tgc gac ctg gcg ccc ccc      1546
Phe Ala Ala Cys Leu Glu Pro Tyr Thr Ala Cys Asp Leu Ala Pro Pro
        485                 490                 495

gcc ggc acc acc gac gcc gcg cac ccg ggg ccg tcc gtg gtc ccc gcg      1594
Ala Gly Thr Thr Asp Ala Ala His Pro Gly Pro Ser Val Val Pro Ala
    500                 505                 510

ttg ctt cct ctg ctg gca ggg acc ttg ctg ctg ctg ggg acg gcc act      1642
Leu Leu Pro Leu Leu Ala Gly Thr Leu Leu Leu Leu Gly Thr Ala Thr
515                 520                 525                 530

gct ccc tga gtgtcccgtc cctgggggct cctgcttccc catcccggag              1691
Ala Pro

ttcccctgct ccccacctcc agttctgcct gccggacctc cacctggagc tgtcaccccc    1751

ggagtcgcca cacagactgt cctgccatgg aaccttccct cccggtgcac cctggggacc    1811

gagcccttga caccacgccc tttgctttat cttgctctta aattttggcc ccaactccag    1871

ggactgggga tttgtgcctg gcagctgcct gcatttcagg aaaagaggag gctcagacca    1931

tccagccccc cgcccatatc ctgaggtgga tcaggcaggc tctctccccg gggacatgag    1991

gcacccatac ctaggacccc ctgcgccttt tttagcttca gtcatggcag cacctgaggg    2051

acacaaggac ttgggtgcat caggacgcct tggagaagcg tggcttcctg ccaccctgca    2111

acccaccctc ccagccaagg aggctgctgt ggtggggatc cccaggggct ttgacacagt    2171

cctctgctgt ccctccactg ggctaattct acacccctgt cgcctcctag gggcccatga     2231

gtcagagagg cttgccccaa gtcacagcca ctcagatgtt cgacgccccc taaggtccat    2291

tccagcaccc acctgagttc cgaggagcac ctgggaagct ctgggtgcag gatagcagtc    2351

cagagtccat ggccccgcct aggccatctg ggtgctgggc atggatttct cagcaaggaa    2411

gactcattac cttccctccc tgggcctcca ttcttctggg aaacacaaag caataataaa    2471

aggaagtgtt agac                                                      2485

<210>  26
<211>  532
<212>  PRT
<213>  Homo sapiens

<400>  26

Met Gln Gly Pro Trp Val Leu Leu Leu Leu Gly Leu Arg Leu Gln Leu
1               5                   10                  15

Ser Leu Gly Ile Ile Pro Val Glu Glu Glu Asn Pro Asp Phe Trp Asn
            20                  25                  30

Arg Gln Ala Ala Glu Ala Leu Gly Ala Ala Lys Lys Leu Gln Pro Ala
        35                  40                  45

Gln Thr Ala Ala Lys Asn Leu Ile Ile Phe Leu Gly Asp Gly Met Gly

50                  55                  60

Val Ser Thr Val Thr Ala Ala Arg Ile Leu Lys Gly Gln Lys Lys Asp
65                  70                  75                  80

Lys Leu Gly Pro Glu Thr Phe Leu Ala Met Asp Arg Phe Pro Tyr Val
                85                  90                  95

Ala Leu Ser Lys Thr Tyr Ser Val Asp Lys His Val Pro Asp Ser Gly
            100                 105                 110

Ala Thr Ala Thr Ala Tyr Leu Cys Gly Val Lys Gly Asn Phe Gln Thr
        115                 120                 125

Ile Gly Leu Ser Ala Ala Ala Arg Phe Asn Gln Cys Asn Thr Thr Arg
    130                 135                 140

Gly Asn Glu Val Ile Ser Val Val Asn Arg Ala Lys Lys Ala Gly Lys
145                 150                 155                 160

Ser Val Gly Val Val Thr Thr Thr Arg Val Gln His Ala Ser Pro Ala
                165                 170                 175

Gly Thr Tyr Ala His Thr Val Asn Arg Asn Trp Tyr Ser Asp Ala Asp
            180                 185                 190

Val Pro Ala Ser Ala Arg Gln Glu Gly Cys Gln Asp Ile Ala Thr Gln
        195                 200                 205

Leu Ile Ser Asn Met Asp Ile Asp Val Ile Leu Gly Gly Gly Arg Lys
    210                 215                 220

Tyr Met Phe Pro Met Gly Thr Pro Asp Pro Glu Tyr Pro Asp Asp Tyr
225                 230                 235                 240

Ser Gln Gly Gly Thr Arg Leu Asp Gly Lys Asn Leu Val Gln Glu Trp
                245                 250                 255

Leu Ala Lys His Gln Gly Ala Arg Tyr Val Trp Asn Arg Thr Glu Leu
            260                 265                 270

Leu Gln Ala Ser Leu Asp Pro Ser Val Thr His Leu Met Gly Leu Phe
        275                 280                 285

Glu Pro Gly Asp Met Lys Tyr Glu Ile His Arg Asp Ser Thr Leu Asp
    290                 295                 300

Pro Ser Leu Met Glu Met Thr Glu Ala Ala Leu Arg Leu Leu Ser Arg
305                 310                 315                 320

Asn Pro Arg Gly Phe Phe Leu Phe Val Glu Gly Gly Arg Ile Asp His

325                 330                 335

Gly His His Glu Ser Arg Ala Tyr Arg Ala Leu Thr Glu Thr Ile Met
            340                 345                 350

Phe Asp Asp Ala Ile Glu Arg Ala Gly Gln Leu Thr Ser Glu Glu Asp
        355                 360                 365

Thr Leu Ser Leu Val Thr Ala Asp His Ser His Val Phe Ser Phe Gly
    370                 375                 380

Gly Tyr Pro Leu Arg Gly Ser Ser Ile Phe Gly Leu Ala Pro Gly Lys
385                 390                 395                 400

Ala Arg Asp Arg Lys Ala Tyr Thr Val Leu Leu Tyr Gly Asn Gly Pro
                405                 410                 415

Gly Tyr Val Leu Lys Asp Gly Ala Arg Pro Asp Val Thr Glu Ser Glu
            420                 425                 430

Ser Gly Ser Pro Glu Tyr Arg Gln Gln Ser Ala Val Pro Leu Asp Gly
        435                 440                 445

Glu Thr His Ala Gly Glu Asp Val Ala Val Phe Ala Arg Gly Pro Gln
    450                 455                 460

Ala His Leu Val His Gly Val Gln Glu Gln Thr Phe Ile Ala His Val
465                 470                 475                 480

Met Ala Phe Ala Ala Cys Leu Glu Pro Tyr Thr Ala Cys Asp Leu Ala
                485                 490                 495

Pro Pro Ala Gly Thr Thr Asp Ala Ala His Pro Gly Pro Ser Val Val
            500                 505                 510

Pro Ala Leu Leu Pro Leu Leu Ala Gly Thr Leu Leu Leu Leu Gly Thr
        515                 520                 525

Ala Thr Ala Pro
    530

<210>  27
<211>  4665
<212>  DNA
<213>  Homo sapiens

<220>
<221>  CDS
<222>  (73)..(3117)

<400>  27
agccagcccg aggacgcgag cggcaggtgt gcacagaggt tctccacttt gttttctgaa      60

ctcgcggtca gg atg gtt ttc tct gtc agg cag tgt ggc cat gtt ggc aga 111
Met Val Phe Ser Val Arg Gln Cys Gly His Val Gly Arg
1               5                   10

act gaa gaa gtt tta ctg acg ttc aag ata ttc ctt gtc atc att tgt 159
Thr Glu Glu Val Leu Leu Thr Phe Lys Ile Phe Leu Val Ile Ile Cys
    15                  20                  25

ctt cat gtc gtt ctg gta aca tcc ctg gaa gaa gat act gat aat tcc 207
Leu His Val Val Leu Val Thr Ser Leu Glu Glu Asp Thr Asp Asn Ser
30                  35                  40                  45

agt ttg tca cca cca cct gct aaa tta tct gtt gtc agt ttt gcc ccc 255
Ser Leu Ser Pro Pro Pro Ala Lys Leu Ser Val Val Ser Phe Ala Pro
                50                  55                  60

tcc tcc aat gag gtt gaa aca aca agc ctc aat gat gtt act tta agc 303
Ser Ser Asn Glu Val Glu Thr Thr Ser Leu Asn Asp Val Thr Leu Ser
            65                  70                  75

tta ctc cct tca aac gaa aca gaa aaa act aaa atc act ata gta aaa 351
Leu Leu Pro Ser Asn Glu Thr Glu Lys Thr Lys Ile Thr Ile Val Lys
        80                  85                  90

acc ttc aat gct tca ggc gtc aaa ccc cag aga aat atc tgc aat ttg 399
Thr Phe Asn Ala Ser Gly Val Lys Pro Gln Arg Asn Ile Cys Asn Leu
    95                  100                 105

tca tct att tgc aat gac tca gca ttt ttt aga ggt gag atc atg ttt 447
Ser Ser Ile Cys Asn Asp Ser Ala Phe Phe Arg Gly Glu Ile Met Phe
110                 115                 120                 125

caa tat gat aaa gaa agc act gtt ccc cag aat caa cat ata acg aat 495
Gln Tyr Asp Lys Glu Ser Thr Val Pro Gln Asn Gln His Ile Thr Asn
                130                 135                 140

ggc acc tta act gga gtc ctg tct cta agt gaa tta aaa cgc tca gag 543
Gly Thr Leu Thr Gly Val Leu Ser Leu Ser Glu Leu Lys Arg Ser Glu
            145                 150                 155

ctc aac aaa acc ctg caa acc cta agt gag act tac ttt ata atg tgt 591
Leu Asn Lys Thr Leu Gln Thr Leu Ser Glu Thr Tyr Phe Ile Met Cys
        160                 165                 170

gct aca gca gag gcc caa agc aca tta aat tgt aca ttc aca ata aaa 639
Ala Thr Ala Glu Ala Gln Ser Thr Leu Asn Cys Thr Phe Thr Ile Lys
    175                 180                 185

ctg aat aat aca atg aat gca tgt gct gca ata gcc gct ttg gaa aga 687
Leu Asn Asn Thr Met Asn Ala Cys Ala Ala Ile Ala Ala Leu Glu Arg
190                 195                 200                 205

gta aag att cga cca atg gaa cac tgc tgc tgt tct gtc agg ata ccc 735
Val Lys Ile Arg Pro Met Glu His Cys Cys Cys Ser Val Arg Ile Pro
                210                 215                 220

tgc cct tcc tcc cca gaa gag ttg gga aag ctt cag tgt gac ctg cag 783
Cys Pro Ser Ser Pro Glu Glu Leu Gly Lys Leu Gln Cys Asp Leu Gln
            225                 230                 235

gat ccc att gtc tgt ctt gct gac cat cca cgt ggc cca cca ttt tct 831
Asp Pro Ile Val Cys Leu Ala Asp His Pro Arg Gly Pro Pro Phe Ser
        240                 245                 250

tcc agc caa tcc atc cca gtg gtg cct cgg gcc act gtg ctt tcc cag 879
Ser Ser Gln Ser Ile Pro Val Val Pro Arg Ala Thr Val Leu Ser Gln
    255                 260                 265

gtc ccc aaa gct acc tct ttt gct gag cct cca gat tat tca cct gtg 927
Val Pro Lys Ala Thr Ser Phe Ala Glu Pro Pro Asp Tyr Ser Pro Val
270                 275                 280                 285

acc cac aat gtt ccc tct cca ata ggg gag att caa ccc ctt tca ccc 975
Thr His Asn Val Pro Ser Pro Ile Gly Glu Ile Gln Pro Leu Ser Pro
                290                 295                 300

cag cct tca gct ccc ata gct tcc agc cct gcc att gac atg ccc cca 1023
Gln Pro Ser Ala Pro Ile Ala Ser Ser Pro Ala Ile Asp Met Pro Pro
            305                 310                 315

cag tct gaa acg atc tct tcc cct atg ccc caa acc cat gtc tcc ggc 1071
Gln Ser Glu Thr Ile Ser Ser Pro Met Pro Gln Thr His Val Ser Gly
        320                 325                 330

acc cca cct cct gtg aaa gcc tca ttt tcc tct ccc acc gtg tct gcc 1119
Thr Pro Pro Pro Val Lys Ala Ser Phe Ser Ser Pro Thr Val Ser Ala
    335                 340                 345

cct gcg aat gtc aac act acc agc gca cct cct gtc cag aca gac atc 1167
Pro Ala Asn Val Asn Thr Thr Ser Ala Pro Pro Val Gln Thr Asp Ile
350                 355                 360                 365

gtc aac acc agc agt att tct gat ctt gag aac caa gtg ttg cag atg 1215
Val Asn Thr Ser Ser Ile Ser Asp Leu Glu Asn Gln Val Leu Gln Met
                370                 375                 380

gag aag gct ctg tcc ttg ggc agc ctg gag cct aac ctc gca gga gaa 1263
Glu Lys Ala Leu Ser Leu Gly Ser Leu Glu Pro Asn Leu Ala Gly Glu
            385                 390                 395

atg atc aac caa gtc agc aga ctc ctt cat tcc ccg cct gac atg ctg 1311
Met Ile Asn Gln Val Ser Arg Leu Leu His Ser Pro Pro Asp Met Leu
        400                 405                 410

gcc cct ctg gct caa aga ttg ctg aaa gta gtg gat gac att ggc cta 1359
Ala Pro Leu Ala Gln Arg Leu Leu Lys Val Val Asp Asp Ile Gly Leu
    415                 420                 425

cag ctg aac ttt tca aac acg act ata agt cta acc tcc cct tct ttg 1407
Gln Leu Asn Phe Ser Asn Thr Thr Ile Ser Leu Thr Ser Pro Ser Leu
430                 435                 440                 445

gct ctg gct gtg atc aga gtg aat gcc agt agt ttc aac aca act acc 1455
Ala Leu Ala Val Ile Arg Val Asn Ala Ser Ser Phe Asn Thr Thr Thr
                450                 455                 460

ttt gtg gcc caa gac cct gca aat ctt cag gtt tct ctg gaa acc caa 1503
Phe Val Ala Gln Asp Pro Ala Asn Leu Gln Val Ser Leu Glu Thr Gln
            465                 470                 475

gct cct gag aac agt att ggc aca att act ctt cct tca tcg ctg atg 1551
Ala Pro Glu Asn Ser Ile Gly Thr Ile Thr Leu Pro Ser Ser Leu Met
        480                 485                 490

aat aat tta cca gct cat gac atg gag cta gct tcc agg gtt cag ttc 1599
Asn Asn Leu Pro Ala His Asp Met Glu Leu Ala Ser Arg Val Gln Phe
    495                 500                 505

aat ttt ttt gaa aca cct gct ttg ttt cag gat cct tcc ctg gag aac 1647
Asn Phe Phe Glu Thr Pro Ala Leu Phe Gln Asp Pro Ser Leu Glu Asn
510                 515                 520                 525

ctc tct ctg atc agc tac gtc ata tca tcg agt gtt gca aac ctg acc 1695
Leu Ser Leu Ile Ser Tyr Val Ile Ser Ser Ser Val Ala Asn Leu Thr
                530                 535                 540

gtc agg aac ttg aca aga aac gtg aca gtc aca tta aag cac atc aac 1743
Val Arg Asn Leu Thr Arg Asn Val Thr Val Thr Leu Lys His Ile Asn
            545                 550                 555

ccg agc cag gat gag tta aca gtg aga tgt gta ttt tgg gac ttg ggc 1791
Pro Ser Gln Asp Glu Leu Thr Val Arg Cys Val Phe Trp Asp Leu Gly
        560                 565                 570

aga aat ggt ggc aga gga ggc tgg tca gac aat ggc tgc tct gtc aaa 1839
Arg Asn Gly Gly Arg Gly Gly Trp Ser Asp Asn Gly Cys Ser Val Lys
    575                 580                 585

gac agg aga ttg aat gaa acc atc tgt acc tgt agc cat cta aca agc 1887
Asp Arg Arg Leu Asn Glu Thr Ile Cys Thr Cys Ser His Leu Thr Ser
590                 595                 600                 605

ttc ggc gtt ctg ctg gac cta tct agg aca tct gtg ctg cct gct caa 1935
Phe Gly Val Leu Leu Asp Leu Ser Arg Thr Ser Val Leu Pro Ala Gln
                610                 615                 620

atg atg gct ctg acg ttc att aca tat att ggt tgt ggg ctt tca tca 1983
Met Met Ala Leu Thr Phe Ile Thr Tyr Ile Gly Cys Gly Leu Ser Ser
            625                 630                 635

att ttt ctg tca gtg act ctt gta acc tac ata gct ttt gaa aag atc 2031
Ile Phe Leu Ser Val Thr Leu Val Thr Tyr Ile Ala Phe Glu Lys Ile
        640                 645                 650

cgg agg gat tac cct tcc aaa atc ctc atc cag ctg tgt gct gct ctg 2079
Arg Arg Asp Tyr Pro Ser Lys Ile Leu Ile Gln Leu Cys Ala Ala Leu
    655                 660                 665

ctt ctg ctg aac ctg gtc ttc ctc ctg gac tcg tgg att gct ctg tat 2127
Leu Leu Leu Asn Leu Val Phe Leu Leu Asp Ser Trp Ile Ala Leu Tyr
670                 675                 680                 685

aag atg caa ggc ctc tgc atc tca gtg gct gta ttt ctt cat tat ttt 2175
Lys Met Gln Gly Leu Cys Ile Ser Val Ala Val Phe Leu His Tyr Phe
                690                 695                 700

ctc ttg gtc tca ttc aca tgg atg ggc cta gaa gca ttc cat atg tac 2223
Leu Leu Val Ser Phe Thr Trp Met Gly Leu Glu Ala Phe His Met Tyr
            705                 710                 715

ctg gcc ctt gtc aaa gta ttt aat act tac atc cga aaa tac atc ctt 2271
Leu Ala Leu Val Lys Val Phe Asn Thr Tyr Ile Arg Lys Tyr Ile Leu
        720                 725                 730

aaa ttc tgc att gtc ggt tgg ggg gta cca gct gtg gtt gtg acc atc 2319
Lys Phe Cys Ile Val Gly Trp Gly Val Pro Ala Val Val Val Thr Ile
    735                 740                 745

atc ctg act ata tcc cca gat aac tat ggg ctt gga tcc tat ggg aaa 2367
Ile Leu Thr Ile Ser Pro Asp Asn Tyr Gly Leu Gly Ser Tyr Gly Lys
750                 755                 760                 765

ttc ccc aat ggt tca ccg gat gac ttc tgc tgg atc aac aac aat gca 2415
Phe Pro Asn Gly Ser Pro Asp Asp Phe Cys Trp Ile Asn Asn Asn Ala
                770                 775                 780

gta ttc tac att acg gtg gtg gga tat ttc tgt gtg ata ttt ttg ctg 2463
Val Phe Tyr Ile Thr Val Val Gly Tyr Phe Cys Val Ile Phe Leu Leu
            785                 790                 795

aac gtc agc atg ttc att gtg gtc ctg gtt cag ctc tgt cga att aaa 2511
Asn Val Ser Met Phe Ile Val Val Leu Val Gln Leu Cys Arg Ile Lys
        800                 805                 810

aag aag aag caa ctg gga gcc cag cga aaa acc agt att caa gac ctc 2559
Lys Lys Lys Gln Leu Gly Ala Gln Arg Lys Thr Ser Ile Gln Asp Leu
    815                 820                 825

agg agt atc gct ggc ctt aca ttt tta ctg gga ata act tgg ggc ttt 2607
Arg Ser Ile Ala Gly Leu Thr Phe Leu Leu Gly Ile Thr Trp Gly Phe
830                 835                 840                 845

gcc ttc ttt gcc tgg gga cca gtt aac gtg acc ttc atg tat ctg ttt 2655
Ala Phe Phe Ala Trp Gly Pro Val Asn Val Thr Phe Met Tyr Leu Phe
                850                 855                 860

gcc atc ttt aat acc tta caa gga ttt ttc ata ttc atc ttt tac tgt 2703
Ala Ile Phe Asn Thr Leu Gln Gly Phe Phe Ile Phe Ile Phe Tyr Cys
            865                 870                 875

gtg gcc aaa gaa aat gtc agg aag caa tgg agg cgg tat ctt tgt tgt 2751
Val Ala Lys Glu Asn Val Arg Lys Gln Trp Arg Arg Tyr Leu Cys Cys
        880                 885                 890

gga aag tta cgg ctg gct gaa aat tct gac tgg agt aaa act gct act 2799
Gly Lys Leu Arg Leu Ala Glu Asn Ser Asp Trp Ser Lys Thr Ala Thr
    895                 900                 905

aat ggt tta aag aag cag act gta aac caa gga gtg tcc agc tct tca 2847
Asn Gly Leu Lys Lys Gln Thr Val Asn Gln Gly Val Ser Ser Ser Ser
910                 915                 920                 925

aat tcc tta cag tca agc agt aac tcc act aac tcc acc aca ctg cta 2895
Asn Ser Leu Gln Ser Ser Ser Asn Ser Thr Asn Ser Thr Thr Leu Leu
                930                 935                 940

gtg aat aat gat tgc tca gta cac gca agc ggg aat gga aat gct tct 2943
Val Asn Asn Asp Cys Ser Val His Ala Ser Gly Asn Gly Asn Ala Ser
            945                 950                 955

aca gag agg aat ggg gtc tct ttt agt gtt cag aat gga gat gtg tgc 2991
Thr Glu Arg Asn Gly Val Ser Phe Ser Val Gln Asn Gly Asp Val Cys
        960                 965                 970

ctt cac gat ttc act gga aaa cag cac atg ttt aac gag aag gaa gat 3039
Leu His Asp Phe Thr Gly Lys Gln His Met Phe Asn Glu Lys Glu Asp
    975                 980                 985

tcc tgc aat ggg aaa ggc cgt atg gct ctc aga agg act tca aag cgg 3087
Ser Cys Asn Gly Lys Gly Arg Met Ala Leu Arg Arg Thr Ser Lys Arg
990                 995                 1000                1005

gga agc tta cac ttt att gag caa atg tga ttcctttctt ctaaaatcaa 3137
Gly Ser Leu His Phe Ile Glu Gln Met
                1010

agcatgatgc ttgacagtgt gaaatgtcca attttacctt ttacacaatg tgagatgtat 3197

gaaaatcaac tcattttatt ctcggcaaca tctggagaag cataagctaa ttaagggcga 3257

tgattattat tacaagaaga aaccaagaca ttacaccatg gtttttagac atttctgatt 3317

tggtttctta tctttcattt tataagaagg ttggttttaa acaatacact aagaatgact 3377

cctataaaga aaacaaaaaa aggtagtgaa ctttcagcta ccttttaaag aggctaagtt 3437

atctttgata acatcatata aagcaactgt tgacttcagc ctgttggtga gtttagttgt 3497

gcatgccttt gttgtatata agctaaattc tagtgaccca tgtgtcaaaa atcttacttc 3557

tacatttttt tgtatttatt ttctactgtg taaatgtatt cctttgtaga atcatggttg 3617

ttttgtctca cgtgataatt cagaaaatcc ttgctcgttc cgcaaatcct aaagctcctt 3677
ttggagatga tataggatgt gaaatacaga aacctcagtg aaatcaagaa ataatgatcc 3737
cagccagact gagaaaatgt aagcagacag tgccacagtt agctcataca gtgcctttga 3797
gcaagttagg aaaagatgcc cccactgggc agacacagcc ctatgggtca tggtttgaca 3857
aacagagtga gagaccatat tttagcccca ctcaccctct tgggtgcacg acctgtacag 3917
ccaaacacag catccaatat gaatacccat cccctgaccg catccccagt agtcagatta 3977
tagaatctgc accaagatgt ttagctttat accttggcca cagagaggga tgaactgtca 4037
tccagaccat gtgtcaggaa aattgtgaac gtagatgagg tacatacact gccgcttctc 4097
aaatccccag agcctttagg aacaggagag tagactagga ttccttctct taaaaaggta 4157
catatatatg gaaaaaaatc atattgccgt tctttaaaag gcaactgcat ggtacattgt 4217
tgattgttat gactggtaca ctctggccca gccagagcta taattgtttt ttaaatgtgt 4277
cttgaagaat gcacagtgac aaggggagta gctattggga acagggaact gtcctacact 4337
gctattgttg ctacatgtat cgagccttga ttgctcctag ttatatacag ggtctatctt 4397
gcttcctacc tacatctgct tgagcagtgc ctcaagtaca tccttattag gaacatttca 4457
aacccctttt agttaagtct ttcactaagg ttctcttgca tatatttcaa gtgaatgttg 4517
gatctcagac taaccatagt aataatacac atttctgtga gtgctgactt gtctttgcaa 4577
tatttctttt ctgatttatt taattttctt gtatttatat gttaaaatca aaaatgttaa 4637
aatcaatgaa ataaatttgc agttaaga 4665

<210> 28
<211> 1014
<212> PRT
<213> Homo sapiens

<400> 28

Met Val Phe Ser Val Arg Gln Cys Gly His Val Gly Arg Thr Glu Glu
1               5                   10                  15

Val Leu Leu Thr Phe Lys Ile Phe Leu Val Ile Ile Cys Leu His Val
            20                  25                  30

Val Leu Val Thr Ser Leu Glu Glu Asp Thr Asp Asn Ser Ser Leu Ser
        35                  40                  45

Pro Pro Pro Ala Lys Leu Ser Val Val Ser Phe Ala Pro Ser Ser Asn
    50                  55                  60

Glu Val Glu Thr Thr Ser Leu Asn Asp Val Thr Leu Ser Leu Leu Pro
65                  70                  75                  80

Ser Asn Glu Thr Glu Lys Thr Lys Ile Thr Ile Val Lys Thr Phe Asn
                85                  90                  95

Ala Ser Gly Val Lys Pro Gln Arg Asn Ile Cys Asn Leu Ser Ser Ile
            100                 105                 110

Cys Asn Asp Ser Ala Phe Phe Arg Gly Glu Ile Met Phe Gln Tyr Asp
        115                 120                 125

Lys Glu Ser Thr Val Pro Gln Asn Gln His Ile Thr Asn Gly Thr Leu
    130                 135                 140

Thr Gly Val Leu Ser Leu Ser Glu Leu Lys Arg Ser Glu Leu Asn Lys
145                 150                 155                 160

Thr Leu Gln Thr Leu Ser Glu Thr Tyr Phe Ile Met Cys Ala Thr Ala
                165                 170                 175

Glu Ala Gln Ser Thr Leu Asn Cys Thr Phe Thr Ile Lys Leu Asn Asn
            180                 185                 190

Thr Met Asn Ala Cys Ala Ala Ile Ala Ala Leu Glu Arg Val Lys Ile
        195                 200                 205

Arg Pro Met Glu His Cys Cys Cys Ser Val Arg Ile Pro Cys Pro Ser
    210                 215                 220

Ser Pro Glu Glu Leu Gly Lys Leu Gln Cys Asp Leu Gln Asp Pro Ile
225                 230                 235                 240

Val Cys Leu Ala Asp His Pro Arg Gly Pro Pro Phe Ser Ser Ser Gln
                245                 250                 255

Ser Ile Pro Val Val Pro Arg Ala Thr Val Leu Ser Gln Val Pro Lys
            260                 265                 270

Ala Thr Ser Phe Ala Glu Pro Pro Asp Tyr Ser Pro Val Thr His Asn
        275                 280                 285

Val Pro Ser Pro Ile Gly Glu Ile Gln Pro Leu Ser Pro Gln Pro Ser
    290                 295                 300

Ala Pro Ile Ala Ser Ser Pro Ala Ile Asp Met Pro Pro Gln Ser Glu
305                 310                 315                 320

Thr Ile Ser Ser Pro Met Pro Gln Thr His Val Ser Gly Thr Pro Pro
                325                 330                 335

Pro Val Lys Ala Ser Phe Ser Ser Pro Thr Val Ser Ala Pro Ala Asn
            340                 345                 350

Val Asn Thr Thr Ser Ala Pro Pro Val Gln Thr Asp Ile Val Asn Thr
        355                 360                 365

Ser Ser Ile Ser Asp Leu Glu Asn Gln Val Leu Gln Met Glu Lys Ala
    370                 375                 380

Leu Ser Leu Gly Ser Leu Glu Pro Asn Leu Ala Gly Glu Met Ile Asn
385                 390                 395                 400

Gln Val Ser Arg Leu Leu His Ser Pro Pro Asp Met Leu Ala Pro Leu
                405                 410                 415

Ala Gln Arg Leu Leu Lys Val Val Asp Asp Ile Gly Leu Gln Leu Asn
            420                 425                 430

Phe Ser Asn Thr Thr Ile Ser Leu Thr Ser Pro Ser Leu Ala Leu Ala
        435                 440                 445

Val Ile Arg Val Asn Ala Ser Ser Phe Asn Thr Thr Thr Phe Val Ala
    450                 455                 460

Gln Asp Pro Ala Asn Leu Gln Val Ser Leu Glu Thr Gln Ala Pro Glu
465                 470                 475                 480

Asn Ser Ile Gly Thr Ile Thr Leu Pro Ser Ser Leu Met Asn Asn Leu
                485                 490                 495

Pro Ala His Asp Met Glu Leu Ala Ser Arg Val Gln Phe Asn Phe Phe
            500                 505                 510

Glu Thr Pro Ala Leu Phe Gln Asp Pro Ser Leu Glu Asn Leu Ser Leu
        515                 520                 525

Ile Ser Tyr Val Ile Ser Ser Ser Val Ala Asn Leu Thr Val Arg Asn
    530                 535                 540

Leu Thr Arg Asn Val Thr Val Thr Leu Lys His Ile Asn Pro Ser Gln
545                 550                 555                 560

Asp Glu Leu Thr Val Arg Cys Val Phe Trp Asp Leu Gly Arg Asn Gly
                565                 570                 575

Gly Arg Gly Gly Trp Ser Asp Asn Gly Cys Ser Val Lys Asp Arg Arg
            580                 585                 590

Leu Asn Glu Thr Ile Cys Thr Cys Ser His Leu Thr Ser Phe Gly Val
        595                 600                 605

Leu Leu Asp Leu Ser Arg Thr Ser Val Leu Pro Ala Gln Met Met Ala
    610                 615                 620

Leu Thr Phe Ile Thr Tyr Ile Gly Cys Gly Leu Ser Ser Ile Phe Leu
625                 630                 635                 640

Ser Val Thr Leu Val Thr Tyr Ile Ala Phe Glu Lys Ile Arg Arg Asp
                645                 650                 655

Tyr Pro Ser Lys Ile Leu Ile Gln Leu Cys Ala Ala Leu Leu Leu Leu
            660                 665                 670

Asn Leu Val Phe Leu Leu Asp Ser Trp Ile Ala Leu Tyr Lys Met Gln
        675                 680                 685

Gly Leu Cys Ile Ser Val Ala Val Phe Leu His Tyr Phe Leu Leu Val
    690                 695                 700

Ser Phe Thr Trp Met Gly Leu Glu Ala Phe His Met Tyr Leu Ala Leu
705                 710                 715                 720

Val Lys Val Phe Asn Thr Tyr Ile Arg Lys Tyr Ile Leu Lys Phe Cys
                725                 730                 735

Ile Val Gly Trp Gly Val Pro Ala Val Val Val Thr Ile Ile Leu Thr
            740                 745                 750

Ile Ser Pro Asp Asn Tyr Gly Leu Gly Ser Tyr Gly Lys Phe Pro Asn
        755                 760                 765

Gly Ser Pro Asp Asp Phe Cys Trp Ile Asn Asn Asn Ala Val Phe Tyr
    770                 775                 780

Ile Thr Val Val Gly Tyr Phe Cys Val Ile Phe Leu Leu Asn Val Ser
785                 790                 795                 800

Met Phe Ile Val Val Leu Val Gln Leu Cys Arg Ile Lys Lys Lys Lys
                805                 810                 815

Gln Leu Gly Ala Gln Arg Lys Thr Ser Ile Gln Asp Leu Arg Ser Ile
            820                 825                 830

Ala Gly Leu Thr Phe Leu Leu Gly Ile Thr Trp Gly Phe Ala Phe Phe
        835                 840                 845

Ala Trp Gly Pro Val Asn Val Thr Phe Met Tyr Leu Phe Ala Ile Phe
    850                 855                 860

Asn Thr Leu Gln Gly Phe Phe Ile Phe Ile Phe Tyr Cys Val Ala Lys
865                 870                 875                 880

Glu Asn Val Arg Lys Gln Trp Arg Arg Tyr Leu Cys Cys Gly Lys Leu
                885                 890                 895

Arg Leu Ala Glu Asn Ser Asp Trp Ser Lys Thr Ala Thr Asn Gly Leu
            900                 905                 910

Lys Lys Gln Thr Val Asn Gln Gly Val Ser Ser Ser Ser Asn Ser Leu
        915                 920                 925

Gln Ser Ser Ser Asn Ser Thr Asn Ser Thr Thr Leu Leu Val Asn Asn
    930                 935                 940

Asp Cys Ser Val His Ala Ser Gly Asn Gly Asn Ala Ser Thr Glu Arg
945                 950                 955                 960

Asn Gly Val Ser Phe Ser Val Gln Asn Gly Asp Val Cys Leu His Asp
                965                 970                 975

Phe Thr Gly Lys Gln His Met Phe Asn Glu Lys Glu Asp Ser Cys Asn
            980                 985                 990

Gly Lys Gly Arg Met Ala Leu Arg  Arg Thr Ser Lys Arg  Gly Ser Leu
        995                 1000                 1005

His Phe  Ile Glu Gln Met
    1010

<210>  29
<211>  3362
<212>  DNA
<213>  Homo sapiens

<220>
<221>  CDS
<222>  (20)..(3319)

<400>  29
aaaaaatcaa ttttggaag atg tca ctg aac aac tct tcc aat gta ttt ctg       52
                     Met Ser Leu Asn Asn Ser Ser Asn Val Phe Leu
                     1               5                   10

gat tca gtg ccc agt aat acc aat cgc ttt caa gtt agt gtc ata aat       100
Asp Ser Val Pro Ser Asn Thr Asn Arg Phe Gln Val Ser Val Ile Asn
            15                  20                  25

gag aac cat gag agc agt gca gct gca gat gac aat act gac cca cca       148
Glu Asn His Glu Ser Ser Ala Ala Ala Asp Asp Asn Thr Asp Pro Pro
        30                  35                  40

cat tat gaa gaa acc tct ttt ggg gat gaa gct cag aaa aga ctc aga       196
His Tyr Glu Glu Thr Ser Phe Gly Asp Glu Ala Gln Lys Arg Leu Arg
    45                  50                  55

atc agc ttt agg cct ggg aat cag gag tgc tat gac aat ttc ctc cac       244
Ile Ser Phe Arg Pro Gly Asn Gln Glu Cys Tyr Asp Asn Phe Leu His
60                  65                  70                  75

agt gga gaa act gct aaa aca gat gcc agt ttt cac gct tat gat tct       292
Ser Gly Glu Thr Ala Lys Thr Asp Ala Ser Phe His Ala Tyr Asp Ser
                80                  85                  90

cac aca aac aca tac tat cta caa act ttt ggc cac aac acc atg gat       340
His Thr Asn Thr Tyr Tyr Leu Gln Thr Phe Gly His Asn Thr Met Asp
            95                  100                 105

gcc gtt ccc aag ata gag tac tat cgt aac acc ggc agc atc agt ggg       388

Ala Val Pro Lys Ile Glu Tyr Tyr Arg Asn Thr Gly Ser Ile Ser Gly
        110                 115                 120

ccc aag gtc aac cga ccc agc ctg ctt gag att cac gag caa ctc gca      436
Pro Lys Val Asn Arg Pro Ser Leu Leu Glu Ile His Glu Gln Leu Ala
    125                 130                 135

aag aat gtg gca gtc acc cca agt tca gct gac aga gtt gct aac ggt      484
Lys Asn Val Ala Val Thr Pro Ser Ser Ala Asp Arg Val Ala Asn Gly
140                 145                 150                 155

gat ggg ata cct gga gat gaa caa gct gaa aat aag gaa gat gat caa      532
Asp Gly Ile Pro Gly Asp Glu Gln Ala Glu Asn Lys Glu Asp Asp Gln
                160                 165                 170

gct ggt gtt gtg aag ttt gga tgg gtg aaa ggt gtg ctg gta aga tgc      580
Ala Gly Val Val Lys Phe Gly Trp Val Lys Gly Val Leu Val Arg Cys
            175                 180                 185

atg ctg aac atc tgg gga gtc atg ctc ttc att cgc ctc tcc tgg att      628
Met Leu Asn Ile Trp Gly Val Met Leu Phe Ile Arg Leu Ser Trp Ile
        190                 195                 200

gtt gga gaa gct gga att ggt ctt gga gtt atc atc att ggc cta tcc      676
Val Gly Glu Ala Gly Ile Gly Leu Gly Val Ile Ile Ile Gly Leu Ser
    205                 210                 215

acc ata gta acg aca atc aca ggt atg tcc acg tct gct att gcc acg      724
Thr Ile Val Thr Thr Ile Thr Gly Met Ser Thr Ser Ala Ile Ala Thr
220                 225                 230                 235

aac gga gtt gtt aga gga ggt ggg gcc tac tat ctt att tcc aga agt      772
Asn Gly Val Val Arg Gly Gly Gly Ala Tyr Tyr Leu Ile Ser Arg Ser
                240                 245                 250

tta ggg ccc gag ttc ggt ggg tca ata ggc ctg atc ttt gct ttt gct      820
Leu Gly Pro Glu Phe Gly Gly Ser Ile Gly Leu Ile Phe Ala Phe Ala
            255                 260                 265

aat gca gtg gct gtt gct atg tat gtg gtg gga ttc gct gaa act gta      868
Asn Ala Val Ala Val Ala Met Tyr Val Val Gly Phe Ala Glu Thr Val
        270                 275                 280

gta gat cta ctt aag gag agt gat tcg atg atg gtg gat cca acc aat      916
Val Asp Leu Leu Lys Glu Ser Asp Ser Met Met Val Asp Pro Thr Asn
    285                 290                 295

gac atc cgg att ata ggc tcc atc aca gtg gtg att ctt cta gga att      964
Asp Ile Arg Ile Ile Gly Ser Ile Thr Val Val Ile Leu Leu Gly Ile
300                 305                 310                 315

tca gta gct gga atg gaa tgg gag gca aag gcc caa gtc att ctt ctg     1012
Ser Val Ala Gly Met Glu Trp Glu Ala Lys Ala Gln Val Ile Leu Leu
                320                 325                 330

gtc att ctt cta att gct att gca aac ttc ttc att gga act gtc att     1060
Val Ile Leu Leu Ile Ala Ile Ala Asn Phe Phe Ile Gly Thr Val Ile
            335                 340                 345

cca tcc aac aat gag aaa aag tcc aga ggt ttc ttt aat tac caa gca     1108
Pro Ser Asn Asn Glu Lys Lys Ser Arg Gly Phe Phe Asn Tyr Gln Ala
        350                 355                 360

tca ata ttt gca gaa aac ttt ggg cca cgc ttc aca aag ggt gaa ggc     1156
Ser Ile Phe Ala Glu Asn Phe Gly Pro Arg Phe Thr Lys Gly Glu Gly
    365                 370                 375

ttc ttc tct gtc ttt gcc att ttt ttc cca gca gct act ggg att ctt     1204

Phe Phe Ser Val Phe Ala Ile Phe Phe Pro Ala Ala Thr Gly Ile Leu
380                 385                 390                 395

gct ggt gcc aat atc tca gga gat ttg gag gat ccc caa gat gcc atc 1252
Ala Gly Ala Asn Ile Ser Gly Asp Leu Glu Asp Pro Gln Asp Ala Ile
                400                 405                 410

ccc aga gga acc atg ctg gcc att ttc atc acc act gtt gcc tac tta 1300
Pro Arg Gly Thr Met Leu Ala Ile Phe Ile Thr Thr Val Ala Tyr Leu
            415                 420                 425

ggg gtt gca att tgt gta ggg gcc tgt gtg gtc cga gat gcc acc ggg 1348
Gly Val Ala Ile Cys Val Gly Ala Cys Val Val Arg Asp Ala Thr Gly
        430                 435                 440

aac atg aat gac acc atc att tct ggg atg aac tgc aat ggt tca gca 1396
Asn Met Asn Asp Thr Ile Ile Ser Gly Met Asn Cys Asn Gly Ser Ala
    445                 450                 455

gca tgt ggg ttg ggc tat gac ttc tca aga tgt cga cat gaa cca tgt 1444
Ala Cys Gly Leu Gly Tyr Asp Phe Ser Arg Cys Arg His Glu Pro Cys
460                 465                 470                 475

cag tac ggg ctg atg aac aat ttc cag gtc atg agc atg gta tca ggg 1492
Gln Tyr Gly Leu Met Asn Asn Phe Gln Val Met Ser Met Val Ser Gly
                480                 485                 490

ttc ggc ccc ctc atc act gcg gga atc ttt tct gca aca ctc tcc tcc 1540
Phe Gly Pro Leu Ile Thr Ala Gly Ile Phe Ser Ala Thr Leu Ser Ser
            495                 500                 505

gcc ctg gcc tcc ctt gtc agc gca ccc aaa gtg ttc cag gct ctg tgc 1588
Ala Leu Ala Ser Leu Val Ser Ala Pro Lys Val Phe Gln Ala Leu Cys
        510                 515                 520

aag gac aac atc tac aaa gcc ctg cag ttt ttt gca aag gga tat ggg 1636
Lys Asp Asn Ile Tyr Lys Ala Leu Gln Phe Phe Ala Lys Gly Tyr Gly
    525                 530                 535

aaa aac aat gaa ccc ctg aga gga tat att ctc act ttt ctt ata gcc 1684
Lys Asn Asn Glu Pro Leu Arg Gly Tyr Ile Leu Thr Phe Leu Ile Ala
540                 545                 550                 555

atg gca ttt att ctt att gcg gaa ctg aac acc att gct ccc atc atc 1732
Met Ala Phe Ile Leu Ile Ala Glu Leu Asn Thr Ile Ala Pro Ile Ile
                560                 565                 570

tcc aac ttt ttc ctg gcc tca tat gca ctt att aat ttc tcc tgc ttc 1780
Ser Asn Phe Phe Leu Ala Ser Tyr Ala Leu Ile Asn Phe Ser Cys Phe
            575                 580                 585

cat gcc tct tat gcc aaa tct cca gga tgg aga cct gcg tat gga att 1828
His Ala Ser Tyr Ala Lys Ser Pro Gly Trp Arg Pro Ala Tyr Gly Ile
        590                 595                 600

tac aac atg tgg gta tct ctt ttt gga gct gtt ttg tgc tgt gca gtc 1876
Tyr Asn Met Trp Val Ser Leu Phe Gly Ala Val Leu Cys Cys Ala Val
    605                 610                 615

atg ttt gtc atc aac tgg tgg gca gct gtc atc acc tat gtc att gaa 1924
Met Phe Val Ile Asn Trp Trp Ala Ala Val Ile Thr Tyr Val Ile Glu
620                 625                 630                 635

ttc ttc ctt tac gtc tat gtg act tgt aag aag cca gat gtg aac tgg 1972
Phe Phe Leu Tyr Val Tyr Val Thr Cys Lys Lys Pro Asp Val Asn Trp
                640                 645                 650

ggc tcc tcc aca cag gct ctt tcc tac gtg agt gct tta gac aat gct 2020

Gly Ser Ser Thr Gln Ala Leu Ser Tyr Val Ser Ala Leu Asp Asn Ala
            655                 660                 665

ctg gaa tta acc aca gtg gaa gac cac gta aaa aac ttc agg ccc cag 2068
Leu Glu Leu Thr Thr Val Glu Asp His Val Lys Asn Phe Arg Pro Gln
        670                 675                 680

tgc att gtc tta aca ggg gga ccc atg aca aga cct gct ctc ctg gac 2116
Cys Ile Val Leu Thr Gly Gly Pro Met Thr Arg Pro Ala Leu Leu Asp
    685                 690                 695

ata act cac gcc ttt acc aag aac agt ggc ctt tgc atc tgc tgt gaa 2164
Ile Thr His Ala Phe Thr Lys Asn Ser Gly Leu Cys Ile Cys Cys Glu
700                 705                 710                 715

gtc ttt gtg gga ccg cgc aaa ctg tgt gtt aag gag atg aac agt ggc 2212
Val Phe Val Gly Pro Arg Lys Leu Cys Val Lys Glu Met Asn Ser Gly
                720                 725                 730

atg gcg aaa aaa cag gcc tgg ctt ata aag aac aaa atc aag gct ttt 2260
Met Ala Lys Lys Gln Ala Trp Leu Ile Lys Asn Lys Ile Lys Ala Phe
            735                 740                 745

tat gct gca gtg gcg gca gac tgt ttc agg gat ggt gtc cga agt ctt 2308
Tyr Ala Ala Val Ala Ala Asp Cys Phe Arg Asp Gly Val Arg Ser Leu
        750                 755                 760

ctt cag gcc tca ggc tta gga aga atg aaa cca aac act ctg gtg att 2356
Leu Gln Ala Ser Gly Leu Gly Arg Met Lys Pro Asn Thr Leu Val Ile
    765                 770                 775

gga tat aag aaa aac tgg agg aaa gct ccc ttg aca gag att gag aac 2404
Gly Tyr Lys Lys Asn Trp Arg Lys Ala Pro Leu Thr Glu Ile Glu Asn
780                 785                 790                 795

tac gtg gga atc ata cat gat gca ttt gat ttt gag att ggc gtg gtt 2452
Tyr Val Gly Ile Ile His Asp Ala Phe Asp Phe Glu Ile Gly Val Val
                800                 805                 810

ata gtc aga atc agc caa gga ttt gac atc tct cag gtt ctt cag gtg 2500
Ile Val Arg Ile Ser Gln Gly Phe Asp Ile Ser Gln Val Leu Gln Val
            815                 820                 825

caa gag gaa tta gag aga tta gaa cag gag aga cta gca ttg gaa gcg 2548
Gln Glu Glu Leu Glu Arg Leu Glu Gln Glu Arg Leu Ala Leu Glu Ala
        830                 835                 840

act atc aaa gat aat gag tgt gaa gag gaa agt gga ggc atc cga ggc 2596
Thr Ile Lys Asp Asn Glu Cys Glu Glu Glu Ser Gly Gly Ile Arg Gly
    845                 850                 855

ttg ttt aaa aaa gct ggc aag ttg aac att act aag aca acg cct aaa 2644
Leu Phe Lys Lys Ala Gly Lys Leu Asn Ile Thr Lys Thr Thr Pro Lys
860                 865                 870                 875

aaa gat ggc agc att aac aca agc cag tcg atg cat gtg gga gag ttc 2692
Lys Asp Gly Ser Ile Asn Thr Ser Gln Ser Met His Val Gly Glu Phe
                880                 885                 890

aac cag aaa ctg gtg gaa gcc agc act caa ttt aaa aag aaa caa gaa 2740
Asn Gln Lys Leu Val Glu Ala Ser Thr Gln Phe Lys Lys Lys Gln Glu
            895                 900                 905

aaa ggc aca att gat gtt tgg tgg ttg ttt gat gat gga ggg tta aca 2788
Lys Gly Thr Ile Asp Val Trp Trp Leu Phe Asp Asp Gly Gly Leu Thr
        910                 915                 920

ctt ctt atc ccc tat atc tta act ctc aga aaa aaa tgg aaa gac tgt 2836

Leu Leu Ile Pro Tyr Ile Leu Thr Leu Arg Lys Lys Trp Lys Asp Cys
    925                 930                 935

aaa tta aga atc tat gtg gga ggg aag atc aac cgc att gaa gaa gaa 2884
Lys Leu Arg Ile Tyr Val Gly Gly Lys Ile Asn Arg Ile Glu Glu Glu
940                 945                 950                 955

aaa att gca atg gct tcc ctt ctg agc aaa ttt agg ata aaa ttt gca 2932
Lys Ile Ala Met Ala Ser Leu Leu Ser Lys Phe Arg Ile Lys Phe Ala
                960                 965                 970

gac atc cat atc atc ggt gac atc aac att agg cca aac aaa gag agc 2980
Asp Ile His Ile Ile Gly Asp Ile Asn Ile Arg Pro Asn Lys Glu Ser
            975                 980                 985

tgg aaa gtc ttt gaa gag atg att gaa cca tat cgt ctc cat gaa agc 3028
Trp Lys Val Phe Glu Glu Met Ile Glu Pro Tyr Arg Leu His Glu Ser
        990                 995                 1000

tgc aaa gat tta aca act gct gag aaa tta aaa aga gaa act ccg 3073
Cys Lys Asp Leu Thr Thr Ala Glu Lys Leu Lys Arg Glu Thr Pro
    1005                1010                1015

tgg aaa att aca gat gca gaa ctg gaa gca gtc aag gaa aag agt 3118
Trp Lys Ile Thr Asp Ala Glu Leu Glu Ala Val Lys Glu Lys Ser
    1020                1025                1030

tac cgc caa gtt cga ctg aat gaa ctc tta cag gag cac tcc aga 3163
Tyr Arg Gln Val Arg Leu Asn Glu Leu Leu Gln Glu His Ser Arg
    1035                1040                1045

gct gct aat ctc att gtc ctg agc ctt ccc gtg gca aga aag gga 3208
Ala Ala Asn Leu Ile Val Leu Ser Leu Pro Val Ala Arg Lys Gly
    1050                1055                1060

tcc ata tcg gat ttg ttg tat atg gct tgg ttg gaa atc ctc aca 3253
Ser Ile Ser Asp Leu Leu Tyr Met Ala Trp Leu Glu Ile Leu Thr
    1065                1070                1075

aag aac ctc cca cct gtc tta cta gtt aga gga aat cac aaa aat 3298
Lys Asn Leu Pro Pro Val Leu Leu Val Arg Gly Asn His Lys Asn
    1080                1085                1090

gtc ttg aca ttt tac tct taa aacatgaaag attggaatac attttaactt 3349
Val Leu Thr Phe Tyr Ser
    1095

aatgtaatgc ata 3362

<210> 30
<211> 1099
<212> PRT
<213> Homo sapiens

<400> 30

Met Ser Leu Asn Asn Ser Ser Asn Val Phe Leu Asp Ser Val Pro Ser
1               5                   10                  15

Asn Thr Asn Arg Phe Gln Val Ser Val Ile Asn Glu Asn His Glu Ser
            20                  25                  30

Ser Ala Ala Ala Asp Asp Asn Thr Asp Pro Pro His Tyr Glu Glu Thr
        35                  40                  45

Ser Phe Gly Asp Glu Ala Gln Lys Arg Leu Arg Ile Ser Phe Arg Pro
    50                  55                  60

Gly Asn Gln Glu Cys Tyr Asp Asn Phe Leu His Ser Gly Glu Thr Ala
65                  70                  75                  80

Lys Thr Asp Ala Ser Phe His Ala Tyr Asp Ser His Thr Asn Thr Tyr
                85                  90                  95

Tyr Leu Gln Thr Phe Gly His Asn Thr Met Asp Ala Val Pro Lys Ile
            100                 105                 110

Glu Tyr Tyr Arg Asn Thr Gly Ser Ile Ser Gly Pro Lys Val Asn Arg
        115                 120                 125

Pro Ser Leu Leu Glu Ile His Glu Gln Leu Ala Lys Asn Val Ala Val
    130                 135                 140

Thr Pro Ser Ser Ala Asp Arg Val Ala Asn Gly Asp Gly Ile Pro Gly
145                 150                 155                 160

Asp Glu Gln Ala Glu Asn Lys Glu Asp Asp Gln Ala Gly Val Val Lys
                165                 170                 175

Phe Gly Trp Val Lys Gly Val Leu Val Arg Cys Met Leu Asn Ile Trp
            180                 185                 190

Gly Val Met Leu Phe Ile Arg Leu Ser Trp Ile Val Gly Glu Ala Gly
        195                 200                 205

Ile Gly Leu Gly Val Ile Ile Ile Gly Leu Ser Thr Ile Val Thr Thr
    210                 215                 220

Ile Thr Gly Met Ser Thr Ser Ala Ile Ala Thr Asn Gly Val Val Arg
225                 230                 235                 240

Gly Gly Gly Ala Tyr Tyr Leu Ile Ser Arg Ser Leu Gly Pro Glu Phe
                245                 250                 255

Gly Gly Ser Ile Gly Leu Ile Phe Ala Phe Ala Asn Ala Val Ala Val
            260                 265                 270

Ala Met Tyr Val Val Gly Phe Ala Glu Thr Val Val Asp Leu Leu Lys
        275                 280                 285

Glu Ser Asp Ser Met Met Val Asp Pro Thr Asn Asp Ile Arg Ile Ile
    290                 295                 300

Gly Ser Ile Thr Val Val Ile Leu Leu Gly Ile Ser Val Ala Gly Met
305                 310                 315                 320

Glu Trp Glu Ala Lys Ala Gln Val Ile Leu Leu Val Ile Leu Leu Ile
                325                 330                 335

Ala Ile Ala Asn Phe Phe Ile Gly Thr Val Ile Pro Ser Asn Asn Glu
            340                 345                 350

Lys Lys Ser Arg Gly Phe Phe Asn Tyr Gln Ala Ser Ile Phe Ala Glu
        355                 360                 365

Asn Phe Gly Pro Arg Phe Thr Lys Gly Glu Gly Phe Phe Ser Val Phe
    370                 375                 380

Ala Ile Phe Phe Pro Ala Ala Thr Gly Ile Leu Ala Gly Ala Asn Ile
385                 390                 395                 400

Ser Gly Asp Leu Glu Asp Pro Gln Asp Ala Ile Pro Arg Gly Thr Met
                405                 410                 415

Leu Ala Ile Phe Ile Thr Thr Val Ala Tyr Leu Gly Val Ala Ile Cys
            420                 425                 430

Val Gly Ala Cys Val Val Arg Asp Ala Thr Gly Asn Met Asn Asp Thr
        435                 440                 445

Ile Ile Ser Gly Met Asn Cys Asn Gly Ser Ala Ala Cys Gly Leu Gly
    450                 455                 460

Tyr Asp Phe Ser Arg Cys Arg His Glu Pro Cys Gln Tyr Gly Leu Met
465                 470                 475                 480

Asn Asn Phe Gln Val Met Ser Met Val Ser Gly Phe Gly Pro Leu Ile
                485                 490                 495

Thr Ala Gly Ile Phe Ser Ala Thr Leu Ser Ser Ala Leu Ala Ser Leu
            500                 505                 510

Val Ser Ala Pro Lys Val Phe Gln Ala Leu Cys Lys Asp Asn Ile Tyr
        515                 520                 525

Lys Ala Leu Gln Phe Phe Ala Lys Gly Tyr Gly Lys Asn Asn Glu Pro
    530                 535                 540

Leu Arg Gly Tyr Ile Leu Thr Phe Leu Ile Ala Met Ala Phe Ile Leu
545                 550                 555                 560

Ile Ala Glu Leu Asn Thr Ile Ala Pro Ile Ile Ser Asn Phe Phe Leu
                565                 570                 575

Ala Ser Tyr Ala Leu Ile Asn Phe Ser Cys Phe His Ala Ser Tyr Ala
            580                 585                 590

Lys Ser Pro Gly Trp Arg Pro Ala Tyr Gly Ile Tyr Asn Met Trp Val
        595                 600                 605

Ser Leu Phe Gly Ala Val Leu Cys Cys Ala Val Met Phe Val Ile Asn
    610                 615                 620

Trp Trp Ala Ala Val Ile Thr Tyr Val Ile Glu Phe Phe Leu Tyr Val
625                 630                 635                 640

Tyr Val Thr Cys Lys Lys Pro Asp Val Asn Trp Gly Ser Ser Thr Gln
                645                 650                 655

Ala Leu Ser Tyr Val Ser Ala Leu Asp Asn Ala Leu Glu Leu Thr Thr
            660                 665                 670

Val Glu Asp His Val Lys Asn Phe Arg Pro Gln Cys Ile Val Leu Thr
        675                 680                 685

Gly Gly Pro Met Thr Arg Pro Ala Leu Leu Asp Ile Thr His Ala Phe
    690                 695                 700

Thr Lys Asn Ser Gly Leu Cys Ile Cys Cys Glu Val Phe Val Gly Pro
705                 710                 715                 720

Arg Lys Leu Cys Val Lys Glu Met Asn Ser Gly Met Ala Lys Lys Gln
                725                 730                 735

Ala Trp Leu Ile Lys Asn Lys Ile Lys Ala Phe Tyr Ala Ala Val Ala
            740                 745                 750

Ala Asp Cys Phe Arg Asp Gly Val Arg Ser Leu Leu Gln Ala Ser Gly
        755                 760                 765

Leu Gly Arg Met Lys Pro Asn Thr Leu Val Ile Gly Tyr Lys Lys Asn
    770                 775                 780

Trp Arg Lys Ala Pro Leu Thr Glu Ile Glu Asn Tyr Val Gly Ile Ile
785                 790                 795                 800

His Asp Ala Phe Asp Phe Glu Ile Gly Val Val Ile Val Arg Ile Ser
                805                 810                 815

Gln Gly Phe Asp Ile Ser Gln Val Leu Gln Val Gln Glu Glu Leu Glu
            820                 825                 830

Arg Leu Glu Gln Glu Arg Leu Ala Leu Glu Ala Thr Ile Lys Asp Asn
        835                 840                 845

Glu Cys Glu Glu Glu Ser Gly Gly Ile Arg Gly Leu Phe Lys Lys Ala
    850                 855                 860

Gly Lys Leu Asn Ile Thr Lys Thr Thr Pro Lys Lys Asp Gly Ser Ile
865                 870                 875                 880

Asn Thr Ser Gln Ser Met His Val Gly Glu Phe Asn Gln Lys Leu Val
                885                 890                 895

Glu Ala Ser Thr Gln Phe Lys Lys Lys Gln Glu Lys Gly Thr Ile Asp
            900                 905                 910

Val Trp Trp Leu Phe Asp Asp Gly Gly Leu Thr Leu Leu Ile Pro Tyr
        915                 920                 925

Ile Leu Thr Leu Arg Lys Lys Trp Lys Asp Cys Lys Leu Arg Ile Tyr
    930                 935                 940

Val Gly Gly Lys Ile Asn Arg Ile Glu Glu Glu Lys Ile Ala Met Ala
945                 950                 955                 960

Ser Leu Leu Ser Lys Phe Arg Ile Lys Phe Ala Asp Ile His Ile Ile
                965                 970                 975

Gly Asp Ile Asn Ile Arg Pro Asn Lys Glu Ser Trp Lys Val Phe Glu
            980                 985                 990

Glu Met Ile Glu Pro Tyr Arg Leu  His Glu Ser Cys Lys  Asp Leu Thr
        995                 1000                1005

Thr Ala  Glu Lys Leu Lys Arg  Glu Thr Pro Trp Lys  Ile Thr Asp
    1010                1015                1020

Ala Glu  Leu Glu Ala Val Lys  Glu Lys Ser Tyr Arg  Gln Val Arg
    1025                1030                1035

Leu Asn  Glu Leu Leu Gln Glu  His Ser Arg Ala Ala  Asn Leu Ile
    1040                1045                1050

Val Leu  Ser Leu Pro Val Ala  Arg Lys Gly Ser Ile  Ser Asp Leu
    1055                1060                1065

Leu Tyr  Met Ala Trp Leu Glu  Ile Leu Thr Lys Asn  Leu Pro Pro
    1070                1075                1080

Val Leu  Leu Val Arg Gly Asn  His Lys Asn Val Leu  Thr Phe Tyr
    1085                1090                1095

Ser

<210>  31
<211>  21
<212>  DNA

<213>  künstliche Sequenz

<220>
<223>  Beschreibung der künstlichen Sequenz: Oligonukleotid

<400>  31
cagcctccag aatgaagaat g                                                21

<210>  32
<211>  18
<212>  DNA
<213>  künstliche Sequenz

<220>
<223>  Beschreibung der künstlichen Sequenz: Oligonukleotid

<400>  32
ggagggagac tgagattt                                                    18

<210>  33
<211>  22
<212>  DNA
<213>  künstliche Sequenz

<220>
<223>  Beschreibung der künstlichen Sequenz: Oligonukleotid

<400>  33
gcggctcact tggacttttt ca                                               22

<210>  34
<211>  20
<212>  DNA
<213>  künstliche Sequenz

<220>
<223>  Beschreibung der künstlichen Sequenz: Oligonukleotid

<400>  34
gcatagtcat ctcttcttca                                                  20

<210>  35
<211>  20
<212>  DNA
<213>  künstliche Sequenz

<220>
<223>  Beschreibung der künstlichen Sequenz: Oligonukleotid

<400>  35
catactgaaa ggtgtgggct                                                  20

<210>  36
<211>  18
<212>  DNA
<213>  künstliche Sequenz

<220>
<223>  Beschreibung der künstlichen Sequenz: Oligonukleotid

<400>  36
ggtcccaaaa gtgtcgtt                                                    18

<210>  37
<211>  19
<212>  DNA
<213>  künstliche Sequenz

<220>
<223>  Beschreibung der künstlichen Sequenz: Oligonukleotid

<400>  37
gagggagatt ggtggtgtt                                                    19

<210>  38
<211>  18
<212>  DNA
<213>  künstliche Sequenz

<220>
<223>  Beschreibung der künstlichen Sequenz: Oligonukleotid

<400>  38
ccagagaaaa tgcccact                                                     18

<210>  39
<211>  20
<212>  DNA
<213>  künstliche Sequenz

<220>
<223>  Beschreibung der künstlichen Sequenz: Oligonukleotid

<400>  39
aatgaacgac tgggtaacag                                                   20

<210>  40
<211>  21
<212>  DNA
<213>  künstliche Sequenz

<220>
<223>  Beschreibung der künstlichen Sequenz: Oligonukleotid

<400>  40
gaggagtatc ttgcttcttc a                                                 21

<210>  41
<211>  22
<212>  DNA
<213>  künstliche Sequenz

<220>
<223>  Beschreibung der künstlichen Sequenz: Oligonukleotid

<400>  41
gtgttcttct ggtggctcaa tc                                                22

<210>  42
<211>  16
<212>  DNA
<213>  künstliche Sequenz

<220>
<223>  Beschreibung der künstlichen Sequenz: Oligonukleotid

<400>  42
ggcttctgtg cgtcat                                                        16

<210>  43
<211>  21
<212>  DNA
<213>  künstliche Sequenz

<220>
<223>  Beschreibung der künstlichen Sequenz: Oligonukleotid

<400>  43
gctacatcat cctggctatc c                                                  21

<210>  44
<211>  17
<212>  DNA
<213>  künstliche Sequenz

<220>
<223>  Beschreibung der künstlichen Sequenz: Oligonukleotid

<400>  44
gcctcgctct cgttgcc                                                       17

<210>  45
<211>  22
<212>  DNA
<213>  künstliche Sequenz

<220>
<223>  Beschreibung der künstlichen Sequenz: Oligonukleotid

<400>  45
acagtgccct accacacaga tc                                                 22

<210>  46
<211>  18
<212>  DNA
<213>  künstliche Sequenz

<220>
<223>  Beschreibung der künstlichen Sequenz: Oligonukleotid

<400>  46
gggaaaaatc gctgttgg                                                      18

<210>  47
<211>  20
<212>  DNA
<213>  künstliche Sequenz

<220>
<223>  Beschreibung der künstlichen Sequenz: Oligonukleotid

<400>  47
aatagagtgc ctgcctggtt                                                    20

<210>  48
<211>  20
<212>  DNA

<213>  künstliche Sequenz

<220>
<223>  Beschreibung der künstlichen Sequenz: Oligonukleotid

<400>  48
tggataatga gtagcctgga                                                    20


<210>  49
<211>  20
<212>  DNA
<213>  künstliche Sequenz

<220>
<223>  Beschreibung der künstlichen Sequenz: Oligonukleotid

<400>  49
ccacaaccaa gaagcaccaa                                                    20


<210>  50
<211>  20
<212>  DNA
<213>  künstliche Sequenz

<220>
<223>  Beschreibung der künstlichen Sequenz: Oligonukleotid

<400>  50
tgcggcttgt tgtagttgaa                                                    20


<210>  51
<211>  17
<212>  DNA
<213>  künstliche Sequenz

<220>
<223>  Beschreibung der künstlichen Sequenz: Oligonukleotid

<400>  51
ttggcttcct ggctaac                                                       17


<210>  52
<211>  20
<212>  DNA
<213>  künstliche Sequenz

<220>
<223>  Beschreibung der künstlichen Sequenz: Oligonukleotid

<400>  52
tggaggtgaa catcgcaaat                                                    20


<210>  53
<211>  20
<212>  DNA
<213>  künstliche Sequenz

<220>
<223>  Beschreibung der künstlichen Sequenz: Oligonukleotid

<400>  53
actgctgtct cgtggtatgc                                                    20

<210>  54
<211>  25
<212>  DNA
<213>  künstliche Sequenz

<220>
<223>  Beschreibung der künstlichen Sequenz: Oligonukleotid

<400>  54
ggcaatttaa caactggttc tcggg                                          25


<210>  55
<211>  20
<212>  DNA
<213>  künstliche Sequenz

<220>
<223>  Beschreibung der künstlichen Sequenz: Oligonukleotid

<400>  55
ctaaaagggc agaagaagga                                                20


<210>  56
<211>  25
<212>  DNA
<213>  künstliche Sequenz

<220>
<223>  Beschreibung der künstlichen Sequenz: Oligonukleotid

<400>  56
ctcccactga ctttcctgct ttctt                                          25


<210>  57
<211>  15
<212>  DNA
<213>  künstliche Sequenz

<220>
<223>  Beschreibung der künstlichen Sequenz: Oligonukleotid

<400>  57
ttctcttggt ctcat                                                     15


<210>  58
<211>  18
<212>  DNA
<213>  künstliche Sequenz

<220>
<223>  Beschreibung der künstlichen Sequenz: Oligonukleotid

<400>  58
atccagcaga agtcatcc                                                  18


<210>  59
<211>  19
<212>  DNA
<213>  künstliche Sequenz

<220>
<223>  Beschreibung der künstlichen Sequenz: Oligonukleotid

<400>  59
catttattct tattgcgga                                                    19


<210>  60
<211>  19
<212>  DNA
<213>  künstliche Sequenz

<220>
<223>  Beschreibung der künstlichen Sequenz: Oligonukleotid

<400>  60
caaaaagaga tacccacat                                                    19


<210>  61
<211>  15
<212>  PRT
<213>  Homo sapiens

<400>  61

Lys Ile Ser Val Ser Leu Pro Leu Ser Leu Ser Gln Ser Val Cys
1               5                   10                  15


<210>  62
<211>  14
<212>  PRT
<213>  Homo sapiens

<400>  62

Gln Leu Ser Lys Asp Thr Ser Val Leu Thr Phe Thr Phe Cys
1               5                   10


<210>  63
<211>  17
<212>  PRT
<213>  Homo sapiens

<400>  63

Cys Ser Asp Ala His Pro Gly Asp Ser Ser Gly Asp Ser Ser Gly Leu
1               5                   10                  15


Asn


<210>  64
<211>  15
<212>  PRT
<213>  Homo sapiens

<400>  64

Arg Gly Glu Val Arg Gln Phe Thr Leu Arg His Trp Leu Lys Val
1               5                   10                  15


<210>  65
<211>  13
<212>  PRT

<213>  Homo sapiens

<400>  65

Gly Asp Tyr Leu Asn Asp Glu Ala Leu Trp Asn Lys Cys
1               5                   10


<210>  66
<211>  15
<212>  PRT
<213>  Homo sapiens

<400>  66

Gly Lys Val Ile Asp Asp Asn Asp His Leu Ser Gln Glu Ile Cys
1               5                   10                  15


<210>  67
<211>  14
<212>  PRT
<213>  Homo sapiens

<400>  67

Leu Met Ala Asn Ser Thr Trp Gly Tyr Pro Phe His Asp Gly
1               5                   10


<210>  68
<211>  14
<212>  PRT
<213>  Homo sapiens

<400>  68

Leu Asn Val Val Pro Trp Asn Leu Thr Leu Phe Ser Ile Leu
1               5                   10


<210>  69
<211>  303
<212>  DNA
<213>  Homo sapiens


<220>
<221>  CDS
<222>  (1)..(303)

<400>  69
atg ctt tgc tac atc att ctg caa tcc cag aag att ttt tgc ata ttt      48
Met Leu Cys Tyr Ile Ile Leu Gln Ser Gln Lys Ile Phe Cys Ile Phe
1               5                   10                  15

ttt tgc tat tac aga aaa tct cag tct ccc tcc ctc tct ctc tct ctc      96
Phe Cys Tyr Tyr Arg Lys Ser Gln Ser Pro Ser Leu Ser Leu Ser Leu
            20                  25                  30

aat ctg tgt gtc tct ttt act cca tat ctc tgt gtg tgt ctc ttt tac      144
Asn Leu Cys Val Ser Phe Thr Pro Tyr Leu Cys Val Cys Leu Phe Tyr
        35                  40                  45

tcc ata tct ctc tgt gtg tgt ctg ttt atg tct ctc tct ctc tct cat      192
Ser Ile Ser Leu Cys Val Cys Leu Phe Met Ser Leu Ser Leu Ser His
    50                  55                  60

cct tcc cat gtt tct ctc tca cac aca cac aca ctc att cac agc ttt 240
Pro Ser His Val Ser Leu Ser His Thr His Thr Leu Ile His Ser Phe
65                  70                  75                  80

caa aag aca cgt ctg tcc tta cct tca ctt ttt gtt tta aac agc aca 288
Gln Lys Thr Arg Leu Ser Leu Pro Ser Leu Phe Val Leu Asn Ser Thr
                85                  90                  95

ctc act tta ctc tga 303
Leu Thr Leu Leu
            100

<210> 70
<211> 100
<212> PRT
<213> Homo sapiens

<400> 70

Met Leu Cys Tyr Ile Ile Leu Gln Ser Gln Lys Ile Phe Cys Ile Phe
1               5                   10                  15

Phe Cys Tyr Tyr Arg Lys Ser Gln Ser Pro Ser Leu Ser Leu Ser Leu
            20                  25                  30

Asn Leu Cys Val Ser Phe Thr Pro Tyr Leu Cys Val Cys Leu Phe Tyr
        35                  40                  45

Ser Ile Ser Leu Cys Val Cys Leu Phe Met Ser Leu Ser Leu Ser His
    50                  55                  60

Pro Ser His Val Ser Leu Ser His Thr His Thr Leu Ile His Ser Phe
65                  70                  75                  80

Gln Lys Thr Arg Leu Ser Leu Pro Ser Leu Phe Val Leu Asn Ser Thr
                85                  90                  95

Leu Thr Leu Leu
            100

<210> 71
<211> 1786
<212> DNA
<213> Homo sapiens

<220>
<221> CDS
<222> (204)..(494)

<400> 71
actcagctct ctcaccatgc gattgccctg caacaccttg gaactctgca gagagtcccc 60

agcaggaagg tctcacctga ggtgaacctt cgaccttgga cttctcagcc tccagaatga 120

agaatggcaa ccatcaaatc aagaaattgg cccaaagccc tacagtctgc aaacatcata 180

acaattcatc ctgaagtttc tcc atg aat tgt gat gct ttg cta cat cat tct 233
Met Asn Cys Asp Ala Leu Leu His His Ser
1               5                   10

gca atc cca gaa gat ttt ttg cat att ttt ttg cta tta cag aaa aat 281
Ala Ile Pro Glu Asp Phe Leu His Ile Phe Leu Leu Leu Gln Lys Asn
                15                  20                  25

ctc agt ctc cct ccc tct ctc tct ctc tca atc tgt gtg tct ctt tta 329
Leu Ser Leu Pro Pro Ser Leu Ser Leu Ser Ile Cys Val Ser Leu Leu
            30                  35                  40

ctc cat atc tct gtg tgt gtc tct ttt act cca tat ctc tct gtg tgt 377
Leu His Ile Ser Val Cys Val Ser Phe Thr Pro Tyr Leu Ser Val Cys
        45                  50                  55

gtc tgt tta tgt ctc tct ctc tct ctc atc ctt ccc atg ttt ctc tct 425
Val Cys Leu Cys Leu Ser Leu Ser Leu Ile Leu Pro Met Phe Leu Ser
    60                  65                  70

cac aca cac aca cac tca ttc aca gct ttc aaa aga cac gtc tgt cct 473
His Thr His Thr His Ser Phe Thr Ala Phe Lys Arg His Val Cys Pro
75                  80                  85                  90

tac ctt cac ttt ttg ttt taa acagcacact cactttactc tgaactatac 524
Tyr Leu His Phe Leu Phe
                95

ctcacatgca cacgagcttt ctgctccatc tgttcatccc acatgtgtct tcacattcaa 584

agcagcacct tccccaagac cagctaccta accacctccc acctccaccc catccctagt 644

cagaggaagg cctggttccc acctgaattc agctttgtca aagagcctcc tggaaagctg 704

tcatcttcag ttagtaggga taatgggatt attctatctg tgtaataata acatgttcaa 764

tttaaagaaa aaaatctgaa gccacttaaa agctactgtt tggcaccgat acattattcc 824

agtaatgaat aatcattaaa gatattattc tggatgcagt taccatgcag tgatgtgaat 884

aaaatgcatt agatggaaaa ttgtatttca agtaaatata tgcactggta gaaatgtatt 944

accacccact aatatgtatt aattcaaaac caaatgccaa ctggagttcg cctacacggg 1004

tttgaatggc aggcagtgat ttggaagtgg gaggaaatag gtttggattt ggtcaaatag 1064

actgagaagt gatagtgggg gcgggggttt atgactcaaa ctttaacagg tgagaagact 1124

atgccatgga cagaacaggc atgaggggct cccctcctac gcctctttaa gagattttta 1184

tctctgacta aggattactg gtagttgttg acatttctga agcagtggat ctttttcctt 1244

tttcactatc tgcatcttca aatattcttt tctgaagaaa gttaaaagga agcctgtaca 1304

ttttttgcta aggtaaatgc cttgccatct tatttcattt tctcattttt ttcttcagtg 1364

cacaacataa gcaactgtcc tccttgtcat actcaagatg agcttggcat atctgaaatc 1424

tgcagggatt ttctcattag cacagggttc caagcccaaa ccgtgaagat ggagttttca 1484

tttttaaatg gcacatcttc aagttcttgc cctgtcctca ctttagtatg ccccagagga 1544

agtcaaagat atggacactc taagactcag aagaactttc tcaggcattc attttcctat 1604

ctattttgag ccattttatt taaaaggtta caattttaaa cctctcttta attaaaagat 1664

accagagtta caatgcaata ctatttggca atcaaaacta atgaagcaca gatgcatgct 1724

acaacacgaa tgaactttga aacgttgtgt taagtgaaat aaaccagtta ttatacaagg 1784

cc 1786

<210>  72
<211>  96
<212>  PRT
<213>  Homo sapiens

<400>  72

Met Asn Cys Asp Ala Leu Leu His His Ser Ala Ile Pro Glu Asp Phe
1               5                   10                  15

Leu His Ile Phe Leu Leu Leu Gln Lys Asn Leu Ser Leu Pro Pro Ser
            20                  25                  30

Leu Ser Leu Ser Ile Cys Val Ser Leu Leu Leu His Ile Ser Val Cys
        35                  40                  45

Val Ser Phe Thr Pro Tyr Leu Ser Val Cys Val Cys Leu Cys Leu Ser
    50                  55                  60

Leu Ser Leu Ile Leu Pro Met Phe Leu Ser His Thr His Thr His Ser
65                  70                  75                  80

Phe Thr Ala Phe Lys Arg His Val Cys Pro Tyr Leu His Phe Leu Phe
                85                  90                  95

<210>  73
<211>  402
<212>  DNA
<213>  Homo sapiens

<220>
<221>  CDS
<222>  (1)..(402)

<400>  73
atg ctt tgc tac atc att ctg caa tcc cag aag att ttt tgc ata ttt      48
Met Leu Cys Tyr Ile Ile Leu Gln Ser Gln Lys Ile Phe Cys Ile Phe
1               5                   10                  15

ttt tgc tat tac aga aaa atc tca gtc tcc ctc cct ctc tct ctc tct      96
Phe Cys Tyr Tyr Arg Lys Ile Ser Val Ser Leu Pro Leu Ser Leu Ser
            20                  25                  30

caa tct gtg tgt ctc ttt tac tcc ata tct ctg tgt gtg tct ctt tta      144
Gln Ser Val Cys Leu Phe Tyr Ser Ile Ser Leu Cys Val Ser Leu Leu
        35                  40                  45

ctc cat atc tct ctg tgt gtg tct gtt tat gtc tct ctc tct ctc tca      192
Leu His Ile Ser Leu Cys Val Ser Val Tyr Val Ser Leu Ser Leu Ser
    50                  55                  60

tcc ttc cca tgt ttc tct ctc aca cac aca cac act cat tca cag ctt      240
Ser Phe Pro Cys Phe Ser Leu Thr His Thr His Thr His Ser Gln Leu
65                  70                  75                  80

tca aaa gac acg tct gtc ctt acc ttc act ttt tgt ttt aaa cag cac      288
Ser Lys Asp Thr Ser Val Leu Thr Phe Thr Phe Cys Phe Lys Gln His
                85                  90                  95

act cac ttt act ctg aac tat acc tca cat gca cac gag ctt tct gct 336
Thr His Phe Thr Leu Asn Tyr Thr Ser His Ala His Glu Leu Ser Ala
            100                 105                 110

cca tct gtt cat ccc aca tgt gtc ttc aca ttc aaa gca gca cct tcc 384
Pro Ser Val His Pro Thr Cys Val Phe Thr Phe Lys Ala Ala Pro Ser
        115                 120                 125

cca aga cca gct acc taa 402
Pro Arg Pro Ala Thr
    130

<210> 74
<211> 133
<212> PRT
<213> Homo sapiens

<400> 74

Met Leu Cys Tyr Ile Ile Leu Gln Ser Gln Lys Ile Phe Cys Ile Phe
1               5                   10                  15

Phe Cys Tyr Tyr Arg Lys Ile Ser Val Ser Leu Pro Leu Ser Leu Ser
            20                  25                  30

Gln Ser Val Cys Leu Phe Tyr Ser Ile Ser Leu Cys Val Ser Leu Leu
        35                  40                  45

Leu His Ile Ser Leu Cys Val Ser Val Tyr Val Ser Leu Ser Leu Ser
    50                  55                  60

Ser Phe Pro Cys Phe Ser Leu Thr His Thr His Thr His Ser Gln Leu
65                  70                  75                  80

Ser Lys Asp Thr Ser Val Leu Thr Phe Thr Phe Cys Phe Lys Gln His
                85                  90                  95

Thr His Phe Thr Leu Asn Tyr Thr Ser His Ala His Glu Leu Ser Ala
            100                 105                 110

Pro Ser Val His Pro Thr Cys Val Phe Thr Phe Lys Ala Ala Pro Ser
        115                 120                 125

Pro Arg Pro Ala Thr
    130

<210> 75
<211> 3579
<212> DNA
<213> Homo sapiens

<220>
<221> CDS
<222> (136)..(3258)

<400> 75
caccacagtt atcacccatg ccctcctaaa agggtgtctc aaagcatatc tttctgtaga 60

gcagaattcg gaactgagaa gacgagggct caaattgaat ctcacaggat ttgcgtgcaa 120

gagaaaccca aagga atg gat tgg ctc ttc ttc aga aac att tgc ctt ttg 171
Met Asp Trp Leu Phe Phe Arg Asn Ile Cys Leu Leu
1               5                   10

atc att cta atg gtg gtg atg gaa gta aac agt gaa ttt att gtt gag 219
Ile Ile Leu Met Val Val Met Glu Val Asn Ser Glu Phe Ile Val Glu
        15                  20                  25

gtg aag gaa ttt gac att gaa aat ggc act aca aaa tgg caa aca gtc 267
Val Lys Glu Phe Asp Ile Glu Asn Gly Thr Thr Lys Trp Gln Thr Val
    30                  35                  40

aga aga caa aag cgg gag tgg atc aag ttt gcc gca gcc tgt cga gaa 315
Arg Arg Gln Lys Arg Glu Trp Ile Lys Phe Ala Ala Ala Cys Arg Glu
45                  50                  55                  60

gga gag gac aac tcg aag agg aac ccc att gcc aaa att cga tca gac 363
Gly Glu Asp Asn Ser Lys Arg Asn Pro Ile Ala Lys Ile Arg Ser Asp
                65                  70                  75

tgc gaa tcg aac cag aag ata aca tac cgg att tct gga gta ggg att 411
Cys Glu Ser Asn Gln Lys Ile Thr Tyr Arg Ile Ser Gly Val Gly Ile
            80                  85                  90

gat cga cca cca tat ggg gta ttc acc att aat cct cgc act ggg gaa 459
Asp Arg Pro Pro Tyr Gly Val Phe Thr Ile Asn Pro Arg Thr Gly Glu
        95                  100                 105

att aac atc act tca gtg gta gac aga gaa ata act cca ctt ttc ttg 507
Ile Asn Ile Thr Ser Val Val Asp Arg Glu Ile Thr Pro Leu Phe Leu
    110                 115                 120

atc tat tgc cgg gct ctg aat tca cgg ggt gaa gat tta gaa agg cct 555
Ile Tyr Cys Arg Ala Leu Asn Ser Arg Gly Glu Asp Leu Glu Arg Pro
125                 130                 135                 140

ctt gag ctt aga gtc aaa gtt atg gac ata aat gat aac gct cca gtc 603
Leu Glu Leu Arg Val Lys Val Met Asp Ile Asn Asp Asn Ala Pro Val
                145                 150                 155

ttt tcg caa agt gta tac aca gcc agc att gaa gaa aat agt gat gcc 651
Phe Ser Gln Ser Val Tyr Thr Ala Ser Ile Glu Glu Asn Ser Asp Ala
            160                 165                 170

aat aca ttg gta gta aag tta tgt gcc aca gat gca gat gaa gaa aat 699
Asn Thr Leu Val Val Lys Leu Cys Ala Thr Asp Ala Asp Glu Glu Asn
        175                 180                 185

cat ctg aat tct aaa att gcc tac aag atc gtc tct cag gag cca tca 747
His Leu Asn Ser Lys Ile Ala Tyr Lys Ile Val Ser Gln Glu Pro Ser
    190                 195                 200

ggt gca ccc atg ttc att ctg aat agg tac act gga gaa gtc tgc acc 795
Gly Ala Pro Met Phe Ile Leu Asn Arg Tyr Thr Gly Glu Val Cys Thr
205                 210                 215                 220

atg tcc agt ttc ttg gac aga gag caa cac agt atg tac aac ctg gtt 843
Met Ser Ser Phe Leu Asp Arg Glu Gln His Ser Met Tyr Asn Leu Val
                225                 230                 235

gtg aga ggc tca gat cgg gat gga gct gca gat gga ctg tct tct gag 891
Val Arg Gly Ser Asp Arg Asp Gly Ala Ala Asp Gly Leu Ser Ser Glu
            240                 245                 250

tgt gac tgt aga atc aag gtt tta gac gtc aac gat aat ttc ccc acc 939

Cys Asp Cys Arg Ile Lys Val Leu Asp Val Asn Asp Asn Phe Pro Thr
        255                 260                 265

tta gag aaa act tca tac tca gcc agt att gaa gag aat tgt tta agt 987
Leu Glu Lys Thr Ser Tyr Ser Ala Ser Ile Glu Glu Asn Cys Leu Ser
    270                 275                 280

tcg gaa ctg ata cga tta caa gca att gat ctt gat gaa gaa ggc act 1035
Ser Glu Leu Ile Arg Leu Gln Ala Ile Asp Leu Asp Glu Glu Gly Thr
285                 290                 295                 300

gat aac tgg ttg gct caa tat tta att ctc tct gga aat gat ggg aat 1083
Asp Asn Trp Leu Ala Gln Tyr Leu Ile Leu Ser Gly Asn Asp Gly Asn
                305                 310                 315

tgg ttc gat att caa aca gat cca caa acc aat gaa ggc att ttg aaa 1131
Trp Phe Asp Ile Gln Thr Asp Pro Gln Thr Asn Glu Gly Ile Leu Lys
            320                 325                 330

gtt gtc aag atg ctg gat tat gaa caa gca cct aac att cag ctt agt 1179
Val Val Lys Met Leu Asp Tyr Glu Gln Ala Pro Asn Ile Gln Leu Ser
        335                 340                 345

atc gga gtt aaa aac caa gct gat ttt cac tac tcc gtt gct tct caa 1227
Ile Gly Val Lys Asn Gln Ala Asp Phe His Tyr Ser Val Ala Ser Gln
    350                 355                 360

ttc caa atg cac cca acc cct gtg aga att caa gtt gtt gat gtg aga 1275
Phe Gln Met His Pro Thr Pro Val Arg Ile Gln Val Val Asp Val Arg
365                 370                 375                 380

gaa gga cct gca ttt cat cca agt act atg gct ttt agt gtg cgg gaa 1323
Glu Gly Pro Ala Phe His Pro Ser Thr Met Ala Phe Ser Val Arg Glu
                385                 390                 395

gga ata aaa gga agt tcc tta ttg aat tat gtg ctt ggc aca tat aca 1371
Gly Ile Lys Gly Ser Ser Leu Leu Asn Tyr Val Leu Gly Thr Tyr Thr
            400                 405                 410

gcc ata gat ttg gac aca gga aac cct gca aca gat gtc aga tat atc 1419
Ala Ile Asp Leu Asp Thr Gly Asn Pro Ala Thr Asp Val Arg Tyr Ile
        415                 420                 425

ata ggg cat gat gca ggc agc tgg tta aaa att gat tca aga act ggt 1467
Ile Gly His Asp Ala Gly Ser Trp Leu Lys Ile Asp Ser Arg Thr Gly
    430                 435                 440

gag ata caa ttt tct aga gaa ttt gat aag aag tca aaa tat att atc 1515
Glu Ile Gln Phe Ser Arg Glu Phe Asp Lys Lys Ser Lys Tyr Ile Ile
445                 450                 455                 460

aat ggg ata tac aca gca gag atc ctg gct ata gat gat ggc tct gga 1563
Asn Gly Ile Tyr Thr Ala Glu Ile Leu Ala Ile Asp Asp Gly Ser Gly
                465                 470                 475

aaa aca gct aca gga acc ata tgt att gag gtt cct gat atc aat gat 1611
Lys Thr Ala Thr Gly Thr Ile Cys Ile Glu Val Pro Asp Ile Asn Asp
            480                 485                 490

tat tgt cca aac att ttt cct gaa aga aga acc atc tgc att gac tct 1659
Tyr Cys Pro Asn Ile Phe Pro Glu Arg Arg Thr Ile Cys Ile Asp Ser
        495                 500                 505

cca tca gtc ctt atc tct gtt aat gaa cat tct tat ggg tct ccg ttt 1707
Pro Ser Val Leu Ile Ser Val Asn Glu His Ser Tyr Gly Ser Pro Phe
    510                 515                 520

act ttc tgt gtt gtt gat gag cca cca gga ata gct gac atg tgg gat 1755

Thr Phe Cys Val Val Asp Glu Pro Pro Gly Ile Ala Asp Met Trp Asp
525                 530                 535                 540

gtc aga tca aca aat gct acc tcg gca atc ctt acg gct aag cag gtt      1803
Val Arg Ser Thr Asn Ala Thr Ser Ala Ile Leu Thr Ala Lys Gln Val
            545                 550                 555

tta tct cca gga ttt tat gaa atc cca atc ctg gtg aag gac agc tat      1851
Leu Ser Pro Gly Phe Tyr Glu Ile Pro Ile Leu Val Lys Asp Ser Tyr
        560                 565                 570

aac aga gca tgt gaa ttg gca caa atg gtg cag tta tat gcc tgt gat      1899
Asn Arg Ala Cys Glu Leu Ala Gln Met Val Gln Leu Tyr Ala Cys Asp
    575                 580                 585

tgc gat gac aac cac atg tgc ctg gac tct ggt gcc gcg ggc atc tac      1947
Cys Asp Asp Asn His Met Cys Leu Asp Ser Gly Ala Ala Gly Ile Tyr
    590                 595                 600

aca gag gac ata act ggt gac acg tat ggg cct gtc act gaa gac caa      1995
Thr Glu Asp Ile Thr Gly Asp Thr Tyr Gly Pro Val Thr Glu Asp Gln
605                 610                 615                 620

gct gga gtt tca aat gtt ggt ctt gga cca gca ggg att ggc atg atg      2043
Ala Gly Val Ser Asn Val Gly Leu Gly Pro Ala Gly Ile Gly Met Met
            625                 630                 635

gtt ctg ggc atc ctg cta ctg att ttg gct cca ctc ttg ctg ctc ctg      2091
Val Leu Gly Ile Leu Leu Leu Ile Leu Ala Pro Leu Leu Leu Leu Leu
        640                 645                 650

tgt tgc tgc aaa cag aga cag cca gaa ggc ctg gga aca aga ttt gct      2139
Cys Cys Cys Lys Gln Arg Gln Pro Glu Gly Leu Gly Thr Arg Phe Ala
    655                 660                 665

cct gtg cct gag ggc gga gaa gga gtg atg cag tct tgg aga att gaa      2187
Pro Val Pro Glu Gly Gly Glu Gly Val Met Gln Ser Trp Arg Ile Glu
    670                 675                 680

ggg gcc cat ccc gag gac agg gat gtg tca aat ata tgt gca ccc atg      2235
Gly Ala His Pro Glu Asp Arg Asp Val Ser Asn Ile Cys Ala Pro Met
685                 690                 695                 700

aca gcc tca aat acc cag gat cgg atg gat tcc tct gaa atc tac acc      2283
Thr Ala Ser Asn Thr Gln Asp Arg Met Asp Ser Ser Glu Ile Tyr Thr
            705                 710                 715

aac acc tat gca gcc ggg ggc acg gtg gaa gga ggt gta tcg gga gtg      2331
Asn Thr Tyr Ala Ala Gly Gly Thr Val Glu Gly Gly Val Ser Gly Val
        720                 725                 730

gag ctc aac aca ggt atg ggg aca gcc gtt ggc ctc atg gcc gca ggg      2379
Glu Leu Asn Thr Gly Met Gly Thr Ala Val Gly Leu Met Ala Ala Gly
    735                 740                 745

gcc gca gga gcc tca ggg gcc gca agg aag agg agc tct acc atg gga      2427
Ala Ala Gly Ala Ser Gly Ala Ala Arg Lys Arg Ser Ser Thr Met Gly
    750                 755                 760

acc ctg cgg gac tac gct gac gca gac atc aac atg gct ttc ttg gac      2475
Thr Leu Arg Asp Tyr Ala Asp Ala Asp Ile Asn Met Ala Phe Leu Asp
765                 770                 775                 780

agc tac ttc tcg gag aaa gcg tat gct tat gca gat gaa gat gaa ggt      2523
Ser Tyr Phe Ser Glu Lys Ala Tyr Ala Tyr Ala Asp Glu Asp Glu Gly
            785                 790                 795

cga cca gcc aat gac tgc ttg ctc att tat gac cac gag gga gtc ggg      2571

Arg Pro Ala Asn Asp Cys Leu Leu Ile Tyr Asp His Glu Gly Val Gly
            800                 805                 810

tct ccc gta ggc tct att ggt tgt tgc agt tgg att gtg gat gac tta      2619
Ser Pro Val Gly Ser Ile Gly Cys Cys Ser Trp Ile Val Asp Asp Leu
        815                 820                 825

gat gaa agc tgc atg gaa act tta gat cca aaa ttt agg act ctt gct      2667
Asp Glu Ser Cys Met Glu Thr Leu Asp Pro Lys Phe Arg Thr Leu Ala
    830                 835                 840

gag atc tgc tta aac aca gaa att gaa cca ttt cct tca cac cag gct      2715
Glu Ile Cys Leu Asn Thr Glu Ile Glu Pro Phe Pro Ser His Gln Ala
845                 850                 855                 860

tgt ata cca atc agt act gac ctc cct ttg ctc gga cct aat tac ttt      2763
Cys Ile Pro Ile Ser Thr Asp Leu Pro Leu Leu Gly Pro Asn Tyr Phe
                865                 870                 875

gtt aat gaa tct tca gga ttg act ccc tca gaa gtt gaa ttc caa gaa      2811
Val Asn Glu Ser Ser Gly Leu Thr Pro Ser Glu Val Glu Phe Gln Glu
            880                 885                 890

gaa atg gca gca tct gaa ccc gtg gtc cat ggg gat att att gtg act      2859
Glu Met Ala Ala Ser Glu Pro Val Val His Gly Asp Ile Ile Val Thr
        895                 900                 905

gag act tac ggt aat gct gat cca tgt gtg caa ccc act aca att att      2907
Glu Thr Tyr Gly Asn Ala Asp Pro Cys Val Gln Pro Thr Thr Ile Ile
    910                 915                 920

ttt gat cct cag ctt gca ccc aat gtt gta gta acc gaa gca gta atg      2955
Phe Asp Pro Gln Leu Ala Pro Asn Val Val Val Thr Glu Ala Val Met
925                 930                 935                 940

gca cct gtc tat gat att caa ggg aat att tgt gta cct gct gag tta      3003
Ala Pro Val Tyr Asp Ile Gln Gly Asn Ile Cys Val Pro Ala Glu Leu
                945                 950                 955

gca gat tac aac aat gta atc tat gct gag aga gta ctg gct agt cct      3051
Ala Asp Tyr Asn Asn Val Ile Tyr Ala Glu Arg Val Leu Ala Ser Pro
            960                 965                 970

ggt gtg cct gac atg agc aat agt agc acg act gag ggt tgt atg gga      3099
Gly Val Pro Asp Met Ser Asn Ser Ser Thr Thr Glu Gly Cys Met Gly
        975                 980                 985

cct gtg atg agc ggc aat att tta gta ggg cca gaa  att caa gtg atg      3147
Pro Val Met Ser Gly Asn Ile Leu Val Gly Pro Glu  Ile Gln Val Met
    990                 995                 1000

caa  atg atg agt cca gac  ctt ccc ata ggc caa  acc gtt ggc tcc      3192
Gln  Met Met Ser Pro Asp  Leu Pro Ile Gly Gln  Thr Val Gly Ser
1005                 1010                 1015

aca  tcc ccc atg aca tct  cga cac aga gta aca  cga tac agt aac      3237
Thr  Ser Pro Met Thr Ser  Arg His Arg Val Thr  Arg Tyr Ser Asn
1020                 1025                 1030

ata  cat tac acc caa cag  taa gtgctttatg gtcagtattc tatgtggaga      3288
Ile  His Tyr Thr Gln Gln
1035                 1040

ccttgcacct tgtaatcatc aatacatcca ccaaaaatat ataatgtacc atatatatta      3348

atagtcaaca aatactcaga tattctaagg tcaatgccat tatttgatta taccattttg      3408

agggtgaata tggctaggca ctttagataa gcctttttaa aattctttct gattttaaat      3468

aatgcgtcaa aaaatgtgca gaaaatgtat tgcatccctt gatactgtct aacgaatagc 3528

acataactca tattgtgaat cctatgggtc ttgaggcctg tagaaccaat c 3579

<210> 76
<211> 1040
<212> PRT
<213> Homo sapiens

<400> 76

Met Asp Trp Leu Phe Phe Arg Asn Ile Cys Leu Leu Ile Ile Leu Met
1               5                   10                  15

Val Val Met Glu Val Asn Ser Glu Phe Ile Val Glu Val Lys Glu Phe
            20                  25                  30

Asp Ile Glu Asn Gly Thr Thr Lys Trp Gln Thr Val Arg Arg Gln Lys
        35                  40                  45

Arg Glu Trp Ile Lys Phe Ala Ala Ala Cys Arg Glu Gly Glu Asp Asn
    50                  55                  60

Ser Lys Arg Asn Pro Ile Ala Lys Ile Arg Ser Asp Cys Glu Ser Asn
65                  70                  75                  80

Gln Lys Ile Thr Tyr Arg Ile Ser Gly Val Gly Ile Asp Arg Pro Pro
                85                  90                  95

Tyr Gly Val Phe Thr Ile Asn Pro Arg Thr Gly Glu Ile Asn Ile Thr
            100                 105                 110

Ser Val Val Asp Arg Glu Ile Thr Pro Leu Phe Leu Ile Tyr Cys Arg
        115                 120                 125

Ala Leu Asn Ser Arg Gly Glu Asp Leu Glu Arg Pro Leu Glu Leu Arg
    130                 135                 140

Val Lys Val Met Asp Ile Asn Asp Asn Ala Pro Val Phe Ser Gln Ser
145                 150                 155                 160

Val Tyr Thr Ala Ser Ile Glu Glu Asn Ser Asp Ala Asn Thr Leu Val
                165                 170                 175

Val Lys Leu Cys Ala Thr Asp Ala Asp Glu Glu Asn His Leu Asn Ser
            180                 185                 190

Lys Ile Ala Tyr Lys Ile Val Ser Gln Glu Pro Ser Gly Ala Pro Met
        195                 200                 205

Phe Ile Leu Asn Arg Tyr Thr Gly Glu Val Cys Thr Met Ser Ser Phe
    210                 215                 220

Leu Asp Arg Glu Gln His Ser Met Tyr Asn Leu Val Val Arg Gly Ser
225                 230                 235                 240

Asp Arg Asp Gly Ala Ala Asp Gly Leu Ser Ser Glu Cys Asp Cys Arg
                245                 250                 255

Ile Lys Val Leu Asp Val Asn Asp Asn Phe Pro Thr Leu Glu Lys Thr
            260                 265                 270

Ser Tyr Ser Ala Ser Ile Glu Glu Asn Cys Leu Ser Ser Glu Leu Ile
        275                 280                 285

Arg Leu Gln Ala Ile Asp Leu Asp Glu Glu Gly Thr Asp Asn Trp Leu
    290                 295                 300

Ala Gln Tyr Leu Ile Leu Ser Gly Asn Asp Gly Asn Trp Phe Asp Ile
305                 310                 315                 320

Gln Thr Asp Pro Gln Thr Asn Glu Gly Ile Leu Lys Val Val Lys Met
                325                 330                 335

Leu Asp Tyr Glu Gln Ala Pro Asn Ile Gln Leu Ser Ile Gly Val Lys
            340                 345                 350

Asn Gln Ala Asp Phe His Tyr Ser Val Ala Ser Gln Phe Gln Met His
        355                 360                 365

Pro Thr Pro Val Arg Ile Gln Val Val Asp Val Arg Glu Gly Pro Ala
    370                 375                 380

Phe His Pro Ser Thr Met Ala Phe Ser Val Arg Glu Gly Ile Lys Gly
385                 390                 395                 400

Ser Ser Leu Leu Asn Tyr Val Leu Gly Thr Tyr Thr Ala Ile Asp Leu
                405                 410                 415

Asp Thr Gly Asn Pro Ala Thr Asp Val Arg Tyr Ile Ile Gly His Asp
            420                 425                 430

Ala Gly Ser Trp Leu Lys Ile Asp Ser Arg Thr Gly Glu Ile Gln Phe
        435                 440                 445

Ser Arg Glu Phe Asp Lys Lys Ser Lys Tyr Ile Ile Asn Gly Ile Tyr
    450                 455                 460

Thr Ala Glu Ile Leu Ala Ile Asp Asp Gly Ser Gly Lys Thr Ala Thr
465                 470                 475                 480

Gly Thr Ile Cys Ile Glu Val Pro Asp Ile Asn Asp Tyr Cys Pro Asn
                485                 490                 495

Ile Phe Pro Glu Arg Arg Thr Ile Cys Ile Asp Ser Pro Ser Val Leu
            500                 505                 510

Ile Ser Val Asn Glu His Ser Tyr Gly Ser Pro Phe Thr Phe Cys Val
        515                 520                 525

Val Asp Glu Pro Pro Gly Ile Ala Asp Met Trp Asp Val Arg Ser Thr
    530                 535                 540

Asn Ala Thr Ser Ala Ile Leu Thr Ala Lys Gln Val Leu Ser Pro Gly
545                 550                 555                 560

Phe Tyr Glu Ile Pro Ile Leu Val Lys Asp Ser Tyr Asn Arg Ala Cys
                565                 570                 575

Glu Leu Ala Gln Met Val Gln Leu Tyr Ala Cys Asp Cys Asp Asp Asn
            580                 585                 590

His Met Cys Leu Asp Ser Gly Ala Ala Gly Ile Tyr Thr Glu Asp Ile
        595                 600                 605

Thr Gly Asp Thr Tyr Gly Pro Val Thr Glu Asp Gln Ala Gly Val Ser
    610                 615                 620

Asn Val Gly Leu Gly Pro Ala Gly Ile Gly Met Met Val Leu Gly Ile
625                 630                 635                 640

Leu Leu Leu Ile Leu Ala Pro Leu Leu Leu Leu Leu Cys Cys Cys Lys
                645                 650                 655

Gln Arg Gln Pro Glu Gly Leu Gly Thr Arg Phe Ala Pro Val Pro Glu
            660                 665                 670

Gly Gly Glu Gly Val Met Gln Ser Trp Arg Ile Glu Gly Ala His Pro
        675                 680                 685

Glu Asp Arg Asp Val Ser Asn Ile Cys Ala Pro Met Thr Ala Ser Asn
    690                 695                 700

Thr Gln Asp Arg Met Asp Ser Ser Glu Ile Tyr Thr Asn Thr Tyr Ala
705                 710                 715                 720

Ala Gly Gly Thr Val Glu Gly Gly Val Ser Gly Val Glu Leu Asn Thr
                725                 730                 735

Gly Met Gly Thr Ala Val Gly Leu Met Ala Ala Gly Ala Ala Gly Ala
            740                 745                 750

Ser Gly Ala Ala Arg Lys Arg Ser Ser Thr Met Gly Thr Leu Arg Asp
        755                 760                 765

Tyr Ala Asp Ala Asp Ile Asn Met Ala Phe Leu Asp Ser Tyr Phe Ser
    770                 775                 780

Glu Lys Ala Tyr Ala Tyr Ala Asp Glu Asp Glu Gly Arg Pro Ala Asn
785                 790                 795                 800

Asp Cys Leu Leu Ile Tyr Asp His Glu Gly Val Gly Ser Pro Val Gly
                805                 810                 815

Ser Ile Gly Cys Cys Ser Trp Ile Val Asp Asp Leu Asp Glu Ser Cys
            820                 825                 830

Met Glu Thr Leu Asp Pro Lys Phe Arg Thr Leu Ala Glu Ile Cys Leu
        835                 840                 845

Asn Thr Glu Ile Glu Pro Phe Pro Ser His Gln Ala Cys Ile Pro Ile
    850                 855                 860

Ser Thr Asp Leu Pro Leu Leu Gly Pro Asn Tyr Phe Val Asn Glu Ser
865                 870                 875                 880

Ser Gly Leu Thr Pro Ser Glu Val Glu Phe Gln Glu Glu Met Ala Ala
                885                 890                 895

Ser Glu Pro Val Val His Gly Asp Ile Ile Val Thr Glu Thr Tyr Gly
            900                 905                 910

Asn Ala Asp Pro Cys Val Gln Pro Thr Thr Ile Ile Phe Asp Pro Gln
        915                 920                 925

Leu Ala Pro Asn Val Val Val Thr Glu Ala Val Met Ala Pro Val Tyr
    930                 935                 940

Asp Ile Gln Gly Asn Ile Cys Val Pro Ala Glu Leu Ala Asp Tyr Asn
945                 950                 955                 960

Asn Val Ile Tyr Ala Glu Arg Val Leu Ala Ser Pro Gly Val Pro Asp
                965                 970                 975

Met Ser Asn Ser Ser Thr Thr Glu Gly Cys Met Gly Pro Val Met Ser
            980                 985                 990

Gly Asn Ile Leu Val Gly Pro Glu Ile Gln Val Met Gln Met Met Ser
        995                 1000                1005

Pro Asp Leu Pro Ile Gly Gln Thr Val Gly Ser Thr Ser Pro Met
    1010                1015                1020

Thr Ser Arg His Arg Val Thr Arg Tyr Ser Asn Ile His Tyr Thr
    1025                1030                1035

Gln Gln
    1040

<210>  77
<211>  26
<212>  DNA
<213>  künstliche Sequenz

<220>
<223>  Beschreibung der künstlichen Sequenz: Oligonukleotid

<400>  77
gggatataca cagcagagat cctggc                                          26

<210>  78
<211>  25
<212>  DNA
<213>  künstliche Sequenz

<220>
<223>  Beschreibung der künstlichen Sequenz: Oligonukleotid

<400>  78
ttagccgtaa ggattgccga ggtag                                           25

<210>  79
<211>  279
<212>  DNA
<213>  Homo sapiens

<400>  79
gggatataca cagcagagat cctggctata gatgatggct ctggaaaaac agctacagga      60

accatatgta ttgaggttcc tgatatcaat gattattgtc caaacatttt tcctgaaaga     120

agaaccatct gcattgactc tccatcagtc cttatctctg ttaatgaaca ttcttatggg     180

tctccgttta ctttctgtgt tgttgatgag ccaccaggaa tagctgacat gtgggatgtc     240

agatcaacaa atgctacctc ggcaatcctt acggctaag                            279

<210>  80
<211>  659
<212>  DNA
<213>  Homo sapiens

<400>  80
cagatatatc atagggcatg atgcaggcag ctggttaaaa attgattcaa gaactggtga      60

gatacaattt tctagagaat ttgataagaa gtcaaaatat attatcaatg ggatatacac     120

agcagagatc ctggctatag atgatggctc tggaaaaaca gctacaggaa ccatatgtat     180

tgaggttcct gatatcaatg attattgtcc aaacattttt cctgaaagaa gaaccatctg     240

cattgactct ccatcagtcc ttatctctgt taatgaacat tcttatgggt ctccgtttac     300

tttctgtgtt gttgatgagc caccaggaat agctgacatg tgggatgtca gatcaacaaa     360

tgctacctcg gcaatcctta cggctaagca ggttttatct ccaggatttt atgaaatccc     420

aatcctggtg aaggacagct ataacagagc atgtgaattg gcacaaatgg tgcagttata     480

tgcctgtgat tgcgatgaca accacatgtg cctggactct ggtgccgcgg gcatctacac 540
agaggacata actggtgaca cgtatgggcc tgtcactgaa gaccaagctg gagtttcaaa 600
tgttggtctt ggaccagcag ggattggcat gatggttctg ggcatcctgc tactgattt 659

<210> 81
<211> 93
<212> PRT
<213> Homo sapiens

<400> 81

Gly Ile Tyr Thr Ala Glu Ile Leu Ala Ile Asp Asp Gly Ser Gly Lys
1               5                   10                  15

Thr Ala Thr Gly Thr Ile Cys Ile Glu Val Pro Asp Ile Asn Asp Tyr
            20                  25                  30

Cys Pro Asn Ile Phe Pro Glu Arg Arg Thr Ile Cys Ile Asp Ser Pro
        35                  40                  45

Ser Val Leu Ile Ser Val Asn Glu His Ser Tyr Gly Ser Pro Phe Thr
    50                  55                  60

Phe Cys Val Val Asp Glu Pro Pro Gly Ile Ala Asp Met Trp Asp Val
65                  70                  75                  80

Arg Ser Thr Asn Ala Thr Ser Ala Ile Leu Thr Ala Lys
                85                  90

<210> 82
<211> 224
<212> PRT
<213> Homo sapiens

<400> 82

Val Arg Tyr Ile Ile Gly His Asp Ala Gly Ser Trp Leu Lys Ile Asp
1               5                   10                  15

Ser Arg Thr Gly Glu Ile Gln Phe Ser Arg Glu Phe Asp Lys Lys Ser
            20                  25                  30

Lys Tyr Ile Ile Asn Gly Ile Tyr Thr Ala Glu Ile Leu Ala Ile Asp
        35                  40                  45

Asp Gly Ser Gly Lys Thr Ala Thr Gly Thr Ile Cys Ile Glu Val Pro
    50                  55                  60

Asp Ile Asn Asp Tyr Cys Pro Asn Ile Phe Pro Glu Arg Arg Thr Ile
65                  70                  75                  80

Cys Ile Asp Ser Pro Ser Val Leu Ile Ser Val Asn Glu His Ser Tyr
                85                  90                  95

Gly Ser Pro Phe Thr Phe Cys Val Val Asp Glu Pro Pro Gly Ile Ala
            100                 105                 110

Asp Met Trp Asp Val Arg Ser Thr Asn Ala Thr Ser Ala Ile Leu Thr
        115                 120                 125

Ala Lys Gln Val Leu Ser Pro Gly Phe Tyr Glu Ile Pro Ile Leu Val
    130                 135                 140

Lys Asp Ser Tyr Asn Arg Ala Cys Glu Leu Ala Gln Met Val Gln Leu
145                 150                 155                 160

Tyr Ala Cys Asp Cys Asp Asp Asn His Met Cys Leu Asp Ser Gly Ala
                165                 170                 175

Ala Gly Ile Tyr Thr Glu Asp Ile Thr Gly Asp Thr Tyr Gly Pro Val
            180                 185                 190

Thr Glu Asp Gln Ala Gly Val Ser Asn Val Gly Leu Gly Pro Ala Gly
        195                 200                 205

Ile Gly Met Met Val Leu Gly Ile Leu Leu Leu Ile Leu Ala Pro Leu
    210                 215                 220

<210>  83
<211>  27
<212>  DNA
<213>  künstliche Sequenz

<220>
<223>  Beschreibung der künstlichen Sequenz: Oligonukleotid

<400>  83
aagacgaggg ctcaaattga atctcac                                          27

<210>  84
<211>  24
<212>  DNA
<213>  künstliche Sequenz

<220>
<223>  Beschreibung der künstlichen Sequenz: Oligonukleotid

<400>  84
cgtgaattca gagcccggca atag                                             24

<210>  85
<211>  973
<212>  DNA
<213>  Homo sapiens

<400>  85
caccacagtt atcacccatg ccctcctaaa agggtgtctc aaagcatatc tttctgtaga      60

gcagaattcg gaactgagaa gacgagggct caaattgaat ctcacaggat ttgcgtgcaa     120

gagaaaccca aaggaatgga ttggctcttc ttcagaaaca tttgcctttt gatcattcta     180

atgcaacaca gtatgtacaa cctggttgtg agaggctcag atcgggatgg agctgcagat 240
ggactgtctt ctgagtgtga ctgtagaatc aaggttttag acgtcaacga taatttcccc 300
accttagaga aaacttcata tatcataggg catgatgcag gcagctggtt aaaaattgat 360
tcaagaactg gtgagataca attttctaga gaatttgata agaagtcaaa atatattatc 420
aatgggatat acacagcaga gatcctggct atagatgatg gctctggaaa aacagctaca 480
ggaaccatat gtattgaggt tcctgatatc aatgattatt gtccaaacat ttttcctgaa 540
agaagaacca tctgcattga ctctccatca gtccttatct ctgttaatga acattcttat 600
gggtctccgt ttactttctg tgttgttgat gagccaccag gaatagctga catgtgggat 660
gtcagatcaa caaatgctac ctcggcaatc cttacggcta agcaggtttt atctccagga 720
ttttatgaaa tcccaatcct ggtgaaggac agctataaca gagcatgtga attggcacaa 780
atggtgcagt tatatgcctg tgattgcgat gacaaccaca tgtgcctgga ctctggtgcc 840
gcgggcatct acacagagga cataactggt gacacgtatg ggcctgtcac tgaagaccaa 900
gctggagttt caaatgttgg tcttggacca gcagggattg gcatgatggt tctgggcatc 960
ctgctactga ttt 973

<210> 86
<211> 279
<212> PRT
<213> Homo sapiens

<400> 86

Met Asp Trp Leu Phe Phe Arg Asn Ile Cys Leu Leu Ile Ile Leu Met
1               5                   10                  15

Gln His Ser Met Tyr Asn Leu Val Val Arg Gly Ser Asp Arg Asp Gly
            20                  25                  30

Ala Ala Asp Gly Leu Ser Ser Glu Cys Asp Cys Arg Ile Lys Val Leu
        35                  40                  45

Asp Val Asn Asp Asn Phe Pro Thr Leu Glu Lys Thr Ser Tyr Ile Ile
    50                  55                  60

Gly His Asp Ala Gly Ser Trp Leu Lys Ile Asp Ser Arg Thr Gly Glu
65                  70                  75                  80

Ile Gln Phe Ser Arg Glu Phe Asp Lys Lys Ser Lys Tyr Ile Ile Asn
                85                  90                  95

Gly Ile Tyr Thr Ala Glu Ile Leu Ala Ile Asp Asp Gly Ser Gly Lys
            100                 105                 110

Thr Ala Thr Gly Thr Ile Cys Ile Glu Val Pro Asp Ile Asn Asp Tyr
        115                 120                 125

Cys Pro Asn Ile Phe Pro Glu Arg Arg Thr Ile Cys Ile Asp Ser Pro
    130                 135                 140

Ser Val Leu Ile Ser Val Asn Glu His Ser Tyr Gly Ser Pro Phe Thr
145                 150                 155                 160

Phe Cys Val Val Asp Glu Pro Pro Gly Ile Ala Asp Met Trp Asp Val
                165                 170                 175

Arg Ser Thr Asn Ala Thr Ser Ala Ile Leu Thr Ala Lys Gln Val Leu
            180                 185                 190

Ser Pro Gly Phe Tyr Glu Ile Pro Ile Leu Val Lys Asp Ser Tyr Asn
        195                 200                 205

Arg Ala Cys Glu Leu Ala Gln Met Val Gln Leu Tyr Ala Cys Asp Cys
    210                 215                 220

Asp Asp Asn His Met Cys Leu Asp Ser Gly Ala Ala Gly Ile Tyr Thr
225                 230                 235                 240

Glu Asp Ile Thr Gly Asp Thr Tyr Gly Pro Val Thr Glu Asp Gln Ala
                245                 250                 255

Gly Val Ser Asn Val Gly Leu Gly Pro Ala Gly Ile Gly Met Met Val
            260                 265                 270

Leu Gly Ile Leu Leu Leu Ile
        275

<210>  87
<211>  964
<212>  DNA
<213>  Homo sapiens

<400>  87
caccacagtt atcacccatg ccctcctaaa agggtgtctc aaagcatatc tttctgtaga      60

gcagaattcg gaactgagaa gacgagggct caaattgaat ctcacaggat ttgcgtgcaa     120

gagaaaccca aaggaatgga ttggctcttc ttcagaaaca tttgcctttt gatcattcta     180

atggaatttg acattgaaaa tggcactaca aaatggcaaa cagtcagaag acaaaagcgg     240

gagtggatca agtttgccgc agcctgtcga gaaggagagg acaactcgaa gaggaacccc     300

attgccaaaa ttcgaatagg gcatgatgca ggcagctggt taaaaattga ttcaagaact     360

ggtgagatac aattttctag agaatttgat aagaagtcaa aatatattat caatgggata     420

tacacagcag agatcctggc tatagatgat ggctctggaa aaacagctac aggaaccata     480

tgtattgagg ttcctgatat caatgattat tgtccaaaca tttttcctga aagaagaacc     540

atctgcattg actctccatc agtccttatc tctgttaatg aacattctta tgggtctccg     600

tttactttct gtgttgttga tgagccacca ggaatagctg acatgtggga tgtcagatca     660

acaaatgcta cctcggcaat ccttacggct aagcaggttt tatctccagg attttatgaa 720
atcccaatcc tggtgaagga cagctataac agagcatgtg aattggcaca aatggtgcag 780
ttatatgcct gtgattgcga tgacaaccac atgtgcctgg actctggtgc cgcgggcatc 840
tacacagagg acataactgg tgacacgtat gggcctgtca ctgaagacca agctggagtt 900
tcaaatgttg gtcttggacc agcagggatt ggcatgatgg ttctgggcat cctgctactg 960
attt 964

<210> 88
<211> 276
<212> PRT
<213> Homo sapiens

<400> 88

Met Asp Trp Leu Phe Phe Arg Asn Ile Cys Leu Leu Ile Ile Leu Met
1               5                   10                  15

Glu Phe Asp Ile Glu Asn Gly Thr Thr Lys Trp Gln Thr Val Arg Arg
            20                  25                  30

Gln Lys Arg Glu Trp Ile Lys Phe Ala Ala Ala Cys Arg Glu Gly Glu
        35                  40                  45

Asp Asn Ser Lys Arg Asn Pro Ile Ala Lys Ile Arg Ile Gly His Asp
    50                  55                  60

Ala Gly Ser Trp Leu Lys Ile Asp Ser Arg Thr Gly Glu Ile Gln Phe
65                  70                  75                  80

Ser Arg Glu Phe Asp Lys Lys Ser Lys Tyr Ile Ile Asn Gly Ile Tyr
                85                  90                  95

Thr Ala Glu Ile Leu Ala Ile Asp Asp Gly Ser Gly Lys Thr Ala Thr
            100                 105                 110

Gly Thr Ile Cys Ile Glu Val Pro Asp Ile Asn Asp Tyr Cys Pro Asn
        115                 120                 125

Ile Phe Pro Glu Arg Arg Thr Ile Cys Ile Asp Ser Pro Ser Val Leu
    130                 135                 140

Ile Ser Val Asn Glu His Ser Tyr Gly Ser Pro Phe Thr Phe Cys Val
145                 150                 155                 160

Val Asp Glu Pro Pro Gly Ile Ala Asp Met Trp Asp Val Arg Ser Thr
                165                 170                 175

Asn Ala Thr Ser Ala Ile Leu Thr Ala Lys Gln Val Leu Ser Pro Gly
            180                 185                 190

Phe Tyr Glu Ile Pro Ile Leu Val Lys Asp Ser Tyr Asn Arg Ala Cys
        195                 200                 205

Glu Leu Ala Gln Met Val Gln Leu Tyr Ala Cys Asp Cys Asp Asp Asn
    210                 215                 220

His Met Cys Leu Asp Ser Gly Ala Ala Gly Ile Tyr Thr Glu Asp Ile
225                 230                 235                 240

Thr Gly Asp Thr Tyr Gly Pro Val Thr Glu Asp Gln Ala Gly Val Ser
                245                 250                 255

Asn Val Gly Leu Gly Pro Ala Gly Ile Gly Met Met Val Leu Gly Ile
            260                 265                 270

Leu Leu Leu Ile
        275

<210>  89
<211>  26
<212>  DNA
<213>  künstliche Sequenz

<220>
<223>  Beschreibung der künstlichen Sequenz: Oligonukleotid

<400>  89
aatgataacg ctccagtctt ttcgca                                          26

<210>  90
<211>  27
<212>  DNA
<213>  künstliche Sequenz

<220>
<223>  Beschreibung der künstlichen Sequenz: Oligonukleotid

<400>  90
gccttcttca tcaagatcaa ttgcttg                                         27

<210>  91
<211>  26
<212>  DNA
<213>  künstliche Sequenz

<220>
<223>  Beschreibung der künstlichen Sequenz: Oligonukleotid

<400>  91
caccatgaat tgtgatgctt tgctac                                          26

<210>  92
<211>  22
<212>  DNA
<213>  künstliche Sequenz

<220>
<223>  Beschreibung der künstlichen Sequenz: Oligonukleotid

<400>  92
ctaggtagct ggtcttgggg aa                                              22

<210>  93
<211>  21
<212>  DNA
<213>  künstliche Sequenz

<220>
<223>  Beschreibung der künstlichen Sequenz: Oligonukleotid

<400>  93
ttctcagcct ccagaatgaa g                                               21

<210>  94
<211>  21
<212>  DNA
<213>  künstliche Sequenz

<220>
<223>  Beschreibung der künstlichen Sequenz: Oligonukleotid

<400>  94
gaaggtaagg acagacgtgt c                                               21

<210>  95
<211>  27
<212>  DNA
<213>  künstliche Sequenz

<220>
<223>  Beschreibung der künstlichen Sequenz: Oligonukleotid

<400>  95
accaatgaag gcattttgaa agttgtc                                         27

<210>  96
<211>  12
<212>  PRT
<213>  Homo sapiens

<400>  96

Thr His Ser Phe Thr Ala Phe Lys Arg His Val Cys
1               5                   10

<210>  97
<211>  13
<212>  PRT
<213>  Homo sapiens

<400>  97

Asn Leu Ser Leu Pro Pro Ser Leu Ser Leu Ser Ile Cys
1               5                   10

<210>  98
<211>  16
<212>  PRT
<213>  Homo sapiens

<400>  98

Glu Arg Pro Ser Ser Val Leu Thr Ile Tyr Asp Ile Gly Ile Gln Cys
1               5                   10                  15

<210>  99
<211>  15
<212>  PRT
<213>  Homo sapiens

<400>  99

Cys Tyr Gln Gln Tyr Thr Asn Leu Gln Glu Arg Pro Ser Ser Val
1               5                   10                  15

<210>  100
<211>  15
<212>  PRT
<213>  Homo sapiens

<400>  100

<210>  101
<211>  17
<212>  PRT
<213>  Homo sapiens

<400>  101

Cys Ser Arg Lys Lys Arg Ala Asp Lys Lys Glu Asn Gly Thr Lys Leu
1               5                   10                  15

Leu

<210>  102
<211>  3439
<212>  DNA
<213>  Homo sapiens

<400>  102
caccacagtt atcacccatg ccctcctaaa agggtgtctc aaagcatatc tttctgtaga      60

gcagaattcg gaactgagaa gacgagggct caaattgaat ctcacaggat ttgcgtgcaa     120

gagaaaccca aaggaatgga ttggctcttc ttcagaaaca tttgcctttt gatcattcta     180

atggtggtga tggaagtaaa cagtgaattt attgttgagg tgaaggaatt tgacattgaa     240

aatggcacta caaaatggca aacagtcaga agacaaaagc gggagtggat caagtttgcc     300

gcagcctgtc gagaaggaga ggacaactcg aagaggaacc ccattgccaa aattcgatca     360

gactgcgaat cgaaccagaa gataacatac cggatttctg gagtagggat tgatcgacca     420

ccatatgggg tattcaccat taatcctcgc actggggaaa ttaacatcac ttcagtggta     480

gacagagaaa taactccact tttcttgatc tattgccggg ctctgaattc acggggtgaa     540

gatttagaaa ggcctcttga gcttagagtc aaagttatgg acataaatga taacgctcca     600

gtcttttcgc aaagtgtata cacagccagc attgaagaaa atagtgatgc caatacattg 660
gtagtaaagt tatgtgccac agatgcagat gaagaaaatc atctgaattc taaaattgcc 720
tacaagatcg tctctcagga gccatcaggt gcacccatgt tcattctgaa taggtacact 780
ggagaagtct gcaccatgtc cagtttcttg gacagagagc aacacagtat gtacaacctg 840
gttgtgagag gctcagatcg ggatggagct gcagatggac tgtcttctga gtgtgactgt 900
agaatcaagg ttttagacgt caacgataat ttccccacct tagagaaaac ttcatactca 960
gccagtattg aagagaattg tttaagttcg gaactgatac gattacaagc aattgatctt 1020
gatgaagaag gcactgataa ctggttggct caatatttaa ttctctctgg aaatgatggg 1080
aattggttcg atattcaaac agatccacaa accaatgaag gcattttgaa agttgtcaag 1140
atgctggatt atgaacaagc acctaacatt cagcttagta tcggagttaa aaaccaagct 1200
gattttcact actccgttgc ttctcaattc caaatgcacc caacccctgt gagaattcaa 1260
gttgttgatg tgagagaagg acctgcattt catccaagta ctatggcttt tagtgtgcgg 1320
gaaggaataa aaggaagttc cttattgaat tatgtgcttg gcacatatac agccatagat 1380
ttggacacag gaaaccctgc aacagatgtc agatggctct ggaaaaacag ctacaggaac 1440
catatgtatt gaggttcctg atatcaatga ttattgtcca aacatttttc ctgaaagaag 1500
aaccatctgc attgactctc catcagtcct tatctctgtt aatgaacatt cttatgggtc 1560
tccgtttact ttctgtgttg ttgatgagcc accaggaata gctgacatgt gggatgtcag 1620
atcaacaaat gctacctcgg caatccttac ggctaagcag gttttatctc caggatttta 1680
tgaaatccca atcctggtga aggacagcta taacagagca tgtgaattgg cacaaatggt 1740
gcagttatat gcctgtgatt gcgatgacaa ccacatgtgc ctggactctg gtgccgcggg 1800
catctacaca gaggacataa ctggtgacac gtatgggcct gtcactgaag accaagctgg 1860
agtttcaaat gttggtcttg gaccagcagg gattggcatg atggttctgg gcatcctgct 1920
actgattttg gctccactct tgctgctcct gtgttgctgc aaacagagac agccagaagg 1980
cctgggaaca agatttgctc ctgtgcctga gggcggagaa ggagtgatgc agtcttggag 2040
aattgaaggg gcccatcccg aggacaggga tgtgtcaaat atatgtgcac ccatgacagc 2100
ctcaaatacc caggatcgga tggattcctc tgaaatctac accaacacct atgcagccgg 2160
gggcacggtg gaaggaggtg tatcgggagt ggagctcaac acaggtatgg ggacagccgt 2220
tggcctcatg gccgcagggg ccgcaggagc ctcaggggcc gcaaggaaga ggagctctac 2280
catgggaacc ctgcgggact acgctgacgc agacatcaac atggctttct tggacagcta 2340
cttctcggag aaagcgtatg cttatgcaga tgaagatgaa ggtcgaccag ccaatgactg 2400
cttgctcatt tatgaccacg agggagtcgg gtctcccgta ggctctattg gttgttgcag 2460
ttggattgtg gatgacttag atgaaagctg catggaaact ttagatccaa aatttaggac 2520
tcttgctgag atctgcttaa acacagaaat tgaaccattt ccttcacacc aggcttgtat 2580
accaatcagt actgacctcc ctttgctcgg acctaattac tttgttaatg aatcttcagg 2640

attgactccc tcagaagttg aattccaaga agaaatggca gcatctgaac ccgtggtcca 2700

tggggatatt attgtgactg agacttacgg taatgctgat ccatgtgtgc aacccactac 2760

aattattttt gatcctcagc ttgcacccaa tgttgtagta accgaagcag taatggcacc 2820

tgtctatgat attcaaggga atatttgtgt acctgctgag ttagcagatt acaacaatgt 2880

aatctatgct gagagagtac tggctagtcc tggtgtgcct gacatgagca atagtagcac 2940

gactgagggt tgtatgggac ctgtgatgag cggcaatatt ttagtagggc cagaaattca 3000

agtgatgcaa atgatgagtc cagaccttcc cataggccaa accgttggct ccacatcccc 3060

catgacatct cgacacagag taacacgata cagtaacata cattacaccc aacagtaagt 3120

gctttatggt cagtattcta tgtggagacc ttgcaccttg taatcatcaa tacatccacc 3180

aaaaatatat aatgtaccat atatattaat agtcaacaaa tactcagata ttctaaggtc 3240

aatgccatta tttgattata ccattttgag ggtgaatatg gctaggcact ttagataagc 3300

ctttttaaaa ttctttctga ttttaaataa tgcgtcaaaa aatgtgcaga aaatgtattg 3360

catcccttga tactgtctaa cgaatagcac ataactcata ttgtgaatcc tatgggtctt 3420

gaggcctgta gaaccaatc 3439

<210> 103
<211> 438
<212> PRT
<213> Homo sapiens

<400> 103

Met Asp Trp Leu Phe Phe Arg Asn Ile Cys Leu Leu Ile Ile Leu Met
1               5                   10                  15

Val Val Met Glu Val Asn Ser Glu Phe Ile Val Glu Val Lys Glu Phe
            20                  25                  30

Asp Ile Glu Asn Gly Thr Thr Lys Trp Gln Thr Val Arg Arg Gln Lys
        35                  40                  45

Arg Glu Trp Ile Lys Phe Ala Ala Ala Cys Arg Glu Gly Glu Asp Asn
    50                  55                  60

Ser Lys Arg Asn Pro Ile Ala Lys Ile Arg Ser Asp Cys Glu Ser Asn
65                  70                  75                  80

Gln Lys Ile Thr Tyr Arg Ile Ser Gly Val Gly Ile Asp Arg Pro Pro
                85                  90                  95

Tyr Gly Val Phe Thr Ile Asn Pro Arg Thr Gly Glu Ile Asn Ile Thr
            100                 105                 110

Ser Val Val Asp Arg Glu Ile Thr Pro Leu Phe Leu Ile Tyr Cys Arg
        115                 120                 125

Ala Leu Asn Ser Arg Gly Glu Asp Leu Glu Arg Pro Leu Glu Leu Arg
    130                 135                 140

Val Lys Val Met Asp Ile Asn Asp Asn Ala Pro Val Phe Ser Gln Ser
145                 150                 155                 160

Val Tyr Thr Ala Ser Ile Glu Glu Asn Ser Asp Ala Asn Thr Leu Val
                165                 170                 175

Val Lys Leu Cys Ala Thr Asp Ala Asp Glu Glu Asn His Leu Asn Ser
            180                 185                 190

Lys Ile Ala Tyr Lys Ile Val Ser Gln Glu Pro Ser Gly Ala Pro Met
        195                 200                 205

Phe Ile Leu Asn Arg Tyr Thr Gly Glu Val Cys Thr Met Ser Ser Phe
    210                 215                 220

Leu Asp Arg Glu Gln His Ser Met Tyr Asn Leu Val Val Arg Gly Ser
225                 230                 235                 240

Asp Arg Asp Gly Ala Ala Asp Gly Leu Ser Ser Glu Cys Asp Cys Arg
                245                 250                 255

Ile Lys Val Leu Asp Val Asn Asp Asn Phe Pro Thr Leu Glu Lys Thr
            260                 265                 270

Ser Tyr Ser Ala Ser Ile Glu Glu Asn Cys Leu Ser Ser Glu Leu Ile
        275                 280                 285

Arg Leu Gln Ala Ile Asp Leu Asp Glu Glu Gly Thr Asp Asn Trp Leu
    290                 295                 300

Ala Gln Tyr Leu Ile Leu Ser Gly Asn Asp Gly Asn Trp Phe Asp Ile
305                 310                 315                 320

Gln Thr Asp Pro Gln Thr Asn Glu Gly Ile Leu Lys Val Val Lys Met
                325                 330                 335

Leu Asp Tyr Glu Gln Ala Pro Asn Ile Gln Leu Ser Ile Gly Val Lys
            340                 345                 350

Asn Gln Ala Asp Phe His Tyr Ser Val Ala Ser Gln Phe Gln Met His
        355                 360                 365

Pro Thr Pro Val Arg Ile Gln Val Val Asp Val Arg Glu Gly Pro Ala
    370                 375                 380

Phe His Pro Ser Thr Met Ala Phe Ser Val Arg Glu Gly Ile Lys Gly
385                 390                 395                 400

Ser Ser Leu Leu Asn Tyr Val Leu Gly Thr Tyr Thr Ala Ile Asp Leu
                405                 410                 415

Asp Thr Gly Asn Pro Ala Thr Asp Val Arg Trp Leu Trp Lys Asn Ser
            420                 425                 430

Tyr Arg Asn His Met Tyr
        435

<210>  104
<211>  3360
<212>  DNA
<213>  Homo sapiens

<400>  104
caccacagtt atcacccatg ccctcctaaa agggtgtctc aaagcatatc tttctgtaga      60
gcagaattcg gaactgagaa gacgagggct caaattgaat ctcacaggat ttgcgtgcaa     120
gagaaaccca aaggaatgga ttggctcttc ttcagaaaca tttgcctttt gatcattcta     180
atggtggtga tggaagtaaa cagtgaattt attgttgagg tgaaggaatt tgacattgaa     240
aatggcacta caaaatggca aacagtcaga agacaaaagc gggagtggat caagtttgcc     300
gcagcctgtc gagaaggaga ggacaactcg aagaggaacc ccattgccaa aattcgatca     360
gactgcgaat cgaaccagaa gataacatac cggatttctg gagtagggat tgatcgacca     420
ccatatgggg tattcaccat taatcctcgc actggggaaa ttaacatcac ttcagtggta     480
gacagagaaa taactccact tttcttgatc tattgccggg ctctgaattc acggggtgaa     540
gatttagaaa ggcctcttga gcttagagtc aaagttatgg acataaatga taacgctcca     600
gtcttttcgc aaagtgtata cacagccagc attgaagaaa atagtgatgc caatacattg     660
gtagtaaagt tatgtgccac agatgcagat gaagaaaatc atctgaattc taaaattgcc     720
tacaagatcg tctctcagga gccatcaggt gcacccatgt tcattctgaa taggtacact     780
ggagaagtct gcaccatgtc cagtttcttg gacagagagc aacacagtat gtacaacctg     840
gttgtgagag gctcagatcg ggatggagct gcagatggac tgtcttctga gtgtgactgt     900
agaatcaagg ttttagacgt caacgataat ttccccacct tagagaaaac ttcatactca     960
gccagtattg aagagaattg tttaagttcg gaactgatac gattacaagc aattgatctt    1020
gatgaagaag gcactgataa ctggttggct caatatttaa ttctctctgg aaatgatggg    1080
aattggttcg atattcaaac agatccacaa accaatgaag gcattttgaa agttgtcaag    1140
atgctggatt atgaacaagc acctaacatt cagcttagta tcggagttaa aaaccaagct    1200
gattttcact actccgttgc ttctcaattc caaatgcacc caacccctgt gagaattcaa    1260
gttgttgatg tgagagaagg acctgcattt catccaagta ctatggcttt tagtgtgcgg    1320
gaaggaataa aaggaagttc cttattgaat tatgtgcttg gcacatatac agccatagat    1380
ttggacacag gaaaccctgc aacagatgtc agatatatca tagggcatga tgcaggcagc    1440
tggttaaaaa ttgattcaag aactggtgag atacaatttt ctagagaatt tgataagaag    1500

tcaaaatata ttatcaatgg gatatacaca gcagagatcc tggctataga tgctacctcg 1560
gcaatcctta cggctaagca ggttttatct ccaggatttt atgaaatccc aatcctggtg 1620
aaggacagct ataacagagc atgtgaattg gcacaaatgg tgcagttata tgcctgtgat 1680
tgcgatgaca accacatgtg cctggactct ggtgccgcgg gcatctacac agaggacata 1740
actggtgaca cgtatgggcc tgtcactgaa gaccaagctg gagtttcaaa tgttggtctt 1800
ggaccagcag ggattggcat gatggttctg ggcatcctgc tactgatttt ggctccactc 1860
ttgctgctcc tgtgttgctg caaacagaga cagccagaag gcctgggaac aagatttgct 1920
cctgtgcctg agggcggaga aggagtgatg cagtcttgga gaattgaagg ggcccatccc 1980
gaggacaggg atgtgtcaaa tatatgtgca cccatgacag cctcaaatac ccaggatcgg 2040
atggattcct ctgaaatcta caccaacacc tatgcagccg ggggcacggt ggaaggaggt 2100
gtatcgggag tggagctcaa cacaggtatg gggacagccg ttggcctcat ggccgcaggg 2160
gccgcaggag cctcaggggc cgcaaggaag aggagctcta ccatgggaac cctgcgggac 2220
tacgctgacg cagacatcaa catggctttc ttggacagct acttctcgga gaaagcgtat 2280
gcttatgcag atgaagatga aggtcgacca gccaatgact gcttgctcat ttatgaccac 2340
gagggagtcg ggtctcccgt aggctctatt ggttgttgca gttggattgt ggatgactta 2400
gatgaaagct gcatggaaac tttagatcca aaatttagga ctcttgctga gatctgctta 2460
aacacagaaa ttgaaccatt tccttcacac caggcttgta taccaatcag tactgacctc 2520
cctttgctcg gacctaatta ctttgttaat gaatcttcag gattgactcc ctcagaagtt 2580
gaattccaag aagaaatggc agcatctgaa cccgtggtcc atggggatat tattgtgact 2640
gagacttacg gtaatgctga tccatgtgtg caacccacta caattatttt tgatcctcag 2700
cttgcaccca atgttgtagt aaccgaagca gtaatggcac ctgtctatga tattcaaggg 2760
aatatttgtg tacctgctga gttagcagat tacaacaatg taatctatgc tgagagagta 2820
ctggctagtc ctggtgtgcc tgacatgagc aatagtagca cgactgaggg ttgtatggga 2880
cctgtgatga gcggcaatat tttagtaggg ccagaaattc aagtgatgca aatgatgagt 2940
ccagaccttc ccataggcca aaccgttggc tccacatccc ccatgacatc tcgacacaga 3000
gtaacacgat acagtaacat acattacacc caacagtaag tgctttatgg tcagtattct 3060
atgtggagac cttgcacctt gtaatcatca atacatccac caaaaatata taatgtacca 3120
tatatattaa tagtcaacaa atactcagat attctaaggt caatgccatt atttgattat 3180
accattttga gggtgaatat ggctaggcac tttagataag cctttttaaa attctttctg 3240
attttaaata atgcgtcaaa aaatgtgcag aaaatgtatt gcatcccttg atactgtcta 3300
acgaatagca cataactcat attgtgaatc ctatgggtct tgaggcctgt agaaccaatc 3360

<210> 105
<211> 967
<212> PRT
<213> Homo sapiens

<400> 105

Met Asp Trp Leu Phe Phe Arg Asn Ile Cys Leu Leu Ile Ile Leu Met
1               5                   10                  15

Val Val Met Glu Val Asn Ser Glu Phe Ile Val Glu Val Lys Glu Phe
            20                  25                  30

Asp Ile Glu Asn Gly Thr Thr Lys Trp Gln Thr Val Arg Arg Gln Lys
        35                  40                  45

Arg Glu Trp Ile Lys Phe Ala Ala Ala Cys Arg Glu Gly Glu Asp Asn
    50                  55                  60

Ser Lys Arg Asn Pro Ile Ala Lys Ile Arg Ser Asp Cys Glu Ser Asn
65                  70                  75                  80

Gln Lys Ile Thr Tyr Arg Ile Ser Gly Val Gly Ile Asp Arg Pro Pro
                85                  90                  95

Tyr Gly Val Phe Thr Ile Asn Pro Arg Thr Gly Glu Ile Asn Ile Thr
            100                 105                 110

Ser Val Val Asp Arg Glu Ile Thr Pro Leu Phe Leu Ile Tyr Cys Arg
        115                 120                 125

Ala Leu Asn Ser Arg Gly Glu Asp Leu Glu Arg Pro Leu Glu Leu Arg
    130                 135                 140

Val Lys Val Met Asp Ile Asn Asp Asn Ala Pro Val Phe Ser Gln Ser
145                 150                 155                 160

Val Tyr Thr Ala Ser Ile Glu Glu Asn Ser Asp Ala Asn Thr Leu Val
                165                 170                 175

Val Lys Leu Cys Ala Thr Asp Ala Asp Glu Glu Asn His Leu Asn Ser
            180                 185                 190

Lys Ile Ala Tyr Lys Ile Val Ser Gln Glu Pro Ser Gly Ala Pro Met
        195                 200                 205

Phe Ile Leu Asn Arg Tyr Thr Gly Glu Val Cys Thr Met Ser Ser Phe
    210                 215                 220

Leu Asp Arg Glu Gln His Ser Met Tyr Asn Leu Val Val Arg Gly Ser
225                 230                 235                 240

Asp Arg Asp Gly Ala Ala Asp Gly Leu Ser Ser Glu Cys Asp Cys Arg
                245                 250                 255

Ile Lys Val Leu Asp Val Asn Asp Asn Phe Pro Thr Leu Glu Lys Thr

            260                 265                 270

Ser Tyr Ser Ala Ser Ile Glu Glu Asn Cys Leu Ser Ser Glu Leu Ile
        275                 280                 285

Arg Leu Gln Ala Ile Asp Leu Asp Glu Glu Gly Thr Asp Asn Trp Leu
    290                 295                 300

Ala Gln Tyr Leu Ile Leu Ser Gly Asn Asp Gly Asn Trp Phe Asp Ile
305                 310                 315                 320

Gln Thr Asp Pro Gln Thr Asn Glu Gly Ile Leu Lys Val Val Lys Met
                325                 330                 335

Leu Asp Tyr Glu Gln Ala Pro Asn Ile Gln Leu Ser Ile Gly Val Lys
            340                 345                 350

Asn Gln Ala Asp Phe His Tyr Ser Val Ala Ser Gln Phe Gln Met His
        355                 360                 365

Pro Thr Pro Val Arg Ile Gln Val Val Asp Val Arg Glu Gly Pro Ala
    370                 375                 380

Phe His Pro Ser Thr Met Ala Phe Ser Val Arg Glu Gly Ile Lys Gly
385                 390                 395                 400

Ser Ser Leu Leu Asn Tyr Val Leu Gly Thr Tyr Thr Ala Ile Asp Leu
                405                 410                 415

Asp Thr Gly Asn Pro Ala Thr Asp Val Arg Tyr Ile Ile Gly His Asp
            420                 425                 430

Ala Gly Ser Trp Leu Lys Ile Asp Ser Arg Thr Gly Glu Ile Gln Phe
        435                 440                 445

Ser Arg Glu Phe Asp Lys Lys Ser Lys Tyr Ile Ile Asn Gly Ile Tyr
    450                 455                 460

Thr Ala Glu Ile Leu Ala Ile Asp Ala Thr Ser Ala Ile Leu Thr Ala
465                 470                 475                 480

Lys Gln Val Leu Ser Pro Gly Phe Tyr Glu Ile Pro Ile Leu Val Lys
                485                 490                 495

Asp Ser Tyr Asn Arg Ala Cys Glu Leu Ala Gln Met Val Gln Leu Tyr
            500                 505                 510

Ala Cys Asp Cys Asp Asp Asn His Met Cys Leu Asp Ser Gly Ala Ala
        515                 520                 525

Gly Ile Tyr Thr Glu Asp Ile Thr Gly Asp Thr Tyr Gly Pro Val Thr

530                 535                 540

Glu Asp Gln Ala Gly Val Ser Asn Val Gly Leu Gly Pro Ala Gly Ile
545                 550                 555                 560

Gly Met Met Val Leu Gly Ile Leu Leu Leu Ile Leu Ala Pro Leu Leu
                565                 570                 575

Leu Leu Leu Cys Cys Cys Lys Gln Arg Gln Pro Glu Gly Leu Gly Thr
            580                 585                 590

Arg Phe Ala Pro Val Pro Glu Gly Gly Glu Gly Val Met Gln Ser Trp
        595                 600                 605

Arg Ile Glu Gly Ala His Pro Glu Asp Arg Asp Val Ser Asn Ile Cys
    610                 615                 620

Ala Pro Met Thr Ala Ser Asn Thr Gln Asp Arg Met Asp Ser Ser Glu
625                 630                 635                 640

Ile Tyr Thr Asn Thr Tyr Ala Ala Gly Gly Thr Val Glu Gly Gly Val
                645                 650                 655

Ser Gly Val Glu Leu Asn Thr Gly Met Gly Thr Ala Val Gly Leu Met
            660                 665                 670

Ala Ala Gly Ala Ala Gly Ala Ser Gly Ala Ala Arg Lys Arg Ser Ser
        675                 680                 685

Thr Met Gly Thr Leu Arg Asp Tyr Ala Asp Ala Asp Ile Asn Met Ala
    690                 695                 700

Phe Leu Asp Ser Tyr Phe Ser Glu Lys Ala Tyr Ala Tyr Ala Asp Glu
705                 710                 715                 720

Asp Glu Gly Arg Pro Ala Asn Asp Cys Leu Leu Ile Tyr Asp His Glu
                725                 730                 735

Gly Val Gly Ser Pro Val Gly Ser Ile Gly Cys Cys Ser Trp Ile Val
            740                 745                 750

Asp Asp Leu Asp Glu Ser Cys Met Glu Thr Leu Asp Pro Lys Phe Arg
        755                 760                 765

Thr Leu Ala Glu Ile Cys Leu Asn Thr Glu Ile Glu Pro Phe Pro Ser
    770                 775                 780

His Gln Ala Cys Ile Pro Ile Ser Thr Asp Leu Pro Leu Leu Gly Pro
785                 790                 795                 800

Asn Tyr Phe Val Asn Glu Ser Ser Gly Leu Thr Pro Ser Glu Val Glu

805                 810                 815

Phe Gln Glu Glu Met Ala Ala Ser Glu Pro Val Val His Gly Asp Ile
            820                 825                 830

Ile Val Thr Glu Thr Tyr Gly Asn Ala Asp Pro Cys Val Gln Pro Thr
        835                 840                 845

Thr Ile Ile Phe Asp Pro Gln Leu Ala Pro Asn Val Val Val Thr Glu
    850                 855                 860

Ala Val Met Ala Pro Val Tyr Asp Ile Gln Gly Asn Ile Cys Val Pro
865                 870                 875                 880

Ala Glu Leu Ala Asp Tyr Asn Asn Val Ile Tyr Ala Glu Arg Val Leu
                885                 890                 895

Ala Ser Pro Gly Val Pro Asp Met Ser Asn Ser Ser Thr Thr Glu Gly
            900                 905                 910

Cys Met Gly Pro Val Met Ser Gly Asn Ile Leu Val Gly Pro Glu Ile
        915                 920                 925

Gln Val Met Gln Met Met Ser Pro Asp Leu Pro Ile Gly Gln Thr Val
    930                 935                 940

Gly Ser Thr Ser Pro Met Thr Ser Arg His Arg Val Thr Arg Tyr Ser
945                 950                 955                 960

Asn Ile His Tyr Thr Gln Gln
                965

<210>  106
<211>  3636
<212>  DNA
<213>  Homo sapiens

<400>  106
caccacagtt atcacccatg ccctcctaaa agggtgtctc aaagcatatc tttctgtaga      60

gcagaattcg gaactgagaa gacgagggct caaattgaat ctcacaggat ttgcgtgcaa     120

gagaaaccca aaggaatgga ttggctcttc ttcagaaaca tttgcctttt gatcattcta     180

atggtggtga tggaagtaaa cagtgaattt attgttgagg tgaaggaatt tgacattgaa     240

aatggcacta caaaatggca aacagtcaga agacaaaagc gggagtggat caagtttgcc     300

gcagcctgtc gagaaggaga ggacaactcg aagaggaacc ccattgccaa aattcgatca     360

gactgcgaat cgaaccagaa gataacatac cggatttctg gagtagggat tgatcgacca     420

ccatatgggg tattcaccat taatcctcgc actggggaaa ttaacatcac ttcagtggta     480

gacagagaaa taactccact tttcttgatc tattgccggg ctctgaattc acggggtgaa     540

gatttagaaa ggcctcttga gcttagagtc aaagttatgg acataaatga taacgctcca     600

gtcttttcgc aaagtgtata cacagccagc attgaagaaa atagtgatgc caatacattg 660
gtagtaaagt tatgtgccac agatgcagat gaagaaaatc atctgaattc taaaattgcc 720
tacaagatcg tctctcagga gccatcaggt gcacccatgt tcattctgaa taggtacact 780
ggagaagtct gcaccatgtc cagtttcttg gacagagagc aacacagtat gtacaacctg 840
gttgtgagag gctcagatcg ggatggagct gcagatggac tgtcttctga gtgtgactgt 900
agaatcaagg ttttagacgt caacgataat ttccccacct tagagaaaac ttcatactca 960
gccagtattg aagagaattg tttaagttcg gaactgatac gattacaagc aattgatctt 1020
gatgaagaag gcactgataa ctggttggct caatatttaa ttctctctgg aaatgatggg 1080
aattggttcg atattcaaac agatccacaa accaatgaag gcattttgaa agttgtcaag 1140
atgctggatt atgaacaagc acctaacatt cagcttagta tcggagttaa aaaccaagct 1200
gattttcact actccgttgc ttctcaattc caaatgcacc caacccctgt gagaattcaa 1260
gttgttgatg tgagagaagg acctgcattt catccaagta ctatggcttt tagtgtgcgg 1320
gaaggaataa aaggaagttc cttattgaat tatgtgcttg gcacatatac agccatagat 1380
ttggacacag gaaaccctgc aacagatgtc agatatatca tagggcatga tgcaggcagc 1440
tggttaaaaa ttgattcaag aactggtgag atacaatttt ctagagaatt tgataagaag 1500
tcaaaatata ttatcaatgg gatatacaca gcagagatcc tggctataga tgatggctct 1560
ggaaaaacag ctacaggaac catatgtatt gaggttcctg atatcaatga ttattgtcca 1620
aacatttttc ctgaaagaag aaccatctgc attgactctc catcagtcct tatctctgtt 1680
aatgaacatt cttatgggtc tccgtttact ttctgtgttg ttgatgagcc accaggaata 1740
gctgacatgt gggatgtcag atcaacaaat gctacctcgg caatccttac ggctaagcag 1800
gttttatctc caggatttta tgaaatccca atcctggtga aggacagcta taacagagca 1860
tgtgaattgg cacaaatggt gcagttatat gcctgtgatt gcgatgacaa ccacatgtgc 1920
ctggactctg gtgccgcggg catctacaca gaggacataa ctggtgacac gtatgggcct 1980
gtcactgaag accaagctgg agtttcaaat gttggtcttg gaccagcagg gattggcatg 2040
atggttctgg gcatcctgct actgattttg gctccactct tgctgctcct gtgttgctgc 2100
aaacagagac agccagaagg cctgggaaca agatttgctc ctgtgcctga gggcggagaa 2160
ggagtgatgc agtcttggag aattgaaggg gcccatcccg aggacaggct ttttagcgcc 2220
tacgccttgc cgggtggtgg tggcaccgca gacggcggcg gcagcgtttt aggcagatgc 2280
gcgctgcagg caactccagc gctgttaaac caacatcctc ccttttcaga aatctacacc 2340
aacacctatg cagccggggg cacggtggaa ggaggtgtat cgggagtgga gctcaacaca 2400
ggtatgggga cagccgttgg cctcatggcc gcaggggccg caggagcctc aggggccgca 2460
aggaagagga gctctaccat gggaaccctg cgggactacg ctgacgcaga catcaacatg 2520
gctttcttgg acagctactt ctcggagaaa gcgtatgctt atgcagatga agatgaaggt 2580
cgaccagcca atgactgctt gctcatttat gaccacgagg gagtcgggtc tcccgtaggc 2640

tctattggtt gttgcagttg gattgtggat gacttagatg aaagctgcat ggaaacttta 2700
gatccaaaat ttaggactct tgctgagatc tgcttaaaca cagaaattga accatttcct 2760
tcacaccagg cttgtatacc aatcagtact gacctccctt tgctcggacc taattacttt 2820
gttaatgaat cttcaggatt gactccctca gaagttgaat tccaagaaga aatggcagca 2880
tctgaacccg tggtccatgg ggatattatt gtgactgaga cttacggtaa tgctgatcca 2940
tgtgtgcaac ccactacaat tatttttgat cctcagcttg cacccaatgt tgtagtaacc 3000
gaagcagtaa tggcacctgt ctatgatatt caagggaata tttgtgtacc tgctgagtta 3060
gcagattaca acaatgtaat ctatgctgag agagtactgg ctagtcctgg tgtgcctgac 3120
atgagcaata gtagcacgac tgagggttgt atgggacctg tgatgagcgg caatatttta 3180
gtagggccag aaattcaagt gatgcaaatg atgagtccag accttcccat aggccaaacc 3240
gttggctcca catcccccat gacatctcga cacagagtaa cacgatacag taacatacat 3300
tacacccaac agtaagtgct ttatggtcag tattctatgt ggagaccttg caccttgtaa 3360
tcatcaatac atccaccaaa aatatataat gtaccatata tattaatagt caacaaatac 3420
tcagatattc taaggtcaat gccattattt gattatacca ttttgagggt gaatatggct 3480
aggcacttta gataagcctt tttaaaattc tttctgattt taaataatgc gtcaaaaaat 3540
gtgcagaaaa tgtattgcat cccttgatac tgtctaacga atagcacata actcatattg 3600
tgaatcctat gggtcttgag gcctgtagaa ccaatc 3636

<210> 107
<211> 1059
<212> PRT
<213> Homo sapiens

<400> 107

Met Asp Trp Leu Phe Phe Arg Asn Ile Cys Leu Leu Ile Ile Leu Met
1               5                   10                  15

Val Val Met Glu Val Asn Ser Glu Phe Ile Val Glu Val Lys Glu Phe
            20                  25                  30

Asp Ile Glu Asn Gly Thr Thr Lys Trp Gln Thr Val Arg Arg Gln Lys
        35                  40                  45

Arg Glu Trp Ile Lys Phe Ala Ala Ala Cys Arg Glu Gly Glu Asp Asn
    50                  55                  60

Ser Lys Arg Asn Pro Ile Ala Lys Ile Arg Ser Asp Cys Glu Ser Asn
65                  70                  75                  80

Gln Lys Ile Thr Tyr Arg Ile Ser Gly Val Gly Ile Asp Arg Pro Pro
                85                  90                  95

Tyr Gly Val Phe Thr Ile Asn Pro Arg Thr Gly Glu Ile Asn Ile Thr

            100                 105                 110

Ser Val Val Asp Arg Glu Ile Thr Pro Leu Phe Leu Ile Tyr Cys Arg
        115                 120                 125

Ala Leu Asn Ser Arg Gly Glu Asp Leu Glu Arg Pro Leu Glu Leu Arg
    130                 135                 140

Val Lys Val Met Asp Ile Asn Asp Asn Ala Pro Val Phe Ser Gln Ser
145                 150                 155                 160

Val Tyr Thr Ala Ser Ile Glu Glu Asn Ser Asp Ala Asn Thr Leu Val
                165                 170                 175

Val Lys Leu Cys Ala Thr Asp Ala Asp Glu Glu Asn His Leu Asn Ser
            180                 185                 190

Lys Ile Ala Tyr Lys Ile Val Ser Gln Glu Pro Ser Gly Ala Pro Met
        195                 200                 205

Phe Ile Leu Asn Arg Tyr Thr Gly Glu Val Cys Thr Met Ser Ser Phe
    210                 215                 220

Leu Asp Arg Glu Gln His Ser Met Tyr Asn Leu Val Val Arg Gly Ser
225                 230                 235                 240

Asp Arg Asp Gly Ala Ala Asp Gly Leu Ser Ser Glu Cys Asp Cys Arg
                245                 250                 255

Ile Lys Val Leu Asp Val Asn Asp Asn Phe Pro Thr Leu Glu Lys Thr
            260                 265                 270

Ser Tyr Ser Ala Ser Ile Glu Glu Asn Cys Leu Ser Ser Glu Leu Ile
        275                 280                 285

Arg Leu Gln Ala Ile Asp Leu Asp Glu Glu Gly Thr Asp Asn Trp Leu
    290                 295                 300

Ala Gln Tyr Leu Ile Leu Ser Gly Asn Asp Gly Asn Trp Phe Asp Ile
305                 310                 315                 320

Gln Thr Asp Pro Gln Thr Asn Glu Gly Ile Leu Lys Val Val Lys Met
                325                 330                 335

Leu Asp Tyr Glu Gln Ala Pro Asn Ile Gln Leu Ser Ile Gly Val Lys
            340                 345                 350

Asn Gln Ala Asp Phe His Tyr Ser Val Ala Ser Gln Phe Gln Met His
        355                 360                 365

Pro Thr Pro Val Arg Ile Gln Val Val Asp Val Arg Glu Gly Pro Ala

370                 375                 380

Phe His Pro Ser Thr Met Ala Phe Ser Val Arg Glu Gly Ile Lys Gly
385                 390                 395                 400

Ser Ser Leu Leu Asn Tyr Val Leu Gly Thr Tyr Thr Ala Ile Asp Leu
                405                 410                 415

Asp Thr Gly Asn Pro Ala Thr Asp Val Arg Tyr Ile Ile Gly His Asp
            420                 425                 430

Ala Gly Ser Trp Leu Lys Ile Asp Ser Arg Thr Gly Glu Ile Gln Phe
        435                 440                 445

Ser Arg Glu Phe Asp Lys Lys Ser Lys Tyr Ile Ile Asn Gly Ile Tyr
    450                 455                 460

Thr Ala Glu Ile Leu Ala Ile Asp Asp Gly Ser Gly Lys Thr Ala Thr
465                 470                 475                 480

Gly Thr Ile Cys Ile Glu Val Pro Asp Ile Asn Asp Tyr Cys Pro Asn
                485                 490                 495

Ile Phe Pro Glu Arg Arg Thr Ile Cys Ile Asp Ser Pro Ser Val Leu
            500                 505                 510

Ile Ser Val Asn Glu His Ser Tyr Gly Ser Pro Phe Thr Phe Cys Val
        515                 520                 525

Val Asp Glu Pro Pro Gly Ile Ala Asp Met Trp Asp Val Arg Ser Thr
    530                 535                 540

Asn Ala Thr Ser Ala Ile Leu Thr Ala Lys Gln Val Leu Ser Pro Gly
545                 550                 555                 560

Phe Tyr Glu Ile Pro Ile Leu Val Lys Asp Ser Tyr Asn Arg Ala Cys
                565                 570                 575

Glu Leu Ala Gln Met Val Gln Leu Tyr Ala Cys Asp Cys Asp Asp Asn
            580                 585                 590

His Met Cys Leu Asp Ser Gly Ala Ala Gly Ile Tyr Thr Glu Asp Ile
        595                 600                 605

Thr Gly Asp Thr Tyr Gly Pro Val Thr Glu Asp Gln Ala Gly Val Ser
    610                 615                 620

Asn Val Gly Leu Gly Pro Ala Gly Ile Gly Met Met Val Leu Gly Ile
625                 630                 635                 640

Leu Leu Leu Ile Leu Ala Pro Leu Leu Leu Leu Leu Cys Cys Cys Lys

                645                 650                 655

Gln Arg Gln Pro Glu Gly Leu Gly Thr Arg Phe Ala Pro Val Pro Glu
            660                 665                 670

Gly Gly Glu Gly Val Met Gln Ser Trp Arg Ile Glu Gly Ala His Pro
        675                 680                 685

Glu Asp Arg Leu Phe Ser Ala Tyr Ala Leu Pro Gly Gly Gly Gly Thr
    690                 695                 700

Ala Asp Gly Gly Gly Ser Val Leu Gly Arg Cys Ala Leu Gln Ala Thr
705                 710                 715                 720

Pro Ala Leu Leu Asn Gln His Pro Pro Phe Ser Glu Ile Tyr Thr Asn
                725                 730                 735

Thr Tyr Ala Ala Gly Gly Thr Val Glu Gly Gly Val Ser Gly Val Glu
            740                 745                 750

Leu Asn Thr Gly Met Gly Thr Ala Val Gly Leu Met Ala Ala Gly Ala
        755                 760                 765

Ala Gly Ala Ser Gly Ala Ala Arg Lys Arg Ser Ser Thr Met Gly Thr
    770                 775                 780

Leu Arg Asp Tyr Ala Asp Ala Asp Ile Asn Met Ala Phe Leu Asp Ser
785                 790                 795                 800

Tyr Phe Ser Glu Lys Ala Tyr Ala Tyr Ala Asp Glu Asp Glu Gly Arg
                805                 810                 815

Pro Ala Asn Asp Cys Leu Leu Ile Tyr Asp His Glu Gly Val Gly Ser
            820                 825                 830

Pro Val Gly Ser Ile Gly Cys Cys Ser Trp Ile Val Asp Asp Leu Asp
        835                 840                 845

Glu Ser Cys Met Glu Thr Leu Asp Pro Lys Phe Arg Thr Leu Ala Glu
    850                 855                 860

Ile Cys Leu Asn Thr Glu Ile Glu Pro Phe Pro Ser His Gln Ala Cys
865                 870                 875                 880

Ile Pro Ile Ser Thr Asp Leu Pro Leu Leu Gly Pro Asn Tyr Phe Val
                885                 890                 895

Asn Glu Ser Ser Gly Leu Thr Pro Ser Glu Val Glu Phe Gln Glu Glu
            900                 905                 910

Met Ala Ala Ser Glu Pro Val Val His Gly Asp Ile Ile Val Thr Glu

915                 920                 925

Thr Tyr Gly Asn Ala Asp Pro Cys Val Gln Pro Thr Thr Ile Ile Phe
    930                 935                 940

Asp Pro Gln Leu Ala Pro Asn Val Val Val Thr Glu Ala Val Met Ala
945                 950                 955                 960

Pro Val Tyr Asp Ile Gln Gly Asn Ile Cys Val Pro Ala Glu Leu Ala
                965                 970                 975

Asp Tyr Asn Asn Val Ile Tyr Ala Glu Arg Val Leu Ala Ser Pro Gly
            980                 985                 990

Val Pro Asp Met Ser Asn Ser Ser  Thr Thr Glu Gly Cys  Met Gly Pro
        995                 1000                1005

Val Met  Ser Gly Asn Ile Leu  Val Gly Pro Glu Ile  Gln Val Met
    1010                1015                1020

Gln Met  Met Ser Pro Asp Leu  Pro Ile Gly Gln Thr  Val Gly Ser
    1025                1030                1035

Thr Ser  Pro Met Thr Ser Arg  His Arg Val Thr Arg  Tyr Ser Asn
    1040                1045                1050

Ile His  Tyr Thr Gln Gln
    1055

<210>  108
<211>  2074
<212>  DNA
<213>  Homo sapiens

<400>  108
tatacagcca tagatttgga cacaggaaac cctgcaacag atgtcagatg gctctggaaa      60

aacagctaca ggaaccatat gtattgaggt tcctgatatc aatgattatt gtccaaacat     120

ttttcctgaa agaagaacca tctgcattga ctctccatca gtccttatct ctgttaatga     180

acattcttat gggtctccgt ttactttctg tgttgttgat gagccaccag gaatagctga     240

catgtgggat gtcagatcaa caaatgctac ctcggcaatc cttacggcta agcaggtttt     300

atctccagga ttttatgaaa tcccaatcct ggtgaaggac agctataaca gagcatgtga     360

attggcacaa atggtgcagt tatatgcctg tgattgcgat gacaaccaca tgtgcctgga     420

ctctggtgcc gcgggcatct acacagagga cataactggt gacacgtatg ggcctgtcac     480

tgaagaccaa gctggagttt caaatgttgg tcttggacca gcagggattg gcatgatggt     540

tctgggcatc ctgctactga ttttggctcc actcttgctg ctcctgtgtt gctgcaaaca     600

gagacagcca gaaggcctgg gaacaagatt tgctcctgtg cctgagggcg gagaaggagt     660

gatgcagtct tggagaattg aaggggccca tcccgaggac agggatgtgt caaatatatg     720

tgcacccatg acagcctcaa atacccagga tcggatggat tcctctgaaa tctacaccaa 780
cacctatgca gccgggggca cggtggaagg aggtgtatcg ggagtggagc tcaacacagg 840
tatggggaca gccgttggcc tcatggccgc aggggccgca ggagcctcag gggccgcaag 900
gaagaggagc tctaccatgg gaaccctgcg ggactacgct gacgcagaca tcaacatggc 960
tttcttggac agctacttct cggagaaagc gtatgcttat gcagatgaag atgaaggtcg 1020
accagccaat gactgcttgc tcatttatga ccacgaggga gtcgggtctc ccgtaggctc 1080
tattggttgt tgcagttgga ttgtggatga cttagatgaa agctgcatgg aaactttaga 1140
tccaaaattt aggactcttg ctgagatctg cttaaacaca gaaattgaac catttccttc 1200
acaccaggct tgtataccaa tcagtactga cctccctttg ctcggaccta attactttgt 1260
taatgaatct tcaggattga ctccctcaga agttgaattc caagaagaaa tggcagcatc 1320
tgaacccgtg gtccatgggg atattattgt gactgagact tacggtaatg ctgatccatg 1380
tgtgcaaccc actacaatta tttttgatcc tcagcttgca cccaatgttg tagtaaccga 1440
agcagtaatg gcacctgtct atgatattca agggaatatt tgtgtacctg ctgagttagc 1500
agattacaac aatgtaatct atgctgagag agtactggct agtcctggtg tgcctgacat 1560
gagcaatagt agcacgactg agggttgtat gggacctgtg atgagcggca atattttagt 1620
agggccagaa attcaagtga tgcaaatgat gagtccagac cttcccatag gccaaaccgt 1680
tggctccaca tcccccatga catctcgaca cagagtaaca cgatacagta acatacatta 1740
cacccaacag taagtgcttt atggtcagta ttctatgtgg agaccttgca ccttgtaatc 1800
atcaatacat ccaccaaaaa tatataatgt accatatata ttaatagtca acaaatactc 1860
agatattcta aggtcaatgc cattatttga ttataccatt ttgagggtga atatggctag 1920
gcactttaga taagcctttt taaaattctt tctgatttta aataatgcgt caaaaaatgt 1980
gcagaaaatg tattgcatcc cttgatactg tctaacgaat agcacataac tcatattgtg 2040
aatcctatgg gtcttgaggc ctgtagaacc aatc 2074

<210> 109
<211> 570
<212> PRT
<213> Homo sapiens

<400> 109

Met Ser Asp Gly Ser Gly Lys Thr Ala Thr Gly Thr Ile Cys Ile Glu
1               5                   10                  15

Val Pro Asp Ile Asn Asp Tyr Cys Pro Asn Ile Phe Pro Glu Arg Arg
            20                  25                  30

Thr Ile Cys Ile Asp Ser Pro Ser Val Leu Ile Ser Val Asn Glu His
        35                  40                  45

Ser Tyr Gly Ser Pro Phe Thr Phe Cys Val Val Asp Glu Pro Pro Gly

50                  55                  60

Ile Ala Asp Met Trp Asp Val Arg Ser Thr Asn Ala Thr Ser Ala Ile
65                  70                  75                  80

Leu Thr Ala Lys Gln Val Leu Ser Pro Gly Phe Tyr Glu Ile Pro Ile
                85                  90                  95

Leu Val Lys Asp Ser Tyr Asn Arg Ala Cys Glu Leu Ala Gln Met Val
            100                 105                 110

Gln Leu Tyr Ala Cys Asp Cys Asp Asp Asn His Met Cys Leu Asp Ser
        115                 120                 125

Gly Ala Ala Gly Ile Tyr Thr Glu Asp Ile Thr Gly Asp Thr Tyr Gly
    130                 135                 140

Pro Val Thr Glu Asp Gln Ala Gly Val Ser Asn Val Gly Leu Gly Pro
145                 150                 155                 160

Ala Gly Ile Gly Met Met Val Leu Gly Ile Leu Leu Leu Ile Leu Ala
                165                 170                 175

Pro Leu Leu Leu Leu Leu Cys Cys Cys Lys Gln Arg Gln Pro Glu Gly
            180                 185                 190

Leu Gly Thr Arg Phe Ala Pro Val Pro Glu Gly Gly Glu Gly Val Met
        195                 200                 205

Gln Ser Trp Arg Ile Glu Gly Ala His Pro Glu Asp Arg Asp Val Ser
    210                 215                 220

Asn Ile Cys Ala Pro Met Thr Ala Ser Asn Thr Gln Asp Arg Met Asp
225                 230                 235                 240

Ser Ser Glu Ile Tyr Thr Asn Thr Tyr Ala Ala Gly Gly Thr Val Glu
                245                 250                 255

Gly Gly Val Ser Gly Val Glu Leu Asn Thr Gly Met Gly Thr Ala Val
            260                 265                 270

Gly Leu Met Ala Ala Gly Ala Ala Gly Ala Ser Gly Ala Ala Arg Lys
        275                 280                 285

Arg Ser Ser Thr Met Gly Thr Leu Arg Asp Tyr Ala Asp Ala Asp Ile
    290                 295                 300

Asn Met Ala Phe Leu Asp Ser Tyr Phe Ser Glu Lys Ala Tyr Ala Tyr
305                 310                 315                 320

Ala Asp Glu Asp Glu Gly Arg Pro Ala Asn Asp Cys Leu Leu Ile Tyr

325                    330                    335

Asp His Glu Gly Val Gly Ser Pro Val Gly Ser Ile Gly Cys Cys Ser
            340                 345                 350

Trp Ile Val Asp Asp Leu Asp Glu Ser Cys Met Glu Thr Leu Asp Pro
        355                 360                 365

Lys Phe Arg Thr Leu Ala Glu Ile Cys Leu Asn Thr Glu Ile Glu Pro
    370                 375                 380

Phe Pro Ser His Gln Ala Cys Ile Pro Ile Ser Thr Asp Leu Pro Leu
385                 390                 395                 400

Leu Gly Pro Asn Tyr Phe Val Asn Glu Ser Ser Gly Leu Thr Pro Ser
                405                 410                 415

Glu Val Glu Phe Gln Glu Glu Met Ala Ala Ser Glu Pro Val Val His
            420                 425                 430

Gly Asp Ile Ile Val Thr Glu Thr Tyr Gly Asn Ala Asp Pro Cys Val
        435                 440                 445

Gln Pro Thr Thr Ile Ile Phe Asp Pro Gln Leu Ala Pro Asn Val Val
    450                 455                 460

Val Thr Glu Ala Val Met Ala Pro Val Tyr Asp Ile Gln Gly Asn Ile
465                 470                 475                 480

Cys Val Pro Ala Glu Leu Ala Asp Tyr Asn Asn Val Ile Tyr Ala Glu
                485                 490                 495

Arg Val Leu Ala Ser Pro Gly Val Pro Asp Met Ser Asn Ser Ser Thr
            500                 505                 510

Thr Glu Gly Cys Met Gly Pro Val Met Ser Gly Asn Ile Leu Val Gly
        515                 520                 525

Pro Glu Ile Gln Val Met Gln Met Met Ser Pro Asp Leu Pro Ile Gly
    530                 535                 540

Gln Thr Val Gly Ser Thr Ser Pro Met Thr Ser Arg His Arg Val Thr
545                 550                 555                 560

Arg Tyr Ser Asn Ile His Tyr Thr Gln Gln
                565                 570

<210>  110
<211>  1995
<212>  DNA
<213>  Homo sapiens

<400> 110
tatacagcca tagatttgga cacaggaaac cctgcaacag atgtcagata tatcataggg 60
catgatgcag gcagctggtt aaaaattgat tcaagaactg gtgagataca attttctaga 120
gaatttgata agaagtcaaa atatattatc aatgggatat acacagcaga gatcctggct 180
atagatgcta cctcggcaat ccttacggct aagcaggttt tatctccagg attttatgaa 240
atcccaatcc tggtgaagga cagctataac agagcatgtg aattggcaca aatggtgcag 300
ttatatgcct gtgattgcga tgacaaccac atgtgcctgg actctggtgc cgcgggcatc 360
tacacagagg acataactgg tgacacgtat gggcctgtca ctgaagacca agctggagtt 420
tcaaatgttg gtcttggacc agcagggatt ggcatgatgg ttctgggcat cctgctactg 480
attttggctc cactcttgct gctcctgtgt tgctgcaaac agagacagcc agaaggcctg 540
ggaacaagat ttgctcctgt gcctgagggc ggagaaggag tgatgcagtc ttggagaatt 600
gaaggggccc atcccgagga cagggatgtg tcaaatatat gtgcacccat gacagcctca 660
aatacccagg atcggatgga ttcctctgaa atctacacca acacctatgc agccgggggc 720
acggtggaag gaggtgtatc gggagtggag ctcaacacag gtatggggac agccgttggc 780
ctcatggccg caggggccgc aggagcctca ggggccgcaa ggaagaggag ctctaccatg 840
ggaaccctgc gggactacgc tgacgcagac atcaacatgg ctttcttgga cagctacttc 900
tcggagaaag cgtatgctta tgcagatgaa gatgaaggtc gaccagccaa tgactgcttg 960
ctcatttatg accacgaggg agtcgggtct cccgtaggct ctattggttg ttgcagttgg 1020
attgtggatg acttagatga aagctgcatg gaaactttag atccaaaatt taggactctt 1080
gctgagatct gcttaaacac agaaattgaa ccatttcctt cacaccaggc ttgtatacca 1140
atcagtactg acctcccttt gctcggacct aattactttg ttaatgaatc ttcaggattg 1200
actccctcag aagttgaatt ccaagaagaa atggcagcat ctgaacccgt ggtccatggg 1260
gatattattg tgactgagac ttacggtaat gctgatccat gtgtgcaacc cactacaatt 1320
atttttgatc ctcagcttgc acccaatgtt gtagtaaccg aagcagtaat ggcacctgtc 1380
tatgatattc aagggaatat ttgtgtacct gctgagttag cagattacaa caatgtaatc 1440
tatgctgaga gagtactggc tagtcctggt gtgcctgaca tgagcaatag tagcacgact 1500
gagggttgta tgggacctgt gatgagcggc aatattttag tagggccaga aattcaagtg 1560
atgcaaatga tgagtccaga ccttcccata ggccaaaccg ttggctccac atcccccatg 1620
acatctcgac acagagtaac acgatacagt aacatacatt acacccaaca gtaagtgctt 1680
tatggtcagt attctatgtg gagaccttgc accttgtaat catcaataca tccaccaaaa 1740
atatataatg taccatatat attaatagtc aacaaatact cagatattct aaggtcaatg 1800
ccattatttg attataccat tttgagggtg aatatggcta ggcactttag ataagccttt 1860
ttaaaattct ttctgatttt aaataatgcg tcaaaaaatg tgcagaaaat gtattgcatc 1920
ccttgatact gtctaacgaa tagcacataa ctcatattgt gaatcctatg ggtcttgagg 1980
cctgtagaac caatc 1995

<210>  111
<211>  460
<212>  PRT
<213>  Homo sapiens

<400>  111

Met Val Gln Leu Tyr Ala Cys Asp Cys Asp Asp Asn His Met Cys Leu
1               5                   10                  15

Asp Ser Gly Ala Ala Gly Ile Tyr Thr Glu Asp Ile Thr Gly Asp Thr
            20                  25                  30

Tyr Gly Pro Val Thr Glu Asp Gln Ala Gly Val Ser Asn Val Gly Leu
        35                  40                  45

Gly Pro Ala Gly Ile Gly Met Met Val Leu Gly Ile Leu Leu Leu Ile
    50                  55                  60

Leu Ala Pro Leu Leu Leu Leu Leu Cys Cys Cys Lys Gln Arg Gln Pro
65                  70                  75                  80

Glu Gly Leu Gly Thr Arg Phe Ala Pro Val Pro Glu Gly Gly Glu Gly
                85                  90                  95

Val Met Gln Ser Trp Arg Ile Glu Gly Ala His Pro Glu Asp Arg Asp
            100                 105                 110

Val Ser Asn Ile Cys Ala Pro Met Thr Ala Ser Asn Thr Gln Asp Arg
        115                 120                 125

Met Asp Ser Ser Glu Ile Tyr Thr Asn Thr Tyr Ala Ala Gly Gly Thr
    130                 135                 140

Val Glu Gly Gly Val Ser Gly Val Glu Leu Asn Thr Gly Met Gly Thr
145                 150                 155                 160

Ala Val Gly Leu Met Ala Ala Gly Ala Ala Gly Ala Ser Gly Ala Ala
                165                 170                 175

Arg Lys Arg Ser Ser Thr Met Gly Thr Leu Arg Asp Tyr Ala Asp Ala
            180                 185                 190

Asp Ile Asn Met Ala Phe Leu Asp Ser Tyr Phe Ser Glu Lys Ala Tyr
        195                 200                 205

Ala Tyr Ala Asp Glu Asp Glu Gly Arg Pro Ala Asn Asp Cys Leu Leu
    210                 215                 220

Ile Tyr Asp His Glu Gly Val Gly Ser Pro Val Gly Ser Ile Gly Cys
225                 230                 235                 240

Cys Ser Trp Ile Val Asp Asp Leu Asp Glu Ser Cys Met Glu Thr Leu
                245                 250                 255

Asp Pro Lys Phe Arg Thr Leu Ala Glu Ile Cys Leu Asn Thr Glu Ile
            260                 265                 270

Glu Pro Phe Pro Ser His Gln Ala Cys Ile Pro Ile Ser Thr Asp Leu
        275                 280                 285

Pro Leu Leu Gly Pro Asn Tyr Phe Val Asn Glu Ser Ser Gly Leu Thr
    290                 295                 300

Pro Ser Glu Val Glu Phe Gln Glu Glu Met Ala Ala Ser Glu Pro Val
305                 310                 315                 320

Val His Gly Asp Ile Ile Val Thr Glu Thr Tyr Gly Asn Ala Asp Pro
                325                 330                 335

Cys Val Gln Pro Thr Thr Ile Ile Phe Asp Pro Gln Leu Ala Pro Asn
            340                 345                 350

Val Val Val Thr Glu Ala Val Met Ala Pro Val Tyr Asp Ile Gln Gly
        355                 360                 365

Asn Ile Cys Val Pro Ala Glu Leu Ala Asp Tyr Asn Asn Val Ile Tyr
    370                 375                 380

Ala Glu Arg Val Leu Ala Ser Pro Gly Val Pro Asp Met Ser Asn Ser
385                 390                 395                 400

Ser Thr Thr Glu Gly Cys Met Gly Pro Val Met Ser Gly Asn Ile Leu
                405                 410                 415

Val Gly Pro Glu Ile Gln Val Met Gln Met Met Ser Pro Asp Leu Pro
            420                 425                 430

Ile Gly Gln Thr Val Gly Ser Thr Ser Pro Met Thr Ser Arg His Arg
        435                 440                 445

Val Thr Arg Tyr Ser Asn Ile His Tyr Thr Gln Gln
    450                 455                 460

<210> 112
<211> 2367
<212> DNA
<213> Homo sapiens

<400> 112
tccgttgctt ctcaattcca aatgcaccca acccctgtga gaattcaagt tgttgatgtg 60

agagaaggac ctgcatttca tccaagtact atggctttta gtgtgcggga aggaataaaa 120

ggaagttcct tattgaatta tgtgcttggc acatatacag ccatagattt ggacacagga 180

aaccctgcaa cagatgtcag atatatcata gggcatgatg caggcagctg gttaaaaatt 240
gattcaagaa ctggtgagat acaattttct agagaatttg ataagaagtc aaaatatatt 300
atcaatggga tatacacagc agagatcctg gctatagatg atggctctgg aaaaacagct 360
acaggaacca tatgtattga ggttcctgat atcaatgatt attgtccaaa catttttcct 420
gaaagaagaa ccatctgcat tgactctcca tcagtcctta tctctgttaa tgaacattct 480
tatgggtctc cgtttacttt ctgtgttgtt gatgagccac caggaatagc tgacatgtgg 540
gatgtcagat caacaaatgc tacctcggca atccttacgg ctaagcaggt tttatctcca 600
ggattttatg aaatcccaat cctggtgaag gacagctata acagagcatg tgaattggca 660
caaatggtgc agttatatgc ctgtgattgc gatgacaacc acatgtgcct ggactctggt 720
gccgcgggca tctacacaga ggacataact ggtgacacgt atgggcctgt cactgaagac 780
caagctggag tttcaaatgt tggtcttgga ccagcaggga ttggcatgat ggttctgggc 840
atcctgctac tgattttggc tccactcttg ctgctcctgt gttgctgcaa acagagacag 900
ccagaaggcc tgggaacaag atttgctcct gtgcctgagg gcggagaagg agtgatgcag 960
tcttggagaa ttgaaggggc ccatcccgag gacagggatg tgtcaaatat atgtgcaccc 1020
atgacagcct caaataccca ggatcggatg gattcctctg aaatctacac caacacctat 1080
gcagccgggg gcacggtgga aggaggtgta tcgggagtgg agctcaacac aggtatgggg 1140
acagccgttg gcctcatggc cgcaggggcc gcaggagcct caggggccgc aaggaagagg 1200
agctctacca tgggaaccct gcgggactac gctgacgcag acatcaacat ggctttcttg 1260
gacagctact tctcggagaa agcgtatgct tatgcagatg aagatgaagg tcgaccagcc 1320
aatgactgct tgctcattta tgaccacgag ggagtcgggt ctcccgtagg ctctattggt 1380
tgttgcagtt ggattgtgga tgacttagat gaaagctgca tggaaacttt agatccaaaa 1440
tttaggactc ttgctgagat ctgcttaaac acagaaattg aaccatttcc ttcacaccag 1500
gcttgtatac caatcagtac tgacctccct ttgctcggac ctaattactt tgttaatgaa 1560
tcttcaggat tgactccctc agaagttgaa ttccaagaag aaatggcagc atctgaaccc 1620
gtggtccatg gggatattat tgtgactgag acttacggta atgctgatcc atgtgtgcaa 1680
cccactacaa ttatttttga tcctcagctt gcacccaatg ttgtagtaac cgaagcagta 1740
atggcacctg tctatgatat tcaagggaat atttgtgtac ctgctgagtt agcagattac 1800
aacaatgtaa tctatgctga gagagtactg gctagtcctg gtgtgcctga catgagcaat 1860
agtagcacga ctgagggttg tatgggacct gtgatgagcg gcaatatttt agtagggcca 1920
gaaattcaag tgatgcaaat gatgagtcca gaccttccca taggccaaac cgttggctcc 1980
acatccccca tgacatctcg acacagagta acacgataca gtaacataca ttacacccaa 2040
cagtaagtgc tttatggtca gtattctatg tggagacctt gcaccttgta atcatcaata 2100
catccaccaa aaatatataa tgtaccatat atattaatag tcaacaaata ctcagatatt 2160
ctaaggtcaa tgccattatt tgattatacc attttgaggg tgaatatggc taggcacttt 2220

agataagcct ttttaaaatt ctttctgatt ttaaataatg cgtcaaaaaa tgtgcagaaa 2280

atgtattgca tcccttgata ctgtctaacg aatagcacat aactcatatt gtgaatccta 2340

tgggtcttga ggcctgtaga accaatc 2367

<210> 113
<211> 674
<212> PRT
<213> Homo sapiens

<400> 113

Met His Pro Thr Pro Val Arg Ile Gln Val Val Asp Val Arg Glu Gly
1               5                   10                  15

Pro Ala Phe His Pro Ser Thr Met Ala Phe Ser Val Arg Glu Gly Ile
            20                  25                  30

Lys Gly Ser Ser Leu Leu Asn Tyr Val Leu Gly Thr Tyr Thr Ala Ile
        35                  40                  45

Asp Leu Asp Thr Gly Asn Pro Ala Thr Asp Val Arg Tyr Ile Ile Gly
    50                  55                  60

His Asp Ala Gly Ser Trp Leu Lys Ile Asp Ser Arg Thr Gly Glu Ile
65                  70                  75                  80

Gln Phe Ser Arg Glu Phe Asp Lys Lys Ser Lys Tyr Ile Ile Asn Gly
                85                  90                  95

Ile Tyr Thr Ala Glu Ile Leu Ala Ile Asp Asp Gly Ser Gly Lys Thr
            100                 105                 110

Ala Thr Gly Thr Ile Cys Ile Glu Val Pro Asp Ile Asn Asp Tyr Cys
        115                 120                 125

Pro Asn Ile Phe Pro Glu Arg Arg Thr Ile Cys Ile Asp Ser Pro Ser
    130                 135                 140

Val Leu Ile Ser Val Asn Glu His Ser Tyr Gly Ser Pro Phe Thr Phe
145                 150                 155                 160

Cys Val Val Asp Glu Pro Pro Gly Ile Ala Asp Met Trp Asp Val Arg
                165                 170                 175

Ser Thr Asn Ala Thr Ser Ala Ile Leu Thr Ala Lys Gln Val Leu Ser
            180                 185                 190

Pro Gly Phe Tyr Glu Ile Pro Ile Leu Val Lys Asp Ser Tyr Asn Arg
        195                 200                 205

Ala Cys Glu Leu Ala Gln Met Val Gln Leu Tyr Ala Cys Asp Cys Asp

210                 215                 220

Asp Asn His Met Cys Leu Asp Ser Gly Ala Ala Gly Ile Tyr Thr Glu
225                 230                 235                 240

Asp Ile Thr Gly Asp Thr Tyr Gly Pro Val Thr Glu Asp Gln Ala Gly
                245                 250                 255

Val Ser Asn Val Gly Leu Gly Pro Ala Gly Ile Gly Met Met Val Leu
            260                 265                 270

Gly Ile Leu Leu Leu Ile Leu Ala Pro Leu Leu Leu Leu Leu Cys Cys
        275                 280                 285

Cys Lys Gln Arg Gln Pro Glu Gly Leu Gly Thr Arg Phe Ala Pro Val
    290                 295                 300

Pro Glu Gly Gly Glu Gly Val Met Gln Ser Trp Arg Ile Glu Gly Ala
305                 310                 315                 320

His Pro Glu Asp Arg Asp Val Ser Asn Ile Cys Ala Pro Met Thr Ala
                325                 330                 335

Ser Asn Thr Gln Asp Arg Met Asp Ser Ser Glu Ile Tyr Thr Asn Thr
            340                 345                 350

Tyr Ala Ala Gly Gly Thr Val Glu Gly Gly Val Ser Gly Val Glu Leu
        355                 360                 365

Asn Thr Gly Met Gly Thr Ala Val Gly Leu Met Ala Ala Gly Ala Ala
    370                 375                 380

Gly Ala Ser Gly Ala Ala Arg Lys Arg Ser Ser Thr Met Gly Thr Leu
385                 390                 395                 400

Arg Asp Tyr Ala Asp Ala Asp Ile Asn Met Ala Phe Leu Asp Ser Tyr
                405                 410                 415

Phe Ser Glu Lys Ala Tyr Ala Tyr Ala Asp Glu Asp Glu Gly Arg Pro
            420                 425                 430

Ala Asn Asp Cys Leu Leu Ile Tyr Asp His Glu Gly Val Gly Ser Pro
        435                 440                 445

Val Gly Ser Ile Gly Cys Cys Ser Trp Ile Val Asp Asp Leu Asp Glu
    450                 455                 460

Ser Cys Met Glu Thr Leu Asp Pro Lys Phe Arg Thr Leu Ala Glu Ile
465                 470                 475                 480

Cys Leu Asn Thr Glu Ile Glu Pro Phe Pro Ser His Gln Ala Cys Ile

485                 490                 495

Pro Ile Ser Thr Asp Leu Pro Leu Leu Gly Pro Asn Tyr Phe Val Asn
            500                 505                 510

Glu Ser Ser Gly Leu Thr Pro Ser Glu Val Glu Phe Gln Glu Glu Met
        515                 520                 525

Ala Ala Ser Glu Pro Val Val His Gly Asp Ile Ile Val Thr Glu Thr
    530                 535                 540

Tyr Gly Asn Ala Asp Pro Cys Val Gln Pro Thr Thr Ile Ile Phe Asp
545                 550                 555                 560

Pro Gln Leu Ala Pro Asn Val Val Val Thr Glu Ala Val Met Ala Pro
                565                 570                 575

Val Tyr Asp Ile Gln Gly Asn Ile Cys Val Pro Ala Glu Leu Ala Asp
            580                 585                 590

Tyr Asn Asn Val Ile Tyr Ala Glu Arg Val Leu Ala Ser Pro Gly Val
        595                 600                 605

Pro Asp Met Ser Asn Ser Ser Thr Thr Glu Gly Cys Met Gly Pro Val
    610                 615                 620

Met Ser Gly Asn Ile Leu Val Gly Pro Glu Ile Gln Val Met Gln Met
625                 630                 635                 640

Met Ser Pro Asp Leu Pro Ile Gly Gln Thr Val Gly Ser Thr Ser Pro
                645                 650                 655

Met Thr Ser Arg His Arg Val Thr Arg Tyr Ser Asn Ile His Tyr Thr
            660                 665                 670

Gln Gln